(19) Europäisches Patentamt
European Patent Office
Office européen des brevets

(11) Veröffentlichungsnummer: **0 671 401 A1**

(12) **EUROPÄISCHE PATENTANMELDUNG**

(21) Anmeldenummer: **95102819.0**

(22) Anmeldetag: **28.02.95**

(51) Int. Cl.⁶: **C07D 513/14**, C07D 507/08, C07D 498/14, C07D 463/10, C07D 487/04, A61K 31/10, A61K 31/535, A61K 31/54

(30) Priorität: **11.03.94 CH 751/94**
**31.01.95 CH 260/95**

(43) Veröffentlichungstag der Anmeldung:
**13.09.95 Patentblatt 95/37**

(84) Benannte Vertragsstaaten:
**AT BE CH DE DK ES FR GB GR IE IT LI LU MC NL PT SE**

(71) Anmelder: **F. HOFFMANN-LA ROCHE AG**
**Postfach 3255**
**CH-4002 Basel (CH)**

(72) Erfinder: **Hubschwerlen, Christian**
**Rue de la Gendarmerie**
**F-68200 Durmenach (FR)**
Erfinder: **Böhringer, Markus**
**Föhrenstrasse, 8**
**CH-4313 Möhlin (CH)**
Erfinder: **Hubschwerlen, Christian**
**Rue de la Gendarmerie**
**F-68200 Durmenach (FR)**
Erfinder: **Pflieger, Philippe**
**12, rue du Césarhof**
**F-68220 Folgensburg (FR)**
Erfinder: **Specklin, Jean-Luc**
**rue du 6ème Ric.**
**F-68680 Kembs-Loechle (FR)**

(74) Vertreter: **Martin, Björn et al**
**Grenzacherstrasse 124**
**Postfach 3255**
**CH-4002 Basel (CH)**

(54) **Beta-Lactame.**

(57) Es werden $\beta$-Lactame beschrieben, und zwar der allgemeinen Formel

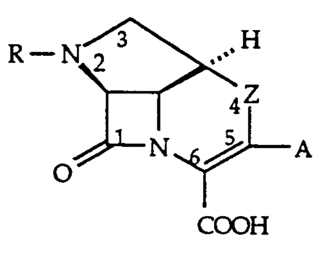

I

in der Z Methylen, Sauerstoff oder Schwefel und R Wasserstoff, ggfs. durch Carboxy, niederes Alkoxycarbonyl, Carbamoyl, niederes Alkylcarbamoyl, Phenylcarbamoyl oder Hydroxyphenylcarbamoyl substituiertes niederes (Cyclo)alkyl, niederes Alkenylmethyl, niederes Alkenylmethoxycarbonyl, Formyl, ggfs. durch Halogen, Cyan, Carbamoyl-niederes Alkoxy, Carbamoyl-niederes Alkylthio oder Carbamoyl-niederes Alkylamino substituiertes niederes (Cyclo)alkanoyl bzw. (Cyclo)alkylsulfonyl, ggfs. durch niederes (Cyclo)alkyl, niederes Alkoxycarbonyl-niederes Alkyl, Benzyloxycarbonyl-niederes Alkyl oder Carboxy-niederes Alkyl substituiertes Carbamoyl oder eine Ringstruktur der allgemeinen Formeln

Q-X-CO- (a1)

EP 0 671 401 A1

$Q\text{-}X\text{-}SO_2\text{-}$ (a2)

bedeutet, worin Q einen 5- oder 6-gliedrigen, ggfs. stickstoff-, schwefel- und/oder sauerstoffhaltigen Ring und X eine direkte Bindung oder eine der Gruppen $-CH_2-$, $-CH_2CH_2-$, $-CH=CH-$, -NH-, $-NHCH_2-$, $-CH_2NH-$, $-CH(NH_2)-$, $-CH_2CH_2NH-$, $-C(=NOCH_3)-$, $-OCH_2-$ und $-SCH_2-$ darstellt; und worin ferner A niederes Alkyl, Hydroxy-niederes Alkyl, Vinyl, Cyanvinyl, niederes Alkoxy, ggfs. phenylsubstituiertes nieder-(cyclo)alkanoyloxy bzw. (Cyclo)alkylsulfonyloxy, ggfs. niederes (Cyclo)alkyl-substituiertes Benzoyloxy bzw. Phenylsulfonyloxy, einen Rest -S-Het oder $-S-CH_2-$Het, worin Het einen 5- oder 6-gliedrigen, stickstoff-, schwefel- und/oder sauerstoffhaltigen Heterocyclus darstellt, oder einen Rest $-CH_2-L$ bedeutet, worin L ggfs. phenylsubstituiertes niederes (Cyclo)-alkanoyloxy bzw. (Cyclo)alkylsulfonyloxy, ggfs. nieder-(cyclo)alkyl-substituiertes Benzoyloxy bzw. Phenylsulfonyloxy, Carbamoyloxy, niederes (Cyclo)alkoxycarbonyl, Carboxy, Azido, Amino, niederes (Cyclo)alkanoylamino, niederes (Cyclo)alkylsulfonylamino, niederes (Cyclo)alkylamino, di-niederes (Cyclo)alkylamino, einen 5- oder 6-gliedrigen, an einem Stickstoffatom gebundenen Ring oder einen Rest -S-Het oder $-S-CH_2-$Het, worin Het die obige Bedeutung hat, darstellt,
und pharmazeutisch verträgliche, leicht hydrolysierbare Ester und Salze dieser Verbindungen.

Ebenfalls beschrieben wird ein Verfahren zur Herstellung der oben definierten $\beta$-Lactame sowie dabei anfallende Zwischenprodukte, des weiteren auch entsprechende Arzneimittel.

Die Produkte besitzen $\beta$-Lactamase hemmende Eigenschaften und sind nützlich bei der Bekämpfung von $\beta$-Lactamase bildenden Krankheitserregern in Kombination mit $\beta$-Lactam-Antibiotika. Sie zeigen ebenfalls eigene antibakterielle Wirkung und können demnach für sich allein bei der Bekämpfung von Infektionskrankheiten eingesetzt werden.

Die vorliegende Erfindung betrifft β-Lactame der allgemeinen Formel

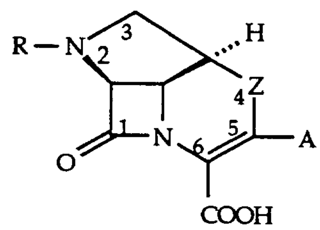

I

in der Z Methylen, Sauerstoff oder Schwefel und R Wasserstoff, ggfs. durch Carboxy, niederes Alkoxycarbonyl, Carbamoyl, niederes Alkylcarbamoyl, Phenylcarbamoyl oder Hydroxyphenylcarbamoyl substituiertes niederes (Cyclo)alkyl, niederes Alkenylmethyl, niederes Alkenylmethoxycarbonyl, Formyl, ggfs. durch Halogen, Cyan, Carbamoyl-niederes Alkoxy, Carbamoyl-niederes Alkylthio oder Carbamoyl-niederes Alkylamino substituiertes niederes (Cyclo)alkanoyl bzw. (Cyclo)alkylsulfonyl, ggfs. durch niederes (Cyclo)-alkyl, niederes Alkoxycarbonyl-niederes Alkyl, Benzyloxycarbonyl-niederes Alkyl oder Carboxy-niederes Alkyl substituiertes Carbamoyl oder eine Ringstruktur der allgemeinen Formeln

Q-X-CO- (a1)

Q-X-$SO_2$- (a2)

bedeutet, worin Q einen 5- oder 6-gliedrigen, ggfs. stickstoff-, schwefel- und/oder sauerstoffhaltigen Ring und X eine direkte Bindung oder eine der Gruppen -$CH_2$-, -$CH_2CH_2$-, -CH=CH-, -NH-, -$NHCH_2$-, -$CH_2NH$-, -CH($NH_2$)-, -$CH_2CH_2NH$-, -C(=$NOCH_3$)-, -$OCH_2$- und -$SCH_2$- darstellt; und worin ferner A niederes Alkyl, Hydroxy-niederes Alkyl, Vinyl, Cyanvinyl, niederes Alkoxy, ggfs. phenylsubstituiertes niederes (Cyclo)alkanoyloxy bzw. (Cyclo)alkylsulfonyloxy, ggfs. nieder-(cyclo)alkyl-substituiertes Benzoyloxy bzw. Phenylsulfonyloxy, einen Rest -S-Het oder -S-$CH_2$-Het, worin Het einen 5- oder 6-gliedrigen, stickstoff-, schwefel- und/oder sauerstoffhaltigen Heterocyclus darstellt, oder einen Rest -$CH_2$-L bedeutet, worin L ggfs. phenylsubstituiertes niederes (Cyclo)alkanoyloxy bzw. (Cyclo)alkylsulfonyloxy, ggfs. nieder(cyclo)alkyl-substituiertes Benzoyloxy bzw. Phenylsulfonyloxy, Carbamoyloxy, niederes (Cyclo)alkoxycarbonyl, Carboxy, Azido, Amino, niederes (Cyclo)alkanoylamino, niederes (Cyclo)alkylsulfonylamino, niederes (Cyclo)alkylamino, di-niederes (Cyclo)alkylamino, einen 5- oder 6-gliedrigen, an einem Stickstoffatom gebundenen Ring oder einen Rest -S-Het oder -S-$CH_2$-Het, worin Het die obige Bedeutung hat, darstellt,
und pharmazeutisch verträgliche, leicht hydrolysierbare Ester und Salze dieser Verbindungen.

Diese Verbindungen zeichnen sich durch therapeutisch wertvolle Eigenschaften aus. Insbesondere besitzen sie ausgeprägte β-Lactamase hemmende Eigenschaften und sind somit nützlich bei der Bekämpfung von β-Lactamase bildenden Krankheitserregern in Kombination mit β-Lactam-Antibiotika, wie den Penicillinen, Cephalosporinen, Penemen und Carbapenemen. Sie besitzen auch an sich eine antibakterielle Wirkung und können somit auch allein gegen Krankheitserreger eingesetzt werden.

Gegenstand der vorliegenden Erfindung sind β-Lactame der obigen allgemeinen Formel I und pharmazeutisch verträgliche Salze davon als solche und als pharmazeutische Wirkstoffe, die Herstellung dieser Verbindungen und Zwischenprodukte für die Herstellung dieser Verbindungen, ferner Arzneimittel enthaltend eine Verbindung der allgemeinen Formel I oder pharmazeutisch verträgliche Salze davon und die Herstellung solcher Arzneimittel, sowie die Verwendung von Verbindungen der allgemeinen Formel I und von pharmazeutisch verträglichen Salzen davon bei der Bekämpfung bzw. Verhütung von Krankheiten.

Die in der obigen Definition aufgeführten Ausdrücke in Klammern, z.B. "niederes (Cyclo)alkyl", "niederes (Cyclo)alkanoyl", "niederes (Cyclo)alkyl-phenylsulfonyloxy" sind als fakultativ zu verstehen, sodass sowohl "niederes Alkyl", "niederes Alkanoyl" und "niederes Alkylphenylsulfonyloxy" als auch "niederes Cycloalkyl", "niederes Cycloalkanoyl" und "niederes Cycloalkyl-phenylsulfonyloxy" vorgesehen sind. Der Ausdruck "niederes Alkyl", für sich allein genommen oder in Kombinationen, wie "niederes Alkoxy", "niederes Alkylamino", "di-niederes Alkylamino", "niederes Alkylsulfonyloxy", "niederes Alkoxycarbonyl", "niederes Alkanoyl" (= "niederes Alkylcarbonyl"), "niederes Alkanoyloxy" und dergleichen, bedeutet geradkettige oder verzweigte gesättigte Kohlenwasserstoffreste mit höchstens 7, vorzugsweise höchstens 4, Kohlenstoffatomen, wie Methyl, Aethyl, n-Propyl, Isopropyl, n-Butyl, Isobutyl, sec-Butyl, t-Butyl und dergleichen. "Niederes Cycloalkyl" für sich allein genommen oder in entsprechenden Kombinationen bedeutet cyclische Kohlenwasserstoffreste mit 3-6 Kohlenwasserstoffatomen, d.h. Cyclopropyl, Cyclobutyl,

Cyclopentyl oder Cyclohexyl. "Halogen" bedeutet Fluor, Chlor, Brom oder Jod, insbesondere Fluor. Die "5- oder 6-gliedrigen, ggfs. stickstoff-, schwefel- und/oder sauerstoffhaltigen Ringe" sind z.B. Phenyl, gesättigte Heterocyclen wie Pyrrolidinyl, Piperidinyl, Piperazinyl, Morpholinyl, Thiomorpholinyl, Tetrahydrothienyl, Tetrahydrofuryl, aromatische Heterocyclen wie 2-Furyl, 3-Furyl, Thiazolyl, Thiadiazolyl, Oxadiazolyl, 2-Thienyl, 3-Thienyl, 2-Pyrrolyl, 3-Pyrrolyl, 1-Pyridinio, 2-Pyridyl, 3-Pyridyl, 4-Pyridyl, Oxazolyl, Isoxazolyl, Imidazolyl, Pyrazolyl, Triazolyl, Tetrazolyl, Pyrazinyl, Pyridazinyl und Pyrimidinyl. Diese Gruppen können auch substituiert sein, z.B. durch niederes Alkyl, niederes Alkoxy, niederes Alkylthio, Hydroxy, Carbamoyl, Carbamoylmethyl, Carbamoylamino, Sulfamoyl, niederes Alkanoyloxy, Sulfonyloxy, Halogen, Amino, Methylamino, Dimethylamino, Chloracetylamino, Pyridin-1-ylio-acetylamino. N-Heterocyclen können zusätzlich auch durch Oxo substituiert sein. Beispiele solcher substituierter Ringe sind 4-Tolyl, 4-Sulfamoylphenyl, 4-Hydroxyphenyl, 4-Carbamoylphenyl, 3,4-Dihydroxyphenyl, 3-Methyl-(2-furyl), 1-Methyl-1H-tetrazol-5-yl, 4-Anisyl, 3,4,5-Trimethoxyphenyl, 4-Chlorphenyl, 4-Fluor-(2-pyridyl), 2-Amino-4-thiazolyl, 5-Methyl-1,3,4-thiadiazol-2-yl, p-Amino-phenyl, p-(Chloracetylamino)-phenyl, 3,4-Disulfonyloxy-phenyl, 3,4-Diacetoxyphenyl, 2-Oxo-pyrrolidinyl-3-yl, 2-Oxo-tetrahydrothien-3-yl, 3-Methoxy-isoxazol-5-yl, 1,1-Dioxo-tetrahydrothien-3-yl, 3-Hydroxy-isoxazol-5-yl, 5-Amino-1,3,4-thiadiazol-2-yl, 5-(Pyridin-1-ylio-acetylamino)-1,3,4-thiadiazol-2-yl, 1-Methyl-pyridin-4-ylio. Ein weiterer Ring kann ankondensiert sein, insbesondere ein Phenylring, wie z.B. in Indolyl, 1H-Benzotriazol-2-yl, 2-Oxo-2H-1-benzopyran-7-yl, 2-Oxo-4-(trifluormethyl)-2H-1-Benzopyran-7-yl, ein gesättigter 5- oder 6-gliedriger carbocyclischer Ring, wie z.B. in 2,3-Cyclopenteno-4-pyridyl (1-Pyrindin-4-yl), 2,3-Cyclohexeno-4-pyridyl, oder auch ein 5- oder 6-gliedriger Heterocyclus, wie z.B. in Benzimidazol-5-yl, 1H-Benzotriazol-4-yl oder 2-Carbamoyl-5-methyl[1,2,4]triazolo[1,5-a]pyrimidin-7-yl. Unter "5- oder 6-gliedrige, an einem Stickstoffatom gebundene Ringe" versteht man N-quaternäre Ringe, welche sowohl aromatisch (wie bei 1-Pyridinio) oder auch gesättigt (wie bei 1-Methyl-1-Pyrrolidinio oder 1-Methyl-1-Piperidinio) sein können; oder auch N-tertiäre Ringe (wie z.B. 5-Methylsulfanyl-1H-tetrazol-1-yl).

Eine bevorzugte Untergruppe von R besitzt die Formel

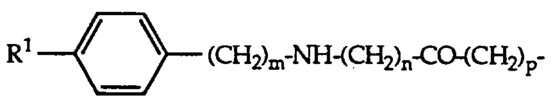 (b)

worin $R^1$ Wasserstoff, Hydroxy, Carbamoyl oder Sulfamoyl und m, n und p jeweils 0 oder 1 darstellen.

Bevorzugte Untergruppen von (b) sind:

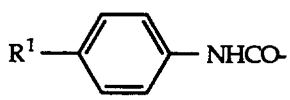 (b1)

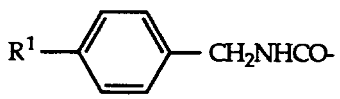 (b2)

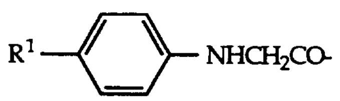 (b3)

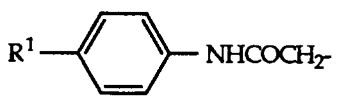 (b4)

Bevorzugt sind:

(b1) mit $R^1$ = Hydroxy oder Carbamoyl, d.h. 4-Hydroxyphenylcarbamoyl und 4-Carbamoylphenylcarbamoyl.

Weitere bevorzugte Gruppen R sind die ggfs. Fluor- oder Cyan-substituierten niederen Alkanoyl- bzw. niederen Alkylsulfonylgruppen. Insbesondere bevorzugt sind Formyl, Acetyl, Trifluoracetyl, Cyanacetyl und Methylsulfonyl.

Weitere bevorzugte Bedeutungen für R sind:
Wasserstoff
2-Oxo-pyrrolidin-3-ylcarbamoyl
Thien-2-yl-methylcarbamoyl
3,4-Dihydroxy-benzylcarbamoyl
2-Oxo-tetrahydrothien-3-ylcarbamoyl
4-Sulfamoyl-benzylcarbamoyl
3-Methoxy-isoxazol-5-ylmethylcarbamoyl
3-Hydroxy-isoxazol-5-ylmethylcarbamoyl
1,1-Dioxo-tetrahydrothien-3-ylmethylcarbamoyl
(2-Amino-thiazol-4-yl)-methoxyiminoacetyl
1-Methyl-1H-tetrazol-5-ylsulfanylacetyl
3-Carbamoyl-pyridin-1-ylioacetyl
2-t-Butoxycarbonyl-äthylcarbamoyl
4-Hydroxy-benzylcarbamoyl
Trifluormethylsulfonyl
Benzyloxycarbonylmethylcarbamoyl
Benzylcarbamoyl
Cyclopropylcarbamoyl
4-Sulfamoyl-benzylcarbamoyl
2-Thiophen-2-yl-äthylcarbamoyl
5-Methyl-1,3,4-thiadiazol-2-yl-sulfonylacetyl
5-Amino-1,3,4-thiadiazol-2-yl-sulfonylacetyl
Pyridin-4-ylsulfanylacetyl
Phenylaminoacetyl
4-Hydroxy-phenylcarbamoylmethyl
Methoxycarbonylmethyl
Aethyl
Carbamoylmethyl
Pyridin-4-ylsulfanylacetyl
3-Carbamoyl-pyridin-1-ylioacetyl
Carbamoylmethylsulfanylacetyl
(R)-(N-Benzyloxycarbonyl)-2-phenylglycyl
(R)-2-Phenylglycyl
Carboxymethylcarbamoyl.

Die im unter A aufgeführten Rest $-CH_2-L$ vorhandene Gruppe L kann u.a. auch "Carbamoyloxy" bedeuten. Solche Carbamoylgruppen können durch die allgemeinen Formel

$-OCONR^2R^3$ (c)

charakterisiert sein,

worin $R^2$ Wasserstoff und $R^3$ Wasserstoff, niederes (Cyclo)alkyl, Halogen-niederes Alkyl, Carbamoylmethyl oder einen Rest $-(CH_2)_qQ$ bedeutet, worin q 0, 1 oder 2 ist und Q obige Bedeutung hat, oder auch $R^2$ und $R^3$ zusammen mit dem Stickstoffatom einen gesättigten, ggfs. Schwefel, Sauerstoff oder zusätzlichen Stickstoff enthaltenden N-Heterocyclus darstellt.

Beispiele für Substituenten $R^3$ sind:
Methyl
Cyclopropyl
2,2,2-Trifluoräthyl
Phenyl
p-Hydroxyphenyl
Benzyl
p-Hydroxybenzyl
4-Pyridylmethyl
Carbamoylmethyl
1H-Tetrazol-5-yl.

Beispiele für gesättigte Heterocyclen $-NR^2R^3$ sind:
Piperazinyl

4-Methyl-piperazinyl
4-Morpholinyl
4-Thiomorpholinyl.

Vorzugsweise sind $R^2$ und $R^3$ beide Wasserstoff, indem (c) die Carbamoyloxygruppe darstellt, d.h. A ist vorzugsweise Carbamoyloxymethyl.

Die unter A fallende Definition -S-Het, $-SCH_2$-Het, $-CH_2S$-Het und $-CH_2SCH_2$-Het beinhalten eine Untergruppe von Substituenten der allgemeinen Formel

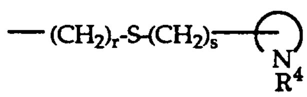 (d)

in der r und s jeweils 0 oder 1 und

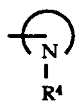

einen 5- oder 6-gliedrigen, ggfs. ein Schwefel- oder Sauerstoffatom enthaltenden N-Heterocyclus darstellt; und worin $R^4$ niederes Alkyl, Sulfonylmethyl oder eine Gruppe der allgemeinen Formel

$-CH_2CONR^5R^6$ (d1)

bedeutet und $R^5$ Wasserstoff und $R^6$ Wasserstoff, niederes (Cyclo)alkyl, Hydroxy, Carbamoylmethyl, Halogen-niederes Alkyl oder einen Rest $-(CH_2)_qQ$ bedeutet, worin q 0, 1 oder 2 ist und Q obige Bedeutung hat, oder auch $R^5$ und $R^6$ zusammen mit dem Stickstoffatom einen gesättigten, ggfs. Schwefel, Sauerstoff oder zusätzlichen Stickstoff enthaltenden N-Heterocyclus darstellt.

Der obige N-Heterocyclus

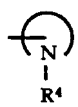

ist vorzugsweise ein 1-$R^4$-substituierter 1H-Tetrazol-5-ylrest der Formel

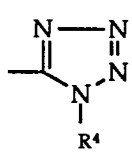

Beispiele für Substituenten $R^6$ sind:
Methyl
Cyclopropyl
Phenyl
p-Hydroxyphenyl
Benzyl
Phenäthyl
Carbamoylmethyl
Hydroxy.

Beispiele für gesättigte Heterocyclen $-NR^5R^6$ sind:
Piperazinyl

4-Methyl-piperazinyl
4-Morpholinyl
4-Thiomorpholinyl.

Vorzugsweise ist $R^4$ Methyl oder Carbamoylmethyl (d.h. $R^5$ und $R^6$ beide Wasserstoff); besonders bevorzugte Gruppen A sind 1-Methyl-1H-tetrazol-5-ylsulfanylmethyl und 1-Carbamoylmethyl-1H-tetrazol-5-ylsulfanylmethyl.

Weitere bevorzugte Untergruppen von A sind der Formel

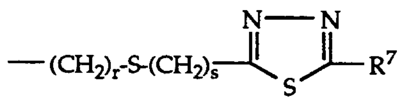 (e)

in der r und s jeweils 0 oder 1 und $R^7$ Methyl, Amino, Acetylamino oder Pyridinioacetylamino darstellt.

Besonders bevorzugt sind 5-Amino-1,3,4-thiadiazol-2-yl-sulfanylmethyl und 5-Amino-1,3,4-thiadiazol-2-yl-sulfanyl.

Weitere bevorzugte Untergruppen von A sind der Formel

$-(CH_2)_r-S-(CH_2)_s-R^8$ (f)

in der r und s jeweils 0 oder 1 ist und $R^8$ die Pyridin-4-yl-Gruppe oder die Gruppe

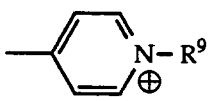 (g)

darstellt und $R^9$ Methyl, Benzyl, Carboxymethyl oder Carbamoylmethyl bedeutet.

Bevorzugt ist A Pyridin-4-yl-sulfanylmethyl.

Weitere bevorzugte Bedeutungen für A sind:
1-Methyl-1H-tetrazol-5-ylsulfanyl
5-Methyl-1,3,4-thiadiazol-2-ylsulfanyl
5-(Pyridin-1-ylioacetylamino)-1,3,4-thiadiazol-2-ylsulfanyl
1-Methyl-pyridin-4-yliosulfanylmethyl
Pyridin-1-yliomethyl
Methylsulfonyloxy
4-Methyl-phenylsulfonyloxy
Carboxymethyl
Methoxycarbonylmethyl
Methyl
Vinyl
Acetoxymethyl
2-Carbamoyl-5-methyl[1,2,4]triazolo[1,5-a]pyrimidin-7-ylsulfanylmethyl
1-(Cyclopropyl-carbamoylmethyl)-1H-tetrazol-5-ylsulfanylmethyl
1-(Phenyläthyl-carbamoylmethyl)-1H-tetrazol-5-ylsulfanylmethyl
1-(Carbamoylmethyl-carbamoylmethyl)-1H-tetrazol-5-ylsulfanylmethyl
1-Methylcarbamoylmethyl-1H-tetrazol-5-ylsulfanylmethyl
1-(2-Morpholin-4-yl-2-oxo-äthyl)-1H-tetrazol-5-ylsulfanylmethyl
1-(4-Hydroxyphenyl-carbamoylmethyl)-1H-tetrazol-5-ylsulfanylmethyl
1-(Hydroxy-carbamoylmethyl)-1H-tetrazol-5-ylsulfanylmethyl
1-Sulfonylmethyl-1H-tetrazol-5-ylsulfanylmethyl
1-Methyl-imidazol-2-ylsulfanylmethyl
5-Hydroxy-4-methyl-4H-[1,2,4]-triazol-3-ylsulfanylmethyl
6,7-Dihydro-5H-1-pyrindin-4-ylsulfanylmethyl
5-Methyl-1,3,4-thiadiazol-2-ylsulfanylmethyl
1-Methyl-1H-tetrazol-5-yl-methylsulfanylmethyl
5-Methylsulfanyl-1H-tetrazol-1-ylmethyl

4-Methyl-4-pyridiniosulfanyl
Carbamoylmethylsulfanyl
5-(1,4-Dimethyl-1H-1,2,4-triazol-4-ium)-methylsulfanyl
Pyridin-4-ylsulfanyl
5-Acetylamino-1,3,4-thiadiazol-2-ylsulfanylmethyl
2-Cyanvinyl (Z- und E-Isomere)
1-Carboxymethyl-pyridin-4-yliosulfanylmethyl
1-Carbamoylmethyl-pyridin-4-yliosulfanylmethyl
1-Benzyl-pyridin-4-yliosulfanylmethyl
2,5-Dihydro-6-hydroxy-2-methyl-5-oxo-1,2,4-triazin-3-ylsulfanylmethyl
Piperidin-1-ylmethyl
3-Benzyloxycarbonylmethyl-pyridin-1-yliomethyl
3-Carboxymethyl-pyridin-1-yliomethyl
4-Pyridin-3-yl-thiazol-2-ylsulfanylmethyl
Pyrimidin-2-ylsulfanyl
1H-1,2,4-Triazol-3-ylsulfanylmethyl
Azidomethyl
Acetylaminomethyl
Methylsulfonylaminomethyl
4-Hydroxy-phenylcarbamoyloxymethyl
2,2,2-Trifluoräthylcarbamoyloxymethyl
Cyclopropylcarbamoyloxymethyl
Carbamoylmethylcarbamoyloxymethyl
Methylcarbamoyloxymethyl
Pyridincarbamoyloxymethyl
4-Hydroxy-benzylcarbamoyloxymethyl
4-Methyl-piperazin-1-ylcarbonyloxymethyl
1H-Tetrazol-5-yl-amino-carbonyloxymethyl
Methoxy.

Wie oben bereits angegeben, sind ganz besonders bevorzugte Bedeutungen für A:
Carbamoyloxymethyl
Pyridin-4-ylsulfanylmethyl
1-Methyl-1H-tetrazol-5-ylsulfanylmethyl
1-Carbamoylmethyl-1H-tetrazol-5-ylsulfanylmethyl
5-Amino-1,3,4-thiadiazol-2-ylsulfanylmethyl
5-Amino-1,3,4-thiadiazol-2-ylsulfanyl.

Besonders bevorzugte Verbindungen der Formel I bzw. ihre Salze sind

(1aS,3aR,6bR)-5-Carbamoyloxymethyl-2-(4-hydroxy-phenylcarbamoyl)-1-oxo-1a,2,3,3a,4,6b-hexahydro-1H-2,6a-diazacyclobut[cd]inden-6-carbonsäure

(1aS,3aR,6bR)-5-Carbamoyloxymethyl-2-(4-carbamoyl-phenylcarbamoyl)-1-oxo-1a,2,3,3a,4,6b-hexahydro-1H-2,6a-diazacyclobut[cd]inden-6-carbonsäure

(1aS,3aR,6bR)-2-(4-Carbamoyl-phenylcarbamoyl)-1-oxo-5-(pyridin-4-ylsulfanylmethyl)-1a,2,3,3a,4,6b-hexahydro-1H-2,6a-diazacyclobut[cd]inden-6-carbonsäure,

(1aS,3aR,6bR)-5-(5-Amino-1,3,4-thiadiazol-2-ylsulfanylmethyl)-2-(4-hydroxy-phenylcarbamoyl)-1-oxo-1a,2,3,3a,4,6b-hexahydro-1H-2,6a-diazacyclobut[cd]inden-6-carbonsäure

(1aS,3aR,6bR)-5-(5-Amino-1,3,4-thiadiazol-2-ylsulfanyl)-2-(4-hydroxyphenylcarbamoyl)-1-oxo-1a,2,3,3a,4,6b-hexahydro-1H-2,6a-diazacyclobut[cd]inden-6-carbonsäure

(1aS,3aR,6bR)-5-Carbamoyloxymethyl-1-oxo-2-trifluoracetyl-1a,2,3,3a,4,6b-hexahydro-1H-2,6a-diaza-cyclobut[cd]inden-6-carbonsäure

(1aS,3aR,6bR)-1-Oxo-5-(pyridin-4-ylsulfanylmethyl)-2-trifluoracetyl-1a,2,3,3a,4,6b-hexahydro-1H-2,6a-diaza-cyclobut[cd]inden-6-carbonsäure

(1aS,3aR,6bR)-5-(1-Carbamoylmethyl-1H-tetrazol-5-ylsulfanylmethyl)-2-formyl-1-oxo-1a,2,3,3a,4,6b-hexahydro-1H-2,6a-diaza-cyclobut[cd]inden-6-carbonsäure

(1aS,3aR,6bR)-2-Methylsulfonyl-5-(1-methyl-1H-tetrazol-5-ylsulfanylmethyl)-1-oxo-1a,2,3,3a,4,6b-hexahydro-1H-2,6a-diaza-cyclobut[cd]inden-6-carbonsäure

(1aS,3aR,6bR)-2-Acetyl-5-(1-carbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure

(1aS,3aR,6bS)-2-Acetyl-5-(1-methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure
sowie entsprechende, pharmazeutisch verträgliche Salze davon, insbesondere die Natriumsalze.

Andere bevorzugte Verbindungen der Formel I bzw. ihre Salze sind:

(1aS,3aR,6bR)-2-(4-Hydroxy-phenylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure

(1aS,3aR,6bR)-2-(4-Carbamoyl-phenylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure

(1aS,3aR,6bR)-2-[(S)-2-Oxo-pyrrolidin-3-ylcarbamoyl]-5-(1-methyl-1H-tetrazol-5-ylsulfanyl)-1-oxo-1a,2,3,3a,4,6b-hexahydro-1H-2,6a-diazacyclobut[cd]inden-6-carbonsäure

(1aS,3aR,6bR)-5-(1-Methyl-1H-tetrazol-5-ylsulfanyl)-1-oxo-2-(thien-2-yl-methylcarbamoyl)-1a,2,3,3a,4,6b-hexahydro-1H-2,6a-diazacyclobut[cd]inden-6-carbonsäure

(1aS,3aR,6bR)-2-(3,4-Dihydroxy-benzylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure

(1aS,3aR,6bR)-5-(1-Methyl-1H-tetrazol-5-ylsulfanyl)-1-oxo-2-[(R)- und [(S)-2-oxo-tetrahydro-thien-3-ylcarbamoyl]-1a,2,3,3a,4,6b-hexahydro-1H-2,6a-diazacyclobut[cd]inden-6-carbonsäure

(1aS,3aR,6bR)-5-(1-Methyl-1H-tetrazol-5-ylsulfanyl)-1-oxo-2-(4-sulfamoyl-benzylcarbamoyl)-1a,2,3,3a,4,6b-hexahydro-1H-2,6a-diazacyclobut[cd]inden-6-carbonsäure

(1aS,3aR,6bR)-2-(3-Methoxy-isoxazol-5-ylmethylcarbamoyl)-5-(1-methyl-1H-tetrazol-5-ylsulfanyl)-1-oxo-1a,2,3,3a,4,6b-hexahydro-1H-2,6a-diazacyclo but[cd]inden-6-carbonsäure

(1aS,3aR,6bR)-2-[(R)- und [(S)-1,1-Dioxo-tetrahydrothien-3-ylcarbamoyl]-5-(1-methyl-1H-tetrazol-5-ylsulfanyl)-1-oxo-1a,2,3,3a,4,6b-hexahydro-1H-2,6a-diazacyclobut[cd]inden-6-carbonsäure

(1aS,3aR,6bR)-2-(4-Hydroxyphenylcarbamoyl)-5-(5-methyl-1,3,4-thiadiazol-2-ylsulfanyl)-1-oxo-1a,2,3,3a,4,6b-hexahydro-1H-2,6a-diazacyclobut[cd]inden-6-carbonsäure

(1aS,3aR,6bR)-2-(4-Carbamoyl-phenylcarbamoyl)-5-(5-methyl-1,3,4-thiadiazol-2-ylsulfanyl)-1-oxo-1a,2,3,3a,4,6b-hexahydro-1H-2,6a-diazacyclobut[cd]inden-6-carbonsäure

(1aS,3aR,6bR)-5-(5-Amino-1,3,4-thiadiazol-2-ylsulfanyl)-2-(4-carbamoylamino-phenylcarbamoyl)-1-oxo-1a,2,3,3a,4,6b-hexahydro-1H-2,6a-diazacyclobut[cd]inden-6-carbonsäure

(1aS,3aR,6bR)-2-(4-Hydroxy-phenylcarbamoyl)-1-oxo-5-(5-pyridin-1-ylacetylamino-1,3,4-thiadiazol-2-ylsulfanyl)-1a,2,3,3a,4,6b-hexahydro-1H-2,6a-diazacyclobut[cd]inden-6-carbonsäure

(Z)-(1aS,3aR,6bR)-2-[(2-Amino-thiazol-4-yl)-methoxyiminoacetyl]-1-oxo-5-(1-methyl-1H-tetrazol-5-ylsulfanyl)-1a,2,3,3a,4,6b-hexahydro-1H-2,6a-diazacyclobut[cd]inden-6-carbonsäure

(1aS,3aR,6bR)-2-(4-Hydroxy-phenylcarbamoyl)-5-methylsulfonyloxy-1-oxo-1a,2,3,3a,4,6b-hexahydro-1H-2,6a-diazacyclobut[cd]inden-6-carbonsäure

(1aS,3aR,6bR)-2-(4-Carbamoyl-phenylcarbamoyl)-5-methylsulfonyloxy-1-oxo-1a,2,3,3a,4,6b-hexahydro-1H-2,6a-diazacyclobut[cd]inden-6-carbonsäure

(1aS,3aR,6bR)-5-Methylsulfonyloxy-2-(thien-2-ylmethylcarbamoyl)-1-oxo-1a,2,3,3a,4,6b-hexahydro-1H-2,6a-diazacyclobut[cd]inden-6-carbonsäure

(1aS,3aR,6bR)-2-(3,4-Dihydroxy-benzylcarbamoyl)-5-methylsulfonyloxy-1-oxo-1a,2,3,3a,4,6b-hexahydro-1H-2,6a-diazacyclobut[cd]inden-6-carbonsäure

(1aS,3aR,6bR)-5-Methylsulfonyloxy-1-oxo-2-[(S)-2-oxo-pyrrolidin-3-ylcarbamoyl]-1a,2,3,3a,4,6b-hexahydro-1H-2,6a-diazacyclobut[cd]inden-6-carbonsäure

(1aS,3aR,6bR)-5-Methylsulfonyloxy-1-oxo-2-[(R) und -[(S)-2-oxo-tetrahydro-thien-3-ylcarbamoyl]-1a,2,3,3a,4,6b-hexahydro-1H-2,6a-diazacyclobut[cd]inden-6-carbonsäure

(1aS,3aR,6bR)-2-[(R)- und [(S)-1,1-Dioxo-tetrahydrothien-3-ylcarbamoyl]-5-methylsulfonyloxy-1-oxo-1a,2,3,3a,4,6b-hexahydro-1H-2,6a-diazacyclobut[cd]inden-6-carbonsäure

(1aS,3aR,6bR)-5-Methylsulfonyloxy-1-oxo-2-(4-sulfamoylbenzylcarbamoyl)-1a,2,3,3a,4,6b-hexahydro-1H-2,6a-diazacyclobut[cd]inden-6-carbon säure

(1aS,3aR,6bR)-2-(4-Hydroxy-phenylcarbamoyl)-5-(4-methyl-phenylsulfonyloxy)-1-oxo-1a,2,3,3a,4,6b-hexahydro-1H-2,6a-diazacyclobut[cd]inden-6-carbonsäure

(1aS,3aR,6bR)-2-(3-Carbamoyl-pyridin-1-ylioacetyl)-5-methylsulfonyloxy-1-oxo-1a,2,3,3a,4,6b-hexahydro-1H-2,6a-diazacyclobut[cd]inden-6-carboxylat

(1aS,3aR,6bR)-5-Methylsulfonyloxy-2-(1-methyl-1H-tetrazol-5-ylsulfanylacetyl)-1-oxo-1a,2,3,3a,4,6b-hexahydro-1H-2,6a-diazacyclobut[cd]inden-6-carbonsäure

(1aS,3aR,6bR)-5-Carboxymethyl-2-(4-hydroxy-phenylcarbamoyl)-1-oxo-1a,2,3,3a,4,6b-hexahydro-1H-2,6a-diazacyclobut[cd]inden-6-carbonsäure

(1aS,3aR,6bR)-2-(4-Hydroxy-phenylcarbamoyl)-5-methoxycarbonylmethyl-1-oxo-1a,2,3,3a,4,6b-hexahydro-1H-2,6a-diazacyclobut[cd]inden-6-carbonsäure

(1aS,3aR,6bR)-2-(4-Hydroxy-phenylcarbamoyl)-5-(1-methyl-1H-tetrazol-5-ylsulfanylmethyl)-1-oxo-1a,2,3,3a,4,6b-hexahydro-1H-2,6a-diazacyclobut[cd]inden-6-carbonsäure

(1aS,3aR,6bR)-2-(4-Carbamoyl-phenylcarbamoyl)-5-(1-methyl-1H-tetrazol-5-ylsulfanylmethyl)-1-oxo-1a,2,3,3a,4,6b-hexahydro-1H-2,6a-diazacyclobut[cd]inden-6-carbonsäure

(1aS,3aR,6bR)-2-[(S)-2-Oxo-pyrrolidin-3-ylcarbamoyl]-5-(1-methyl-1H-tetrazol-5-ylsulfanylmethyl]-1-oxo-1a,2,3,3a,4,6b-hexahydro-1H-2,6a-diazacyclobut[cd]inden-6-carbonsäure

(1aS,3aR,6bR)-2-Acetyl-5-(1-methyl-1H-tetrazol-5-ylsulfanylmethyl)-1-oxo-1a,2,3,3a,4,6b-hexahydro-1H-2,6a-diazacyclobut[cd]inden-6-carbonsäure

(1aS,3aR,6bR)-2-Acetyl-1-oxo-5-(pyridin-4-ylsulfanylmethyl)-1a,2,3,3a,4,6b-hexahydro-1H-2,6a-diazacyclobut[cd]inden-6-carbonsäure

(1aS,3aR,6bR)-2-(4-Carbamoyl-phenylcarbamoyl)-5-(1-methyl-pyridin-1-yliosulfanylmethyl)-1-oxo-1a,2,3,3a,4,6b-hexahydro-1H-2,6a-diazacyclobut[cd]inden-6-carboxylat

(1aS,3aR,6bR)-2-(4-Hydroxy-phenylcarbamoyl)-1-oxo-5-(pyridin-1-yliomethyl)-1a,2,3,3a,4,6b-hexahydro-1H-2,6a-diazacyclobut[cd]inden-6-carboxylat

(1aS,3aR,6bR)-2-(3-Hydroxy-isoxazol-5-ylmethylcarbamoyl)-1-oxo-5-(pyridin-1-yliomethyl)-1a,2,3,3a,4,6b-hexahydro-1H-2,6a-diazacyclobut[cd]inden-6-carboxylat

(1aS,3aR,6bR)-2-(4-Carbamoyl-phenylcarbamoyl)-1-oxo-5-(pyridin-1-yliomethyl)-1a,2,3,3a,4,6b-hexahydro-1H-2,6a-diazacyclobut[cd]inden-6-carboxylat

(1aS,3aR,6bR)-2-Acetyl-1-oxo-5-(pyridin-1-yliomethyl)-1a,2,3,3a,4,6b-hexahydro-1H-2,6a-diazacyclobut-[cd]inden-6-carboxylat

(1aS,3aR,6bR)-1-Oxo-5-(pyridin-1-yliomethyl)-2-trifluoracetyl-1a,2,3,3a,4,6b-hexahydro-1H-2,6a-diazacyclobut[cd]inden-6-carboxylat

(1aS,3aR,6bR)-5-Acetoxymethyl-2-(4-hydroxy-phenylcarbamoyl)-1-oxo-1a,2,3,3a,4,6b-hexahydro-1H-2,6a-diazacyclobut[cd]inden-6-carbonsäure

sowie entsprechende, pharmazeutisch verträgliche Salze davon, insbesondere die Natriumsalze.

Als leicht hydrolysierbare Ester der Verbindungen der Formel I sind Verbindungen der Formel I zu verstehen, deren Carboxygruppe bzw. Carboxygruppen (z.B. die 6-Carboxygruppe) in Form einer leicht hydrolysierbaren Estergruppe vorliegt/vorliegen. Beispiele solcher Ester, die herkömmlicher Art sein können, sind die niederen Alkanoyloxyalkylester, z.B. der Acetoxymethyl-, Pivaloyloxymethyl-, 1-Acetoxyäthyl- und der 1-Pivaloyloxyäthylester; die niederen Alkoxycarbonyloxyalkylester, z.B. der Methoxycarbonyloxymethyl-, 1-Aethoxycarbonyloxyäthyl- und der 1-Isopropoxycarbonyloxyäthylester; der 1-Cyclohexyloxycarbonyloxyäthylester; der (5-Methyl-2-oxo-1,3-dioxol-4-yl)-methylester; die Lactonylester, z.B. der Phthalidyl und der Thiophthalidylester; die niederen Alkoxymethylester, z.B. der Methoxymethylester und die niederen Alkanoylaminomethylester, z.B. der Acetamidomethylester. Auch andere Ester, z.B. die Benzyl- und Cyanmethylester können brauchbar sein. Weitere leicht hydrolysierbare Ester sind die (2,2-Dimethyl-1-oxopropoxy)methylester, die 2-[(2-Methylpropoxy)carbonyl]-2-pentenylester, die 1-[[(1-Methyläthoxy)-carbonyl]oxy]äthylester und die 3,3-Dimethyl-2-oxobutylester.

Beispiele von Salzen der Verbindungen der Formel I sind Alkalimetallsalze, wie das Natrium- und das Kaliumsalz, Ammoniumsalze; Erdalkalimetallsalze, wie das Calciumsalz; Salze mit organischen Basen, wie die Salze mit Aminen, z.B. Salze mit N-Aethylpiperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin.

Die Verbindungen der Formel I, sofern sie eine basische funktionelle Gruppe, wie z.B. eine Aminogruppe besitzen, bilden ebenfalls Additionssalze mit organischen oder anorganischen Säuren. Beispiele für solche Salze sind Hydrohalogenide, beispielsweise Hydrochloride, Hydrobromide, Hydrojodide, sowie andere Mineralsäuresalze, wie Sulfate, Nitrate, Phosphate und dergleichen, Alkyl- und Monoarylsulfonate, wie Aethansulfonate, Toluolsulfonate, Benzolsulfonate und dergleichen und auch andere organische Säuresalze wie Acetate, Trifluoracetate, Tartrate, Maleate, Citrate, Benzoate, Salicylate, Ascorbate und dergleichen.

Die erfindungsgemässen $\beta$-Lactame der Formel I sowie deren pharmazeutisch verträgliche Salze können erfindungsgemäss hergestellt werden, indem man

a) in einer Verbindung der allgemeinen Formel

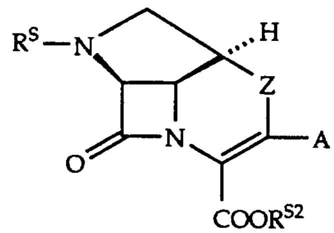

II

in der A und Z die oben gegebene Bedeutung haben, $R^S$ die für R angegebene Bedeutung hat oder eine Aminoschutzgruppe bedeutet und $R^{S2}$ eine Carboxyschutzgruppe darstellt,
die Carboxyschutzgruppe und eine allfällige Aminoschutzgruppe abspaltet und ein allenfalls erhaltenes Säureadditionssalz einer Verbindung der allgemeinen Formel

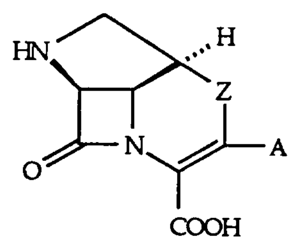

III

in der A und Z die oben gegebene Bedeutung haben,
gewünschtenfalls mit den Rest R abgebenden Mitteln behandelt und ggfs. allfällig noch vorhandene Schutzgruppen abspaltet, oder dass man
b) zur Herstellung eines leicht hydrolysierbaren Esters einer Verbindung der Formel I eine Carbonsäure der Formel I einer entsprechenden Veresterung unterwirft, oder dass man
c) zur Herstellung von pharmazeutisch verträglichen Salzen einer Verbindung der Formel I eine Verbindung der Formel I in ein solches Salz überführt.

Bei Z = Methylen erfolgt die bevorzugte Abspaltung von Schutzgruppen in Verbindungen der Formel II mit $R^{S2}$ = t-Butyl bzw. $R^S$ = t-Butoxycarbonyl und durch Behandlung mit einem sauren Mittel, vorzugsweise mit Trifluoressigsäure, in einem organischen Lösungsmittel wie Methylenchlorid, ggfs. in Gegenwart von Anisol, Phenol, Cresol oder Triäthylsilan, oder auch mit Chlorwasserstoff in einem organischen Lösungsmittel, wie Dioxan, Tetrahydrofuran oder Methylenchlorid. Die Temperatur liegt vorzugsweise zwischen -20 °C und Raumtemperatur; bei tieferer Temperatur, etwa -20 °C bis -10 °C wird vorzugsweise nur die t-Butoxycarbonylgruppe in 2-Stellung abgespalten, sodass im Reaktionsprodukt nach Einführung eines Restes R die t-Butylschutzgruppe in Stellung 6 in der obigen Weise abgespalten werden muss. Der Rest R bleibt dabei erhalten.

Bei Z = Sauerstoff oder Schwefel erfolgt die bevorzugte Abspaltung von Schutzgruppen in Verbindungen der Formel II mit $R^{S2}$ = Allyl bzw. $R^S$ = Allyloxycarbonyl und durch Behandlung mit einem Palladiumkatalysator, wie z.B. Bis-(triphenyl-phosphin)-palladium(II)dichlorid oder Tetrakispalladiumtriphenylphosphin, und einem $\pi$-Allylkomplex-Abfanger ("Scavenger"), wie Tributylzinnhydrid. Die Reaktion erfolgt in einem aprotischen organischen Lösungsmittel, wie Aethylacetat, Tetrahydrofuran oder Methylenchlorid, und, vorzugsweise, bei Raumtemperatur.

Für die Abspaltung von Allyloxycarbonyl- bzw. Allylgruppen, siehe auch J. Org. Chem. 1982, 47, 587.

Für die obige Schutzgruppenabspaltung eignen sich auch analoge Zwischenprodukte mit anderen Schutzgruppen (z.B. Benzyloxycarbonyl oder Chloracetyl in Stellung 2; p-Nitrobenzyl, Benzyl, Benzylhydryl in Stellung 6). Die Ausgangsverbindungen werden analog hergestellt und die Schutzgruppenabspaltung in an sich bekannter Weise durchgeführt, z.B.:

Stellung 2

Benzyloxycarbonyl: Hydrierung mit Palladiumkohle oder Behandeln mit Palladiumkohle und 1,4-Cyclohexadien in einem organischen Lösungsmittel wie Aethanol, Tetrahydrofuran, Dioxan, Aethylacetat, Dimethylformamid (ggfs. wässrig) bei etwa 0-80 °C;

Chloracetyl: Mit Thioharnstoff in einem polaren Lösungsmittel, vorzugsweise in Wasser bei neutralem pH, und etwa 0-30 °C; oder auch mit einem Alkalimetallbicarbonat, z.B. Natriumbicarbonat, in Methanol und/oder Tetrahydrofuran (ggfs. wässrig) bei etwa 0-30 °C.

Stellung 6

Benzyl und p-Nitrobenzyl: Hydrierung mit Palladiumkohle oder Palladiumoxid bei etwa 0 °C bis 80 °C in einem organischen Lösungsmittel wie Aethylacetat, Methanol oder Tetrahydrofuran, oder in Wasser, ggfs. in Gegenwart einer Säure, wie Essigsäure oder Salzsäure; oder Hydrolyse in Gegenwart von Natriumsulfid bei (oder unterhalb) 0 °C bis Zimmertemperatur in einem Lösungsmittel, wie z.B. Dimethylformamid (vorzugsweise wässrig).

Benzhydryl: Mit m-Kresol bei etwa 50 °C.

Es versteht sich, dass die Wahl der Schutzgruppen in Stellung 2 bzw. 6 von der Reaktivität der anderen Gruppen im Molekül abhängt. Beispielsweise sind bei einer Doppelbindung im Endprodukt (z.B. bei A = niederes Alkenyl) keine hydrogenolytisch abspaltbare Schutzgruppen für die Stellungen 2 (Benzyloxycarbonyl) und 6 (Benzyl oder p-Nitrobenzyl) zu wählen, weil die niedere Alkenylgruppe A gesättigt würde. Auch ist zu beachten, dass die olefinischen Schutzgruppen (Allyloxycarbonyl bzw. Allyl) nicht einer Hydrierung unterworfen werden können, weil sie dann gesättigt werden und anschliessend nicht mit brauchbaren Methoden abgespalten werden können.

Zur Einführung eines Restes R in Stellung 2 wird z.B. die Verbindung der Formel III mit einer Säure der Formel ROH oder mit einem ihrer reaktionsfähigen Derivate acyliert. Als Acylierungsmittel kommen in Frage: entsprechende Säuren der Formel ROH in Gegenwart von 2-Halogenpyridiniumsalzen, z.B. von 2-Chlor- oder 2-Fluor-1-methylpyridiniumchlorid oder -tosylat, oder auch in Gegenwart von Carbonyldiimidazol oder N,N'-Dicyclohexylcarbodiimid, letzteres bevorzugt zusammen mit N-Hydroxybenztriazol, N-Hydroxysuccinimid oder N-Hydroxyphthalimid. Es können auch entsprechende, reaktionsfähige Derivate der Carbonsäure eingesetzt werden, wie z.B. das Säurehalogenid (vorzugsweise das Chlorid), Säureanhydrid oder Säureazid. Ebenfalls verwendbar sind die entsprechenden Thiolester, wie z.B. 2-Benzthiazolylthioester, wie auch Hydroxybenztriazolester, N-Hydroxysuccinimidester oder N-Hydroxyphthalimidester. Die Umsetzung wird vorzugsweise in einem organischen Lösungsmittel oder Lösungsmittelgemisch, durchgeführt, z.B. Aceton, Methylenchlorid, Tetrahydrofuran, Dioxan, Dimethylacetamid, Dimethylformamid, Dimethylsulfoxid oder Acetonitril. Die Temperatur liegt im allgemeinen zwischen -30 °C und Raumtemperatur.

Zur Herstellung der leicht hydrolysierbaren Ester der Carbonsäuren der Formel I gemäss Variante b) des erfindungsgemässen Verfahrens wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triäthylamin, beschleunigt werden. Die Veresterungsreaktion wird vorzugsweise in einem inerten organischen Lösungsmittel, wie Dimethylacetamid, Hexamethylphosphorsäuretriamid, Dimethylsulfoxid oder, vorzugsweise, Dimethylformamid durchgeführt. Die Temperatur liegt vorzugsweise im Bereiche von etwa 0-40 °C.

Die Herstellung der Salze der Verbindungen der Formel I gemäss Variante c) des erfindungsgemässen Verfahrens kann in an sich bekannter Weise erfolgen, z.B. durch Umsetzen der Carbonsäure der Formel I mit einer äquimolaren Menge der gewünschten Base, zweckmässig in einem Lösungsmittel, wie Wasser, oder in einem organischen Lösungsmittel wie Aethanol, Methanol, Aceton und dergleichen. Entsprechend wird Salzbildung durch Addition einer organischen oder anorganischen Säure herbeigeführt. Die Temperatur der Salzbildung ist nicht kritisch, sie liegt im allgemeinen bei Raumtemperatur, kann aber auch leicht darüber oder darunter, etwa im Bereiche von 0 °C bis +50 °C, liegen.

Die folgenden Reaktionsschemas I und II veranschaulichen das Verfahren zur Herstellung der erfindungsgemässen Produkte bzw. der in der Synthese anfallenden Zwischenprodukte.

Reaktionsschema I

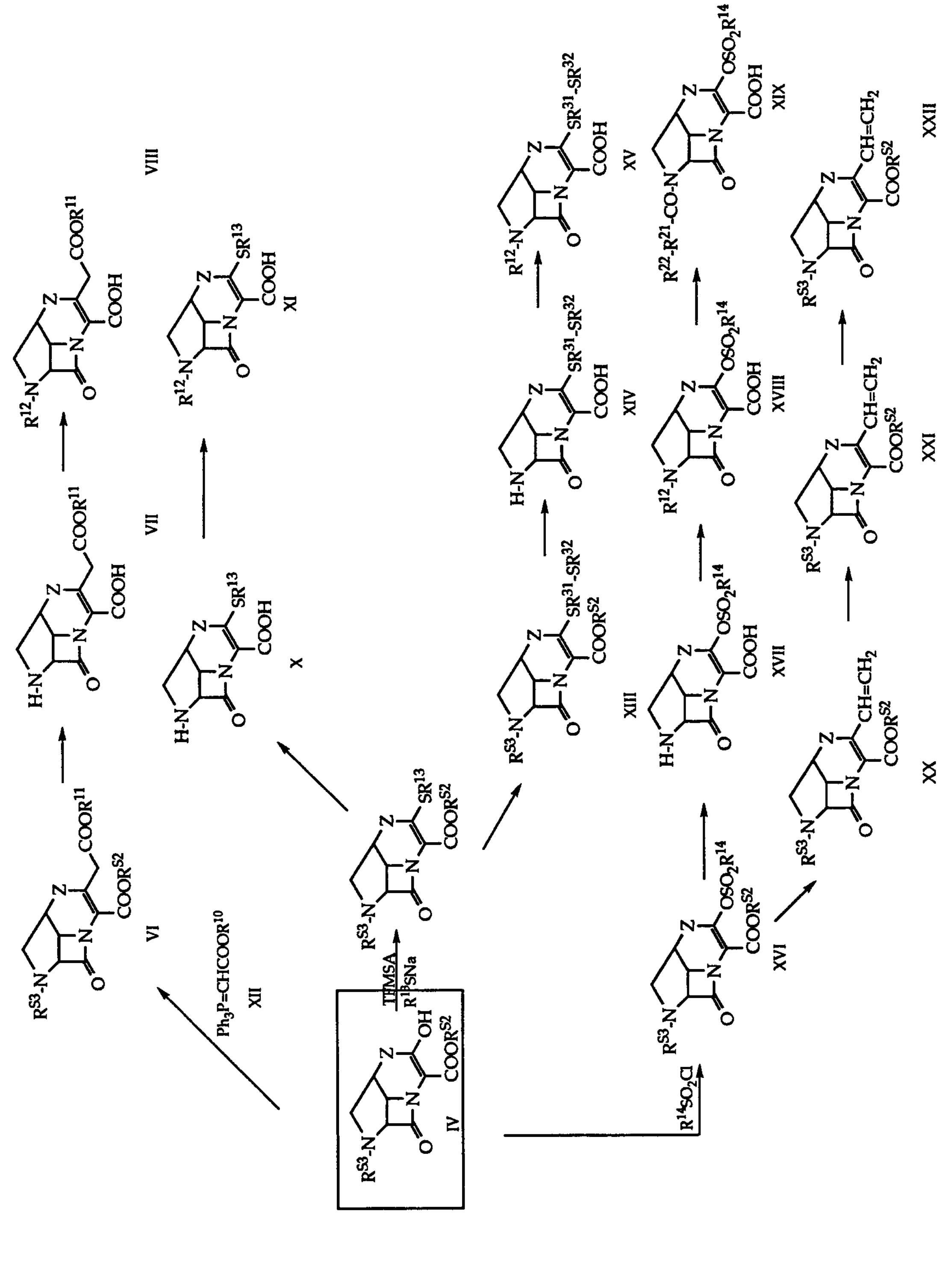


IV, VI, VII, VIII, X, XI, XIII, XIV, XV, XVI, XVII, XVIII, XIX, XX, XXI, XXII

Reaktionsschema II

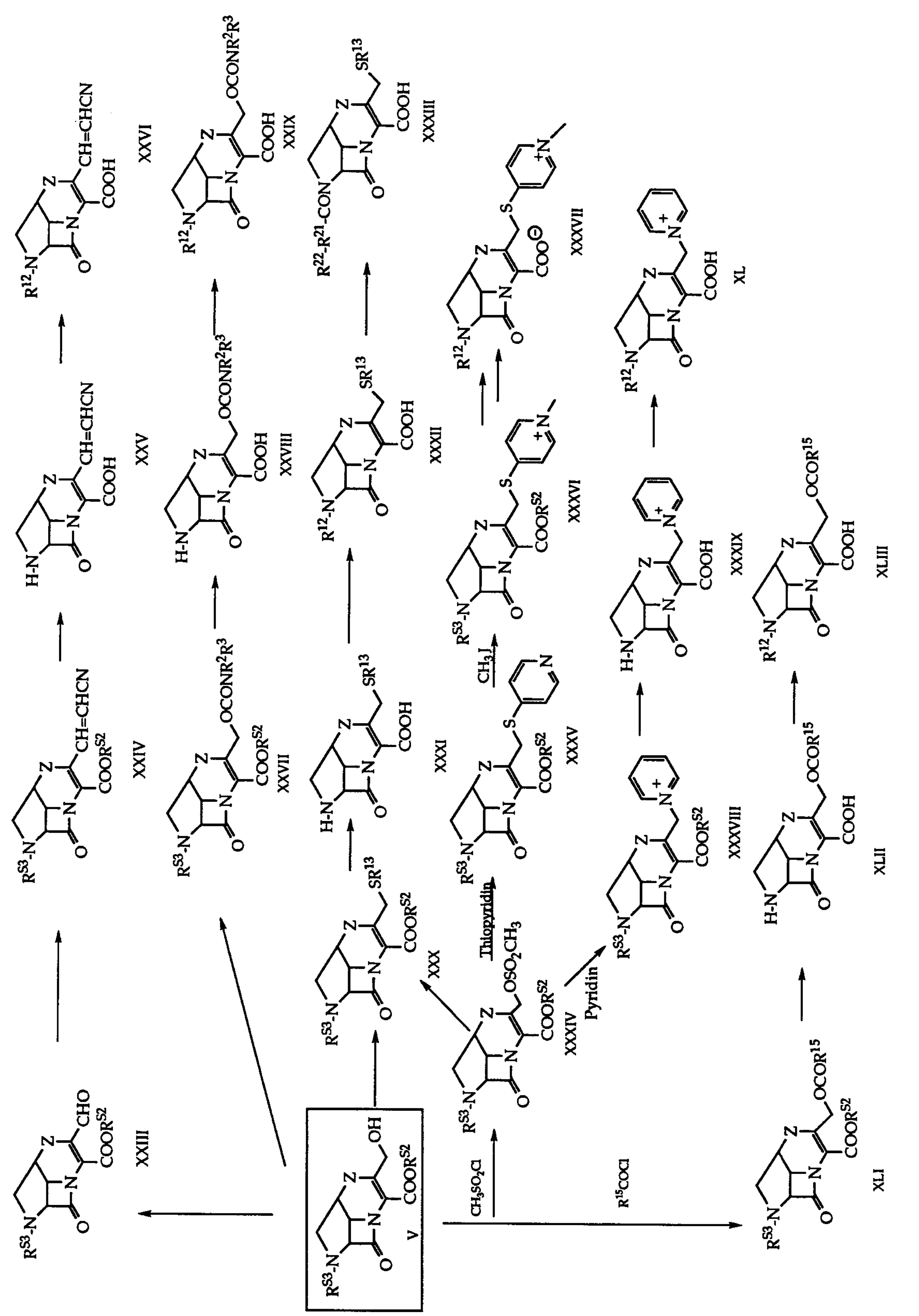


In den Reaktionsschemas I und II bedeuten

| | |
|---|---|
| Z = | Methylen, Sauerstoff oder Schwefel |
| $R^{S2}$ = | eine Carboxyschutzgruppe, vorzugsweise t-Butyl (für Z = Methylen) oder Allyl (für Z = Sauerstoff oder Schwefel) |
| $R^{S3}$ = | Eine Aminoschutzgruppe, vorzugsweise t-Butoxycarbonyl (für Z = Methylen) oder |

| | |
|---|---|
| | Allyloxycarbonyl (für Z = Sauerstoff oder Schwefel) |
| TFMSA = | Trifluormethansulfonsäureanhydrid |
| $R^{10}$ = | Benzyl oder niederes Alkyl |
| $R^{11}$ = | Wasserstoff oder niederes Alkyl |
| $R^{12}$ = | die gleiche Bedeutung wie R, ausgenommen Wasserstoff |
| $R^{22}$-$R^{21}$-CO = | eine mit einem nachträglich eingeführten Ring versehene Gruppe (a) |
| $R^{13}$ = | "Het" mit der obigen Bedeutung |
| $R^{31}$-$R^{32}$ = | eine mit einem nachträglich eingeführten Substituenten versehene Gruppe "Het" |
| $R^{14}$ = | niederes (Cyclo)alkyl, Phenyl oder niederes (Cyclo)alkylphenyl |
| $R^{15}$ = | niederes Alkyl |

Wird in Reaktionsschema II bei der Umsetzung XXXIV → XL anstelle von Pyridin eine andere N-tertiäre, N-heterocyclische Verbindung (z.B. 1-Methyl-1-Pyrrolidin) eingesetzt, erhält man entsprechend substituierte Reaktionsprodukte mit dem entsprechenden quaternären Stickstoffring. Setzt man anstelle von Pyridin eine N-gesättigte Ringverbindung ein (z.B. 1H-Tetrazol, 5-Methylsulfanyl-1H-tetrazol) erhält man entsprechende Verbindungen mit tertiärem Stickstoffring. Setzt man anstelle von Pyridin Ammoniak oder ein niederes Alkylamin bzw. di-niederes Alkyl-amin ein, erhält man entsprechende Verbindungen der Formel I, worin A Aminomethyl, niederes Alkylaminomethyl oder di-niederes Alkyl-aminomethyl darstellt. Azidomethyl- bzw. Aminomethylgruppen A können eingeführt werden, indem man die Verbindung XXXIV mit Natriumazid umsetzt und die erhaltene Azidomethylverbindung gewünschtenfalls hydriert, z.B. mit Palladiumkohle.

Die Synthese kann, wie erwähnt, ebenfalls über Zwischenprodukte, die in 2- und 6-Stellung durch andere Schutzgruppen als t-Butoxycarbonyl bzw. t-Butyl substituiert sind (z.B. Benzyloxycarbonyl oder Chloracetyl in Stellung 2; p-Nitrobenzyl, Benzyl oder Benzylhydryl in Stellung 6) laufen. Diese Zwischenprodukte sind in Analogie zu den beschriebenen Verfahren erhältlich.

Die in den Reaktionsschemas I und II aufgeführten "Bausteine" der Formeln IV und V können in der in den Beispielen 1, 14, 28 und 40 beschriebenen Weise, oder in analoger Weise, hergestellt werden. Es versteht sich, dass in Verbindungen IV und V als Aminoschutzgruppen $R^{S3}$ auch unter der Definition von R fallende Gruppen, die sich in der Synthese inert verhalten, gezählt werden können. Solche Gruppen sind z.B. niedere Alkanoylgruppen, z.B. Acetyl, die im Endprodukt aufscheinen sollen und demgemäss nicht abzuspalten sind. Die Herstellung eines solchen "Bausteins" der Formel V (mit $R^{S3}$ = Acetyl) ist in Beispiel 35 beschrieben worden.

Wie bereits eingangs erwähnt, zeigen die erfindungsgemässen Verbindungen der allgemeinen Formel I und pharmazeutisch verträgliche Salze davon mit Basen ausgeprägte β-Lactamase hemmende Aktivitäten gegen β-Lactamasen aus verschiedenen Bakterienstämmen. Diese therapeutisch wertvollen Eigenschaften können, wie nachstehend erläutert, an isolierten β-Lactamasen in vitro bestimmt werden:

A. Isolierung der β-Lactamasen

Verschiedene β-Lactamasen können aus penicillin- bzw. cephalosporin-resistenten Bakterienstämmen, wie Klebsiella pneumoniae NCTC 418, Proteus vulgaris 1028, Bacillus licheniformis 749/C, Escherichia coli SN01, Pseudomonas aeruginosa 18SH und Citrobacter freundii 1203 isoliert werden. Hierzu werden die entsprechenden Stämme in Tryptic Soy Broth (Difco) kultiviert und durch Zentrifugation in der späten logarithmischen Wachstumsphase geerntet (wenn nötig gibt man dem Medium gegen Ende der log-Phase 50-100 mg/l Ampicillin hinzu, um die β-Lactamase zu induzieren). Die so erhaltene Bakterienmasse wird mit 20 mM Tris-HCl-Puffer (pH 7,0) versetzt; unter Kühlung werden die Zellen mit French Press aufgebrochen. Man zentrifugiert (20'000 U/min.) während 20-30 Minuten und erhält einen klaren Rohextrakt. Die Reinigung der Proteine erfolgt nach der Methode von Cartwright, S.J. & Waley, S.G. [Biochem. J. 221, 505-512 (1980)] und, für B. licheniformis, Ellerby, L.M. et al. [Biochemistry 29, 5797-5806 (1990)].

B. Bestimmung der β-Lactamase-Aktivität

Die Bestimmung der Aktivität der isolierten β-Lactamasen kann nach der Methode von O'Callaghan, C.H. et al. [Antimicr. Ag. Chemother. 1, 283-288 (1972)] mit dem chromogenen Cephalosporin Nitrocefin (87/312 von Glaxo) durchgeführt werden. Der benötigte Versuchsansatz enthält pro ml Wasser: 50 mM Phosphatpuffer (pH 7,0), 0,1 mM Nitrocefin und genügend Enzym (β-Lactamase) um eine ΔA/min. von ca. 0,1 zu erreichen. Die Spaltung des Substrates, die mit einer Farbänderung verbunden ist, erfolgt bei 37 °C und wird bei 482 nm mit einem Spektralphotometer quantitativ verfolgt.

C. Bestimmung der β-Lactamase hemmenden Wirkung der Verbindungen der allgemeinen Formel I

Die oben beschriebene Spaltung des chromogenen Substrates durch β-Lactamasen (Versuch B.) kann durch Zugabe von Verbindungen der allgemeinen Formel I (Inhibitoren) gehemmt werden. Da es sich zeigte, dass die Inhibitoren die β-Lactamase in einer zeitabhängigen Reaktion irreversibel inaktivieren, wird die Reaktion (Spaltung des Substrates), jeweils nach einer Vorinkubationszeit von β-Lactamase mit Inhibitor von 15 Minuten, durch Zugabe des Substrates gestartet. Als Mass für die Affinität des jeweils getesteten Inhibitors zur β-Lactamase, die ein Mass für die Stärke des Inhibitors darstellt, dient diejenige Konzentration, welche die unter obigen Versuchsbedingungen (Versuch B.) in Abwesenheit eines Inhibitors erfolgende Spaltung des Substrates (Nitrocefin) zu 50% hemmt (IC 50 in µM). Zur Bestimmung der IC 50 wurden 4 bis 6 Versuche mit verschiedenen Konzentrationen an Inhibitor durchgeführt. Die Ermittlung der IC 50 erfolgte graphisch.

Die in obigem Versuch (Versuch C) erhaltenen Resultate sind in der nachfolgenden Tabelle 1 dargestellt.

## Tabelle 1

(Testorganismus: Citrobacter freundii 1982)

Der IC 50 Wert in µM wird für die Endprodukte der folgenden Ausführungsbeispiele angegeben. Dies ist ein Mass für die β-Lactamase-Hemmung. Ein IC 50 Wert von 1 µM (Micromolar) oder weniger wird als signifikant angesehen.

| Beispiel No. | IC 50 µM | Beispiel No. | IC 50 µM |
|---|---|---|---|
| 2(b) | 0,074 | 9(e) | 0,012 |
| 3(a) | 0,010 | 9(f) | 0,041 |
| 3(b) | 0,004 | 9(g) | 0,027 |
| 3(c) | 0,012 | 9(h) | 0,018 |
| 3(d) | 0,022 | 9(i) | 0,013 |
| 3(e) | 0,010 | 10(a) | 0,010 |
| 3(f) | 0,006 | 10(d) | 0,015 |
| 3(g) | 0,009 | 11(a) | 0,060 |
| 3(h) | 0,010 | 11(b) | 0,009 |
| 3(i) | 0,009 | 12(b) | 35 |
| 3(j) | 0,007 | 13(a) | 0,038 |
| 3(k) | 0,013 | 13(b) | 0,014 |
| 3(l) | 0,010 | 18(a) | 0,011 |
| 3(m) | 0,007 | 18(b) | 0,012 |
| 4 | 2,7 | 18(c) | 0,010 |
| 5 | 0,011 | 19(a) | 0,021 |
| 7 | 4,16 | 20 | 58 |
| 8 | 29,3 | 21 | 0,043 |
| 9(a) | 0,012 | 22 | 2,7 |
| 9(b) | 0,010 | 23(a) | 0,082 |
| 9(c) | 0,026 | 23(b) | 0,15 |
| 9(d) | 0,017 | 23(c) | 0,023 |
| | | 25 | 0,004 |

D. Bestimmung der $\beta$-Lactamase hemmender Wirkung durch Kombination einer Verbindung der allgemeinen Formel I mit Ceftriaxon

Die Mindesthemmkonzentration in vitro (MHK in $\mu$g/ml) einer 1:4 Kombination von Ceftriaxon mit einer Verbindung der Formel I gegen Citrobacter freundii 1982 wird gemessen und in der nachstehenden Tabelle 2 zusammengestellt.

Tabelle 2

| Beispiel No. | MHK C. freundii 1982 $\mu$g/ml | Beispiel No. | MHK C. freundii 1982 $\mu$g/ml |
|---|---|---|---|
| 2(b) | 1 | 9(f) | 0,12 |
| 3(a) | 0,25 | 9(g) | 0,25 |
| 3(b) | 0,25 | 9(h) | 0,25 |
| 3(c) | 0,25 | 9(i) | 2 |
| 3(d) | 1 | 10(a) | 1 |
| 3(e) | 2 | 10(d) | 8 |
| 3(f) | 0,06 | 11(b) | 0,5 |
| 3(g) | 0,5 | 12(b) | 4 |
| 3(h) | 2 | 13(a) | 1 |
| 3(i) | 0,06 | 13(b) | 0,25 |
| 3(k) | 0,5 | 18(a) | 0,25 |
| 3(l) | 0,5 | 18(b) | 0,25 |
| 3(m) | 0,25 | 18(c) | 0,25 |
| 5 | 2 | 20 | 1 |
| 7 | 16 | 21 | 0,5 |
| 8 | 1 | 22 | 4 |
| 9(a) | 0,12 | 23(a) | 1 |
| 9(b) | 0,25 | 23(b) | 1 |
| 9(c) | 1 | 23(c) | 0,25 |
| 9(d) | 2 | 25 | 0,25 |
| 9(e) | 0,25 | Ceftriaxon allein (Kontrolle) | 128 |

Die erfindungsgemässen Verbindungen zeigen ebenfalls eigene anti-bakterielle Wirkung, die anhand der nachstehenden Versuchsergebnisse veranschaulicht wird:

E. Antibakterielle Wirkung

Die antibakterielle Wirkung der Endprodukte an sich wird anhand der nachstehenden Tabelle 3 veranschaulicht, wobei die Mindesthemmkonzentration ($\mu$g/ml) in vitro gegen E. coli 1346 unter Verwendung der Reihenverdünnungsmethode in Flüssigmedium ermittelt wurde:

Tabelle 3

| Beispiel No. | MHK E. coli 1346 $\mu$g/ml | Beispiel No. | MHK E. coli 1346 $\mu$g/ml |
|---|---|---|---|
| 2(a) | 4 | 9(f) | 32 |
| 2(b) | 4 | 9(h) | 32 |
| 3(a) | 128 | 9(i) | 16 |
| 3(d) | 32 | 11(a) | 32 |
| 3(e) | 32 | 11(b) | 16 |
| 3(g) | 16 | 12(b) | 8 |
| 3(h) | 16 | 13(a) | 4 |
| 3(j) | 32 | 15 | 2 |
| 3(l) | 8 | 18(a) | 32 |
| 7 | 64 | 18(b) | 32 |
| 8 | 2 | 18(c) | 32 |
| 9(a) | 32 | 19(a) | 0,5 |
| 9(b) | 0,5 | 20 | ≦0,5 |
| 9(c) | 8 | 22 | 4 |
| 9(d) | 32 | | |

In der nachstehenden Tabelle 4 sind entsprechende Versuchsdaten für weitere Endverbindungen aus den nachstehend angegebenen Ausführungsbeispielen in bezug auf die obigen Versuche C, D und E (vgl. die Tabellen 1, 2 und 3) zusammengeführt:

<u>Tabelle 4</u>

| Beispiel No. | IC 50 µM (C) | MHK C. freundii 1982 µg/ml (D) | MHK E. coli 1346 µg/ml (E) |
|---|---|---|---|
| 2(d) | 0,34 | 64 | >32 |
| 2(f) | 13,2 | 2 | 8 |
| 3(n) | 0,008 | 1 | >32 |
| 3(o) | 0,016 | 1 | >32 |
| 3(p) | 0,014 | 1 | 32 |
| 3(q) | 0,242 | 2 | >32 |
| 3(aa) | 0,033 | 2 | 8 |
| 3(ab) | 0,027 | 16 | 32 |
| 3(ac) | 0,012 | 4 | 8 |
| 3(ad) | 0,039 | 8 | 16 |
| 3(ae) | 0,030 | 4 | 4 |
| 3(ag) | 0,028 | 2 | 8 |
| 3(ah) | 0,012 | 2 | 8 |
| 3(ai) | 0,026 | 32 | >32 |
| 3(ak) | 0,461 | 4 | 16 |
| 3(al) | - | 2 | 8 |
| 3(an) | 0,071 | 32 | 32 |
| 3(ao) | 1,020 | 8 | >32 |
| 18(f) | 0,013 | 1 | 4 |
| 18(g) | 0,011 | 0,5 | - |
| 18(h) | 0,004 | 0,5 | 16 |
| 18(i) | 0,002 | 4 | 32 |
| 18(j) | 0,006 | 0,5 | >32 |
| 19(c) | 0,005 | 0,5 | 2 |
| 19(d) | 0,009 | 0,25 | 2 |
| 19(f) | 0,034 | 4 | - |
| 19(h) | 0,018 | 0,5 | 2 |
| 19(i) | 0,031 | 1 | 0,5 |
| 19(j) | 0,034 | 2 | 0,5 |
| 19(k) | 0,038 | 0,25 | 0,5 |
| 23(f) | 0,320 | 4 | >32 |

**Tabelle 4** (Forts.)

| Beispiel No. | IC 50 µM (C) | MHK C. freundii 1982 µg/ml (D) | MHK E. coli 1346 µg/ml (E) |
|---|---|---|---|
| 25(b) | 0,018 | 0,25 | 4 |
| 25(c) | 0,033 | 1 | 4 |
| 25(d) | 0,019 | 0,5 | 4 |
| 27(c) | 0,008 | 0,25 | >32 |
| 27(d) | 0,011 | 0,25 | 32 |
| 27(e) | 0,006 | 0,5 | 32 |
| 27(f) | 0,008 | 0,25 | ≤0,25 |
| 27(g) | 0,005 | 0,5 | >32 |
| 27(h) | 0,003 | 0,25 | >32 |
| 29(a) | 0,790 | 1 | 1 |
| 29(b) | 0,275 | 2 | >16 |
| 29(c) | 0,380 | 0,5 | 2 |
| 29(d) | 0,430 | 8 | 4 |
| 29(e) | 0,036 | 0,5 | 1 |
| 29(e) Nebenprodukt | 0,003 | 1 | 4 |
| 29(g) | 0,056 | 8 | 8 |
| 29(h) | 0,218 | 32 | >32 |
| 29(i) | 0,823 | 8 | 8 |
| 29(j) | 0,045 | 4 | 8 |
| 29(k) | 0,034 | 8 | 32 |
| 29(l) | 0,056 | 32 | >32 |
| 30 | 0,019 | 1 | 8 |
| 31(a) | 0,010 | 0,5 | 8 |
| 31(b) | 0,011 | 0,2 | 8 |
| 31(c) | 0,016 | - | - |
| 31(d) | 0,005 | 1 | >32 |
| 31(e) | 0,011 | 0,5 | >32 |
| 31(g) | 51 | 16 | 0,5 |
| 31(h) | 0,006 | 0,5 | 8 |
| 31(i) | 0,011 | 0,5 | 8 |

Tabelle 4 (Forts.)

| Beispiel No. | IC 50 µM (C) | MHK C. freundii 1982 µg/ml (D) | MHK E. coli 1346 µg/ml (E) |
|---|---|---|---|
| 32(a) | 0,516 | 16 | >32 |
| 32(c) | 0,030 | 32 | 4 |
| 32(d) | 0,017 | 16 | 16 |
| 32(e) | 0,019 | 2 | 4 |
| 32(f) | - | 8 | 4 |
| 32(h) | 0,022 | 64 | >32 |
| 33 | 0,037 | 2 | 2 |
| 34 | 0,025 | 1 | 8 |
| 37 | 0,171 | 32 | 32 |
| 38(b) | 0,222 | 4 | 8 |
| 38(d) | 0,326 | 128 | >32 |
| 39 | 0,010 | 2 | 8 |
| 40(a) | 0,011 | 0,5 | 16 |
| 40(c) | 0,053 | 0,5 | >32 |
| 40(d) | 0,218 | 2 | 8 |
| 40(e) | 0,078 | 1 | 32 |
| 40(f) | 0,029 | 2 | 32 |
| 40(g) | 0,317 | 4 | >32 |
| 40(h) | 0,069 | 0,5 | 8 |
| 41(a) | 0,001 | 2 | >32 |
| 41(b) | 0,019 | 4 | >32 |
| 41(c) | 0,010 | 1 | >32 |
| 41(d) | 0,010 | 0,5 | >32 |
| 41(d) Nebenprodukt | 0,090 | 1 | >32 |
| 42(a) | 0,020 | 1 | 2 |
| 42(b) | 0,050 | 2 | 0,5 |
| 42(c) | 0,011 | 0,2 | 16 |
| 43 | 12,6 | 4 | >32 |
| 44 | 13,1 | 4 | 32 |
| 46(a) | 0,004 | 1 | 64 |
| 46(b) | 0,004 | 0,5 | >32 |

**Tabelle 4** (Forts.)

| Beispiel No. | IC 50 µM (C) | MHK C. freundii 1982 µg/ml (D) | MHK E. coli 1346 µg/ml (E) |
|---|---|---|---|
| 47 | 0,025 | 4 | 8 |
| 48(a) | 3,48 | 4 | 16 |
| 48(b) | 0,53 | 2 | 4 |
| 49(a) | 0,010 | 2 | >32 |
| 49(b) | 0,010 | 1 | 4 |
| 50 | 3 | 2 | 4 |
| 51 | 0,198 | 1 | >32 |
| 52(a) | 0,298 | 4 | 8 |
| 52(b) | 0,024 | 1 | 2 |
| 52(c) | 0,033 | - | - |
| 52(d) | 0,022 | 1 | 2 |
| 53 | 0,338 | 4 | 2 |
| 54 | 0,007 | 0,25 | 32 |
| 57(a) | 0,007 | - | - |
| 57(b) | 0,041 | 0,25 | >32 |
| 58(a) | 0,011 | 2 | >32 |
| 58(b) | 0,011 | 0,5 | 16 |
| 58(c) | 0,020 | 0,5 | 0,5 |
| 58(e) | 0,009 | 0,25 | 8 |
| 58(f) | 0,013 | 0,12 | 8 |
| 58(g) | 0,013 | 0,25 | 8 |
| 58(h) | 0,009 | 1 | 32 |
| 59(a) | 0,019 | 1 | 2 |
| 59(b) | 0,006 | 0,5 | 2 |
| 59(c) | 0,163 | 2 | 8 |
| 59(d) | 0,030 | 1 | 8 |
| 60 | 0,149 | 2 | >32 |
| 61(a) | 0,004 | 0,25 | - |
| 61(b) | 0,004 | 2 | 16 |
| 62 | 0,008 | 0,5 | 16 |
| 63 | 0,032 | 2 | 8 |
| 64 | 0,010 | 0,5 | >32 |

<u>Tabelle 4</u> (Forts.)

| Beispiel No. | IC 50 µM (C) | MHK C. freundii 1982 µg/ml (D) | MHK E. coli 1346 µg/ml (E) |
|---|---|---|---|
| 65(a) | 0,032 | 1 | 0,5 |
| 65(b) | 0,010 | 1 | 2 |
| 65(c) | 0,003 | 1 | 2 |
| 65(d) | 0,124 | 2 | 16 |
| 65(e) | 0,045 | 0,5 | 1 |
| 65(f) | 0,008 | 0,5 | 2 |
| 65(g) | ≤0,001 | 0,5 | 8 |
| 66 | 0,053 | 8 | 4 |
| 67(a) | 0,013 | 8 | 0,5 |
| 67(b) | 0,030 | 8 | 4 |
| 69 | 0,209 | 0,5 | 2 |
| 70(b) | 0,012 | 0,5 | 1 |
| 71 | 0,119 | 2 | 8 |
| 72(a) | 0,041 | 4 | 2 |
| 72(b) | 0,011 | 2 | 4 |
| 73 | 0,159 | 1 | 1 |
| 74(a) | 0,006 | 1 | 2 |
| 74(b) | 0,005 | 1 | 1 |
| 75 | 0,127 | 1 | 4 |
| 76(a) | 0,037 | 1 | 2 |
| 76(b) | 0,072 | 0,5 | ≤0,25 |
| 77 | 0,322 | 2 | 2 |
| 78(a) | 0,014 | 0,5 | 1 |
| 78(b) | 0,013 | 1 | 1 |
| 79 | 0,134 | 4 | 2 |
| 80(a) | 0,008 | 4 | 0,5 |
| 80(b) | 0,004 | - | - |
| 81 | 0,127 | 1 | 1 |
| 82 | 0,013 | 0,25 | 16 |
| 83(a) | 0,016 | 0,5 | 2 |
| 83(b) | 0,010 | 0,5 | 0,5 |
| 84 | 50 | 4 | 2 |

Tabelle 4 (Forts.)

| Beispiel No. | IC 50 µM (C) | MHK C. freundii 1982 µg/ml (D) | MHK E. coli 1346 µg/ml (E) |
|---|---|---|---|
| 85 | 0,019 | 0,5 | >32 |
| 86 | 0,060 | 2 | 16 |
| 87 | 8,7 | 4 | 16 |
| 88(a) | 0,436 | 4 | 16 |
| 88(b) | 0,092 | 2 | 4 |
| 89 | 0,040 | 2 | 4 |
| 90(a) | 0,015 | 1 | 0,5 |
| 90(b) | 0,015 | 1 | 0,5 |
| 91 | 0,080 | 2 | 4 |
| 92(a) | 0,020 | 2 | 4 |
| 92(b) | 0,014 | 2 | 4 |
| 93 | 0,068 | 2 | 8 |
| 94(a) | 0,021 | 2 | 2 |
| 94(b) | 0,012 | 1 | 2 |
| 95 | 0,150 | 0,5 | 4 |
| 96(a) | 0,042 | 2 | 8 |
| 96(b) | 0,005 | 0,5 | 2 |
| 97 | 0,124 | 1 | 4 |
| 98(a) | 0,033 | 1 | 2 |
| 98(b) | 0,010 | 1 | 2 |
| 99 | 0,059 | 2 | 4 |
| 100(a) | 0,018 | 1 | 1 |
| 100(b) | 0,004 | 1 | 2 |
| 100(d) | 0,019 | 2 | 0,5 |
| 101 | 0,038 | 1 | 2 |
| 102(a) | 0,016 | 2 | 0,5 |
| 102(b) | 0,013 | 2 | 1 |
| 103 | 0,047 | 2 | 1 |
| 108 | 0,107 | 1 | 32 |

Versuchsdaten für besonders bevorzugte Endverbindungen sind in der nachstehenden Tabelle 5 zusammengestellt:

Tabelle 5

| Beispiel No. | IC 50 µM (C) | MHK C. freundii 1982 µg/ml (D) | MHK E. coli 1346 µg/ml (E) |
|---|---|---|---|
| 3(l) | 0,010 | 0,5 | 8 |
| 18(d) | 0,009 | ≦0,06 | 32 |
| 18(e) | 0,007 | 0,12 | >32 |
| 19(e) | 0,015 | 0,25 | 1 |
| 19(g) | 0,010 | 1 | 1 |
| 27(a) | 0,031 | ≦0,06 | >32 |
| 27(b) | 0,006 | ≦0,06 | >32 |
| 35 | 0,015 | 1 | 0,5 |
| 58(d) | 0,005 | 0,5 | 1 |
| 70(a) | 0,024 | 0,5 | 1 |
| 70(c) | 0,018 | 0,25 | 1 |

Die erfindungsgemässen Produkte können als Heilmittel, z.B. in Form pharmazeutischer Präparate Verwendung finden, welche sie oder ihre Salze in Mischung mit einem für die parenterale oder enterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaestetica oder Puffer. Sie kommen für die parenterale Applikation sowie auch für die enterale Verabreichung in Betracht.

Wie eingangs erwähnt, kann man die erfindungsgemässen Verbindungen bei der Bekämpfung bzw. Verhütung von Krankheiten verwenden, insbesondere bei der Bekämpfung von β-Lactamase bildenden Krankheitserregern, allein oder, insbesondere, in Kombination mit β-Lactam-Antibiotika, d.h. Antibiotika welche einen β-Lactamring enthalten, beispielsweise Penicilline, wie Benzylpenicillin, Piperacillin, Phenoxymethylpenicillin, Carbenicillin, Apalcillin, Methicillin, Propicillin, Tricarcillin, Ampicillin, Anioxicillin oder Mecillinam, oder Cephalosporine, wie Ceftriaxon, Ceftazidim, Cefetamet, Cefatamet Pivoxil, Cefotaxim, Cefmenoxim, Cetfizoxim, Cefuroxim, Cephaloridin, Cephalotin, Cefazolin, Cephalexin, Cefoxitin, Cephacetril, Cefamandol, Cephapirin, Cephradin, Cephaloglycin, (6R,7R)-7-[(Z)-2-(2-Amino-4-thiazolyl)-2-(methoxyimino)-acetamido]-3-(azidomethyl)-8-oxo-5-thia-1-azabicyclo[4.2.0]oct-2-en-2-carbonsäure oder (E)-2-(Isobutoxycarbonyl)-2-pentenyl (6R,7R)-7-[(Z)-2-(2-Amino-4-thiazolyl)-2-(methoxyimino)acetamido]-3-(azidomethyl)-8-oxo-5-thia-1-azabicyclo[4.2.0]oct-2-en-2-carboxylat oder auch Peneme oder Carbapeneme, wie Imipenem und Meropenem. Dabei können die Verbindungen der allgemeinen Formel I oder pharmazeutisch verträgliche Salze davon mit Basen vor, gleichzeitig mit oder nach der Verabreichung bzw. Einnahme von β-Lactam-Antibiotika verabreicht werden. Werden die erfindungsgemässen Produkte gleichzeitig mit einem β-Lactam-Antibiotikum verabreicht, so kann dies durch Verabreichung als ad-hoc-Kombination oder in Form einer pharmazeutischen Kombination erfolgen, welche eine Verbindung der allgemeinen Formel I oder ein pharmazeutisch verträgliches Salz davon mit Base und ein β-Lactam-Antibiotikum enthält; derartige pharmazeutische Kombinationen sind ebenfalls Gegenstand der vorliegenden Erfindung.

Die Dosierung der Verbindungen der allgemeinen Formel I und der pharmazeutisch verträglichen Salze davon mit Basen kann innerhalb weiter Grenzen variieren und ist natürlich in jedem einzelnen Fall den individuellen Gegebenheiten und dem zu bekämpfenden β-Lactamase produzierenden Krankheitserreger anzupassen. Im allgemeinen dürfte eine Tagesdosis von etwa 0,1 bis etwa 2,0 g angemessen sein. Das Verhältnis von β-Lactamase-Inhibitor (Verbindung der Formel I oder pharmazeutisch veträgliches Salz davon mit Base) zu β-Lactam-Antibiotikum kann ebenfalls innerhalb weiter Grenzen variieren und ist in jedem einzelnen Fall den individuellen Gegebenheiten anzupassen. Im allgemeinen dürfte ein Verhältnis von etwa 1:20 bis etwa 1:1 angemessen sein.

Wie eingangs erwähnt, sind Arzneimittel enthaltend eine Verbindung der allgemeinen Formel I oder einen pharmazeutisch verträglichen, leicht hydrolysierbaren Ester oder entsprechendes Salz davon ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der allgemeinen Formel I oder pharmazeutisch verträgliche Ester bzw. Salze davon und gegebenenfalls einen oder mehrere therapeutisch wertvolle Stoffe in eine galenische Darreichungsform bringt; in diesem Zusammenhang sei

nochmals auf die weiter oben erwähnten pharmazeutischen Kombinationen hingewiesen, welche ebenfalls Gegenstand der vorliegenden Erfindung sind. Insbesondere sind pharmazeutische Kombinationen enthaltend eine Verbindung der allgemeinen Formel I oder einen pharmazeutisch verträglichen, leicht hydrolysierbaren Ester oder entsprechendes Salz davon und ein β-Lactam-Antibiotikum, z.B. ein Penicillin, wie Benzylpenicillin, Piperacillin, Phenoxymethylpenicillin, Carbenicillin, Apalcillin, Methicillin, Propicillin, Tricarcillin, Ampicillin, Amoxicillin oder Mecillinam, oder ein Cephalosporin, wie Ceftriaxon, Ceftazidim, Cefetamet, Cefatamet Pivoxil, Cefotaxim, Cefmenoxim, Ceftizoxim, Cefuroxim, Cephaloridin, Cephalotin, Cefazolin, Cephalexin, Cefoxitin, Cephacetril, Cefamandol, Cephapirin, Cephradin, Cephaloglycin, (6R,7R)-7-[(Z)-2-(2-Amino-4-thiazolyl)-2-(methoxyimino)acetamido]-3-(azidomethyl)-8-oxo-5-thia-1-azabicyclo[4.2.0]oct-2-en-2-carbonsäure oder (E)-2-(Isobutoxycarbonyl)-2-pentenyl (6R,7R)-7-[(Z)-2-(2-Amino-4-thiazolyl)-2-(methoxyimino)acetamido]-3-(azidomethyl)-8-oxo-5-thia-1-azabicyclo[4.2.0]oct-2-en-2-carboxylat, oder auch ein Penem oder Carbapenem, wie Imipenem oder Meropenem, Gegenstand der vorliegenden Erfindung. Derartige Kombinationen eignen sich zur Bekämpfung von β-Lactamase bildenden Krankheitserregern.

In den nachfolgenden Beispielen bedeutet DMF Dimethylformamid und THF Tetrahydrofuran.

Beispiel 1

(1a S,3aR,6R)-5-Hydroxy-1-oxo-1a,2,3,3a,4,6b-hexahydro-2,6a-diazacyclobut[cd]inden-2,6-dicarbonsäure-di-t-butylester (5-Hydroxy "Baustein")

Diese Verbindung kann durch folgende Reaktionssequenz a)-j) erhalten werden:

a) Benzyl (2S,3S)-1-(3,4-dimethoxybenzyl)-2-[(R)-2-oxo-1,3-dioxolan-4-yl]-4-oxo-3-azetidincarbamat

In 3 l THF werden bei Siedetemperatur 215,23 g (0,5 Mol) Benzyl (2S,3S)-1-(3,4-dimethoxybenzyl)-2-[1-(R),2-dihydroxyäthyl]-4-oxo-3-azetidincarbamat gelöst. Hierzu gibt man 121,61 g (0,75 Mol) 1,1'-Carbonyldiimidazol. Man lässt 4 Stunden unter Rückfluss kochen. Das THF wird anschliessend durch Einengen entfernt, der ölige Pest in 1,5 l Dichlormethan aufgenommen, einmal mit 500 ml 1N wässriger Salzsäure, zweimal mit je 1 l Wasser und einmal mit 500 ml gesättigter, wässriger Kochsalzlösung gewaschen und über Magnesiumsulfat unter Zusatz von ca. 7 g Bleicherde getrocknet. Das Lösungsmittel wird durch Einengen entfernt. Man erhält nach dem Trocknen ohne weitere Reinigung 220,3 g (Ausbeute: 96%) des reinen Produkts. Smp: 135-136 °C.
MS (EI): 456 ($M^+$)

| Mikroanalyse: $C_{23}H_{24}N_2O_8$ | | | |
|---|---|---|---|
| Ber. | C 60,52 | H 5,30 | N 6,14 |
| Gef. | C 60,48 | H 5,39 | N 6,28 |

b) (1S,4S,5S)-6-(3,4-Dimethoxybenzyl)-4-hydroxy-7-oxo-2,6-diazabicyclo[3.2.0]heptan-2-carbonsäure-benzylester

Benzyl (2S,3S)-1-(3,4-dimethoxybenzyl)-2-[(R)-2-oxo-1,3-dioxolan-4-yl]-4-oxo-3-azetidincarbamat (220,3 g, 0,483 Mol) werden in 3 l DMF gelöst, mit 25,4 g (0,121 Mol) Tetraäthylammoniumbromid versetzt und bei 140 °C Innentemperatur unter Argon 5 Stunden lang heftig gerührt. Das DMF wird bei 60 °C durch Einengen unter stark vermindertem Druck entfernt. Den gelben öligen Pest nimmt man in 1 l Aethylacetat auf und extrahiert zweimal mit 1 l Wasser und einmal mit 500 ml gesättigter wässriger Kochsalzlösung. Unter Zusatz von 7 g Bleicherde wird über Magnesiumsulfat getrocknet. Nach dem Einengen erhält man ein gelbes Oel, das durch Chromatographie mit 1) Aethylacetat/n-Hexan (3:1) und 2) Aethylacetat über 2 kg Kieselgel gereinigt wird. Ausbeute: 172 g (86%) als leicht gelbes Oel, das aus Aethylacetat oder Isopropanol kristallisiert. Smp: 100-102 °C (Aethylacetat).
IR (KBr): 1731, 1707 $cm^{-1}$

| Mikroanalyse: $C_{22}H_{24}N_2O_6$ | | | |
|---|---|---|---|
| Ber.<br>Gef. | C 64,07<br>C 64,00 | H 5,87<br>H 5,80 | N 6,79<br>N 6,80 |

c) (1S,5S)-6-(3,4-Dimethoxybenzyl)-4,7-dioxo-2,6-diazabicyclo[3.2.0]heptan-2-carbonsäure-benzylester

Oxalylchlorid (25,0 ml, 290,7 mmol) wird in abs. Methylenchlorid gelöst und auf -78°C gekühlt. Innerhalb einer Stunde wird abs. Dimethylsulfoxid (40,8 ml, 572 mmol) zwischen -70 und -76°C zugetropft. Nach 30 Minuten bei dieser Temperatur wird (1S,4S,5S)-6-(3,4-Dmiethoxybenzyl)-4-hydroxy-7-oxo-2,6-diazabicyclo[3.2.0]heptan-2-carbonsäure-benzylester (100 g, 242,4 mmol) in Methylenchlorid (300 ml) während 2 Stunden zwischen -74 und -76°C zugetropft. Nach 1 Stunde bei dieser Temperatur wird mit Methylenchlorid (640 ml) unterhalb von -70°C verdünnt und mit Aethyldiisopropylamin (120 ml, 701 mmol) zwischen -74°C und -78°C innerhalb 1 Stunde versetzt. Nach 30 Minuten bei dieser Temperatur lässt man die Temperatur auf -40°C steigen. Das Reaktionsgemisch wird anschliessend unter Rühren auf 1N wässrige Salzsäure gegossen. Die organische Phase wird abgetrennt und nacheinander mit einer 1N wässrigen Salzsäure (600 ml), einer gesättigten wässrigen Kochsalzlösung (600 ml), einer gesättigtern wässrigen Natriumbicarbonatlösung (1200 ml), erneut mit einer gesättigten wässrigen Kochsalzlösung (1200 ml) gewaschen, mit Magnesiumsulfat getrocknet und eingeengt. Ausbeute: 98,4 g (99%) als farbloser fester Schaum.
IR (KBr): 1760, 1709 $cm^{-1}$
MS (EI): ($M^+$) 410

d) (Z)- und (E)-(1S,5R)-4-Benzyloxycarbonylmethylen-6-(3,4-dimethoxybenzyl)-7-oxo-2,6-diazabicyclo[3.2.0]-heptan-2-carbonsäure-benzylester

(1S,5S)-6-(3,4-Dimethoxybenzyl)-4,7-dioxo-2,6-diazabicyclo[3.2.0]heptan-2-carbonsäure-benzylester (70,1 g; 170,8 mmol) wird in abs. THF (570 ml) gelöst und auf -10°C gekühlt. Benzyloxycarbonylmethylentriphenylphosphoran (70,1 g; 170,8 mmol) wird portionsweise innerhalb von 15 Minuten zugegeben, ohne dass die Temperatur über 0°C steigt. Nach 3 Stunden bei -10°C wird die Suspension abgenutscht und die Mutterlauge eingeengt. Das erhaltene Oel wird in Methylenchlorid (20 ml) gelöst und über Kieselgel (600 g; 0,040-0,063 mm Korngrösse) mit Aethylacetat/n-Hexan 2:8 bis 1:1 chromatographiert. Die vereinten reinen Fraktionen werden dann bis auf ca. 200 ml konzentriert, das ausgefallene Triphenylphosphinoxid wird abgenutscht und die Mutterlauge eingeengt. Ausbeute: 78 g (84%) als farbloser Schaum.
IR (Film): 2835, 1762, 1710, 1590, 1516, 1237, 1132 $cm^{-1}$

| Mikroanalyse: $C_{30}H_{28}N_2O_7$ | | | |
|---|---|---|---|
| Ber.<br>Gef. | C 68,17<br>C 68,11 | H 5,34<br>H 5,54 | N 5,30<br>N 4,99 |

e) (1S,4R,5R)-2-t-Butoxycarbonyl-6-(3,4-dimethoxybenzyl)-7-oxo-2,6-diazabicyclo[3.2.0]hept-4-yl-essigsäure

(Z)- und (E)-(1S,5R)-4-Benzyloxycarbonylmethylen-6-(3,4-dimethoxybenzyl)-7-oxo-2,6-diazabicyclo-[3.2.0]heptan-2-carbonsäure-benzylester (78 g; 143,8 mmol) wird in DMF (400 ml) und Methanol (1400 ml) gelöst. Nach Zugabe von Di-t-butyl-dicarbonat (50 ml; 215,6 mmol) wird das Reaktionsgemisch über 10% Pd/C (26 g) über Nacht hydriert. Die erhaltene schwarze Suspension wird filtriert und eingeengt. Das erhaltene viskose Oel wird mit Wasser (850 ml) versetzt, mit gesättigter wässriger Natriumbicarbonatlösung (150 ml) verrührt und mit Aether (4x1000 ml) gewaschen. Nach Zugabe von 1N wässriger Salzsäure (100 ml; pH = 5) wird die milchartige Emulsion mit Aethylacetat (1000 ml) extrahiert. Das gleiche Verfahren wird wiederholt, und die vereinten organischen Phasen werden über Magnesiumsulfat getrocknet und eingeengt. Ausbeute: 42,2 g (71%) als gelblicher Schaum.
IR (Film): 2600, 1757, 1699, 1675, 1594, 1571 $cm^{-1}$
MS (EI): (M-tBuO) 347

f) (1S,4R,5R)-4-Benzyloxycarbonylmethyl-6-(3,4-dimethoxybenzyl)-7-oxo-2,6-diazabicyclo[3.2.0]heptan-2-carbonsäure-t-butylester

(1S,4R,5R)-2-t-Butoxycarbonyl-6-(3,4-dimethoxybenzyl)-7-oxo-2,6-diazabicyclo[3.2.0]hept-4-yl-essigsäure (41,2 g; 98,9 mmol) wird in Methylenchlorid (70 ml) gelöst. Benzylalkohol (12 ml; 118,7 mmol) und 4-Dimethylaminopyridin (1,3 g; 9,89 mmol) werden zugegeben. Anschliessend wird die Lösung auf -10 °C gekühlt, und Dicyclohexylcarbodiimid (24,7 g; 118,7 mmol) wird portionsweise so zugegeben, dass die Temperatur nicht über +10 °C steigt. Die erhaltene Suspension wird während 20 Stunden bei Raumtemperatur gerührt und anschliessend abgenutscht und eingeengt. Das erhaltene Oel wird über Kieselgel (800 g; 0,040-0,063 mm Korngrösse) mit Aethylacetat/n-Hexan 1:1 chromatographiert. Ausbeute: 37,8 g (75,5%) als farbloser Schaum.
IR (KBr): 1758, 1697, 1517, 1261, 1160, 1027 $cm^{-1}$
MS (ISP): $(M+H^+)$ 511,6

g) (1S,4R,5R)-4-Benzyloxycarbonylmethyl-7-oxo-2,6-diazabicyclo[3.2.0]heptan-2-carbonsäure-t-butylester

(1S,4R,5R)-4-Benzyloxycarbonylmethyl-6-(3,4-dimethoxybenzyl)-7-oxo-2,6-diazabicyclo[3.2.0]heptan-2-carbonsäure-t-butylester (37,8 g; 74,18 mmol) wird in Acetonitril (500 ml) und Wasser (240 ml) vorgelegt. Die Lösung wird auf 60 °C aufgewärmt und mit Kaliumpersulfat (84 g; 310 mmol) in 4 Portionen mit jeweils 1 Stunde Abstand versetzt. Gleichzeitig wird der pH-Wert mit einer 15%iger wässriger Natriumcarbonatlösung bei 5 gehalten. Nach 3 Stunden bei 60 °C wird die erhaltene Suspension abgekühlt, der pH auf 7 eingestellt, dann mit Wasser (200 ml) verdünnt und mit Aethylacetat (2x1000 ml) extrahiert. Die vereinigten organischen Phasen werden mit gesättigter wässriger Kochsalzlösung (500 ml) gewaschen, über Magnesiumsulfat getrocknet und eingeengt. Der Rückstand wird über Kieselgel (1000 g; 0,040-0,063 mm Korngrösse) mit Aethylacetat/n-Hexan 1:1 chromatographiert. Ausbeute: 18,5 g (69,1%) als farbloser Feststoff. Smp: 135 °C.
IR (KBr): 3196, 1750, 1733, 1705, 1160 $cm^{-1}$

| Mikroanalyse: $C_{19}H_{24}N_2O_5$ | | | |
|---|---|---|---|
| Ber. | C 63,32 | H 6,71 | N 7,77 |
| Gef. | C 63,22 | H 6,88 | N 7,58 |

h) (1S,4R,5R)-4-Benzyloxycarbonylmethyl-6-t-butoxycarbonylmethyl-7-oxo-2,6-diazabicyclo[3.2.0]heptan-2-carbonsäure-t-butylester

(1S,4R,5R)-4-Benzyloxycarbonylmethyl-7-oxo-2,6-diazabicyclo[3.2.0]heptan-2-carbonsäure-t-butylester (18,0 g; 49,94 mmol) wird in abs. THF (200 ml) bei -78 °C vorgelegt. Eine 1M Bistrimethylsilyllithiumamidlösung in THF (55,1 ml; 55,1 mmol) wird so zugetropft, dass die Temperatur nicht über -70 °C steigt (20 Minuten). Nach 10 Minuten bei dieser Temperatur wird Bromessigsäure-t-butylester (8,8 ml; 60,1 mmol) zugegeben, und das Reaktionsgemisch wird während 1 Stunde bei 0 °C weitergerührt. Die erhaltene orange Lösung wird auf 1N wässrige Salzsäure (250 ml) und Eis (150 g) gegossen und anschliessend mit Aethylacetat extrahiert (2x450 ml). Die vereinigten organischen Phasen werden mit gesättigter wässriger Kochsalzlösung (3x300 ml) gewaschen, über Magnesiumsulfat getrocknet und eingeengt. Das dickflüssige Oel wird anschliessend mit n-Hexan (250 ml) verrührt und die erhaltenen Kristalle abgenutscht. Ausbeute: 19,2 g (81,1%) als farblose Kristalle.
IR (KBr): 1771, 1737, 1700, 1157 $cm^{-1}$
MS (FAB): $(M+H^+)$ 475,4

i) (1S,4R,5R)-2-t-Butoxycarbonyl-6-t-butoxycarbonylmethyl-7-oxo-2,6-diazabicyclo[3.2.0]hept-4-yl-essigsäure

(1S,4R,5R)-4-Benzyloxycarbonylmethyl-6-t-butoxycarbonylmethyl-7-oxo-2,6-diazabicyclo[3.2.0]heptan-2-carbonsäure-t-butylester (19,0 g; 40,0 mmol) wird in Aethylacetat (500 ml) über 10% Pd/C (2 g) hydriert. Die erhaltene Suspension wird abgenutscht, eingeengt, dann mit Wasser (220 ml), gesättigter wässriger Natriumbicarbonatlösung (110 ml), und Aether (220 ml) verrührt. Die Wasserphase wird mit 1N wässriger Salzsäure (ca. 110 ml) versetzt, bis eine beständige, starke Trübung entsteht (pH = 5). Nach Extraktion mit Aethylacetat (300 ml) wird erneut 1N wässrige Salzsäure zugegeben (ca. 22 ml; pH = 1) und das Gemisch

mit Aethylacetat (300 ml) extrahiert. Die vereinigten organischen Phasen werden mit gesättigter wässriger Kochsalzlösung (270 ml) gewaschen und anschliessend über Magnesiumsulfat getrocknet und eingeengt. Ausbeute: 14,5 g (94,3%) als farbloser Feststoff. Smp: 46-48°C.
IR (KBr): 3247, 2626, 1772, 1738, 1704, 1158 $cm^{-1}$
MS (ISP): $(M+H^+)$ 385,3

j) (1aS,3aR,6R)-5-Hydroxy-1-oxo-1a,2,3,3a,4,6b-hexahydro-2,6a-diazacyclobut[cd]inden-2,6-dicarbonsäure-di-t-butylester

(1S,4R,5R)-2-t-Butoxycarbonyl-6-t-butoxycarbonylmethyl-7-oxo-2,6-diazabicyclo[3.2.0]hept-4-yl-essigsäure (13,13 g; 34,2 mmol) wird in abs. THF (200 ml) gelöst und mit 1,1-Carbonyldiimidazol (8,33 g; 51,33 mmol) versetzt. Die erhaltene Suspension wird 5 Stunden bei Raumtemperatur gerührt. Anschliessend wird das Reaktionsgemisch auf -78°C gekühlt, und eine 1M Bistrimethylsilyllithiumamidlösung in THF (75,3 ml; 75,33 mmol) wird während 2 Stunden zugetropft, ohne dass die Temperatur über -74°C steigt. Nach 7 Stunden wird das Reaktionsgemisch auf 1N wässrige Salzsäure (150 ml) und Eis (50 g) gegossen und mit Aethylacetat extrahiert. Die vereinigten organischen Phasen werden nacheinander mit gesättigter wässriger Natriumbicarbonatlösung (150 ml) und gesättigter wässriger Kochsalzlösung (2x150 ml) gewaschen und anschliessend über Magnesiumsulfat getrocknet und eingeengt. Der Rückstand wird in Aether (175 ml) suspendiert und nacheinander mit 1N wässriger Salzsäure (3x175 ml), gesättigter wässriger Natriumbicarbonatlösung (2x50 ml) und gesättigter wässriger Kochsalzlösung (2x100 ml) gründlich gewaschen. Die organische Phase wird über Magnesiumsulfat getrocknet und eingeengt. Anschliessend wird der erhaltene beige Feststoff mit n-Hexan (50 ml) während 2 Stunden verrührt und abgenutscht. Ausbeute: 6,2 g (49%) als farblose Kristalle. Smp: 127-129°C.
IR: 3440, 2979, 1772, 1703, 1657, 1619 $cm^{-1}$

| Mikroanalyse: $C_{18}H_{26}N_2O_6$ | | | |
|---|---|---|---|
| Ber. | C 59,00 | H 7,15 | N 7,65 |
| Gef. | C 59,18 | H 7,30 | N 7,35 |

Beispiel 2

(a) (1aS,3aR,6bR)-5-(1-Methyl-1H-tetrazol-5-ylsulfanyl)-1-oxo-1a,2,3,3a,4,6b-hexahydro-1H-2,6a-diazacyclobut[cd]inden-6-carbonsäure-trifluoracetat

Eine Lösung von (1aS,3aR,6bR)-5-(1-Methyl-1H-tetrazol-5-ylsulfanyl)-1-oxo-1a,2,3,3a,4,6b-hexahydro-1H-2,6a-diazacyclobut[cd]inden-2,6-dicarbonsäure-di-t-butylester (3,0 g; 6,46 mmol) in abs. Methylenchlorid (8 ml) wird zu abs. Trifluoressigsäure (32 ml), zwischen -20°C und -18°C, getropft. Nach 2 Stunden bei dieser Temperatur wird die Lösung mit abs. Methylenchlorid (64 ml) verdünnt und während 2 Stunden bei Raumtemperatur weitergerührt. Das Reaktionsgemisch wird eingeengt. Der Rückstand wird mit abs. Aether (300 ml) verrührt, abgenutscht und mit Aether (2x50 ml) gewaschen. Die Kristalle werden 10 Stunden am Hochvakuum getrocknet. Ausbeute: 2,9 g (98%) als beiger Feststoff.
IR (KBr): 2683, 1788, 1675 $cm^{-1}$
MS (ISN): $(M-H)^-$ 307,0

Der als Ausgangsprodukt eingesetzte (1aS,3aR,6bR)-5-(1-Methyl-1H-tetrazol-5-ylsulfanyl)-1-oxo-1a,2,3,3a,4,6b-hexahydro-1H-2,6a-diazacyclobut[cd]inden-2,6-dicarbonsäure-di-t-butylester kann wie folgt hergestellt werden:

(1aS,3aR,6bR)-5-Hydroxy-1-oxo-1a,2,3,3a,4,6b-hexahydro-2,6a-diazacyclobut[cd]inden-2,6-dicarbonsäure-di-t-butylester (4,0 g; 10,92 mmol; aus Beispiel 1) wird in abs. Methylenchlorid (110 ml) bei Raumtemperatur gelöst und auf -78°C gekühlt. N-Aethyldiisopropylamin (2,1 ml; 12 mmol) wird vorsichtig zugetropft, ohne dass die Temperatur über -76°C steigt (ca. 10 Minuten). Nach 10 Minuten wird Trifluormethansulfonsäureanhydrid (2,0 ml; 12 mmol) zwischen -78°C und -76°C zugetropft und anschliessend noch 20 Minuten bei -78°C weitergerührt. Das Reaktionsgemisch wird mit Methylenchlorid (300 ml) verdünnt und nacheinander mit Wasser (3x160 ml) und gesättigter wässriger Kochsalzlösung (80 ml) gewaschen. Die organische Phase wird über Magnesiumsulfat getrocknet und eingeengt. Der Rückstand wird in abs. THF (110 ml) gelöst und mit 5-Mercapto-1-methyl-1H-tetrazol-Natriumsalz (1,51 g; 10,92 mmol) bei Raumtemperatur versetzt. Nach 6 Tagen wird erneut 5-Mercapto-1-methyltetrazol-Natriumsalz (0,75 g;

5,46 mmol) zugegeben. Anschliessend wird nochmals während 9 Tagen weitergerührt. Die Suspension wird abgenutscht und der erhaltene Feststoff mit etwas Aethylacetat gewaschen. Ausbeute: 3,0 g (60%) als farbloser Feststoff. Smp. 222 ° C.
IR (KBr): 1784, 1692, 1248, 1163 $cm^{-1}$

| Mikroanalyse: $C_{20}H_{28}N_6O_5S$ | | | |
|---|---|---|---|
| Ber. | C 51,71 | H 6,08 | N 18,09 |
| Gef. | C 51,61 | H 6,09 | N 18,08 |

In Analogie hierzu werden hergestellt:

(b) (1aS,3aR,6bR)-5-(5-Methyl-1,3,4-thiadiazol-2-ylsulfanyl)-1-oxo-1a,2,3,3a,4,6b-hexahydro-1H-2,6a-diazacyclobut[cd]inden-6-carbonsäure-trifluoracetat

Ausgehend von (1aS,3aR,6bR)-5-(5-Methyl-1,3,4-thiadiazol-2-ylsulfanyl)-1-oxo-1a,2,3,3a,4,6b-hexahydro-1H-2,6a-diazacyclo[cd]inden-2,6-dicarbonsäure-di-t-butylester (660 mg; 0,37 mmol) werden 360 mg (62%) als farbloser Feststoff erhalten.
IR (KBr): 1785, 1674 $cm^{-1}$
MS (ISN): $(M-H)^{-} + NH_3$: 340,0 (MS-Artefact)
Der als Ausgangsverbindung eingesetzte (1aS,3aR,6bR)-5-(5-Methyl-1,3,4-thiadiazol-2-ylsulfanyl)-1-oxo-1a,2,3,3a,4,6b-hexahydro-1H-2,6a-diazacyclobut[cd]inden-2,6-dicarbonsäure-di-t-butylester wird ausgehend von (1aS,3aR,6bR)-5-Hydroxy-1-oxo-1a,2,3,3a,4,6b-hexahydro-2,6a-diazacyclobut[cd]inden-2,6-dicarbonsäure-di-t-butylester (2,15 g; 5,87 mmol; aus Beispiel 1) erhalten: 960 mg (40%) Produkt als farbloser Feststoff. Smp: 175 ° C.
IR (KBr): 1779, 1701, 1243, 1161 $cm^{-1}$

| Mikroanalyse: $C_{21}H_{28}N_4O_5S_2$ | | | |
|---|---|---|---|
| Ber. | C 52,48 | H 5,87 | N 11,66 |
| Gef. | C 52,34 | H 5,91 | N 11,62 |

(c) (1aS,3aR,6bR)-5-(5-Amino-1,3,4-thiadiazol-2-ylsulfanyl)-1-oxo-1a,2,3,3a,4,6b-hexahydro-1H-2,6a-diazacyclobut[cd]inden-6-carbonsäure-trifluoracetat

Ausgehend von (1aS,3aR,6bR)-5-(5-Amino-1,3,4-thiadiazol-2-ylsulfanyl)-1-oxo-1a,2,3,3a,4,6b-hexahydro-1H-2,6a-diazacyclobut[cd]inden-2,6-dicarbonsäure-di-t-butylester (510 mg; 1,03 mmol) werden 400 mg (89%) als beiger Feststoff erhalten.
IR (KBr): 1776, 1678, 1619, 1390, 1204 $cm^{-1}$
MS (ISN): $(M-H)^{-}$ 324,2
Der als Ausgangsverbindung eingesetzte (1aS,3aR,6bR)-5-(5-Amino-1,3,4-thiadiazol-2-ylsulfanyl)-1-oxo-1a,2,3,3a,4,6b-hexahydro-1H-2,6a-diazacyclobut[cd]inden-2,6-di-carbonsäure-di-t-butylester wird ausgehend von (1aS,3aR,6bR)-5-Hydroxy-1-oxo-1a,2,3,3a,4,6b-hexahydro-2,6a-diazacyclobut[cd]inden-2,6-dicarbonsäure-di-t-butylester (500 mg; 1,36 mmol; aus Beispiel 1) erhalten: 280 mg (41%) Produkt als gelber Feststoff. Smp: 204 ° C (Zers.).
IR (KBr): 3317, 1777, 1700, 1616, 1244, 1161 $cm^{-1}$

| Mikroanalyse: $C_{20}H_{27}N_5O_6S_2$ | | | |
|---|---|---|---|
| Ber. | C 48,28 | H 5,47 | N 14,07 |
| Gef. | C 48,58 | H 5,60 | N 14,21 |

(d) (1aS,3aR,6bR)-1-Oxo-5-(pyridin-4-ylsulfanyl)-1a,2,3,3a,4,6b-hexahydro-1H-2,6a-diaza-cyclobut[cd]inden-6-carbonsäure-trifluoracetat

Ausgehend von (1aS,3aR,6bR)-1-Oxo-5-(pyridin-4-ylsulfanyl)-1a,2,3,3a,4,6b-hexahydro-1H-2,6a-diaza-cyclobut[cd]inden-2,6-dicarbonsäure-di-t-butylester (177mg; 0,38mmol) werden 119mg (58%) als gelblicher Festkörper erhalten.
IR(KBr): 1786, 1678, 1624, 1479, 1199, 1135 $cm^{-1}$
MS (ISP): $(M+H)^+$ 304,3

Der als Ausgangsverbindung eingesetzte (1aS,3aR,6bR)-1-Oxo-5-(pyridin-4-ylsulfanyl)-1a,2,3,3a,4,6b-hexahydro-1H-2,6a-diaza-cyclobut[cd]inden-2,6-dicarbonsäure-di-t-butylester kann wie folgt hergestellt werden:

(1aS,3aR,6bR)-1-Oxo-5-trifluormethylsulfonyloxy-1a,2,3,3a,4,6b-hexahydro-1H-2,6a-diaza-cyclobut[cd]inden-2,6-dicarbonsäure-di-t-butylester

Bei Raumtemperatur wird (1aS,3aR,6bR)-5-Hydroxy-1-oxo-1a,2,3,3a,4,6b-hexahydro-2,6a-diazacyclobut-[cd]inden-2,6-dicarbonsäure-di-t-butylester (1,8 g; 4,91 mmol; aus Beispiel 1) in absolutem Methylenchlorid (50 ml) gelöst und auf -78°C gekühlt. Bei einer Temperatur von < -70° C wird zuerst N-Aethyldiisopropylamin (0,99 ml; 5,78 mmol) zugetropft, dann Trifluormethansulfonsäureanhydrid (0,90 ml; 5,49 mmol) und schliesslich wird noch 1,5 Stunden weitergerührt. Das Reaktionsgemisch wird mit Wasser (1x100 ml, 2x50ml) gewaschen. Die organische Phase wird über Magnesiumsulfat getrocknet und eingeengt. Der Rückstand wird in Diethylaether gelöst. Zugabe von n-Hexan liefert 2.25 g (90 %) eines beigen Niederschlages.
MS(EI): 425 $(M\text{-}OC_4H_9)^+$

(1aS,3aR,6bR)-1-Oxo-5-(pyridin-4-ylsulfanyl)-1a,2,3,3a,4,6b-hexahydro-1H-2,6a-diaza-cyclobut[cd]inden-2,6-dicarbonsäure-di-t-butylester

Ein Gemisch von 4-Mercaptopyridin (118 mg; 1.06 mmol) und Natriumhydrid (51 mg; ca. 1.16 mmol) wird in THF (10 ml) suspendiert. Bei -45°C - -40°C wird eine Lösung von (1aS,3aR,6bR)-1-Oxo-5-trifluormethylsulfonyloxy-1a,2,3,3a,4,6b-hexahydro-1H-2,6a-diaza-cyclobut[cd]inden-2,6-dicarbonsäure-di-t-butylester (499 mg; 1.00 mmol) in THF (5ml) zugetropft und das Ganze anschliessend 7.5 Stunden gerührt. Das Reaktionsgemisch wird verdünnt mit 40ml eines Gemisches bestehend aus 20 ml gesättigter, wässriger Kochsalzlösung, 10 ml Wasser und 10 ml eines wässrigen 2M Dikaliumhydrogenphosphat/Kaliumdihydrogenphosphat-Puffers, pH 6. Das Reaktionsgemisch wird mit Aethylacetat (2x60 ml) extrahiert. Die organischen Phasen werden mit gesättigter, wässriger Kochsalzlösung (40 ml) gewaschen, vereinigt, über Magnesiumsulfat getrocknet und eingeengt. Der Rückstand wird zweimal an Kieselgel chromatographiert, wobei bei der ersten Chromatographie als Laufmittel Methylenchlorid/Aceton 9:1, resp. 4:1 verwendet wird, bei der zweiten Chromatographie Aethylacetat/n-Hexan 4:1. Man erhält 407 mg (88%) als weissen Schaum.
IR (KBr): 1779, 1703, 1572, 1406, 1368, 1162 $cm^{-1}$
MS (MALDI): $(M+H)^+$ 460,8

(e) (1aS,3aR,6bR)-4-(6-Carboxy-1-oxo-1a,2,3,3a,4,6b-hexahydro-1H-2,6a-diaza-cyclobut[cd]inden-5-ylsulfanyl)-1-methyl-pyridiniumtrifluormethansulfonat-trifluoracetat (1:1)

Ausgehend von (1aS,3aR,6bR)-4-(2,6-Bis-t-butoxycarbonyl-1-oxo-1a,2,3,3a,4,6b-hexahydro-1H-2,6a-diaza-cyclobut[cd]inden-5-ylsulfanyl)-1-methyl-pyridinium-trifluormethansulfonat (840mg; 1,35mmol) werden 640mg (78%) als gelblicher Festkörper erhalten.
IR(KBr): 1782, 1731, 1225, 1175, 827 $cm^{-1}$
MS (ISP): $M^+$ 318,3

| Mikroanalyse: $C_{16}H_{16}N_3O_6S_2F_3 \cdot 0,95\ CF_3COOH \cdot 0,11\ CH_3OSO_2CF_3 \cdot 0,07\ (C_2H_5)_2O \cdot 0,66\ H_2O$ | | | | | |
|---|---|---|---|---|---|
| Ber. | C 36,18 | H 3,18 | N 6,88 | S 11,07 | F 19,22 |
| Gef. | C 36,19 | H 3,35 | N 6,80 | S 11,17 | F 19,23 |

Das als Ausgangsverbindung eingesetzte (1aS,3aR,6bR)-4-(2,6-Bis-t-butoxycarbonyl-1-oxo-1a,2,3,3a,4,6b-hexahydro-1H-2,6a-diaza-cyclobut[cd]inden-5-ylsulfanyl)-1-methyl-pyridinium-trifluormethansulfonat kann wie folgt hergestellt werden:

Bei 0°C wird zu einer Lösung von (1aS,3aR,6bR)-1-Oxo-5-(pyridin-4-ylsulfanyl)-1a,2,3,3a,4,6b-hexahydro-1H-2,6a-diaza-cyclobut[cd]inden-2,6-dicarbonsäure-di-t-butylester (730 mg; 1,6 mmol; aus Beispiel 2-(d)) in Methylenchlorid (10ml) Trifluormethansulfonsäure-methylester (0,21 ml; 1,91 mmol) zugetropft. Noch während 2 Stunden wird bei 0°C gerührt, dann das Solvens abgezogen. Der Rückstand wird mit n-Hexan versetzt und ausgerührt. Der dabei gebildete Niederschlag wird abgenutscht und getrocknet. Man erhält 870 mg (82%) als gelblichen Festkörper.

IR(KBr): 1783, 1722, 1699, 1369, 1263, 1160 $cm^{-1}$

MS (ISP): $M^+$ 474,5

| Mikroanalyse: $C_{25}H_{32}N_3O_8S_2F_3 \cdot 0,1\ CH_3OSO_2CF_3 \cdot 0,25\ C_6H_{14} \cdot 0,5\ H_2O$ | | | | | |
|---|---|---|---|---|---|
| Ber. | C 47,82 | H 5,53 | N 6,27 | S 10,04 | F 9,35 |
| Gef. | C 47,60 | H 5,69 | N 6,21 | S 10,32 | F 9,69 |

(f) (1aS,3aR,6bR)-5-Carbamoylmethylsulfanyl-1-oxo-1a,2,3,3a,4,6b-hexahydro-1H-2,6a-diaza-cyclobut[cd]-inden-6-carbonsäure-trifluoracetat

Ausgehend von (1aS,3aR,6bR)-5-Carbamoylmethylsulfanyl-1-oxo-1a,2,3,3a,4,6b-hexahydro-1H-2,6a-diaza-cyclobut[cd]inden-2,6-dicarbonsäure-di-t-butylester (140mg; 0,32mmol) werden 88mg (70%) als bräunlicher Festkörper erhalten.

IR(KBr): 3429, 1776, 1677, 1378, 1202 $cm^{-1}$

MS (ISN): $(M-H)^-$ 396,3

Der als Ausgangsverbindung eingesetzte (1aS,3aR,6bR)-5-Carbamoylmethylsulfanyl-1-oxo-1a,2,3,3a,4,6b-hexahydro-1H-2,6a-diaza-cyclobut[cd]inden-2,6-dicarbonsäure-di-t-butylester kann in Analogie zu Beispiel 2(d) aus (1aS,3aR,6bR)-1-Oxo-5-trifluormethylsulfonyloxy-1a,2,3,3a,4,6b-hexahydro-1H-2,6a-diaza-cyclobut[cd]inden-2,6-dicarbonsäure-di-t-butylester (600 mg; 1.2 mmol; aus Beispiel 2(d)) und Mercaptoacetamid (120 mg; 1.3 mmol) hergestellt werden. Man erhält 120 mg (23%) eines gelblichen Festkörpers.

IR(KBr): 1773, 1691, 1618, 1252 $cm^{-1}$

MS(ISP): 457,4 $(M+NH_4)^+$; 440,4 $(M+H)^+$

(g) (1aS,3aR,6bR)-5-(6-Carboxy-1-oxo-1a,2,3,3a,4,6b-hexahydro-1H-2,6a-diaza-cyclobut[cd]inden-5-ylsulfanylmethyl)-1,4-dimethyl-1H-1,2,4-triazol-4-ium-trifluormethansulfonat-trifluoroacetat

Ausgehend von (1aS,3aR,6bR)-5-(2,6-Bis-t-butoxycarbonyl-1-oxo-1a,2,3,3a,4,6b-hexahydro-1H-2,6a-diaza-cyclobut[cd]inden-5-ylsulfanylmethyl)-1,4-dimethyl-1H-1,2,4-triazol-4-ium-trifluormethansulfonat (980mg; 1,5mmol) werden 865mg (94%) als gelber Festkörper erhalten.

IR(KBr): 1781, 1682, 1629, 1264 $cm^{-1}$

MS (ISP): $(M)^+$ 336,2

Das als Ausgangsverbindung eingesetzte (1aS,3aR,6bR)-5-(2,6-Bis-t-butoxycarbonyl-1-oxo-1a,2,3,3a,4,6b-hexahydro-1H-2,6a-diaza-cyclobut[cd]inden-5-ylsulfanylmethyl)-1,4-dimethyl-1H-1,2,4-triazol-4-ium-trifluormethansulfonat kann wie folgt hergestellt werden:

Bei -76°C wird eine Lösung von (1aS,3aR,6bR)-1-Oxo-5-trifluormethylsulfonyloxy-1a,2,3,3a,4,6b-hexahydro-1H-2,6a-diaza-cyclobut[cd]inden-2,6-dicarbonsäure-di-t-butylester (205 mg; 0,41 mmol; aus Beispiel 2-(d)) in THF (4 ml) mit einer Lösung von 1,4-Dimethyl-5-mercaptomethyl-1H-1,2,4-triazol-4-ium-trifluormethansulfonat (137 mg; 0,47 mmol) in THF (3 ml) und einer Lösung von Diisopropylaethylamin in THF (2 ml) versetzt. Innert 3 Stunden wird auf Raumtemperatur erwärmt und dann noch 3 Stunden nachgerührt. Das Reaktionsgemisch wird mit Aethylacetat (100 ml) verdünnt und extrahiert mit 50 ml eines Gemisches bestehend aus 25 ml gesättigter, wässriger Kochsalzlösung, 12,5 ml Wasser und 12,5 ml eines wässrigen 2M Dikaliumhydrogenphosphat/Kaliumdihydrogenphosphat-Puffers von pH 6. Die wässrige Phase wird mit Aethylacetat (100 ml) rückextrahiert. Die organischen Phasen werden mit gesättigter, wässriger Kochsalzlösung (50 ml) gewaschen, vereinigt, über Magnesiumsulfat getrocknet und eingeengt. Chromatographie des Rückstandes an Kieselgel (Laufmittel Methylenchlorid/Methanol 9:1, dann 6:1) ergibt 111 mg (42%) als weissen Festkörper.

IR(KBr): 1773, 1694, 1566, 1260, 1160 $cm^{-1}$

MS(ISP): 492,4 (M-trifluormethansulfonat)$^+$

Beispiel 3

(a) (1aS,3aR,6bR)-2-(4-Hydroxy-phenylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure-Natriumsalz

(1aS,3aR,6bR)-5-(1-Methyl-1H-tetrazol-5-ylsulfanyl)-1-oxo-1a,2,3,3a,4,6b-hexahydro-1H-2,6a-diazacyclo[cd]inden-6-carbonsäure-trifluoracetat (200 mg; 0,474 mmol; aus Beispiel 2(a)) wird in Acetonitril/Wasser 1:1 (6 ml) vorgelegt und auf 0°C gekühlt. Die Lösung wird mit Natriumbicarbonat (80 mg; 0,948 mmol) und 4-Hydroxy-phenylcarbaminsäure-2,5-dioxopyrrolidin-1-yl-ester (118 mg; 0,474 mmol) versetzt. Nach 10 Minuten bei 0°C wird 1 bis 2 Stunden bei Raumtemperatur weitergerührt (wird dünnschichtchromatographisch verfolgt). Anschliessend wird das Reaktionsgemisch mit Wasser (5 ml) verdünnt, mit Methylenchlorid (3x10 ml) gewaschen und lyophilisiert. Der Rückstand wird in wenig Wasser gelöst und über ein polymeres hydrophobes Gel mit Wasser chromatographiert und lyophilisiert.
Ausbeute: 78 mg (35%) als farbloses Pulver.
IR (KBr): 3416, 1756, 1615, 1513, 1389, 1240 cm$^{-1}$
MS (ISN): (M-Na$^+$) 442,4

In Analogie hierzu, ausgehend von (1aS,3aR,6bR)-5-(1-Methyl-1H-tetrazol-5-ylsulfanyl)-1-oxo-1a,2,3,3a,4,6b-hexahydro-1H-2,6a-diazacyclo[cd]inden-6-carbonsäure-trifluoracetat, werden hergestellt:

(b) (1aS,3aR,6bR)-2-(4-Carbamoyl-phenylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure-Natriumsalz. Ausbeute: 21% als farbloses Pulver.
IR (KBr): 3424, 1756, 1658, 1608, 1526, 1390 cm$^{-1}$

| Mikroanalyse: $C_{19}H_{17}N_8O_5SNa$ | | | |
|---|---|---|---|
| Ber. | C 42,41 | H 4,13 | N 20,82 |
| Gef. | C 42,59 | H 4,01 | N 20,61 |

(c) (1aS,3aR,6bR)-2-[(S)-2-Oxo-pyrrolidin-3-ylcarbamoyl]-5-(1-methyl-1H-tetrazol-5-ylsulfanyl)-1-oxo-1a,2,3,3a,4,6b-hexahydro-1H-2,6a-diazacyclobut[cd]inden-6-carbonsäure-Natriumsalz. Ausbeute: 17% als farbloses Pulver.
IR (KBr): 1758, 1702 cm$^{-1}$
MS (ISN): (M-Na)$^-$ 433,2

(d) (1aS,3aR,6bR)-5-(1-Methyl-1H-tetrazol-5-ylsulfanyl)-1-oxo-2-(thien-2-ylmethylcarbamoyl)-1a,2,3,3a,4,6b-hexahydro-1H-2,6a-diazacyclobut[cd]inden-6-carbonsäure-Natriumsalz. Ausbeute: 19% als farbloses Pulver.
IR (KBr): 3410, 1756, 1619, 1525, 1394 cm$^{-1}$
MS (ISN): (M-Na)$^-$ 446,2

(e) (1aS,3aR,6bR)-2-(3,4-Dihydroxy-benzylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure-Natriumsalz. Ausbeute: 16% als farbloses Pulver.
IR (KBr): 3411, 1756, 1618, 1529, 1392 cm$^{-1}$
MS (ISN): (M-Na)$^-$ 472,3

(f) (1aS,3aR,6bR)-5-(1-Methyl-1H-tetrazol-5-ylsulfanyl)-1-oxo-2-[(R)- und [(S)-2-oxo-tetrahydro-thien-3-ylcarbamoyl]-1a,2,3,3a,4,6b-hexahydro-1H-2,6a-diazacyclobut[cd]inden-6-carbonsäure-Natriumsalz. Ausbeute: 40% als farbloses Pulver.
IR (KBr): 3426, 1758, 1699, 1620, 1534, 1393 cm$^{-1}$
MS (ISN): (M-Na)$^-$ 450,3

(g) (1aS,3aR,6bR)-5-(1-Methyl-1H-tetrazol-5-ylsulfanyl)-1-oxo-2-(4-sulfamoyl-benzylcarbamoyl)-1a,2,3,3a,4,6b-hexahydro-1H-2,6a-diazacyclobut[cd]inden-6-carbonsäure-Natriumsalz. Ausbeute: 36% als farbloses Pulver.
IR (KBr): 3371, 1756, 1615, 1539, 1377, 1323, 1160 cm$^{-1}$

| Mikroanalyse: $C_{19}H_{19}N_8O_6S_2Na$ | | | |
|---|---|---|---|
| Ber. | C 42,06 | H 3,53 | N 20,65 |
| Gef. | C 41,81 | H 3,71 | N 20,32 |

(h) (1aS,3aR,6bR)-2-(3-Methoxy-isoxazol-5-ylmethylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure-Natriumsalz. Ausbeute: 28% als farbloses Pulver.
IR (KBr): 3280, 1751, 1620, 1518, 1409 $cm^{-1}$
MS (ISN): $[(M\text{-}Na)^{-} + NH_3]$ 478,3 (MS-Artefact), $(M+Na)^{-}$ 461,5
(i) (1aS,3aR,6bR)-2-[(R)- und [(S)-1,1-Dioxo-tetrahydrothien-3-ylcarbamoyl)-5-(1-methyl-1H-tetrazol-5-yl-sulfanyl)-1-oxo-1a,2,3,3a,4,6b-hexahydro-1H-2,6a-diazacyclobut[cd]inden-6-carbonsäure-Natriumsalz. Ausbeute: 30% als farbloses Pulver.
IR (KBr): 3300, 1764, 1633, 1534, 1394, 1305, 1118 $cm^{-1}$
MS (ISN): $(M+Na)^{-}$ 468,6

In Analogie zu Beispiel 3(a) werden ausgehend von (1aS,3aR,6bR)-5-(5-Methyl-1,3,4-thiadiazol-2-ylsulfanyl)-1-oxo-1a,2,3,3a,4,6b-hexabydro-1H-2,6a-diazacyclobut[cd]inden-6-carbonsäure-trifluoracetat (aus Beispiel 2b) hergestellt:
(j) (1aS,3aR,6bR)-2-(4-Hydroxyphenylcarbamoyl)-5-(5-methyl-1,3,4-thiadiazol-2-ylsulfanyl)-1-oxo-1a,2,3,3a,4,6b-hexahydro-1H-2,6a-diazacyclobut[cd]inden-6-carbonsäure-Natriumsalz. Ausbeute: 35% als farbloses Pulver.
IR (KBr): 3280, 1756, 1612, 1539, 1387 $cm^{-1}$

| Mikroanalyse: $C_{19}H_{16}N_5O_5S_2Na$ | | | |
|---|---|---|---|
| Ber. | C 47,40 | H 3,35 | N 14,55 |
| Gef. | C 47,44 | H 3,22 | N 14,45 |

(k) (1aS,3aR,6bR)-2-(4-Carbamoyl-phenylcarbamoyl)-5-(5-methyl-1,3,4-thiadiazol-2-ylsulfanyl)-1-oxo-1a,2,3,3a,4,6b-hexahydro-1H-2,6a-diazacyclobut[cd]inden-6-carbonsäure-Natriumsalz. Ausbeute: 19% als farbloses Pulver.
IR (KBr): 3416, 3240, 1756, 1660, 1607, 1525, 1385 $cm^{-1}$

| Mikroanalyse: $C_{20}H_{17}N_6O_5S_2Na$ | | | |
|---|---|---|---|
| Ber. | C 47,24 | H 3,37 | N 16,53 |
| Gef. | C 47,16 | H 3,74 | N 16,35 |

In Analogie zu Beispiel 3(a) werden ausgehend von (1aS,3aR,6bR)-5-(5-Amino-1,3,4-thiadiazol-2-ylsulfanyl)-1-oxo-1a,2,3,3a,4,6b-hexahydro-1H-2,6a-diazacyclobut[cd]inden-6-carbonsäure-trifluoracetat (aus Beispiel 2c) hergestellt:
(l) (1aS,3aR,6bR)-5-(5-Amino-1,3,4-thiadiazol-2-ylsulfanyl)-2-(4-hydroxyphenylcarbamoyl)-1-oxo-1a,2,3,3a,4,6b-hexahydro-1H-2,6a-diazacyclobut[cd]inden-6-carbonsäure-Natriumsalz. Ausbeute: 32% als farbloses Pulver.
IR (KBr): 3411, 3300, 3180, 1751, 1611, 1513, 1389, 1238 $cm^{-1}$
MS (ISN): $(M+Na)^{-}$ 458,9
(m) (1aS,3aR,6bR)-5-(5-Amino-1,3,4-thiadiazol-2-ylsulfanyl)-2-(4-carbamoylamino-phenylcarbamoyl)-1-oxo-1a,2,3,3a,4,6b-hexahydro-1H-2,6a-diazacyclobut[cd]inden-6-carbonsäure-Natriumsalz. Ausbeute: 26% als farbloses Pulver.
IR (KBr): 3394, 1751, 1657, 1606, 1523, 1389 $cm^{-1}$
MS (ISN): $(M+Na)^{-}$ 486,2

| Mikroanalyse: $C_{19}H_{16}N_7O_5S_2Na$ | | | |
|---|---|---|---|
| Ber. | C 44,79 | H 3,17 | N 19,24 |
| Gef. | C 44,82 | H 3,29 | N 19,60 |

In Analogie zu Beispiel 3(a) werden hergestellt:

(n) (1aS,3aR,6bR)-2-(4-Carbamoyl-phenylcarbamoyl)-1-oxo-5-(pyridin-4-ylsulfanyl)-1a,2,3,3a,4,6b-hexahydro-1H-2,6a-diaza-cyclobut[cd]inden-6-carbonsäure-Natriumsalz

Ausgehend von (1aS,3aR,6bR)-1-Oxo-(5-pyridin-4-ylsulfanyl)-1a,2,3,3a,4,6b-hexahydro-1H-2,6a-diaza-cyclobut[cd]inden-6-carbonsäure-trifluoracetat (105 mg; 0,20 mmol; aus Beispiel 2(d)) werden 31 mg (32%) als weisses Pulver isoliert.

IR (KBr): 3422, 1759, 1662, 1611, 1383 $cm^{-1}$

MS (ISN): $(M\text{-}H+NH_3)^-$ 481,4; $(M\text{-}Na)^-$ 464

(o) (1aS,3aR,6bR)-2-(4-Hydroxy-phenylcarbamoyl)-1-oxo-5-(pyridin-4-ylsulfanyl)-1a,2,3,3a,4,6b-hexahydro-1H-2,6a-diaza-cyclobut[cd]inden-6-carbonsäure-Natriumsalz

Ausgehend von (1aS,3aR,6bR)-1-Oxo-5-(pyridin-4-ylsulfanyl)-1a,2,3,3a,4,6b-hexahydro-1H-2,6a-diaza-cyclobut[cd]inden-6-carbonsäure-trifluoracetat (100 mg; 0,19 mmol; aus Beispiel 2(d)) werden 51 mg (54%) als weisses Pulver isoliert.

IR (KBr): 3406, 1756, 1613, 1438, 1237, 832 $cm^{-1}$

MS (ISP): $(M+H+Na)^+$ 461,5; $(M+H)^+$ 439,5

| Mikroanalyse: $C_{21}H_{17}N_4O_5SNa \cdot 1,99\ H_2O$ | | | | |
|---|---|---|---|---|
| Ber. | C 50,83 | H 4,26 | N 11,26 | S 6,46 |
| Gef. | C 49,78 | H 4,18 | N 11,09 | S 6,68 |

(p) (1aS,3aR,6bR)-5-Carbamoylmethylsulfanyl-2-(4-hydroxy-phenylcarbamoyl)-1-oxo-5-(pyridin-4-ylsulfanyl)-1a,2,3,3a,4,6b-hexahydro-1H-2,6a-diaza-cyclobut[cd]inden-6-carbonsäure-Natriumsalz

Ausgehend von (1aS,3aR,6bR)-5-Carbamoylmethylsulfanyl-1-oxo-1a,2,3,3a,4,6b-hexahydro-1H-2,6a-diaza-cyclobut[cd]inden-6-carbonsäure-trifluoracetat (120 mg; 0,30 mmol; aus Beispiel 2(f)) werden 25 mg (19%) als weisses Pulver isoliert.

IR (KBr): 3410, 1748, 1670, 1605, 1512, 1384, 1238, 838 $cm^{-1}$

MS (ISN): $(M+NH_3\text{-}Na)^-$ 434,3; $(M\text{-}Na)^-$ 417,3

(q) (1aS,3aR,6bR)-5-[(1,4-Dimethyl-1H-1,2,4-triazol-5-ylio)-methylsulfanyl]-2-(4-hydroxy-phenylcarbamoyl)-1-oxo-1a,2,3,3a,4,6b-hexahydro-1H-2,6a-diaza-cyclobut[cd]inden-6-carboxylat

Ausgehend von (1aS,3aR,6bR)-5-(6-Carboxy-1-oxo-1a,2,3,3a,4,6b-hexahydro-1H-2,6a-diaza-cyclobut-[cd]inden-5-ylsulfanylmethyl)-1,4-dimethyl-1H-1,2,4-triazol-4-ium-trifluoromethansulfonat-trifluoracetat (150 mg; 0.25 mmol; aus Beispiel 2(g)) werden 22 mg (14%) als weisses Pulver isoliert.

IR (KBr): 3411, 1761, 1711, 1607, 1370, 1240, 1195 $cm^{-1}$

MS (ISN): $(M\text{-}H)^-$ 469,3

(aa) (1aS,3aR,6bR)-2-Acetyl-5-(1-methyl-1H-tetrazol-5-ylsulfanyl)-1-oxo-1a,2,3,3a,4,6b-hexahydro-1H-2,6a-diaza-cyclobut[cd]inden-6-carbonsäure-Natriumsalz

Zu einer Lösung von (1aS,3aR,6bR)-5-(1-Methyl-1H-tetrazol-5-ylsulfanyl)-1-oxo-1a,2,3,3a,4,6b-hexahydro-1H-2,6a-diaza-cyclobut[cd]inden-6-carbonsäure-trifluoracetat (200 mg; 0,36 mmol; aus Beispiel 2(a)) und Natriumhydrogencarbonat (121 mg; 1,44 mmol) in DMF (2 ml) wird bei 0°C Acetylchlorid (26 µl; 0,36 mmol) gegeben. Man lässt bei 0°C 0,5 Stunden rühren und engt dann ein. Der Rückstand wird in wenig Wasser gelöst und über ein hydrophobes Polymer chromatographiert (Laufmittel: Wasser/Acetonitril). Man erhält 24 mg (18%) eines gelblichen Pulvers.

IR (KBr): 1760, 1618, 1395 $cm^{-1}$

MS (ISN): $(M\text{-}Na+NH_3)^-$ 366,3 (MS-Artefakt); $(M\text{-}Na)^-$ 349,3

(ab) (1aS,3aR,6bR)-5-(1-Methyl-1H-tetrazol-5-ylsulfanyl)-1-oxo-2-trifluoracetyl-1a,2,3,3a,4,6b-hexahydro-1H-2,6a-diaza-cyclobut[cd]inden-6-carbonsäure-Natriumsalz

Zu einer Lösung von (1aS,3aR,6bR)-5-(1-Methyl-1H-tetrazol-5-ylsulfanyl)-1-oxo-1a,2,3,3a,4,6b-hexahydro-1H-2,6a-diaza-cyclobut[cd]inden-6-carbonsäure-trifluoracetat (200 mg; 0,36 mmol; aus Beispiel 2(a)) in DMF (4 ml) wird N-Methyl-N-trimethylsilyltrifluoracetamid (80 µl; 0,27 mmol) gegeben. dann Dicyclohexylcarbodiimid (89 mg; 0,43 mmol) und Trifluoressigsäure. Bei Raumtemperatur wird 1 Stunde gerührt. Der entstandene Niederschlag wird abfiltriert und mit wenig DMF nachgewaschen. Das Filtrat wird eingeengt. Der Rückstand wird in wenig Wasser gelöst. Mit Natriumhydrogencarbonat wird auf pH 7 gestellt und über ein hydrophobes Polymer chromatographiert (Laufmittel: Wasser/Acetonitril). Man erhält 12 mg (9%) eines weissen Pulvers.
IR (KBr): 1768, 1696, 1621, 1394, 1172 $cm^{-1}$
MS (ISP): $(M+H)^+$ 427,3; $(M\text{-}Na+H+NH_4)^+$ 422,4; $(M\text{-}Na+2H)^+$ 405,3

(ac) (1aS,3aR,6bR)-2-Acetyl-5-(5-methyl-1,3,4-thiadiazol-2-ylsulfanyl)-1-oxo-1a,2,3,3a,4,6b-hexahydro-1H-2,6a-diaza-cyclobut[cd]inden-6-carbonsäure-Natriumsalz

Ausgehend von (1aS,3aR,6bR)-5-(5-Methyl-1,3,4-thiadiazol-2-ylsulfanyl)-1-oxo-1a,2,3,3a,4,6b-hexahydro-1H-2,6a-diaza-cyclobut[cd]inden-6-carbonsäure-trifluoracetat (100 mg; 0,23 mmol; aus Beispiel 2(b)) werden in Analogie zu Beispiel 3(aa) 30 mg (34%) eines gelblichen Pulvers erhalten.
IR (KBr): 1759, 1620, 1397 $cm^{-1}$
MS (ISN): $(M\text{-}Na)^-$ 365,3

(ad) (1aS,3aR,6bR)-5-(5-Methyl-1,3,4-thiadiazol-2-ylsulfanyl)-1-oxo-2-trifluoracetyl-1a,2,3,3a,4,6b-hexahydro-1H-2,6a-diaza-cyclobut[cd]inden-6-carbonsäure-Natriumsalz

Bei 0°C wird ein Lösung von (1aS,3aR,6bR)-5-(5-Methyl-1,3,4-thiadiazol-2-ylsulfanyl)-1-oxo-1a,2,3,3a,4,6b-hexahydro-1H-2,6a-diaza-cyclobut[cd]inden-6-carbonsäure-trifluoracetat (90 mg; 0,21 mmol; aus Beispiel 2(b)) und Natriumhydrogencarbonat (53 mg; 0,63 mmol) in DMF (1 ml) mit 2-(Trifluoracetoxy)-pyridin (29 µl; 0,21 mmol) versetzt. Man lässt bei Raumtemperatur 1,5 Stunden rühren und engt dann ein. Der Rückstand wird in wenig Wasser gelöst und über ein hydrophobes Polymer chromatographiert (Laufmittel: Wasser/Acetonitril). Man erhält 23 mg (24%) eines weissen Pulvers.
IR (KBr): 1766, 1697, 1618, 1343, 1180 $cm^{-1}$
MS (ISP): $(M+H)^+$ 443,4; $(M\text{-}Na+H+NH_4)^+$ 438,4; $(M\text{-}Na+2H)^+$ 421,4

(ae) (1aS,3aR,6bR)-2-Acetyl-5-(5-amino-1,3,4-thiadiazol-2-ylsulfanyl)-1-oxo-1a,2,3,3a,4,6b-hexahydro-1H-2,6a-diaza-cyclobut[cd]inden-6-carbonsäure-Natriumsalz

Ausgehend von (1aS,3aR,6bR)-5-(5-Amino-1,3,4-thiadiazol-2-ylsulfanyl)-1-oxo-1a,2,3,3a,4,6b-hexahydro-1H-2,6a-diaza-cyclobut[cd]inden-6-carbonsäure-trifluoracetat (100 mg; 0,23 mmol; aus Beispiel 2(c)) werden in Analogie zu Beispiel 3(aa) 61 mg (49%) eines gelblichen Pulvers erhalten.
IR (KBr): 1756, 1617, 1400, 1405, 807 $cm^{-1}$
MS (ISN): $(M\text{-}Na)^-$ 366,3

| Mikroanalyse: $C_{13}H_{12}N_5O_4S_2Na \cdot 2{,}37\ H_2O \cdot 0{,}3\ NaHCO_3$ | | | | | |
|---|---|---|---|---|---|
| Ber.<br>Gef. | C 34,93<br>C 34,90 | H 3,76<br>H 3,45 | N 15,32<br>N 15,41 | S 14,02<br>S 13,43 | Na 6,54<br>Na 6,49 |

(af) (1aS,3aR,6bR)-5-(5-Amino-1,3,4-thiadiazol-2-ylsulfanyl)-2-methylsulfonyl-1-oxo-1a,2,3,3a,4,6b-hexahydro-1H-2,6a-diaza-cyclobut[cd]inden-6-carbonsäure-Natriumsalz

Ausgehend von (1aS,3aR,6bR)-5-(5-Amino-1,3,4-thiadiazol-2-ylsulfanyl)-1-oxo-1a,2,3,3a,4,6b-hexahydro-1H-2,6a-diaza-cyclobut[cd]inden-6-carbonsäure-trifluoracetat (120 mg; 0,27 mmol; aus Beispiel 2(c)) werden in Analogie zu Beispiel 3(aj) 23 mg (20%) eines gelblichen Pulvers erhalten.
IR (KBr): 3400, 3286, 1754, 1613, 1397, 1333, 1154 $cm^{-1}$
MS (ISP): $(M+Na)^+$ 448,3; $(M+H)^+$ 426,4; $(M\text{-}Na+H+NH_4)^+$ 421,4; $(M\text{-}Na+2H)^+$ 404,4

(ag) (1aS,3aR,6bR)-5-(5-Amino-1,3,4-thiadiazol-2-ylsulfanyl)-2-cyanacetyl-1-oxo-1a,2,3,3a,4,6b-hexahydro-1H-2,6a-diaza-cyclobut[cd]inden-6-carbonsäure-Natriumsalz

Ausgehend von (1aS,3aR,6bR)-5-(5-Amino-1,3,4-thiadiazol-2-ylsulfanyl)-1-oxo-1a,2,3,3a,4,6b-hexahydro-1H-2,6a-diaza-cyclobut[cd]inden-6-carbonsäure-trifluoracetat (300 mg; 0,68 mmol; aus Beispiel 2(c)) werden in Analogie zu Beispiel 3(ah) 26 mg (9%) eines weissen Pulvers erhalten.
IR (KBr): 2408, 2236, 1755, 1613, 1395 $cm^{-1}$
MS (ISP): $(M+Na)^+$ 437; $(M+H)^+$ 414

(ah) (1aS,3aR,6bR)-2-Acetyl-1-oxo-5-(pyridin-4-ylsulfanyl)-1a,2,3,3a,4,6b-hexahydro-1H-2,6a-diaza-cyclobut-[cd]inden-6-carbonsäure-Natriumsalz

Zu einer Lösung von (1aS,3aR,6bR)-1-Oxo-5-(pyridin-4-ylsulfanyl)-1a,2,3,3a,4,6b-hexahydro-1H-2,6a-diaza-cyclobut[cd]inden-6-carbonsäure-trifluoracetat (105 mg; 0,20 mmol; aus Beispiel 2(d)) in Acetonitril/Wasser 1:1 (5 ml) wird bei 0 °C Natriumhydrogencarbonat (67 mg; 0,80 mmol) und 2,5-dioxo-pyrrolidin-1-ylacetat (43 mg; 0,28 mmol) gegeben. Man lässt bei Raumtemperatur 3 Stunden rühren und engt dann ein. Der Rückstand wird in Wasser (20 ml) aufgenommen und mit Methylenchlorid (4x10 ml) extrahiert. Die wässrige Phase wird eingeengt. Der Rückstand wird in wenig Wasser gelöst und über ein hydrophobes Polymer chromatographiert (Laufmittel: Wasser/Acetonitril). Man erhält 35 mg (45%) eines gelblichen Pulvers.
IR (KBr): 1760, 1620, 1573, 1405, 807 $cm^{-1}$
MS (ISN): $(M\text{-}Na+NH_3)^-$ 361,4 (MS-Artefakt)

(ai) (1aS,3aR,6bR)-1-Oxo-5-(pyridin-4-ylsulfanyl)-2-trifluoracetyl-1a,2,3,3a,4,6b-hexahydro-1H-2,6a-diaza-cyclobut[cd]inden-6-carbonsäure-Natriumsalz

Ausgehend von (1aS,3aR,6bR)-1-Oxo-5-(pyridin-4-ylsulfanyl)-1a,2,3,3a,4,6b-hexahydro-1H-2,6a-diaza-cyclobut[cd]inden-6-carbonsäure-trifluoracetat (87 mg; 0,18 mmol; aus Beispiel 2(d)) werden in Analogie zu Beispiel 3(ad) 35 mg (47%) als gelbliches Pulver isoliert.
IR (KBr): 1766, 1692, 1618, 1399, 1208, 1179 $cm^{-1}$
MS (ISP): $(M\text{-}Na+2H)^+$ 400,4

(aj) (1aS,3aR,6bR)-2-Methylsulfonyl-1-oxo-5-(pyridin-4-ylsulfanyl)-1a,2,3,3a,4,6b-hexahydro-1H-2,6a-diaza-cyclobut[cd]inden-6-carbonsäure-Natriumsalz

Zu einer Suspension von (1aS,3aR,6bR)-1-Oxo-5-(pyridin-4-ylsulfanyl)-1a,2,3,3a,4,6b-hexahydro-1H-2,6a-diaza-cyclobut[cd]inden-6-carbonsäure-trifluoracetat (120 mg; 0,24 mmol; aus Beispiel 2(d)) in Methylenchlorid (5 ml) wird N-Methyl-N-trimethylsilyltrifluoracetamid (300 µl; 1,6 mmol) gegeben. Die Suspension wird 0,5 Stunden bei Raumtemperatur gerührt, wobei sämtlicher Feststoff in Lösung geht. Man versetzt weiter mit Natriumhydrogencarbonat (28 mg; 0,33 mmol) und Mesylchlorid (21 µl; 0,27 mmol), lässt 23 Stunden rühren und giesst dann auf Wasser (5 ml). Durch Zugabe von Natriumhydrogencarbont wird der pH der wässrigen Phase auf pH 7 eingestellt. Das Solvens wird abgezogen. Der Rückstand wird in wenig Wasser gelöst und über ein hydrophobes Polymer chromatographiert (Laufmittel: Wasser/Acetonitril). Man erhält 17 mg (17%) eines gelblichen Pulvers.
IR (KBr): 1756, 1616, 1577, 1398, 1333, 1153 $cm^{-1}$
MS (ISN): $(M\text{-}Na)^-$ 380,2

(ak) (1aS,3aR,6bR)-2-Acetyl-5-carbamoylmethylsulfanyl-1-oxo-1a,2,3,3a,4,6b-hexahydro-1H-2,6a-diaza-cyclobut[cd]inden-6-carbonsäure-Natriumsalz

Ausgehend von (1aS,3aR,6bR)-5-Carbamoylmethylsulfanyl-1-oxo-1a,2,3,3a,4,6b-hexahydro-1H-2,6a-diaza-cyclobut[cd]inden-6-carbonsäure-trifluoracetat (200 mg; 0,50 mmol; aus Beispiel 2(f)) werden in Analogie zu Beispiel 3(aa) 22 mg (13%) eines gelblichen Pulvers erhalten.
IR (KBr): 1752, 1673, 1614, 1394 $cm^{-1}$
MS (ISP): $(M\text{-}Na+2H)^+$ 326,2; $(M+H)^+$ 348,2

(al) (1aS,3aR,6bR)-5-Carbamoylmethylsulfanyl-1-oxo-2-trifluoracetyl-1a,2,3,3a,4,6b-hexahydro-1H-2,6a-diaza-cyclobut[cd]inden-6-carbonsäure-Natriumsalz

Ausgehend von (1aS,3aR,6bR)-5-Carbamoylmethylsulfanyl-1-oxo-1a,2,3,3a,4,6b-hexahydro-1H-2,6a-diaza-cyclobut[cd]inden-6-carbonsäure-trifluoracetat (120 mg; 0,30 mmol; aus Beispiel 2(f)) werden in Analogie zu Beispiel 3(ab) 12 mg (10%) als gelbliches Pulver isoliert.
IR (KBr): 3425, 1759, 1688, 1605, 1396, 1178 $cm^{-1}$
MS (ISP): $(M+H)^+$ 402,2; $(M\text{-}Na+H+NH_4)^+$ 397,2; $(M\text{-}Na+2H)^+$ 380,2

(am) (1aS,3aR,6bR)-5-Carbamoylmethylsulfanyl-2-methylsulfonyl-1-oxo-1a,2,3,3a,4,6b-hexahydro-1H-2,6a-diaza-cyclobut[cd]inden-6-carbonsäure-Natriumsalz

Ausgehend von (1aS,3aR,6bR)-5-Carbamoylmethylsulfanyl-1-oxo-1a,2,3,3a,4,6b-hexahydro-1H-2,6a-diaza-cyclobut[cd]inden-6-carbonsäure-trifluoracetat (112 mg; 0,28 mmol; aus Beispiel 2(f)) werden in Analogie zu Beispiel 3(aj) 17 mg (16%) eines weissen Pulvers erhalten.
IR (KBr): 3421, 1752, 1675, 1603, 1396, 1329, 1152 $cm^{-1}$
MS (ISN): $(M\text{-}Na+NH_3)^-$ 377,3 (MS-Artefakt)

(an) (1aS,3aR,6bR)-5-[(1,4-Dimethyl-1H-1,2,4-triazol-5-ylio)-methylsulfanyl]-1-oxo-2-trifluoracetyl-1a,2,3,3a,4,6b-hexahydro-1H-2,6a-diazacyclobut[cd]inden-6-carboxylat

Ausgehend von (1aS,3aR,6bR)-5-(6-Carboxy-1-oxo-1a,2,3,3a,4,6b-hexahydro-1H-2,6a-diaza-cyclobut-[cd]inden-5-ylsulfanylmethyl)-1,4-dimethyl-1H-1,2,4-triazol-4-ium-trifluormethansulfonat-trifluoracetat (150 mg; 0,25 mmol; aus Beispiel 2(g)) werden in Analogie zu Beispiel 3(ab) 48 mg (40%) als gelbliches Pulver isoliert.
IR (KBr): 1766, 1693, 1613, 1386, 1180 $cm^{-1}$
MS (ISP): $(M+H)^+$ 432,3

| Mikroanalyse: $C_{16}H_{16}N_5O_4F_3S \bullet 2.5\ H_2O$ | | | | | | |
|---|---|---|---|---|---|---|
| Ber.<br>Gef. | C 40,34<br>C 40,94 | H 4,44<br>H 4,53 | N 14,70<br>N 14,56 | F 11,96<br>F 10.60 | S 6,73<br>S 6,47 | Na 0,00<br>Na 0,12 |

(ao) (1aS,3aR,6bR)-2-Acetyl-5-[(1,4-dimethyl-1H-1,2,4-triazol-5-ylio)methylsulfanyl]-1-oxo-1a,2,3,3a,4,6b-hexahydro-1H-2,6a-diazacyclobut[cd]inden-6-carboxylat

Ausgehend von (1aS,3aR,6bR)-5-(6-Carboxy-1-oxo-1a,2,3,3a,4,6b-hexahydro-1H-2,6a-diaza-cyclobut-[cd]inden-5-ylsulfanylmethyl)-1,4-dimethyl-1H-1,2,4-triazol-4-ium-trifluormethansulfonat-trifluoracetat (150 mg; 0,25 mmol; aus Beispiel 2(g)) werden in Analogie zu Beispiel 3(aa) 23 mg (24%) eines gelblichen Pulvers erhalten.
IR (KBr): 1757, 1614, 1386 $cm^{-1}$
MS (ISP): $(M+H)^+$ 378,3

Beispiel 4

(1aS,3aR,6bR)-1-Oxo-5-[5-(pyridin-1-ylioacetylamino)-1,3,4-thiadiazol-2-ylsulfanyl]-1a,2,3,3a,4,6b-hexahydro-1H-2,6a-diazacyclobut[cd]inden-6-carboxylat-trifluoracetat-hydrobromid

Diese Verbindung wird in der gleichen Weise wie in Beispiel 2(a) angegeben ausgehend von (1aS,3aR,6bR)-1-[5-(2,6-bis-t-Butoxycarbonyl-1-oxo-1a,2,3,3a,4,6b-hexahydro-1H-2,6a-diazacyclobut[cd]-inden-5-ylsulfanyl)-1,3,4-thiadiazol-2-ylcarbamoylmethyl]-pyridiniumbromid (340 mg; 0,49 mmol) hergestellt. Ausbeute: 280 mg (81%) als beiger Feststoff.
IR (KBr): 2744, 1780, 1679, 1551, 1490, 1425, 1203 $cm^{-1}$
MS (ISP): $M^+$ 445,2
Die obige Ausgangsverbindung wird wie folgt hergestellt:

(1aS,3aR,6bR)-5-[5-(2-Brom-acetylamino)-1,3,4-thiadiazol-2-ylsulfanyl]-1-oxo-1a,2,3,3a,4,6b-hexahydro-1H-2,6a-diazacyclobut[cd]inden-2,6-dicarbonsäure-di-t-butylester

(1aS,3aR,6bR)-5-(5-Amino-1,3,4-thiadiazol-2-ylsulfanyl)-1-oxo-1a,2,3,3a,4,6b-hexahydro-1H-2,6a-diazacyclobut[cd]inden-2,6-dicarbonsäure-di-t-butylester (400 mg; 0,804 mmol; Beispiel 2c)) wird in abs. Methylenchlorid bei -20°C vorgelegt und mit Pyridin (0,078 ml; 0,965 mmol) und Bromessigsäurebromid (0,084 ml; 0,965 mmol) versetzt. Nach 30 Minuten bei -20°C wird das Reaktionsgemisch auf 1N wässrige Salzsäure (50 ml) und Eis (20 g) unter heftigem Rührem gegossen. Anschliessend wird mit Aethylacetat (2x100 ml) extrahiert. Die vereinigten organischen Phasen werden mit gesättigter Kochsalzlösung gewaschen, über Magnesiumsulfat getrocknet und eingeengt. Ausbeute: 470 mg (95%) als gelber Feststoff.
IR (KBr): 1771, 1700, 1660, 1544, 1246 $cm^{-1}$
MS (FAB): $(M+H)^+$ 604,1

(1aS,3aR,6bR)-1-[(5-(2,6-bis-t-Butoxycarbonyl-1-oxo-1a,2,3,3a,4,6b-hexahydro-1H-2,6a-diazacyclobut[cd]-inden-5-ylsulfanyl)-1,3,4-thiadiazol-2-ylcarbamoylmethyl]-pyridiniumbromid

(1aS,3aR,6bR)-5-[5-(2-Brom-acetylamino)-1,3,4-thiadiazol-2-ylsulfanyl]-1-oxo-1a,2,3,3a,4,6b-hexahydro-1H-2,6a-diazacyclobut[cd]inden-2,6-dicarbonsäure-di-t-butylester (470 mg; 0,78 mmol) wird in abs. Methylenchlorid (5 ml) gelöst und mit Pyridin (0,14 ml; 1,74 mmol) versetzt. Nach 5 Stunden bei Raumtemperatur wird die Lösung eingeengt, mit Aether verrührt und die entstehenden Kristalle abgenutscht. Ausbeute: 460 mg (86%) als beiger Feststoff Smp: >180°C.
IR (KBr): 1777, 1700, 1635, 1543 $cm^{-1}$

| Mikroanalyse: $C_{27}H_{33}N_6O_7S_2Br$ | | | |
|---|---|---|---|
| Ber. | C 46,49 | H 4,77 | N 12,05 |
| Gef. | C 46,64 | H 5,06 | N 11,96 |

Beispiel 5

(1aS,3aR,6bR)-2-(4-Hydroxy-phenylcarbamoyl)-1-oxo-5-[5-(pyridin-1-ylioacetylamino)-1,3,4-thiadiazol-2-ylsulfanyl]-1a,2,3,3a,4,6b-hexahydro-1H-2,6a-diazacyclobut[cd]inden-6-carbonsäure

Diese Verbindung wird in der gleichen Weise wie in Beispiel 2(a) angegeben aus (1aS,3aR,6bR)-1-Oxo-5-[5-(pyridin-1-ylioacetylamino)-1,3,4-thiadiazol-2-ylsulfanyl]-1a,2,3,3a,4,6b-hexahydro-1H-2,6a-diazacyclobut[cd]inden-6-carboxylat-trifluoracetat-hydrobromid (240 mg; 0,34 mmol; aus Beispiel 4) hergestellt. Ausbeute: 74 mg (37%) als beiger Feststoff.
IR (KBr): 3399, 1761, 1700, 1634, 1610, 1513, 1434, 1234 $cm^{-1}$
MS (EI): $(M+H)^+$ 580,0

Beispiel 6

(1aS,3aR,6bR)-2-t-Butoxycarbonyl-5-(1-methyl-1H-tetrazol-5-ylsulfanyl)-1-oxo-1a,2,3,3a,4,6b-hexahydro-1H-2,6a-diazacyclobut[cd]inden-6-carbonsäure

(1aS,3aR,6bR)-5-(1-Methyl-1H-tetrazol-5-ylsulfanyl)-1-oxo-1a,2,3,3a,4,6b-hexahydro-1H-2,6a-diazacyclobut[cd]inden-6-carbonsäure-trifluoracetat (100 mg; 0,22 mmol; aus Beispiel 2(a)) wird in Dioxan/Wasser (2 ml) gelöst, mit Natriumbicarbonat (41 mg, 0,49 mmol) und Di-t-butyldicarbonat (0,078 ml, 0,34 mmol) versetzt. Nach 2 Stunden wird Wasser addiert (2 ml) und mit Methylenchlorid gewaschen (3x5 ml). Die Wasserphase wird anschliessend über ein polymeres hydrophobes Gel mit Wasser chromatographiert und lyophilisiert. Ausbeute: 53 mg (56%) als farbloses Pulver.
IR (KBr): 1758, 1695, 1615, 1579, 1409, 1163 $cm^{-1}$
MS (ISN): $[(M-Na)^- + NH_3]$: 424,5 (MS-Artefact)

Das Produkt kann mit Trifluoressigsäure gemäss Beispiel 2(a) in (1aS,3aR,6bR)-5-(1-Methyl-1H-tetrazol-5-ylsulfanyl)-1-oxo-1a,2,3,3a,4,6b-hexahydro-1H-2,6a-diazacyclobut[cd]inden-6-carbonsäure-trifluoracetat übergeführt werden.

Beispiel 7

(Z)-(1aS,3aR,6bR)-2-[(2-Amino-thiazol-4-yl)-methoxyiminoacetyl]-1-oxo-5-(1-methyl-1H-tetrazol-5-ylsulfanyl)-1a,2,3,3a,4,6b-hexahydro-1H-2,6a-diazacyclobut[cd]inden-6-carbonsäure-Natriumsalz

(Z)-(1aS,3aR,6bR)-2-[(2-Amino-thiazol-4-yl)-methoxyiminoacetyl]-1-oxo-5-(1-methyl-1H-tetrazol-5-ylsulfanyl)-1a,2,3,3a,4,6b-hexahydro-1H-2,6a-diazacyclobut[cd]inden-6-carbonsäure-t-butylester (180 mg; 0,32 mmol) wird in Phenol/p-Cresol 1:1 (0,7 ml) gelöst und mit Trifluoressigsäure (0,5 ml) versetzt. Nach 3 Stunden bei Raumtemperatur wird die Trifluoressigsäure unter Vakuum entfernt und abs. Aether (10 ml) zugegeben. Die Suspension wird abgenutscht, der Feststoff mit Aether gewaschen (2x10 ml), in Wasser aufgenommen (2 ml) und der pH mit gesättigter wässriger Natriumbicarbonatlösung auf 6 eingestellt. Die erhaltene trübe Lösung wird über ein polymeres hydrophobes Gel mit Wasser chromatographiert; die reinen Fraktionen werden lyophilisiert. Ausbeute: 40 mg (25%) als farbloses Lyophilisat.
IR (KBr): 3426, 3197, 1764, 1622, 1534, 1392, 1048 $cm^{-1}$
MS (ISN): $[(M\text{-}Na)^- + NH_3]$ 507,2 (MS-Artefact)

Der als Ausgangsverbindung verwendete (Z)-(1aS,3aR,6bR)-2-[(2-Amino-thiazol-4-yl)-methoxyiminoacetyl]-1-oxo-5-(1-methyl-1H-tetrazol-5-ylsulfanyl)-1a,2,3,3a,4,6b-hexahydro-1H-2,6a-diazacyclobut[cd]inden-6-carbonsäure-t-butylester wird wie folgt hergestellt:

(1aS,3aR,6bR)-5-(1-Methyl-1H-tetrazol-5-ylsulfanyl)-1-oxo-1a,2,3,3a,4,6b-hexahydro-1H-2,6a-diazacyclobut-[cd]inden-6-carbonsäure-t-butylestertrifluoracetat

(1aS,3aR,6bR)-5-(1-Methyl-1H-tetrazol-5-ylsulfanyl)-1-oxo-1a,2,3,3a,4,6b-hexahydro-1H-2,6a-diazacyclobut[cd]inden-2,6-dicarbonsäure-di-t-butylester (580 mg; 1,24 mmol; aus Beispiel 2(a)) wird in mehreren Portionen zu bei -15 °C vorgekühlter Trifluoressigsäure (2 ml) gegeben (15 Minuten). Danach wird 2 Stunden bei -15 °C gerührt und anschliessend mit abs. Aether (20 ml) verdünnt und abgenutscht. Ausbeute: 510 mg (86%) als farbloser Feststoff. Smp. 157-159 °C (Aether).
IR (KBr): 1783, 1694, 1673, 1620, 1164 $cm^{-}$

| Mikroanalyse: $C_{17}H_{21}N_6O_5F_3S$ | | | |
|---|---|---|---|
| Ber. | C 42,68 | H 4,42 | N 17,57 |
| Gef. | C 42,61 | H 4,38 | N 17,54 |

(Z)-(1aS,3aR,6bR)-2-[(2-Amino-thiazol-4-yl)-methoxyiminoacetyl]-1-oxo-5-(1-methyl-1H-tetrazol-5-ylsulfanyl)-1a,2,3,3a,4,6b-hexahydro-1H-2,6a-diazacyclobut[cd]inden-6-carbonsäure-t-butylester

(1aS,3aR,6bR)-5-(1-Methyl-1H-tetrazol-5-ylsulfanyl)-1-oxo-1a,2,3,3a,4,6b-hexahydro-1H-2,6a-diazacyclobut[cd]inden-6-carbonsäure-di-t-butylestertrifluoracetat (190 mg; 0,397 mmol) wird in Acetonitril/Wasser 1:1 (8 ml) gelöst und mit S-(2-Benzotriazol)-2-amino-4-thiazolthioglyoxylat-(Z)-O-methyloxim (140 mg; 0,397 mmol) in DMF (2 ml) und Natriumbicarbonat (67 mg; 0,794 mmol) versetzt. Nach 3 Stunden bei Raumtemperatur wird das Acetonitril unter Vakuum entfernt und die erhaltene Suspension abgenutscht. Ausbeute: 180 mg (83%) als farbloser Feststoff Smp. >200 °C.
IR (KBr): 3359, 1784, 1721, 1655, 1615, 1533, 1260, 1044 $cm^{-1}$
MS (ISP): $(M + H^+)$ 548,3

Beispiel 8

(a) (1aS,3aR,6bR)-5-Methylsulfonyloxy-1-oxo-1a,2,3,3a,4,6b-hexahydro-1H-2,6a-diazacyclobut[cd]inden-6-carbonsäure-trifluoracetat

In Analogie zu Beispiel 2(a) werden ausgehend von (1aS,3aR,6bR)-5-Methylsulfonyloxy-1-oxo-1a,2,3,3a,4,6b-hexahydro-1H-2,6a-diazacyclobut[cd]inden-2,6-dicarbonsäure-di-t-butylester (780 mg; 1,98 mmol) 880 mg (98%) als farbloser Feststoff isoliert.
IR (KBr): 2662, 1783, 1720, 1680, 1610, 1357 $cm^{-1}$
MS (ISN): $(M\text{-}H)^-$ 287,0

Der als Ausgangsverbindung verwendete (1aS,3aR,6bR)-5-Methylsulfonyloxy-1-oxo-1a,2,3,3a,4,6b-hexahydro-1H-2,6a-diazacyclobut[cd]inden-2,6-dicarbonsäure-di-t-butylester wird wie folgt hergestellt:

(1aS,3aR,6bR)-5-Hydroxy-1-oxo-1a,2,3,3a,4,6b-hexahydro-2,6a-diazacyclobut[cd]inden-2,6-dicarbonsäure-di-t-butylester (360 mg; 1 mmol; aus Beispiel 1) wird in abs. Methylenchlorid (10 ml) vorgelegt, auf -78 °C abgekühlt und mit Methansulfochlorid (0,19 ml; 1,1 mmol) versetzt. Nach 1 Stunde bei dieser Temperatur wird das Reaktionsgemisch auf 1N wässrige Salzsäure (10 ml) gegossen und mit Aethylacetat (3x10 ml) extrahiert. Die vereinigten organischen Phasen werden nacheinander mit gesättigter wässriger Natriumbicarbonatlösung (10 ml) und gesättigter wässriger Kochsalzlösung gewaschen, dann über Magnesiumsulfat getrocknet und eingeengt. Der Rückstand wird mit n-Hexan (10 ml) verrührt und abgenutscht. Ausbeute: 340 mg (77%) als farbloser Feststoff. Smp. 130-133 °C.

| Mikroanalyse: $C_{19}H_{28}N_2O_8S$ 1:0,1 $C_6H_{14}$ | | | |
|---|---|---|---|
| Ber. | C 51,97 | H 6,54 | N 6,18 |
| Gef. | C 52,03 | H 6,41 | N 6,14 |

In analoger Weise wird hergestellt:

(b) (1aS,3aR,6bR)-5-(4-Methyl-phenylsulfonyloxy)-1-oxo-1a,2,3,3a,4,6b-hexahydro-1H-2,6a-diazacyclobut-[cd]inden-6-carbonsäure-trifluoracetat

Ausgehend von (1aS,3aR,6bR)-5-(4-Methyl-phenylsulfonyloxy)-1-oxo-1a,2,3,3a,4,6b-hexahydro-1H-2,6a-diazacyclobut[cd]inden-2,6-dicarbonsäure-di-t-butylester (230 mg; 0,44 mmol) werden 150 mg (86%) als beiger Feststoff erhalten.
IR (KBr): 1789, 1622, 1596, 1364, 1195 $cm^{-1}$
MS (ISP): $(M+H)^+$ 365,0

Der als Ausgangsverbindung verwendete (1aS,3aR,6bR)-5-(4-Methylphenylsulfonyloxy)-1-oxo-1a,2,3,3a,4,6b-hexahydro-1H-2,6a-diazacyclobut[cd]inden-2,6-dicarbonsäure-di-t-butylester wird ausgehend von (1aS,3aR,6bR)-5-Hydroxy-1-oxo-1a,2,3,3a,4,6b-hexahydro-2,6a-diazacyclobut[cd]inden-2,6-dicarbonsäure-di-t-butylester (750 mg; 2,04 mmol; aus Beispiel 1) hergestellt. Es werden 270 mg (25%) als leicht gelber Feststoff erhalten. Smp. 137-140 °C (Aether).

| Mikroanalyse: $C_{25}H_{32}N_2O_8S$ | | | |
|---|---|---|---|
| Ber. | C 57,68 | H 6,20 | N 5,38 |
| Gef. | C 57,86 | H 6,34 | N 5,22 |

Beispiel 9

In Analogie zu Beispiel 3(a) werden ausgehend von (1aS,3aR,6bR)-5-Methylsulfonyloxy-1-oxo-1a,2,3,3a,4,6b-hexahydro-1H-2,6a-diazacyclobut[cd]inden-6-carbonsäure-trifluoracetat (100 mg; 0,25 mmol) folgende Verbindungen hergestellt:

(a) (1aS,3aR,6bR)-2-(4-Hydroxy-phenylcarbamoyl)-5-methylsulfonyloxy-1-oxo-1a,2,3,3a,4,6b-hexahydro-1H-2,6a-diazacyclobut[cd]inden-6-carbonsäure-Natriumsalz. Ausbeute: 40 mg (36%) als farbloses Pulver.
IR (KBr): 3408, 3260, 1751, 1650, 1615, 1513, 1357, 1235, 1154, 833, 809 $cm^{-1}$

| Mikroanalyse: $C_{17}H_{16}N_3O_8SNa$ | | | |
|---|---|---|---|
| Ber. | C 45,85 | H 3,62 | N 9,43 |
| Gef. | C 45,62 | H 3,50 | N 9,50 |

(b) (1aS,3aR,6bR)-2-(4-Carbamoyl-phenylcarbamoyl)-5-methylsulfonyloxy-1-oxo-1a,2,3,3a,4,6b-hexahydro-1H-2,6a-diazacyclobut[cd]inden-6-carbonsäure-Natriumsalz. Ausbeute: 44% als farbloser Feststoff.
IR (KBr): 3434, 3240, 1757, 1657, 1615, 1525, 1412, 1325, 1185 $cm^{-1}$

| Mikroanalyse: $C_{18}H_{17}N_4O_8SNa$ | | | |
|---|---|---|---|
| Ber. | C 45,77 | H 3,63 | N 11,86 |
| Gef. | C 45,65 | H 3,41 | N 11,96 |

(c) (1aS,3aR,6bR)-5-Methylsulfonyloxy-2-(thien-2-ylmethylcarbamoyl)-1-oxo-1a,2,3,3a,4,6b-hexahydro-1H-2,6a-diazacyclobut[cd]inden-6-carbonsäure-Natriumsalz. Ausbeute: 20% als brauner Feststoff.
IR (KBr): 3431, 3280, 1764, 1705, 1629, 1530 $cm^{-1}$
MS (ISN): $(M\text{-}Na)^-$ 426,3
(d) (1aS,3aR,6bR)-2-(3,4-Dihydroxy-benzylcarbamoyl)-5-methylsulfonyloxy-1-oxo-1a,2,3,3a,4,6b-hexahydro-1H-2,6a-diazacyclobut[cd]inden-6-carbonsäure-Natriumsalz. Ausbeute: 23% als farbloser Feststoff.
IR (KBr): 3425, 1758, 1620, 1530, 1396, 1330, 1154 $cm^{-1}$
MS (ISN): $(M\text{-}Na)^-$ 452,2
(e) (1aS,3aR,6bR)-5-Methylsulfonyloxy-1-oxo-2-[(S)-2-oxo-pyrrolidin-3-ylcarbamoyl]-1a,2,3,3a,4,6b-hexahydro-1H-2,6a-diazacyclobut[cd]inden-6-carbonsäure-Natriumsalz. Ausbeute: 12% als farbloser Feststoff.
IR (KBr): 3412, 1762, 1702, 1622, 1538, 1395, 1352, 1153 $cm^{-1}$
MS (ISN): $(M\text{-}Na)^-$ 413,1
(f) (1aS,3aR,6bR)-5-Methylsulfonyloxy-1-oxo-2-[(R) und -[(S)-2-oxo-tetrahydro-thien-3-ylcarbamoyl]-1a,2,3,3a,4,6b-hexahydro-1H-2,6a-diazacyclobut[cd]inden-6-carbonsäure-Natriumsalz. Ausbeute: 18% als farbloser Feststoff.
IR (KBr): 3412, 3300, 1765, 1699, 1644, 1534, 1154 $cm^{-1}$
MS (ISN): $[(M\text{-}Na)^- + NH_3]$ 447,3 (MS-Artefact)
(g) (1aS,3aR,6bR)-2-[(R)- und [(S)-1,1-Dioxo-tetrahydrothien-3-ylcarbamoyl]-5-methylsulfonyloxy-1-oxo-1a,2,3,3a,4,6b-hexahydro-1H-2,6a-diazacyclobut[cd]inden-6-carbonsäure-Natriumsalz. Ausbeute: 15% als farbloser Feststoff.
IR (KBr): 3280, 1768, 1716, 1644, 1536, 1303, 1119 $cm^{-1}$
MS (ISN): $[(M\text{-}H)^- + NH_3]$ 465,1 (MS-Artefact)
(h) (1aS,3aR,6bR)-5-Methylsulfonyloxy-1-oxo-2-(4-sulfamoylbenzylcarbamoyl)-1a,2,3,3a,4,6b-hexahydro-1H-2,6a-diazacyclobut[cd]inden-6-carbonsäure-Natriumsalz. Ausbeute: 13% als farbloser Feststoff.
IR (KBr): 3350, 1762, 1644, 1323, 1300, 1160 $cm^{-1}$
MS (ISN): $(M\text{-}Na)^-$ 499,3

In Analogie zu Beispiel 3(a) wird ebenfalls ausgehend von (1aS,3aR,6bR)-5-(4-Methyl-phenylsulfonyloxy)-1-oxo-1a,2,3,3a,4,6b-hexahydro-1H-2,6a-diazacyclobut[cd]inden-6-carbonsäure-trifluoracetat hergestellt:
(i) (1aS,3aR,6bR)-2-(4-Hydroxy-phenylcarbamoyl)-5-(4-methyl-phenylsulfonyloxy)-1-oxo-1a,2,3,3a,4,6b-hexahydro-1H-2,6a-diazacyclobut[cd]inden-6-carbonsäure-Natriumsalz. Ausbeute: 56% als farbloser Feststoff.
IR (KBr): 3421, 1760, 1619, 1400, 1235 $cm^{-1}$
MS (ISN): $(M\text{-}Na)^-$ 498,4

Beispiel 10

(a) (1aS,3aR,6bR)-2-t-Butoxycarbonyl-5-methylsulfonyloxy-1-oxo-1a,2,3,3a,4,6b-hexahydro-1H-2,6a-diazacyclobut[cd]inden-6-carbonsäure-Natriumsalz

(1aS,3aR,6bR)-5-Methylsulfonyloxy-1-oxo-1a,2,3,3a,4,6b-hexahydro-1H-2,6a-diazacyclobut[cd]inden-6-carbonsäure-trifluoracetat (62 mg; 0,15 mmol); aus Beispiel 8(a)) wird in Dioxan/Wasser 1:1 (2 ml) gelöst und mit Natriumbicarbonat (26 mg; 0,31 mmol) und Di-t-butyldicarbonat (0,053 ml; 0,23 mmol) versetzt. Nach 2 Stunden wird Wasser (2 ml) zugegeben und das Gemisch mit Methylenchlorid (3x5 ml) gewaschen. Der pH-Wert der Wasserphase wird mit 1N wässriger Salzsäure auf 2 eingestellt; anschliessend wird das Gemisch mit Aethylacetat extrahiert (2x10 ml). Die Aethylacetat-Phasen werden über Magnesiumsulfat getrocknet und eingeengt. Der Rückstand wird in Aethylacetat (0,2 ml) gelöst, mit einer 2N Natriumäthylcapronatlösung in Aethylacetat (0,07 ml; 0,14 mmol) versetzt, mit Aether (5 ml) verdünnt und abgenutscht. Ausbeute: 43 mg (68%) als farbloser Feststoff. Smp. 164-172°C.
IR (KBr): 1765, 1699, 1618, 1406, 1364, 1156 $cm^{-1}$

| Mikroanalyse: $C_{15}H_{19}N_2O_8SNa$ | | | |
|---|---|---|---|
| Ber.<br>Gef. | C 43,90<br>C 43,56 | H 4,67<br>H 4,95 | N 6,83<br>N 6,53 |

Das Produkt kann mit Trifluoressigsäure gemäss Beispiel 2(a) in (1aS,3aR,6bR)-5-Methylsulfonyloxy-1-oxo-1a,2,3,3a,4,6b-hexahydro-1H-2,6a-diazacyclobut[cd]inden-6-carbonsäure-trifluoracetat übergeführt werden.

In analoger Weise werden hergestellt:

(b) (1aS,3aR,6bR)-2-Acetyl-5-methylsulfonyloxy-1-oxo-1a,2,3,3a,4,6b-hexahydro-1H-2,6a-diazacyclobut-[cd]inden-6-carbonsäure

(1aS,3aR,6bR)-5-Methylsulfonyloxy-1-oxo-1a,2,3,3a,4,6b-hexahydro-1H-2,6a-diazacyclobut[cd]inden-6-carbonsäure-trifluoracetat (100 mg; 0,25 mmol; aus Beispiel 8(a)) wird in Essigsäure (1 ml) gelöst und mit Essigsäureanhydrid (0,12 ml) bei Raumtemperatur versetzt. Nach 30 Minuten wird die Lösung eingeengt und über ein polymeres hydrophobes Gel mit Wasser/Acetonitril chromatographiert. Ausbeute: 35 mg (43%) als farbloses Pulver.

IR (KBr): 2550, 1772, 1727, 1646, 1360 $cm^{-1}$

MS (ISN): $(M-H+NH_3)^-$ 346,2 (MS-Artefact)

(c) (1aS,3aR,6bR)-2-Formyl-5-methylsulfonyloxy-1-oxo-1a,2,3,3a,4,6-hexahydro-1H-2,6a-diazacyclobut-[cd]inden-6-carbonsäure

(1aS,3aR,6bR)-5-Methylsulfonyloxy-1-oxo-1a,2,3,3a,4,6b-hexahydro-1H-2,6a-diazacyclobut[cd]inden-6-carbonsäure-trifluoracetat (250 mg; 0,62 mmol; aus Beispiel 8(a)) wird in Chloroform (12 ml) und Acetonitril (2 ml) suspendiert und mit Ameisensäure-pentafluorphenylester (395 mg; 1,86 mmol) und Natriumbicarbonat (104 mg; 1,24 mmol) versetzt. Nach 2 Stunden bei Raumtemperatur wird die Suspension eingeengt, mit Aether (12 ml) verrührt und abgenutscht. Der erhaltene beige Feststoff wird in Wasser (2 ml) gelöst und über ein polymeres hydrophobes Gel mit Wasser/Acetonitril chromatographiert. Ausbeute: 67 mg (32%) als beiges Pulver.

IR (KBr): 1761, 1658, 1618, 1395, 1354, 1153 $cm^{-1}$

MS (ISN): $[(M-Na^+ + NH_3]$ 332,2

In Analogie hierzu wird ausgehend von (1aS,3aR,6bR)-5-(4-Methylphenylsulfonyloxy)-1-oxo-1a,2,3,3a,4,6b-hexahydro-1H-2,6a-diazacyclobut[cd]inden-6-carbonsäure-trifluoracetat (aus Beispiel 8(b)) hergestellt:

(d) (1aS,3aR,6bR)-2-t-Butoxycarbonyl-5-(4-methyl-phenylsulfonyloxy)-1-oxo-1a,2,3,3a,4,6b-hexahydro-1H-2,6a-diazacyclobut[cd]inden-6-carbonsäure-Natriumsalz. Ausbeute: 57% als farbloser Feststoff. Smp. 153-166 °C (Zers.).

IR (KBr): 1766, 1700, 1621, 1403, 1366, 1160 $cm^{-1}$

| Mikroanalyse: $C_{21}H_{23}N_2O_8SNa$ | | | |
|---|---|---|---|
| Ber.<br>Gef. | C 51,85<br>C 51,83 | H 4,77<br>H 5,05 | N 5,76<br>N 6,01 |

Das Produkt kann mit Trifluoressigsäure gemäss Beispiel 2(a) in (1aS,3aR,6bR)-5-(4-Methyl-phenylsulfonyloxy)-1-oxo-1a,2,3,3a,4,6b-hexahydro-1H-2,6a-diazacyclobut[cd]inden-6-carbonsäure-trifluoracetat übergeführt werden.

Beispiel 11

(a) (1aS,3aR,6bR)-2-(3-Carbamoyl-pyridin-1-ylioacetyl)-5-methylsulfonyloxy-1-oxo-1a,2,3,3a,4,6b-hexahydro-1H-2,6a-diazacyclobut[cd]inden-6-carboxylat

(1aS,3aR,6bR)-5-Methylsulfonyloxy-1-oxo-1a,2,3,3a,4,6b-hexahydro-1H-2,6a-diazacyclobut[cd]inden-6-carbonsäure-trifluoracetat (500 mg; 1,24 mmol; aus Beispiel 8(a)) wird in abs. Methylenchlorid (10 ml) suspendiert und mit N-Methyl-N-trimethylsilyltrifluoracetamid (0,53 ml) versetzt. Nach 10 Minuten bei Raumtemperatur wird die erhaltene Lösung auf -20 °C gekühlt, mit Pyridin (0,18 ml; 2,2 mmol) und anschliessend mit Bromessigsäurebromid (0,14 ml; 1,6 mmol) versetzt. Das Reaktionsgemisch wird noch zusätzlich 1 Stunde bei 0 °C gerührt, mit Wasser (25 ml) verdünnt und mit Aethylacetat (3x100 ml)

extrahiert. Die vereinigten organischen Phasen werden mit gesättigter wässriger Kochsalzlösung (25 ml) gewaschen, über Magnesiumsulfat getrocknet und eingeengt. Der Rückstand wird mit n-Hexan (20 ml) verrührt und abgenutscht. Man erhält 390 mg (66%) (1aS,3aR,6bR)-2-Bromacetyl-5-methylsulfonyloxy-1-oxo-1a,2,3,3a,4,6a-hexahydro-1H-2,6a-diazacyclobut[cd]inden-6-carbonsäure als farbloser Feststoff.
IR (KBr): 2800, 1778, 1727, 1657, 1350, 1230, 1156 $cm^{-1}$
MS (ISN): M-H)$^-$ 407

(1aS,3aR,6bR)-2-Bromacetyl-5-methylsulfonyloxy-1-oxo-1a,2,3,3a,4,6a-hexahydro-1H-2,6a-diazacyclobut[cd]inden-6-carbonsäure (300 mg; 0,73 mmol) wird in DMF (12 ml) gelöst und mit Nicotinsäureamid (180 mg; 1,47 mmol) versetzt. Nach 20 Stunden bei Raumtemperatur wird die Lösung eingeengt. Der Rückstand wird in Wasser (2 ml) gelöst, und über ein polymeres hydrophobes Gel mit Wasser chromatographiert. Ausbeute: 40 mg (12%) als farbloses Pulver.
IR (KBr): 1764, 1669, 1616, 1506, 1394, 1347, 1153 $cm^{-1}$
MS (ISP): $(M+H^+)$ 451,4

In Analogie hierzu wird hergestellt:

(b) (1aS,3aR,6bR)-5-Methylsulfonyloxy-2-(1-methyl-1H-tetrazol-5-ylsulfanylacetyl)-1-oxo-1a,2,3,3a,4,6b-hexahydro-1H-2,6a-diazacyclobut[cd]inden-6-carbonsäure-Natriumsalz

Ausgehend von (1aS,3aR,6bR)-2-Bromacetyl-5-methylsulfanyloxy-1-oxo-1a,2,3,3a,4,6b-hexahydro-1H-2,6a-diazacyclobut[cd]inden-6-carbonsäure (200 mg; 0,49 mmol) werden 42 mg (18%) als farbloses Lyophilisat erhalten.
IR (KBr): 1761, 1649, 1619, 1398, 1352, 1154 $cm^{-1}$
MS (ISN): $[(M-H)^- + NH_3]$: 460,4 (MS-Artefact)

Beispiel 12

(a) (1aS,3aR,6bR)-5-Carboxymethyl-1-oxo-1a,2,3,3a,4,6b-hexabydro-1H-2,6a-diazacyclobut[cd]inden-6-carbonsäure-trifluoracetat

In der gleichen Weise wie in Beispiel 2(a) angegeben werden ausgehend von (1aS,3aR,6bR)-(2,6-bis-t-Butoxycarbonyl-1-oxo-1a,2,3,3a,4,6,b-hexahydro-1H-2,6a-diazacyclobut[cd]inden-5-yl)-essigsäure (250 mg; 0,6 mmol) 180 mg (81%) als farbloser Feststoff erhalten.
IR (KBr): 2700, 1778, 1711, 1197 $cm^{-1}$
MS (ISN): $(M-H)^-$ 251,2

Das dabei verwendete Ausgangsmaterial wird wie folgt hergestellt:

(1aS,3aR,6bR)-5-Benzyloxycarbonylmethyl-1-oxo-1a,2,3,3a,4,6b-hexahydro-1H-2,6a-diazacyclobut[cd]inden-2,6-dicarbonsäure-di-t-butylester

(1aS,3aR,6bR)-5-Hydroxy-1-oxo-1a,2,3,3a,4,6b-hexahydro-2,6a-diazacyclobut[cd]inden-2,6-dicarbonsäure-di-t-butylester (500 mg; 1,36 mmol; aus Beispiel 1) wird in 1,2-Dichloräthan (50 ml) vorgelegt und mit Benzyloxycarboxymethylentriphenylphosphoran (840 mg; 2,05 mmol) während 48 Stunden unter Rückflussbedingungen erhitzt. Das Reaktionsgemisch wird anschliessend auf 1N wässrige Salzsäure (50 ml) und Eis (50 g) unter heftigem Rühren gegossen und mit Aethylacetat extrahiert. Die vereinigten organischen Phasen werden mit gesättigter wässriger Kochsalzlösung (100 ml) gewaschen, über Magnesiumsulfat getrocknet und eingeengt. Der Rückstand wird über Kieselgel (50 g, 0,040-0,063 mm Korngrösse) mit Aethylacetat/n-Hexan 3:7 chromatographiert. Ausbeute: 480 mg (71%) als farbloser Feststoff.
IR (KBr): 1763, 1725, 1710, 1695 $cm^{-1}$
MS (ISP): $(M+H)^+$ 499,2

(1aS,3aR,6bR)-(2,6-bis-t-Butoxycarbonyl-1-oxo-1a,2,3,3a,4,6b-hexahydro-1H-2,6a-diazacyclobut[cd]inden-5-yl)-essigsäure

(1aS,3aR,6bR)-5-Benzyloxycarbonylmethyl-1-oxo-1a,2,3,3a,4,6b-hexahydro-1H-2,6a-diazacyclobut[cd]-inden-2,6-dicarbonsäure-di-t-butylester (300 mg; 0,60 mmol) wird über 10% Pd/C (50 mg) in Methanol hydriert. Die Suspension wird anschliessend abgenutscht und eingeengt. Ausbeute: 220 mg (90%) als farbloser Feststoff.
IR (KBr): 2700, 1770, 1731, 1703 $cm^{-1}$

MS (ISN): $(M-H)^-$ 407,3

In analoger Weise wird hergestellt:

(b) (1aS,3aR,6bR)-5-Methoxycarbonylmethyl-1-oxo-1a,2,3,3a,4,6b-hexahydro-1H-2,6a-diazacyclobut[cd]-inden-6-carbonsäure-trifluoracetat

Ausgehend von (1aS,3aR,6bR)-5-Methoxycarbonylmethyl-1-oxo-1a,2,3,3a,4,6b-hexahydro-1H-2,6a-diazacyclobut[cd]inden-2,6-dicarbonsäure-di-t-butylester (300 mg; 0,70 mmol) werden 240 mg (91%) als beiger Feststoff erhalten.

IR (KBr): 1779, 1732, 1678, 1640, 1202 $cm^{-1}$

MS (ISN): $(M+H)^+$ 267,3

Die Ausgangsverbindung wird ausgehend von (1aS,3aR,6bR)-5-Hydroxy-1-oxo-1a,2,3,3a,4,6b-hexahydro-2,6a-diazacyclobut[cd]inden-2,6-dicarbonsäure-di-t-butylester (500 mg; 1,36 mmol; aus Beispiel 1) erhalten. 260 mg (45%) als farbloser Feststoff.

IR (KBr): 1765, 1735, 1704, 1638, 1252, 1164 $cm^{-1}$

MS (ISP): $(M+H)^+$ 423,4

Beispiel 13

(a) (1aS,3aR,6bR)-5-Carboxymethyl-2-(4-hydroxy-phenylcarbamoyl)-1-oxo-1a,2,3,3a,4,6b-hexahydro-1H-2,6a-diazacyclobut[cd]inden-6-carbonsäure-Natriumsalz

Diese Verbindung wird wie in Beispiel 3(a) angegeben ausgehend von (1aS,3aR,6bR)-5-Carboxymethyl-1-oxo-1a,2,3,3a,4,6b-hexahydro-1H-2,6a-diazacyclobut[cd]inden-6-carbonsäure-trifluoracetat (120 mg; 0,36 mmol; aus Beispiel 12(a) hergestellt. Ausbeute: 45 mg (31%) als farbloses Pulver.

IR (KBr): 1735, 1635, 1589, 1378 $cm^{-1}$

| Mikroanalyse: $C_{18}H_{15}N_3O_7Na$ | | | |
|---|---|---|---|
| Ber.<br>Gef. | C 52,95<br>C 53,33 | H 3,70<br>H 3,73 | N 10,29<br>N 10,19 |

In Analogie hierzu wird hergestellt:

(b) (1aS,3aR,6bR)-2-(4-Hydroxy-phenylcarbamoyl)-5-methoxycarbonylmethyl-1-oxo-1a,2,3,3a,4,6b-hexahydro-1H-2,6a-diazacyclobut[cd]inden-6-carbonsäure-Natriumsalz

In Analogie zu Beispiel 3(a) wird ausgehend von (1aS,3aR,6bR)-5-Methoxycarbonylmethyl-1-oxo-1a,2,3,3a,4,6b-hexahydro-1H-2,6a-diazacyclobut[cd]inden-6-carbonsäure-trifluoracetat (200 mg; 0,55 mmol; aus Beispiel 12(b)) 82 mg (35%) als farbloses Pulver erhalten.

IR (KBr): 1736, 1638, 1610, 1540, 1513 $cm^{-1}$

MS (ISN): $(M-H)^-$ 400,3

Beispiel 14

(1aS,3aR,6bR)-5-Hydroxymethyl-1-oxo-1a,2,3,3a,4,6b-hexahydro-1H-2,6a-diazacyclobut[cd]inden-2,6-dicarbonsäure-di-t-butylester (5-Hydroxymethyl "Baustein")

Diese Verbindung kann durch folgende Reaktionssequenz a)-f) erhalten werden:

a) Gemisch von (E)- und (Z)-(1S,5R)-6-(3,4-Dimethoxybenzyl)-7-oxo-4-[2-oxo-3-(2-trimethylsilanyl-äthoxy)-propyliden]-2,6-diazabicyclo[3.2.0]heptan-2-carbonsäure-benzylester

Benzyl (1S,5S)-6-(3,4-dimethoxybenzyl)-4,7-dioxo-2,6-diazabicyclo[3.2.0]heptan-2-carboxylat (49 g; 119,4 mmol; aus Beispiel 1) wird in abs. Methylenchlorid (250 ml) vorgelegt und mit 1-[2-(Trimethyl-silanyl)-äthoxy]-3-triphenylphosphoranyliden-propan-2-on (51,9 g; 119,4 mmol) in abs. Methylenchlorid (125 ml) tropfenweise (40 Minuten) versetzt. Nach 2,5 Stunden bei Raumtemperatur wird das Reaktionsgemisch auf 1N wässrige Salzsäure (650 ml) gegossen und mit Methylenchlorid (2x300 ml) extrahiert. Die vereinigten

organischen Phasen werden mit Wasser (3x500 ml) und gesättigter wässriger Kochsalzlösung (500 ml) gewaschen, über Magnesiumsulfat getrocknet und eingeengt. Der Rückstand wird über Kieselgel (1,7 kg, 0,040-0,063 mm Korngrösse) mit Aethylacetat/n-Hexan 7:3 chromatographiert. Ausbeute: 51,4 g (76%) als farbloses Oel.
IR (Film): 2840, 1763, 1711 $cm^{-1}$
MS (ISP): $(M+H)^+$ 567,5

b) (1S,4R,5R)-6-(3,4-Dimethoxybenzyl)-7-oxo-4-[2-oxo-3-(2-trimethylsilanyläthoxy)-propyl]-2,6-diazabicyclo-[3.2.0]heptan-2-carbonsäure-t-butylester

Das oben hergestellte Gemisch von (E)- und (Z)-(1S,5R)-6-(3,4-Dimethoxybenzyl)-7-oxo-4-[2-oxo-3-(2-trimethylsilanyl-äthoxy)-propyliden]-2,6-diazabicyclo[3.2.0]heptan-2-carbonsäure-benzylester (51,4 g; 90,7 mmol) wird in Methanol (2 l) vorgelegt, mit Di-t-butyl-dicarbonat (29,7 ml; 136 mmol) versetzt und über Pd/C (15 g) hydriert. Nach 15 Stunden wird das Reaktionsgemisch abgenutscht, eingeengt und über Kieselgel (1 kg, 0,040-0,063 mm Korngrösse) mit Aethylacetat/n-Hexan 1:1 chromatographiert. Ausbeute: 25,7 g (53%) als farbloser Schaum.
IR (Film): 1760, 1699, 1591, 1517, 1160, 887, 765 $cm^{-1}$
MS (ISP: $(M+H)^+$ 535,4

| Mikroanalyse: $C_{27}H_{42}N_2O_7Si$ | | | |
|---|---|---|---|
| Ber. | C 60,65 | H 7,92 | N 5,24 |
| Gef. | C 60,48 | H 8,27 | N 4,91 |

c) (1S,4R,5R)-7-Oxo-4-[2-oxo-3-(2-trimethylsilanyl-äthoxy)-propyl]-2,6-diazabicyclo[3.2.0]heptan-2-carbonsäure-t-butylester

Diese Verbindung wird in Analogie zu Beispiel 1g) ausgehend von (1S,4R,5R)-6-(3,4-Dimethoxybenzyl)-7-oxo-4-[2-oxo-3-(2-trimethylsilanyl-äthoxy)-propyl]-2,6-diazabicyclo[3.2.0]heptan-2-carbonsäure-t-butylester (25,7 g; 48 mmol) hergestellt. Ausbeute: 12,7 g (69%) als farbloser Feststoff. Smp. 89-91 °C (Aethylacetat).
IR (KBr). 3294, 1784, 1729, 1696, 1514, 1250 $cm^{-1}$

| Mikroanalyse: $C_{18}H_{32}N_2O_5Si$ | | | |
|---|---|---|---|
| Ber. | C 56,22 | H 8,39 | N 7,28 |
| Gef. | C 55,93 | H 8,22 | N 7,00 |

d) (1aS,3aR,6bR)-5-(2-Trimethylsilanyl-äthoxymethyl)-1-oxo-1a,2,3,3a,4,6b-hexahydro-1H-2,6a-diazacyclobut[cd]inden-2,6-dicarbonsäure-di-t-butylester

(1S,4R,5R)-7-Oxo-4-[2-oxo-3-(2-trimethylsilanyl-äthoxy)-propyl]-2,6-diazabicyclo[3.2.0]heptan-2-carbonsäure-t-butylester (12,7 g; 33 mmol) und Methyl-diisopropylamin (7,0 ml; 39,6 mmol) werden in abs. Methylenchlorid (90 ml) auf -5 °C vorgekühlt und zu einer Suspension von Calciumcarbonat (13,1 g; 131 mmol) und t-Butyl-oxalsäurechlorid (6 ml; 39,6 mmol) in abs. Methylenchlorid (30 ml) unter Eisbadkühlung gegeben. Nach 2 Stunden bei 0 °C wird die Suspension mit äthanolfreiem Chloroform (120 ml) verdünnt und über Kieselgel (70 g; 0,040-0,063 mm Korngrösse) filtriert. Anschliessend wird die Säule mit Chloroform (120 ml) nachgespült. Die vereinigten organischen Phasen werden mit abs. Toluol (900 ml) verdünnt, mit Triäthylphosphit (11,5 ml; 66 mmol) bei Raumtemperatur versetzt und 15 Stunden unter Rückflussbedingungen erhitzt. Die erhaltene Lösung wird eingeengt. Der Rückstand wird in Aethylacetat (1200 ml) gelöst, nacheinander mit Wasser (600 ml) und gesättigter wässriger Kochsalzlösung (600 ml) gewaschen und über Magnesiumsulfat getrocknet. Nach Einengen wird der Rückstand über Kieselgel (600 g; 0,040-0,063 mm Korngrösse) mit n-Hexan/Aceton 9:1 chromatographiert. Ausbeute: 8,7 g (55%) als farbloser Feststoff.
IR (KBr): 1764, 1706, 1248, 836, 776 $cm^{-1}$
MS (ISP): $(M+H)^+$ 481,6

e) (1aS,3aR,6bR)-5-Hydroxymethyl-1-oxo-1a,2,3,3a,4,6b-hexahydro-1H-2,6a-diazacyclobut[cd]inden-6-carbonsäure-t-butylester.

(1aS,3aR,6bR)-5-(2-Trimethylsilanyl-äthoxymethyl)-1-oxo-1a,2,3,3a,4,6b-hexahydro-1H-2,6a-diazacyclobut[cd]inden-2,6-dicarbonsäure-di-t-butylester (8,7 g; 18,1 mmol) wird in Methylenchlorid (30 ml) gelöst und unter heftigem Rühren zu auf -20°C vorgekühlter Trifluoressigsäure (80 ml) getropft (die Temperatur wird zwischen -18 und -20°C gehalten). Nach 3 Stunden bei -20°C wird das Reaktionsgemisch bei derselben Temperatur eingeengt, mit abs. Aether (670 ml) versetzt und abgenutscht. Ausbeute: 5,3 g (74%) als beiger Feststoff.
IR (KBr). 3426, 1773, 1710, 1670, 1180, 1077 $cm^{-1}$
MS (ISP): $(M+H)^+$ 281,2

f) (1aS,3aR,6bR)-5-Hydroxymethyl-1-oxo-1a,2,3,3a,4,6b-hexahydro-1H-2,6a-diazacyclobut[cd]inden-2,6-dicarbonsäure-di-t-butylester

(1aS,3aR,6bR)-5-Hydroxymethyl-1-oxo-1a,2,3,3a,4,6b-hexahydro-1H-2,6a-diazacyclobut[cd]inden-6-carbonsäure-t-butylester (5,3 g; 13,4 mmol) wird in Dioxan/Wasser 1:1 (150 ml) vorgelegt, mit Natriumbicarbonat (2,2 g; 26,7 mmol), und Di-t-butyl-dicarbonat (3,7 ml; 16 mmol) bei Raumtemperatur versetzt. Nach 1 Stunde wird das Reaktionsgemisch auf gesättigte wässrige Kochsalzlösung (150 ml) gegossen, mit Aethylacetat extrahiert (3x150 ml), über Magnesiumsulfat getrocknet und eingeengt. Der Rückstand wird über Kieselgel (150 g, 0,040-0,063 mm Korngrösse) mit Aethylacetat chromatographiert. Ausbeute: 3,2 g (63%) als farbloser Feststoff. Smp. 175°C.
IR (KBr). 1761, 1705, 1631, 1253, 1161, 1117, 1087 $cm^{-1}$
MS (ISP): $(M+H)^+$ 381,4

Der 5-Hydroxymethyl "Baustein" kann auch nach der folgenden, verbesserten Methode (Reaktionssequenz a1) - g1)) erhalten werden:

a1) (t-Butyl-dimethyl-silanyloxy)-essigsäure-n-butyl ester

n-Butylglycolat (231 g; 1,75 Mol) und Imidazol (345,1 g; 5,07 Mol) werden bei 0°C vorgelegt. Die erhaltene Suspension wird mit t-Butyldimethylchlorsilan (303 g; 2,01 Mol) portionsweise während 1,5 Stunden versetzt. Nach 20 Stunden bei Raumtemperatur wird das Reaktionsgemisch mit Aether/n-Hexan 1:1 (1 l) verdünnt und abgenutscht. Die Kristalle werden mit Aether/n-Hexan 1:1 (200 ml) gründlich nachgewaschen. Das Filtrat wird nacheinander mit Wasser (2x700 ml) und gesättigter wässriger Kochsalzlösung (500 ml) gewaschen, über Magnesiumsulfat getrocknet und eingeengt. Das erhaltene Öl wird über eine Vigreuxkolonne (7,5 cm) destilliert. Ausbeute: 405 g (94%) als farbloses Öl (Sdp 78°C/0,98 mmHg).
IR (Film): 1760, 1225, 1206, 1148, 838, 780 $cm^{-1}$
MS (EI): $(M+H)^+$ 247

b1) [3-(t-Butyl-dimethyl-silanyloxy)-propyl]-phosphonsäure-dimethylester

Methanphosphonsäure-dimethylester (70 ml; 634,8 mmol) wird in Tetrahydrofuran (1,6 l) bei -75°C vorgelegt und mit 1,6M n-Butyllithium in Tetrahydrofuran (437 ml; 700 mmol) bei dieser Temperatur versetzt. Nach 1,5 Stunden bei -75°C wird (t-Butyl-dimethyl-silanyloxy)-essigsäure-n-butylester (52,1 g; 211,6 mmol) in Tetrahydrofuran (110 ml) zugegeben und das Ganze 2 Stunden bei -30°C gerührt. Das Reaktionsgemisch wird anschliessend auf 1N eiskalte wässrige Salzsäure (800 ml) gegossen und rasch mit Essigester (2x1 l) extrahiert. Die vereinten organischen Phasen werden nacheinander mit Wasser (2x1 l) und gesättigter wässriger Kochsalzlösung (500 ml) gewaschen, über Magnesiumsulfat getrocknet und eingeengt. Der Rückstand wird mit Toluol (2x300 ml) azeotropiert und destilliert (Sdp: 89-95°C; 0,42 mmHg). Ausbeute: 57,2 g (92%) als farbloses Öl.
IR (Film): 1734, 1257, 1033, 840, 780 $cm^{-1}$
MS (EI): $(M+H)^+$ 297

c1) (Z) und (E)-(1S,5R)-4-[3-(t-Butyl-dimethyl-silanyloxy)-2-oxo-propyliden]-6-(2,4-dimethoxy-benzyl)-7-oxo-2,6-diaza-bicyclo[3.2.0] heptan-2-carbonsäure-benzylester

[3-(t-Butyl-dimethyl-silanyloxy)-propyl]-phosphonsäure-dimethylester (39,4 g; 133,2 mmol) wird in THF (177 ml) gelöst und auf 0°C gekühlt. Natriumhydrid (4,25 g einer 55 bis 60%igen Suspension in Öl) wird

portionsweise zugegeben, so dass die Temperatur nicht über +5°C steigt. Nach 40 Minuten bei 0°C wird eine auf -20°C vorgekühlte Lösung von Benzyl-(1S,5S)-6-(2,4-dimethoxybenzyl)-4,7-dioxo-2,6-diazabicyclo-[3.2.0]heptan-2-carboxylat (die Europäische Offenlegungsschrift Nr. 508 234 offenbart die entsprechende 3,4-Dimethoxybenzylverbindung) in Aethylenchlorid (750 ml) auf einmal zugegeben. Das Reaktionsgemisch wird 1 Stunde zwischen -6 und -7°C gerührt, auf 1N eiskalte wässrige Salzsäure (140 ml) gegossen und mit Essigester (2x1 l) extrahiert. Die vereinigten organischen Phasen werden mit gesättigter wässriger Kochsalzlösung (1 l) gewaschen, über Magnesiumsulfat getrocknet und eingeengt. Ausbeute: 76 g als gelbes Öl, das ohne weitere Reinigung in der nächsten Stufe eingesetzt wird.
IR (KBr): 1763, 1711, 1293, 1133, 1034, 838, 781 $cm^{-1}$
MS (ISP): $(M+H)^+$ 581,4

d1) (1S,4R,5R)-4-[3-(t-Butyl-dimethyl-silanyloxy)-2-oxo-propyl]-6-(2,4-dimethoxy-benzyl)-7-oxo-2,6-diaza-bicyclo[3.2.0]heptan-2-carbonsäure-t-butylester

Das oben hergestellte Gemisch von (Z) und (E)-(1S,5R)-4-[3-(t-Butyldimethyl-silanyloxy)-2-oxo-propyliden]-6-(2,4-dimethoxybenzyl)-7-oxo-2,6-diaza-bicyclo[3.2.0]heptan-2-carbonsäure-benzyl ester (76 g; maximal 73,8 mmol) wird in Methanol (900 ml) gelöst, mit Di-t-butyl-dicarbonat (24,4 ml; 112 mmol) versetzt und über 10% Pd/C (9g) hydriert. Nach 1,5 Stunden wird das Reaktionsgemisch abgenutscht, eingeengt und über Kieselgel (400 g; 0,063-0,2 mm Korngrösse) mit Essigester/n-Hexan 1:4 chromatographiert. Der erhaltene feste Rückstand wird mit n-Hexan (200 ml) verrührt und abgenutscht. Ausbeute: 17 g (42%) als farbloses Pulver.
IR (KBr): 1760, 1740, 1688, 1613, 1365, 1261, 1161, 1035, 840, 780 $cm^{-1}$
MS (ISP): $(M+H)^+$ 549,5

e1) (1S,4R,5R)-4-[3-(t-Butyl-dimethyl-silanyloxy)-2-oxo-propyl]-7-oxo-2,6-diaza-bicyclo[3.2.0]heptan-2-carbonsäure-t-butylester

Diese Verbindung wird in Analogie zu Beispiel 1g) ausgehend von (1S,4R,5R)-4-[3-(t-Butyl-dimethyl-silanyloxy)-2-oxo-propyl]-6-(2,4-dimethoxybenzyl)-7-oxo-2,6-diaza-bicyclo[3.2.0]heptan-2-carbonsäure-t-butylester (17 g; 31,0 mmol) hergestellt. Der erhaltene Rückstand wird über Kieselgel (400 g; 0,063-0,2 mm Korngrösse) mit Essigester/n-Hexan 7:3 chromatographiert und anschliessend aus n-Hexan crystallisiert. Ausbeute: 7,17 g (58%) als farbloses Pulver.
IR (KBr): 1772, 1740, 1700, 1257, 1164, 1107, 839, 780 $cm^{-1}$
MS (ISP): $(M+H)^+$ 399,5; $(M+NH_4)^+$ 416,5

f1) (1aS,3aR,6bR)-5-(t-Butyl-dimethyl-silanyloxymethyl)-1-oxo-1a,2,3,3a,4,6b-hexahydro-1H-2,6a-diazacyclobut[cd]inden-2,6-dicarbonsäure-di-t-butylester

(1S,4R,5R)-4-[3-(t-Butyl-dimethyl-silanyloxy)-2-oxo-propyl]-7-oxo-2,6-diaza-bicyclo[3.2.0]heptan-2-carbonsäure-t-butylester (7,17 g; 18,0 mmol) und Aethyl-diisopropylamin (3,7 ml; 21,6 mmol) werden in abs. Methylenchlorid (70 ml) auf 0°C vorgekühlt und zu einer Suspension von Calciumcarbonat (7,1 g; 71 mmol)und t-Butyl-oxalsäurechlorid (3,55 g; 21,6 mmol) in abs. Methylenchlorid (50 ml) unter Eisbadkühlung gegeben. Nach 1,5 Stunden bei 0°C wird das Reaktionsgemisch mit Methylenchlorid (200 ml) verdünnt und nacheinander mit 1N eiskalter wässriger Salzsäure (100 ml), eiskaltem Wasser (2x100 ml) und eiskalter gesättigter wässriger Kochsalzlösung (100 ml) gewaschen, über Magnesiumsulfat getrocknet und eingeengt. Der Rückstand wird in abs. Toluol gelöst (250 ml), bei Raumtemperatur mit Triaethylphosphit (6,26 ml; 36 mmol) in abs. Toluol (50 ml) versetzt und 15 Stunden unter Rückflussbedingungen erhitzt. Das Reaktionsgemisch wird in Essigester (100 ml) aufgenommen und nacheinander mit Wasser (20 ml) und gesättigter wässriger Kochsalzlösung (2x20 ml) gewaschen, über Magnesiumsulfat getrocknet und eingeengt. Der feste Rückstand wird mit n-Hexan (200 ml) verrührt und abgenutscht. Ausbeute: 5,61 g (63%) als farbloses Pulver.
IR (KBr): 1783, 1703, 1695, 1624, 1258, 1163, 1098, 838, 778 $cm^{-1}$
MS (EI): $(M-{}^tBuO\bullet)$421

| Mikroanalyse: $C_{25}H_{42}N_2O_6Si$ | | | |
|---|---|---|---|
| Ber. | C 60,70 | H 8,56 | N 5,66 |
| Gef. | C 60,59 | H 8,76 | N 5,49 |

g1) (1aS,3aR,6bR)-5-Hydroxymethyl-1-oxo-1a,2,3,3a,4,6b-hexahydro-1H-2,6a-diazacyclobut[cd]inden-2,6-dicarbonsäure-di-t-butylester

(1aS,3aR,6bR)-5-(t-Butyl-dimethyl-silanyloxymethyl)-1-oxo-1a,2,3,3a,4,6b-hexahydro-1H-2,6a-diazacyclobut[cd]inden-2,6-dicarbonsäure-di-t-butylester (5,61 g; 11,34 mmol) wird in Tetrahydrofuran (80 ml) gelöst und bei Raumtemperatur mit 1N wässriger Salzsäure (23 ml) versetzt. Nach 1 Stunde wird das Reaktionsgemisch mit Essigester (200 ml) verdünnt und nacheinander mit wässriger Natriumbicarbonatlösung (50 ml) und gesättigter wässriger Kochsalzlösung (50 ml) gewaschen, über Magnesiumsulfat getrocknet und eingeengt. Der Rückstand wird aus n-Hexan kristallisiert. Ausbeute: 3,93 g (91%) als farbloses Pulver. Smp. 184 °C.
IR (KBr). 1761, 1705, 1631, 1253, 1161, 1117, 1087 $cm^{-1}$
MS (ISP): $(M+H)^+$ 381,4

Beispiel 15

(1aS,3aR,6bR)-5-(1-Methyl-1H-tetrazol-5-ylsulfanylmethyl)-1-oxo-1a,2,3,3a,4,6b-hexahydro-1H-2,6a-diazacyclobut[cd]inden-6-carbonsäure-trifluoracetat

Diese Verbindung wird in der gleichen Weise wie in Beispiel 2(a) angegeben ausgehend von (1aS,3aR,6bR)-5-(1-Methyl-1H-tetrazol-5-ylsulfanylmethyl)-1-oxo-1a,1,2,3,3a,4,6b-hexahydro-1H-2,6a-diazacyclobut[cd]inden-2,6-dicarbonsäure-di-t-butylester (190 mg; 0,39 mmol) hergestellt. Ausbeute: 150 mg (87%) als beiger Feststoff.
IR (KBr): 1780, 1677, 1198, 1140 $cm^{-1}$
MS (ISP): $(M+H)^+$ 323,3

Die dabei eingesetzte Ausgangsverbindung wird wie folgt hergestellt:

(1aS,3aR,6bR)-5-Hydroxymethyl-1-oxo-1a,2,3,3a,4,6b-hexahydro-1H-2,6a-diazacyclobut[cd]inden-2,6-dicarbonsäure-di-t-butylester (260 mg; 0,67 mmol; aus Beispiel 14) wird in abs. Acetonitril (5 ml) vorgelegt und mit bis-(5-Mercapto-1-methyl-1H-tetrazolyl)-dithiocarbonat (260 mg; 1 mmol) und Triäthylamin (0,09 ml; 0,67 mmol) versetzt. Nach 10 Minuten wird das Reaktionsgemisch mit Aethylacetat (30 ml) verdünnt und nacheinander mit 1N wässriger Salzsäure (15 ml), gesättigter wässriger Natriumbicarbonatlösung (2x10 ml), und gesättigter wässriger Kochsalzlösung (15 ml) gewaschen. Die organische Phase wird über Magnesiumsulfat getrocknet und eingeengt. Ausbeute: 300 mg (93%) als farbloser Feststoff.
IR (KBr): 1776, 1703, 1629, 1251, 1165 $cm^{-1}$
MS (ISP): $(M+H)^+$ 479,5

Beispiel 16

(1aS,3aR,6bR)-5-(5-Amino-1,3,4-thiadiazol-2-ylsulfanylmethyl)-1-oxo-1a,2,3,3a,4,6b-hexahydro-1H-2,6a-diazacyclobut[cd]inden-6-carbonsäure-trifluoracetat

Diese Verbindung wird in der gleichen Weise wie in Beispiel 2(a) angegeben ausgehend von (1aS,3aR,6bR)-5-(5-Amino-1,3,4-thiadiazol-2-ylsulfanylmethyl)-1-oxo-1a,2,3,3a,4,6-hexahydro-1H-2,6a-diazacyclobut[cd]inden-2,6-dicarbonsäure-di-t-butylester (229 mg; 0,462 mmol) hergestellt. Ausbeute: 175 mg (74%) als beiger Feststoff.
IR (KBr): 1777, 1677, 1629, 1416 $cm^{-1}$
MS (ISP): $(M+H)^-$ 340,2

Die dabei eingesetzte Ausgangsverbindung wird wie folgt hergestellt:

(1aS,3aR,6bR)-5-Hydroxymethyl-1-oxo-1a,2,3,3a,4,6b-hexahydro-1H-2,6a-diazacyclobut[cd]inden-2,6-dicarbonsäure-di-t-butylester (200 mg; 0,53 mmol; aus Beispiel 14) wird in abs. Methylenchlorid (2 ml) bei -40 °C vorgelegt und mit Triäthylamin (0,11 ml; 0,789 mmol) und Mesylchlorid (0,061 ml; 0,789 mmol) versetzt. Nach 20 Minuten wird das Reaktionsgemisch zu einer Suspension von 2-Amino-5-mercapto-1,3,4-thiadiazol (105 mg; 0,788 mmol) und Natriumhydrid (32 mg; 0,789 mmol) in THF (3 ml) bei 0 °C gegeben.

Nach 30 Minuten bei dieser Temperatur wird das Reaktionsgemisch mit Aethylacetat (20 ml) verdünnt und mit gesättigter wässriger Kochsalzlösung gewaschen. Anschliessend wird die organische Phase über Magnesiumsulfat getrocknet, eingeengt und mit abs. Aether (20 ml) versetzt. Die erhaltenen Kristalle werden abgenutscht und die Mutterlauge eingeengt. Ausbeute: 229 mg (88%) als leicht gelber Feststoff.
IR (KBr): 1776, 1705, 1620, 1250, 1164 $cm^{-1}$
MS (ISP): $(M+H)^+$ 496,4

Beispiel 17

(1aS,3aR,6bR)-1-Oxo-5-(pyridin-4-ylsulfanylmethyl)-1a,2,3,3a,4,6b-hexahydro-1H-2,6a-diazacyclobut[cd]-inden-6-carbonsäure-trifluoracetat

Diese Verbindung wird in der gleichen Weise wie in Beispiel 2(a) angegeben ausgehend von (1aS,3aR,6bR)-1-Oxo-5-(pyridin-4-ylsulfanylmethyl)-1a,2,3,3a,4,6b-hexahydro-1H-2,6a-diazacyclobut[cd]-inden-2,6-dicarbonsäure-di-t-butylester (448 mg; 0,946 mmol) hergestellt. Ausbeute: 400 mg (98%) als beiger Feststoff.
IR (KBr): 1781, 1710, 1674, 1196 $cm^{-1}$
MS (ISN): $(M-H)^-$ 316,2

Die hierfür verwendete Ausgangsverbindung wird wie folgt hergestellt:

(1aS,3aR,6bR)-5-Hydroxymethyl-1-oxo-1a,2,3,3a,4,6b-hexahydro-1H-2,6a-diazacyclobut[cd]inden-2,6-dicarbonsäure-di-t-butylester (360 mg; 0,95 mmol; aus Beispiel 14) wird in abs. Methylenchlorid (5 ml) bei -40 °C vorgelegt und mit Triäthylamin (0,19 ml; 1,3 mmol) und Mesylchlorid (0,10 ml; 1,3 mmol) versetzt. Nach 30 Minuten bei dieser Temperatur wird das Reaktionsgemisch mit abs. THF (25 ml) verdünnt und mit Triäthylamin (0,15 ml; 1,04 mmol) und 4-Thiopyridin (160 mg; 1,4 mmol) versetzt. Anschliessend wird die Suspension 2 Stunden bei 0 °C gerührt und abgenutscht. Die Mutterlauge wird mit Aethylacetat (200 ml) verdünnt, nacheinander mit Wasser (50 ml) und gesättigter wässriger Kochsalzlösung (50 ml) gewaschen, über Magnesiumsulfat getrocknet und eingeengt. Ausbeute: 440 mg (98%) als gelber Feststoff.
IR (KBr): 1774, 1704, 1625, 1480, 1250, 1165 $cm^{-1}$
MS (ISP): $(M+H)^+$ 474,4

Beispiel 18

(a) (1aS,3aR,6bR)-2-(4-Hydroxy-phenylcarbamoyl)-5-(1-methyl-1H-tetrazol-5-ylsulfanylmethyl)-1-oxo-1a,2,3,3a,4,6b-hexahydro-1H-2,6a-diazacyclobut[cd]inden-6-carbonsäure-Natriumsalz

Diese Verbindung wird in der gleichen Weise wie Beispiel 3(a) angegeben ausgehend von (1aS,3aR,6bR)-5-(1-Methyl-1H-tetrazol-5-ylsulfanylmethyl)-1-oxo-1a,2,3,3a,4,6b-hexahydro-1H-2,6a-diazacyclobut[cd]inden-6-carbonsäure-trifluoracetat (130 mg; 0,30 mmol; aus Beispiel 15) hergestellt. Ausbeute: 38 mg (28%) als farbloser Feststoff.
IR (KBr): 1747, 1603, 1512 $cm^{-1}$
MS (ISN): $(M-Na)^-$ 456,2

In Analogie hierzu werden ausgehend von der gleichen Ausgangsverbindung hergestellt:

(b) (1aS,3aR,6bR)-2-(4-Carbamoyl-phenylcarbamoyl)-5-(1-methyl-1H-tetrazol-5-ylsulfanylmethyl)-1-oxo-1a,2,3,3a,4,6b-hexahydro-1H-2,6a-diazacyclobut[cd]inden-6-carbonsäure-Natriumsalz. Ausbeute: 17% als farbloses Pulver.
IR (KBr): 1747, 1661, 1603, 1524, 1411 $cm^{-1}$
MS (ISN): $(M-Na)^-$ 483,2

(c) (1aS,3aR,6bR)-2-[(S)-2-Oxo-pyrrolidin-3-ylcarbamoyl)-5-(1-methyl-1H-tetrazol-5-ylsulfanylmethyl]-1-oxo-1a,2,3,3a,4,6b-hexahydro-1H-2,6a-diazacyclobut[cd]inden-6-carbonsäure-Natriumsalz. Ausbeute: 26% als farbloses Pulver.
IR (KBr): 1746, 1696, 1631, 1602, 1536, 1391 $cm^{-1}$
MS (ISN): $(M-Na)^-$ 447,3

In Analogie hierzu wird ausgehend von (1aS,3aR,6bR)-5-(5-Amino-1,3,4-thiadiazol-2-ylsulfanylmethyl)-1-oxo-1a,2,3,3a,4,6b-hexahydro-1H-2,6a-diazacyclobut[cd]inden-6-carbonsäure-trifluoracetat (aus Beispiel 16) hergestellt:

(d) (1aS,3aR,6bR)-5-(5-Amino-1,3,4-thiadiazol-2-ylsulfanylmethyl)-2-(4-hydroxy-phenylcarbamoyl)-1-oxo-1a,2,3,3a,4,6b-hexahydro-1H-2,6a-diazacyclobut[cd]inden-6-carbonsäure-Natriumsalz. Ausbeute: 45% als farbloses Pulver.
IR (KBr): 1743, 1640, 1602, 1513, 1391 $cm^{-1}$

MS (ISN): $(M-Na)^-$ 473,2

In Analogie hierzu wird ausgehend von (1aS,3aR,6bR)-1-Oxo-5-(pyridin-4-ylsulfanylmethyl)-1a,2,3,3a,4,6b-hexahydro-1H-2,6a-diazacyclobut[cd]inden-6-carbonsäure-trifluoracetat (aus Beispiel 17) hergestellt:

(e) (1aS,3aR,6bR)-2-(4-Carbamoyl-phenylcarbamoyl)-1-oxo-5-(pyridin-4-ylsulfanylmethyl)-1a,2,3,3a,4,6b-hexahydro-1H-2,6a-diazacyclobut[cd]inden-6-carbonsäure. Ausbeute: 41% als farbloses Pulver.
IR (KBr): 1748, 1661, 1585, 1525, 1412 $cm^{-1}$
MS (ISN): $(M-H)^-$ 478,2

(f) (1aS,3aR,6bR)-2-(2-t-Butoxycarbonyl-äthylcarbamoyl)-5-(1-methyl-1H-tetrazol-5-ylsulfanylmethyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 3(a) ausgehend von (1aS,3aR,6bR)-5-(1-Methyl-tetrazol-5-yl-sulfanyl-1-oxo-1a,2,3,3a,4,6b-hexahydro-1H-2,6a-diaza-cyclobut[cd]inden-6-carbonsäure-trifluoracetat (400 mg; 0,838 mmol) hergestellt. Ausbeute: 210 mg (49%) als farbloser Feststoff.
IR (KBr): 1738, 1605, 1531, 1392 $cm^{-1}$
MS (ISN): $(M-Na)^-$ 492,5

Durch Behandlung mit Trifluoressigsäure wie in Beispiel 2(a) erhält man die entsprechende 2-(2-Carboxyäthylcarbamoyl)-Verbindung.

In Analogie hierzu werden ausgehend von der gleichen Ausgangsverbindung hergestellt:

(g) (1aS,3aR,6bR)-1-Oxo-5-(1-methyl-1H-tetrazol-5-ylsulfanylmethyl)-2-thiophen-2-ylmethylcarbamoyl-1a,2,3,3a,4,6b-hexahydro-1H-2,6a-diazacyclobut[cd]inden-6-carbonsäure-Natriumsalz
Ausbeute: 14% als farbloser Feststoff.
IR (KBr): 1749, 1634, 1603, 1526, 1393 $cm^{-1}$
MS (ISN): $(M-Na)^-$ 460,4

(h) (1aS,3aR,6bR)-2-(4-Hydroxy-benzylcarbamoyl)-5-(1-methyl-1H-tetrazol-5-ylsulfanylmethyl)-1-oxo-1a,2,3,3a,4,6b-hexahydro-1H-2,6a-diazacyclobut[cd]inden-6-carbonsäure-Natriumsalz
Ausbeute: 64% als leicht gelber Feststoff.
IR (KBr): 1747, 1609, 1515, 1392 $cm^{-1}$
MS (ISN): $(M-Na)^-$ 470,5

In Analogie hierzu werden ausgehend von (1aS,3aR,6bR)-1-Oxo-5-pyridin-4-ylsulfanylmethyl-1a,2,3,3a,4,6b-hexahydro-1H-2,6a-diazacyclobut[cd]inden-6-carbonsäure-trifluoracetat hergestellt:

(i) (1aS,3aR,6bR)-2-(4-Hydroxy-benzylcarbamoyl)-1-oxo-5-(pyridin-4-ylsulfanylmethyl)-1a,2,3,3a,4,6b-hexahydro-1H-2,6a-diaza-cyclobut[cd]inden-6-carbonsäure-Natriumsalz
Ausbeute: 82% als leicht gelber Feststoff.
IR (KBr): 1746, 1609, 1582, 1538, 1482, 1392 $cm^{-1}$
MS (ISN): $(M-Na)^-$465,4

(j) (1aS,3aR,6bR)-2-(4-Hydroxy-phenylcarbamoyl)-1-oxo-5-(pyridin-4-ylsulfanylmethyl)-1a,2,3,3a,4,6b-hexahydro-1H-2,6a-diaza-cyclobut[cd]inden-6-carbonsäure-Natriumsalz
Ausbeute: 83% als farbloses Pulver.
IR (KBr): 1749, 1604, 1481, 1241 $cm^{-1}$
MS (ISN): $(M-Na)^-$451,4

## Beispiel 19

(a) (1aS,3aR,6bR)-2-Acetyl-5-(1-methyl-1H-tetrazol-5-ylsulfanylmethyl)-1-oxo-1a,2,3,3a,4,6b-hexahydro-1H-2,6a-diazacyclobut[cd]inden-6-carbonsäure-Natriumsalz

(1aS,3aR,6bR)-5-(1-Methyl-1H-tetrazol-5-ylsulfanylmethyl)-1-oxo-1a,2,3,3a,4,6b-hexahydro-1H-2,6a-diazacyclobut[cd]inden-6-carbonsäure-trifluoracetat (150 mg; 0,31 mmol; aus Beispiel 15) wird in Methylenchlorid (5 ml) und Acetonitril (2 ml) bei 0°C vorgelegt und mit Acetylchlorid (0,025 ml; 0,35 mmol) und Natriumbicarbonat (62 mg; 0,74 mmol) versetzt. Nach 1 Stunde bei 0°C wird das Reaktionsgemisch mit Wasser (4 ml) verdünnt und der pH-Wert mittels gesättigter wässriger Natriumbicarbonatlösung auf 7 gestellt. Die erhaltene Lösung wird über ein polymeres hydrophobes Gel mit Wasser chromatographiert und die reinen Fraktionen lyophilisiert. Ausbeute: 43 mg (38%) als farbloses Lyophilisat.
IR (KBr): 1749, 1602, 1407 $cm^{-1}$
MS (ISN): $(M-Na)^-$ 363,3

In Analogie hierzu wird hergestellt:

(b) (1aS,3aR,6bR)-2-Acetyl-1-oxo-5-(pyridin-4-ylsulfanylmethyl)-1a,2,3,3a,4,6b-hexahydro-1H-2,6a-diazacyclobut[cd]inden-6-carbonsäure

Ausgehend von (1aS,3aR,6bR)-1-Oxo-5-(pyridin-4-ylsulfanylmethyl)-1a,2,3,3a,4,6b-hexahydro-1H-2,6a-diazacyclobut[cd]inden-6-carbonsäure-trifluoracetat (200 mg; 0,46 mmol; aus Beispiel 17) werden 40 mg (24%) eines farblosen Pulvers erhalten.
IR (KBr): 1764, 1623, 1417 $cm^{-1}$
MS (ISN): $(M-H)^-$ 358,1

(c) (1aS,3aR,6bR)-5-(1-Methyl-1H-tetrazol-5-ylsulfanylmethyl)-1-oxo-2-trifluoracetyl-1a,2,3,3a,4,6b-hexahydro-1H-2,6a-diaza-cyclobut[cd]inden-6-carbonsäure-Natriumsalz

(1aS,3aR,6bR)-5-(1-Methyl-tetrazol-5-yl-sulfanylmethyl)-1-oxo-1a,2,3,3a,4,6b-hexahydro-1H-2,6a-diaza-cyclobut[cd]inden-6-carbonsäure-trifluoracetat (200 mg; 0,415 mmol) wird in Dimethylformamid (4 ml) gelöst und mit Trifluoressigsäure (0,033 ml; 0,41 mmol) und Dicyclohexylcarbodiimid (100 mg; 0,48 mmol) versetzt. Nach 30 Minuten wird die erhaltene Suspension abgenutscht,eingeengt und in wenig Wasser aufgenommen. Der pH-Wert wird mit gesättigter wässriger Natriumbicarbonatlösung auf 7 eingestellt. Die Lösung wird über ein polymeres hydrophobes Gel mit Wasser/Acetonitril chromatographiert und lyophilisiert. Ausbeute: 75 mg (44%) als farbloses Pulver.
IR (KBr): 1765, 1697, 1607, 1397 $cm^{-1}$
MS (ISN): $(M-Na)^-$ 417,3

(d) (1aS,3aR,6bR)-2-Cyanacetyl-5-(1-methyl-1H-tetrazol-5-ylsulfanylmethyl)-1-oxo-1a,2,3,3a,4,6b-hexahydro-1H-2,6a-diaza-cyclobut[cd]inden-6-carbonsäure-Natriumsalz

(1aS,3aR,6bR)-5-(1-Methyl-tetrazol-5-yl-sulfanylmethyl)-1-oxo-1a,2,3,3a,4,6b-hexahydro-1H-2,6a-diaza-cyclobut[cd]inden-6-carbonsäure-trifluoracetat (200 mg; 0,415 mmol) wird in Dimethylformamid (2 ml) gelöst und mit Natriumbicarbonat (91 mg; 1,08 mmol) und 2-Cyanessigsäure-2,5-dioxo-pyrrolidin-1-yl-ester (91 mg; 0,498 mmol) versetzt. Nach 3 Stunden bei Raumtemperatur wird das Reaktionsgemisch eingeengt. Der erhaltene Rückstand wird in wenig Wasser (1 ml) aufgenommen und der pH-Wert mit gesättigter wässriger Natriumbicarbonatlösung auf 7 eingestellt. Die Lösung wird über ein polymeres hydrophobes Gel mit Wasser/Acetonitril chromatographiert und lyophilisiert. Ausbeute: 24 mg (16%) als farbloses Pulver.
IR (KBr): 2260, 1753, 1665, 1605, 1395 $cm^{-1}$
MS (ISN): $(M-Na)^-$ 388,3

(e) (1aS,3aR,6bR)-2-Methylsulfonyl-5-(1-methyl-1H-tetrazol-5-ylsulfanylmethyl)-1-oxo-1a,2,3,3a,4,6b-hexahydro-1H-2,6a-diaza-cyclobut[cd]inden-6-carbonsäure-Natriumsalz

(1aS,3aR,6bR)-5-(1-Methyl-tetrazol-5-yl-sulfanyl-1-oxo-1a,2,3,3a,4,6b-hexahydro-1H-2,6a-diaza-cyclobut-[cd]inden-6-carbonsäure-trifluoracetat (200 mg; 0,415 mmol) wird in Methylenchlorid (5 ml) suspendiert und mit N-Methyl-N-trimethysilyltrifluoracetamid (0,2 ml; 1,08 mmol) versetzt. Nach 5 Minuten wird Methansulfonsäurechlorid (0,039 ml; 0,498 mmol) und N-Aethyldiisopropylamin (0,085 ml; 0,498 mmol) zugegeben. Nach 2 Stunden bei Raumtemperatur wird das Reaktionsgemisch eingeengt und der erhaltene Rückstand in Wasser (1 ml) aufgenommen. Der pH-Wert wird mit gesättigter wässriger Natriumbicarbonatlösung auf 7 eingestellt. Die Lösung wird über ein polymeres hydrophobes Gel mit Wasser/Acetonitril chromatographiert und lyophilisiert. Ausbeute: 29 mg (17%) als farbloses Pulver.
IR (KBr): 1763, 1607, 1388, 1337, 1154 $cm^{-1}$
MS (ISN): $(M-Na)^-$ 399,4

(f) (1aS,3aR,6bR)-5-(1-Methyl-1H-tetrazol-5-ylsulfanylmethyl)-1-oxo-2-trifluormethylsulfonyl-1a,2,3,3a,4,6b-hexahydro-1H-2,6a-diazacyclobut[cd]inden-6-carbonsäure-Natriumsalz

(1aS,3aR,6bR)-5-(1-Methyl-tetrazol-5-yl-sulfanylmethyl)-1-oxo-1a,2,3,3a,4,6b-hexahydro-1H-2,6a-diaza-cyclobut[cd]inden-6-carbonsäure-trifluoracetat (200 mg; 0,415 mmol) wird in Methylenchlorid (5 ml) suspendiert und mit N-Methyl-N-trimethysilyltrifluoracetamid (0,2 ml; 1,08 mmol) versetzt. Nach 5 Minuten wird das Reaktionsgemisch auf 0°C gekühlt und Trifluormethansulfonsäureanhydrid (0,102 ml; 0,623 mmol) und N-Aethyldiisopropylamin (0,107 ml; 0,623 mmol) zugegeben. Nach 1 Stunde bei dieser Temperatur wird das Reaktionsgemisch eingeengt und der erhaltene Rückstand in Wasser (1 ml) aufgenommen. Der pH-Wert

wird mit gesättigter wässriger Natriumbicarbonatlösung auf 7 eingestellt. Die Lösung wird über ein polymeres hydrophobes Gel mit Wasser/Acetonitril chromatographiert und lyophilisiert. Ausbeute: 17 mg (9%) als farbloses Pulver.
IR (KBr): 1777, 1698, 1610, 1393, 1360, 1190, 1144 $cm^{-1}$
MS (ISN): $(M+H)^+$ 455,4

(g) (1aS,3aR,6bR)-1-Oxo-5-(pyridin-4-ylsulfanylmethyl)-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-1-Oxo-5-(pyridin-4-ylsulfanylmethyl)-1a,2,3,3a,4,6b-hexahydro-1H-2,6a-diaza-cyclobut[cd]-inden-6-carbonsäure-trifluoracetat (165 mg; 0,3 mmol) hergestellt. Ausbeute: 54 mg (52 %) als farbloses Pulver.
IR (KBr): 1764, 1696, 1609, 1403, 1180 $cm^{-1}$
MS (ISN): (M-Na)- 412,4

(h) (1aS,3aR,6bR)-5-(5-Amino-1,3,4-thiadiazol-2-ylsulfanylmethyl)-1-oxo-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-(5-Amino-1,3,4-thiadiazol-2-ylsulfanylmethyl)-1-oxo-1a,2,3,3a,4,6b-hexahydro-1H-2,6a-diaza-cyclobut[cd] inden-6-carbonsäure-trifluoracetat (204 mg; 0,4 mmol) hergestellt. Ausbeute: 21 mg (12 %) als farbloses Pulver.
IR (KBr): 1760, 1694, 1606, 1399, 1180 $cm^{-1}$
MS (ISP): $(M+H)^+$ 436,3

(i) (1aS,3aR,6bR)-2-Acetyl-5-(5-amino-1,3,4-thiadiazol-2-ylsulfanylmethyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-(5-Amino-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat (180 mg; 0,35 mmol) in DMF (5 ml) bei -20 °C hergestellt. Ausbeute: 26 mg (19 %) als braunes Pulver.
IR (KBr): 1750, 1605, 1404 $cm^{-1}$
MS (ISN): $(M\text{-}Na)^-$ 380,2

(j) (1aS,3aR,6bR)-2-Acetyl-5-(5-acetylamino-1,3,4-thiadiazol-2-ylsulfanylmethyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-(5-Amino-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat (204 mg; 0,4 mmol) in DMF (5 ml) bei 0 °C hergestellt. Ausbeute: 55 mg (31%) als gelblisches Pulver.
IR (KBr): 1753, 1690, 1606, 1397 $cm^{-1}$
MS (ISP): $(M+H)^+$ 424,2 (ohne Na); $(M+H)^+$ 446,2 (mit Na)

(k) (1aS,3aR,6bR)-2-Formyl-5-(1-methyl-1H-tetrazol-5-ylsulfanylmethyl)-1-oxo-1a,2,3,3a,4,6b-hexahydro-1H-2,6a-diaza-cyclobut[cd]inden-6-carbonsäure-Natriumsalz

(1aS,3aR,6bR)-5-(1-Methyl-1H-tetrazol-5-ylsulfanylmethyl)-1-oxo-1a,2,3,3a,4,6b-hexahydro-1H-2,6a-diaza-cyclobut[cd]inden-6-carbonsäure-trifluoracetat (241 mg; 0,5 mmol) wird in Dimethylformamid (4 ml) bei 0 °C vorgelegt und mit konzentrierter Ameisensäure (0,38 ml; 10 mmol) und Dicyclohexylcarbodiimid (226 mg; 1,1 mmol) versetzt. Nach 3 Stunden bei 0 °C wird die erhaltene Suspension abgenutscht und eingeengt. Der Rückstand wird in Wasser (2 ml) aufgenommen und der pH-Wert mit gesättigter wässriger Natriumbicarbonatlösung auf 7 eingestellt. Die Lösung wird über ein polymeres hydrophobes Gel mit Wasser/Acetonitril chromatographiert und lyophilisiert. Ausbeute: 61 mg (33%) als oranges Pulver.
IR (KBr): 1753, 1660, 1597, 1393 $cm^{-1}$
MS (ISP): $(M+H)^+$ 373,3

Beispiel 20

(1aS,3aR,6bR)-5-(1-Methyl-pyridin-4-yliosulfanylmethyl)-1-oxo-1a,2,3,3a,4,6b-hexahydro-1H-2,6a-diazacyclobut[cd]inden-6-carboxylat-trifluoracetat

Diese Verbindung wird in der gleichen Weise wie in Beispiel 2(a) angegeben ausgehend von (1aS,3aR,6bR)-4-(2,6-bis-t-Butoxycarbonyl-1-oxo-1a,2,3,3a,4,6b-hexahydro-1H-2,6a-diazacyclobut[cd]inden-5-ylmethylsulfanyl)-1-methyl-pyridinium-jodid (355 mg; 0,59 mmol) hergestellt. Ausbeute: 287 mg (100%) als beiger Feststoff.
IR (KBr): 1779, 1681, 1633 $cm^{-1}$
MS (ISP): $M^{+}$ 332,3

Die Ausgangsverbindung wird wie folgt hergestellt:

(1aS,3aR,6bR)-1-Oxo-5-(pyridin-4-ylsulfanylmethyl)-1a,2,3,3a,4,6b-hexahydro-1H-2,6a-diazacyclobut[cd]-inden-2,6-dicarbonsäure-di-t-butylester (440 mg; 0,93 mmol; aus Beispiel 17) wird in Dimethylformamid (3 ml) vorgelegt und bei Raumtemperatur mit Methyljodid (0,17 ml; 2,8 mmol) versetzt. Nach 3 Stunden wird die Lösung eingeengt, mit gesättigter, wässriger Natriumchloridlösung (20 ml) versetzt und mit Methylenchlorid (60 ml) extrahiert. Anschliessend wird die organische Phase über Magnesiumsulfat getrocknet, eingeengt, mit absolutem Aether (20 ml) verrührt und abgenutscht. Ausbeute: 355 mg (63%) als beige-brauner Feststoff.
IR (KBr): 1775, 1702, 1633, 1163 $cm^{-1}$
MS (ISP): $M^{+}$ 488,5

Beispiel 21

(1aS,3aR,6bR)-2-(4-Carbamoyl-phenylcarbamoyl)-5-(1-methyl-pyridin-1-yliosulfanylmethyl)-1-oxo-1a,2,3,3a,4,6b-hexahydro-1H-2,6a-diazacyclobut[cd]inden-6-carboxylat

Diese Verbindung wird in der gleichen Weise wie in Beispiel 3(a) angegeben aus (1aS,3aR,6bR)-5-(1-Methyl-pyridin-4-yliosulfanylmethyl)-1-oxo-1a,2,3,3a,4,6b-hexahydro-1H-2,6a-diazacyclobut[cd]inden-6-carboxylat-trifluoracetat (190 mg, 0,32 mmol; aus Beispiel 20) hergestellt. Ausbeute: 50 mg (32%) als leicht rosa Pulver.
IR (KBr): 1752, 1661, 1633, 1600, 1524 $cm^{-1}$

| Mikroanalyse: $C_{24}H_{23}N_5O_5S$ | | | |
|---|---|---|---|
| Ber. | C 58,41 | H 4,70 | N 14,19 |
| Gef. | C 58,31 | H 4,68 | N 14,10 |

Beispiel 22

(1aS,3aR,6bR)-1-Oxo-5-(pyridin-1-yliomethyl)-1a,2,3,3a,4,6b-hexahydro-1H-2,6a-diazacyclobut[cd]inden-6-carboxylat-trifluoracetat

In Analogie zu Beispiel 2(a) werden ausgehend von (1aS,3aR,6bR)-1-(2,6-bis-t-Butoxycarbonyl-1-oxo-1a,2,3,3a,4,6b-hexahydro-1H-2,6a-diazacyclobut[cd]inden-5-ylmethyl)pyridinium-chlorid (680 mg; 1,42 mmol) 640 mg (98%) als farbloser Feststoff erhalten.
IR (KBr): 2700, 1783, 1719, 1681, 1487 $cm^{-1}$
MS (ISP): $M^{+}$ 286,3

Die Ausgangsverbindung wird wie folgt hergestellt:

(1aS,3aR,6bR)-5-Hydroxymethyl-1-oxo-1a,2,3,3a,4,6b-hexahydro-1H-2,6a-diazacyclobut[cd]inden-2,6-dicarbonsäure-di-t-butylester (900 mg; 2,73 mmol; aus Beispiel 14) wird bei 0 °C in Pyridin (5 ml) gelöst und mit Mesylchlorid (0,25 ml; 3,15 mmol) versetzt. Nach 16 Stunden bei Raumtemperatur wird das Reaktionsgemisch eingeengt. Der Rückstand wird in Methylenchlorid (50 ml) gelöst und mit gesättigter wässriger Kochsalzlösung (3x25 ml) gewaschen. Anschliessend wird die organische Phase über Magnesiumsulfat getrocknet und eingeengt. Der Rückstand wird mit Aether verrührt (2x50 ml) und abgenutscht. Ausbeute: 950 mg (84%) als farbloser Feststoff. Smp. 124 °C (Zers.).
IR (KBr): 1780, 1703, 1630, 1250, 1161 $cm^{-1}$

MS (ISP): $M^+$ 442,5

Beispiel 23

(a) (1aS,3aR,6bR)-2-(4-Hydroxy-phenylcarbamoyl)-1-oxo-5-(pyridin-1-yliomethyl)-1a,2,3,3a,4,6b-hexahydro-1H-2,6a-diazacyclobut[cd]inden-6-carboxylat

Diese Verbindung wird in der gleichen Weise wie in Beispiel 3(a) angegeben ausgehend von (1aS,3aR,6bR)-1-Oxo-5-(pyridin-1-yliomethyl)-1a,2,3,3a,4,6b-hexahydro-1H-2,6a-diazacyclobut[cd]inden-6-carboxylat-trifluoracetat (200 mg; 0,44 mmol; aus Beispiel 22) hergestellt. Ausbeute: 79 mg (41%) als farbloses Pulver.

IR (KBr): 3415, 3259, 1758, 1650, 1611, 1530, 1513, 1385 $cm^{-1}$

| Mikroanalyse: $C_{22}H_{20}N_4O_5$ | | | |
|---|---|---|---|
| Ber. | C 62,85 | H 4,80 | N 13,33 |
| Gef. | C 62,79 | H 4,68 | N 13,08 |

In Analogie hierzu werden ausgehend von (1aS,3aR,6bR)-1-Oxo-5-(pyridin-1-yliomethyl)-1a,2,3,3a,4,6b-hexahydro-1H-2,6a-diazacyclobut[cd]inden-6-carboxylat-trifluoracetat hergestellt:

(b) (1aS,3aR,6bR)-2-(3-Hydroxy-isoxazol-5-ylmethylcarbamoyl)-1-oxo-5-(pyridin-1-yliomethyl)-1a,2,3,3a,4,6b-hexahydro-1H-2,6a-diazacyclobut[cd]inden-6-carboxylat. Ausbeute: 29% als farbloses Pulver.

IR (KBr): 1762, 1705, 1629, 1531, 1391 $cm^{-1}$

| Mikroanalyse: $C_{20}H_{19}N_5O_6$ | | | |
|---|---|---|---|
| Ber. | C 56,47 | H 4,50 | N 16,46 |
| Gef. | C 56,51 | H 4,30 | N 16,35 |

(c) (1aS,3aR,6bR)-2-(4-Carbamoyl-phenylcarbamoyl)-1-oxo-5-(pyridin-1-yliomethyl)-1a,2,3,3a,4,6b-hexahydro-1H-2,6a-diazacyclobut[cd]inden-6-carboxylat. Ausbeute: 43% als farbloses Pulver.

IR (KBr): 3420, 1758, 1662, 1524, 1384 $cm^{-1}$

| Mikroanalyse: $C_{23}H_{21}N_5O_5$ | | | |
|---|---|---|---|
| Ber. | C 61,74 | H 4,73 | N 15,65 |
| Gef. | C 61,67 | H 4,53 | N 15,39 |

(d) (1aS,3aR,6bR)-2-Acetyl-1-oxo-5-(pyridin-1-yliomethyl)-1a,2,3,3a,4,6b-hexahydro-1H-2,6a-diazacyclobut[cd]inden-6-carboxylat

Diese Verbindung wird in der gleichen Weise wie Beispiel 19 angegeben ausgehend von (1aS,3aR,6bR)-1-Oxo-5-(pyridin-1-yliomethyl)-1a,2,3,3a,4,6b-hexahydro-1H-2,6a-diazacyclobut[cd]inden-6-carboxylat-trifluoracetat (200 mg; 0,43 mmol; aus Beispiel 22) hergestellt. Ausbeute: 80 mg (57%) als gelbes Pulver.

IR (KBr): 1770, 1680, 1424 $cm^{-1}$

MS (ISP): $(M+H)^+$ 328,2

(e) (1aS,3aR,6bR)-1-Oxo-5-(pyridin-1-yliomethyl)-2-trifluoracetyl-1a,2,3,3a,4,6b-hexahydro-1H-2,6a-diazacyclobut[cd]inden-6-carboxylat

(1aS,3aR,6bR)-1-Oxo-5-(pyridin-1-yliomethyl)-1a,2,3,3a,4,6b-hexahydro-1H-2,6a-diazacyclobut[cd]-inden-6-carboxylat-trifluoracetat (188 mg; 0,41 mmol) wird in Methylenchlorid (8 ml) bei 0°C vorgelegt und mit N-Methyl-N-trimethylsilyltrifluoracetamid (91 $\mu$l; 0,49 mmol) und Dicyclohexylcarbodiimid (103 mg; 0,49 mmol) versetzt. Nach 2 Stunden bei Raumtemperatur wird das Reaktionsgemisch eingeengt, in Wasser (1 ml) gelöst, der pH-Wert mit gesättigter wässriger Natriumbicarbonatlösung auf 7 eingestellt, und über ein polymeres hydrophobes Gel mit Wasser/Acetonitril chromatographiert und lyophilisiert. Ausbeute: 52 mg (34%) als gelbes Pulver.

IR (KBr): 1770, 1615, 1390, 1336, 1155 $cm^{-1}$

MS (ISP): $[M+H^++H_2O]^+$ 382,3 (MS-Artefact)

f) (1aS,3aR,6bR)-2-(2-t-Butoxycarbonyl-äthylcarbamoyl)-1-oxo-5-(pyridin-1-yliomethyl)-1a,2,3,3a,4,6b-hexahydro-1H-2,6a-diaza-cyclobut[cd]indene-6-carboxylat

Diese Verbindung wird in Analogie zu Beispiel 3(a) ausgehend von (1aS,3aR,6bR)-1-Oxo-5-(pyridin-1-yliomethyl)-1a,2,3,3a,4,6b-hexahydro-1H-2,6a-diaza-cyclobut[cd]inden-6-carboxylat-trifluoracetat (350 mg; 0,702 mmol; aus Beispiel 22) hergestellt. Ausbeute: 154 mg (48%) als oranges Pulver.
IR (KBr): 1762, 1722, 1632, 1536, 1392, 1216 $cm^{-1}$
MS (ISP): $(M+H)^+$ 457,4

(g) (1aS,3aR,6bR)-2-Benzyloxycarbonylmethylcarbamoyl-1-oxo-5-(pyridin-1-yliomethyl)-1a,2,3,3a,4,6b-hexahydro-1H-2,6a-diaza-cyclobut[cd]inden-6-carboxylat

Diese Verbindung wird in Analogie zu Beispiel 3(a) ausgehend von (1aS,3aR,6bR)-1-Oxo-5-(pyridin-1-yliomethyl)-1a,2,3,3a,4,6b-hexahydro-1H-2,6a-diaza-cyclobut[cd]inden-6-carboxylat-trifluoracetat (300 mg; 0,602 mmol; aus Beispiel 22) hergestellt. Ausbeute: 54 mg (19%) als braunes Pulver.
IR (KBr): 1758, 1614, 1536, 1390 $cm^{-1}$
MS (ISP): $(M+H)^+$ 477,4

Beispiel 24

(1aS,3aR,6bR)-5-Acetoxymethyl-1-oxo-1a,2,3,3a,4,6b-hexahydro-1H-2,6a-diazacyclobut[cd]inden-6-carbonsäure-trifluoracetat

Diese Verbindung wird in gleicher Weise wie in Beispiel 2(a) angegeben ausgehend von (1aS,3aR,6bR)-5-Acetoxymethyl-1-oxo-1a,2,3,3a,4,6b-hexahydro-1H-2,6a-diazacyclobut[cd]inden-6-carbonsäure-di-t-butylester (570 mg; 1,35 mmol) hergestellt. Ausbeute: 400 mg (83%) als beiger Feststoff.
IR (KBr): 1784, 1739, 1674, 1234, 1198 $cm^{-1}$
MS (ISN): $(M-H)^-$ 265,2

Die Ausgangsverbindung wird wie folgt hergestellt:

(1aS,3aR,6bR)-5-Hydroxymethyl-1-oxo-1a,2,3,3a,4,6b-hexahydro-1H-2,6a-diazacyclobut[cd]inden-2,6-dicarbonsäure-di-t-butylester (440 mg; 1,08 mmol; aus Beispiel 14) wird in abs. Methylenchlorid (8 ml) bei 0°C vorgelegt und mit Pyridin (0,12 ml; 1,4 mmol) und Acetylchlorid (0,09 ml; 1,3 mmol) versetzt. Nach 2 Stunden bei 0°C wird das Reaktionsgemisch mit Aethylacetat (40 ml) verdünnt, nacheinander mit Wasser (40 ml) und gesättigter wässriger Kochsalzlösung (40 ml) gewaschen, über Magnesiumsulfat getrocknet und eingeengt. Ausbeute: 450 mg (99%) als farbloses Pulver.
IR (KBr): 1775, 1742, 1705, 1636, 1239, 1162 $cm^{-1}$
MS (ISP): $(M+H)^+$ 423,6

Beispiel 25

a) (1aS,3aR,6bR)-5-Acetoxymethyl-2-(4-hydroxy-phenylcarbamoyl)-1-oxo-1a,2,3,3a,4,6b-hexahydro-1H-2,6a-diazacyclobut[cd]inden-6-carbonsäure-Natriumsalz

Diese Verbindung wird in der gleichen Weise wie in Beispiel 3(a) angegeben ausgehend von (1aS,3aR,6bR)-5-Acetoxymethyl-1-oxo-1a,2,3,3a,4,6b-hexahydro-1H-2,6a-diazacyclobut[cd]inden-6-carbonsäure-trifluoracetat (390 mg; 1,09 mmol; aus Beispiel 24) hergestellt. Ausbeute: 160 mg (35%) als farbloses Pulver.
IR (KBr): 1750, 1739, 1638, 1610, 1513, 1382, 1238 $cm^{-1}$
MS (ISN): $M^-$ 400,2

(b) (1aS,3aR,6bR)-5-Acetoxymethyl-2-benzyloxycarbonylmethylcarbamoyl-1-oxo-1a,2,3,3a,4,6b-hexahydro-1H-2,6a-diazacyclobut [cd]inden-6-carbonsäure-Natriumsalz

Diese Verbindung wird in Analogie zu Beispiel 3(a) ausgehend von (1aS,3aR,6bR)-5-Acetoxymethyl-1-oxo-1a,2,3,3a,4,6b-hexahydro-1H-2,6a-diazacyclobut [cd]inden-6-carbonsäure-trifluoracetat (317 mg; 0,86 mmol) hergestellt. Ausbeute: 167 mg (41%) als farbloses Pulver.
IR (KBr): 1742, 1609, 1532, 1398, 1243 $cm^{-1}$
MS (ISN): $(M-Na)^-$ 456,4

(c) (1aS,3aR,6bR)-5-Acetoxymethyl-2-acetyl-1-oxo-1a,2,3,3a,4,6b-hexahydro-1H,2,6a-diazacyclobut[cd]-inden-6-carbonsäure-Natriumsalz

Diese Verbindung wird in Analogie zu Beispiel 19(a) ausgehend von 1aS,3aR,6bR)-5-Acetoxymethyl-1-oxo-1a,2,3,3a,4,6b-hexahydro-1H-2,6a-diazacyclobut[cd]inden-6-carbonsäure-trifluoracetat (148 mg; 0,4 mmol) in Dimethylformamid (5 ml) hergestellt. Ausbeute: 106 mg (80%) als gelbes Pulver.
IR (KBr): 1747, 1610, 1411, 1241 $cm^{-1}$
MS (ISN): $(M+H)^{+}$ 331,3

(d) (1aS,3aR,6bR)-5-Acetoxymethyl-2-trifluoracetyl-1-oxo-1a,2,3,3a,4,6b-hexahydro-1H-2,6a-diazacyclobut-[cd]inden-6-carbonsäure-Natriumsalz

Diese Verbindung wird in Analogie zu Beispiel 19(c) ausgehend von 1aS,3aR,6bR)-5-Acetoxymethyl-1-oxo-1a,2,3,3a,4,6b-hexahydro-1H-2,6a-diazacyclobut[cd]inden-6-carbonsäure-trifluoracetat (148 mg; 0,4 mmol) hergestellt. Ausbeute: 44 mg (29%) als farbloses Pulver.
IR (KBr): 1756, 1699, 1611, 1409, 1168 $cm^{-1}$
MS (ISN): $(M\text{-}Na)^{-}$ 361,3

Beispiel 26

(1aS,3aR,6bR)-5-Carbamoyloxymethyl-1-oxo-1a,2,3,3a,4,6b-hexahydro-1H-2,6a-diazacyclobut[cd]inden-6-carbonsäure-trifluoracetat

Diese Verbindung wird in der gleichen Weise wie in Beispiel 2(a) angegeben ausgehend von (1aS,3aR,6bR)-5-Carbamoyloxymethyl-1-oxo-1a,2,3,3a,4,6b-hexahydro-1H-2,6a-diazacyclobut[cd]inden-2,6-dicarbonsäure-di-t-butylester (1,42 mg; 3,36 mmol) hergestellt. Ausbeute: 1,25 g (98%) als farbloses Pulver.
IR (KBr): 1775, 1678, 1620, 1200 $cm^{-1}$
MS (ISN): $(M\text{-}H)^{-}$ 266,2

Die Ausgangsverbindung wird wie folgt hergestellt:

(1aS,3aR,6bR)-5-(2-Chloracetylaminocarbonyloxymethyl)-1-oxo-1a,2,3,3a,4,6b-hexahydro-1H-2,6a-diazacyclobut[cd]inden-2,6-dicarbonsäure-di-t-butylester

(1aS,3aR,6bR)-5-Hydroxymethyl-1-oxo-1a,2,3,3a,4,6b-hexahydro-1H-2,6a-diazacyclobut[cd]inden-2,6-dicarbonsäure-di-t-butylester (1,9 g; 5,0 mmol; aus Beispiel 14) wird in abs. Methylenchlorid (25 ml) bei 0 °C vorgelegt und mit Chloracetylisocyanat (900 mg; 7,5 mmol) in abs. Methylenchlorid (7 ml) versetzt. Nach 1 Stunde wird das Reaktionsgemisch eingeengt, mit n-Hexan (25 ml) verrührt und abgenutscht. Ausbeute: 2,5 g (100%) als gelbes Pulver. Smp. 124-126 °C.
IR (KBr): 1776, 1707 $cm^{-1}$
MS (ISP): $(M+H)^{+}$ 500,4

(1aS,3aR,6bR)-5-Carbamoyloxymethyl-1-oxo-1a,2,3,3a,4,6b-hexahydro-1H-2,6a-diazacyclobut[cd]inden-2,6-dicarbonsäure-di-t-butylester

(1aS,3aR,6bR)-5-(2-Chloracetylaminocarbonyloxymethyl)-1-oxo-1a,2,3,3a,4,6b-hexahydro-1H-2,6a-diazacyclobut[cd]inden-2,6-dicarbonsäure-di-t-butylester (2,0 g; 4 mmol) wird in THF (20 ml) und Methanol (8,5 ml) gelöst und mit Natriumbicarbonat (670 mg; 8 mmol) in Wasser (8,5 ml) versetzt. Nach 18 Stunden bei Raumtemperatur werden die organischen Lösungsmittel abgedampft, der Rückstand mit gesättigter wässriger Kochsalzlösung (25 ml) versetzt und mit Aethylacetat (2x100 ml) extrahiert. Die vereinigten organischen Phasen werden mit gesättigter wässriger Kochsalzlösung (2x25 ml) gewaschen, über Magnesiumsulfat getrocknet und eingeengt. Der erhaltene harzige Rückstand wird mit n-Hexan (20 ml) wahrend 2 Stunden verrührt und abgenutscht. Ausbeute: 1,47 g (87%) als farbloses Pulver. Smp. 128-133 °C.

| Mikroanalyse: $C_{20}H_{29}N_3O_7$ | | | |
|---|---|---|---|
| Ber. | C 56,73 | H 6,90 | N 9,92 |
| Gef. | C 56,67 | H 6,92 | N 9,63 |

Der (1aS,3aR,6bR)-5-Carbamoyloxymethyl-1-oxo-1a,2,3,3a,4,6b-hexahydro-1H-2,6a-diazacyclobut[cd]-inden-2,6-dicarbonsäure-di-t-butylester kann auch in Analogie zu Beispiel 89 ausgehend von (1aS,3aR,6bR)-5-Hydroxymethyl-1-oxo-1a,2,3,3a,4,6b-hexahydro-1H-2,6a-diazacyclobut[cd]inden-2,6-dicarbonsäure-di-t-butylester (3,93 g; 10,33 mmol) und Ammoniumchlorid (1,1 g; 20,6 mmol) hergestellt werden. Ausbeute 3,84 g (90%) als farbloses Pulver.

Beispiel 27

(a) (1aS,3aR,6bR)-5-Carbamoyloxymethyl-2-(4-hydroxy-phenylcarbamoyl)-1-oxo-1a,2,3,3a,4,6b-hexahydro-1H-2,6a-diazacyclobut[cd]inden-6-carbonsäure-Natriumsalz

Diese Verbindung wird in der gleichen Weise wie in Beispiel 3(a) angegeben ausgehend von (1aS,3aR,6bR)-5-Carbamoyloxymethyl-1-oxo-1a,2,3,3a,4,6b-hexahydro-1H-2,6a-diazacyclobut[cd]inden-6-carbonsäure-trifluoracetat (310 mg; 0,83 mmol; aus Beispiel 26) hergestellt. Ausbeute: 215 mg (61%) als farbloses Pulver.

IR (KBr): 3407, 1758, 1718, 1643, 1610 $cm^{-1}$

MS (ISN): $(M\text{-}Na)^-$ 401,4

In analoger Weise wird hergestellt:

(b) (1aS,3aR,6bR)-5-Carbamoyloxymethyl-2-(4-carbamoyl-phenylcarbamoyl)-1-oxo-1a,2,3,3a,4,6b-hexahydro-1H-2,6a-diazacyclobut[cd]inden-6-carbonsäure-Natriumsalz. Ausbeute: 46% als farbloses Pulver.

IR (KBr): 3371, 1751, 1713, 1658, 1607, 1525 $cm^{-1}$

MS (ISN): $(M\text{-}Na)^-$ 428,3

(c) (1aS,3aR,6bR)-2-Benzylcarbamoyl-5-carbamoyloxymethyl-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 3(a) ausgehend von (1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (372 mg; 1,00 mmol) hergestellt. Ausbeute: 83 mg (20 %) als gelber Feststoff.

IR (KBr): 1741, 1608, 1399 $cm^{-1}$

MS (ISN): $(M\text{-}Na)^-$ 399,3

In analoger Weise werden hergestellt:

(d) (1aS,3aR,6bR)-5-Carbamoyloxymethyl-2-cyclopropylcarbamoyl-1-oxo-1a,2,3,3a,4,6b-hexahydro-1H-2,6a-diaza-cyclobut[cd]inden-6-carbonsäure-Natriumsalz

Ausbeute: 65 % als farbloses Pulver.

IR (KBr): 1747, 1609, 1529, 1400, 1249 $cm^{-1}$

MS (ISN): $(M\text{-}Na)^-$ 349,3

(e) (1aS,3aR,6bR)-5-Carbamoyloxymethyl-2-(4-sulfamoyl-benzylcarbamoyl)-1-oxo-1a,2,3,3a,4,6b-hexahydro-1H-2,6a-diaza-cyclobut[cd]inden-6-carbonsäure-Natriumsalz

Ausbeute: 57 % als farbloses Pulver.

IR (KBr): 1746, 1607, 1534, 1398, 1318, 1160 $cm^{-1}$

MS (ISN): $(M\text{-}Na)^-$ 478,4

(f) (1aS,3aR,6bR)-5-Carbamoyloxymethyl-2-(thiophen-2-ylmethylcarbamoyl)-1-oxo-1a,2,3,3a,4,6b-hexahydro-1H-2,6a-diaza-cyclobut[cd]inden-6-carbonsäure-Natriumsalz

Ausbeute: 53 % als farbloses Pulver.

IR (KBr): 1741, 1609, 1526, 1400, 1246 $cm^{-1}$

MS (ISN): $(M\text{-}Na)^-$ 405,4

(g) (1aS,3aR,6bR)-5-Carbamoyloxymethyl-1-oxo-2-(2-thiophen-2-yl-äthylcarbamoyl)-1a,2,3,3a,4,6b-hexahydro-1H-2,6a-diaza-cyclobut[cd]inden-6-carbonsäure-Natriumsalz

Ausbeute: 64 % als farbloses Pulver.

IR (KBr): 1744, 1710, 1608, 1533, 1400 $cm^{-1}$

MS (ISN): $(M\text{-}Na)^-$ 419,2

(h) (1aS,3aR,6bR)-5-Carbamoyloxymethyl-2-(4-hydroxy-benzylcarbamoyl)-1-oxo-1a,2,3,3a,4,6b-hexahydro-1H-2,6a-diaza-cyclobut[cd]inden-6-carbonsäure-Natriumsalz

Ausbeute: 57 % als farbloses Pulver.

IR (KBr): 1745, 1612, 1514, 1398 $cm^{-1}$

MS (ISN): $(M\text{-}Na)^-$ 415,4

Beispiel 28

(1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (5-Hydroxymethyl "Baustein")

Diese Verbindung kann durch folgende Reaktionssequenz a) - h) erhalten werden:

a) 1:1 Gemisch von (1S,4S,5S)-[2-Benzyloxycarbonyl-7-oxo-4-[(R)- und (S)-tetrahydro-pyran-2-yloxy]-2,6-diaza-bicyclo[3.2.0]heptan-6-yl]essigsäure-Natriumsalz (1:1)

(1S,4S,5S)-2-Benzyloxycarbonyl-4-[(R)-und (S)-tetrahydropyran-2-yloxy]-2,6-diaza-bicyclo[3.2.0]heptan-7-on (1:1 Gemisch; 62.46 g; 0.18 mol; Europäische Offenlegungsschrift 508 234, Beispiel 14) in THF (Tetrahydrofuran; 1 l) wird unter Rühren bei -78 °C mit einer Bistrimethylsiyllithiumamidlösung (396 ml, 1M in THF) versetzt. Bromessigsäure (27.56 g, 0.198 mol) in THF (100 ml) wird zugetropft, und das Reaktionsgemisch wird während zwei Tage bei 0 °C weitergerührt. Das Reaktionsgemisch wird bei -10 °C mit Aethylacetat und Wasser verdünnt. Die organische Phase wird mit Wasser gewaschen und die vereinigten Wasserphasen mit Kohle behandelt und filtriert. Der pH der Lösung wird bei 0 °C auf 3.5 gestellt, und die Lösung wird mit Aethylacetat extrahiert. Die organische Lösung wird mit einer gesättigten wässrigen Kochsalzlösung gewaschen, über Magnesiumsulfat getrocknet, filtriert und eingeengt. Man erhält 65.12 g (89%) eines farbloses Oel. Ein Teil davon (0.5g) wird mit Natriumbicarbonat (105 mg) versetzt und über ein hydrophobes Polymer chromatographiert (Laufmittel: Wasser). Man erhält 160 mg eines weissen Pulvers.
MS (ISN): 403.5 (M-Na)

| Mikroanalyse: $C_{20}H_{23}N_2O_7Na$ | | | |
|---|---|---|---|
| Ber.<br>Gef. | C 56.34<br>C 55.89 | H 5.44<br>H 5.35 | N 6.57<br>N 6.43 |

b) 1:1 Gemisch von (1S,4S,5S)-[7-Oxo-4-[(R)- und (S)-tetrahydropyran-2-yloxy]-2,6-diaza-bicyclo[3.2.0]-heptan-6-yl]-essigsäure

Eine Lösung von (1S,4S,5S)-[2-Benzyloxycarbonyl-7-oxo-4-[(R) und (S)-tetrahydro-pyran-2-yloxy]-2,6-diaza-bicyclo[3.2.0]heptan-6-yl]-essigsäure (61.9 g, 0.153 mol) in Aethanol (1l) wird über Palladiumkohle hydriert. Der Katalysator wird abgenutscht und die Lösung eingeengt. Man erhält 43 g eines farbloses Oel. Ein Teil davon (0.5 g) wird über ein hydrophobes Polymer chromatographiert (Laufmittel: Wasser). Man erhält 131 mg eines weissen Pulvers.
MS (ISN): 269.3 (M-H)

| Mikroanalyse: $C_{12}H_{18}N_2O_5$ | | | |
|---|---|---|---|
| Ber.<br>Gef. | C 53.33<br>C 53.39 | H 6.71<br>H 6.53 | N 10.36<br>N 10.45 |

c) 1:1 Gemisch von (1S,4S,5S)-[2-Allyloxycarbonyl-7-oxo-4-[(R)- und (S)-tetrahydropyran-2-yloxy]-2,6-diaza-bicyclo[3.2.0]heptan-6-yl]-essigsäure-Natriumsalz

Eine Lösung von (1S,4S,5S)-[7-Oxo-4-[(R)- und (S)-tetrahydropyran-2-yloxy]-2,6-diaza-bicyclo[3.2.0]-heptan-6-yl]-essigsäure (43 g; 0.153 mol) in Wasser (400 ml) wird mit 2N NaOH bei 0 °C auf pH 7.5 gestellt. Die Lösung wird mit Chlorameisensäure-allylester (22.13 g, 0.183 mol) versetzt und während zwei Stunden bei 0 °C gerührt. Die Lösung wird mit Kohle behandelt und filtriert. Das Filtrat wird auf pH 3 eingestellt. Die Lösung wird mit Aethylacetat extrahiert, getrocknet und eingeengt. Man erhält 54 g (100%) eines farbloses Oel. Ein Teil davon (400 mg) wird mit Natriumbicarbonat (126 mg) versetzt und über ein hydrophobes Polymer chromatographiert (Laufmittel: Wasser). Man erhält 264 mg eines weissen Pulvers.
MS (ISN): 353.4 (M-Na)

| Mikroanalyse: $C_{12}H_{18}N_2O_5$ | | | |
|---|---|---|---|
| Ber. | C 51.06 | H 5.62 | N 7.44 |
| Gef. | C 50.73 | H 5.90 | N 7.42 |

d) 1:1 Gemisch von (1S,4S,5S)-6-Allyloxycarbonylmethyl-7-oxo-4-[(R)- und (S)-tetrahydropyran-2-yloxy]-2,6-diaza-bicyclo[3.2.0]heptan-2-carbonsäure-allylester

Eine Lösung von (1S,4S,5S)-[2-Allyloxycarbonyl-7-oxo-4[(R)- und (S)-tetrahydropyran-2-yloxy]-2,6a-diaza-bicyclo[3.2.0] heptan-6-yl]-essigsäure (54 g; 0.153 mol) in Aceton (1l) wird mit Triäthylamin (23,4 ml; 0.168 mol) und Allylbromid (28.47 ml; 0.336 mol) versetzt. Die Lösung wird 24 Stunden gerührt und anschliessend eingeengt. Der Rückstand wird in Aethylacetat gelöst, mit Wasser und gesättigter Kochsalzlösung gewaschen, über Magnesiumsulfat getrocknet und eingeengt. Der Rückstand wird über Kieselgel chromatographiert (Laufmittel Aethylacetat/n-Hexan 1:1). Man erhält 43.4 g (72%) eines farblosen Oels.
IR(Film) 1773, 1740, 1707 $cm^{-1}$

| Mikroanalyse: $C_{19}H_{26}N_2O_7$ | | | |
|---|---|---|---|
| Ber. | C 57.86 | H 6.64 | N 7.10 |
| Gef. | C 57.91 | H 6.64 | N 6.93 |

e) 1:1:1:1 Gemisch von (1S,4S,5S)-6-[(R)- und (S)-1-Allyloxycarbonyl-3-(t-butyl-dimethyl-silanyloxy)-2-oxo-propyl]-7-oxo-4-[(R)- und (S)-tetrahydropyran-2-yloxy]-2,6-diaza-bicyclo[3.2.0]heptan-2-carbonsäure-allylester

Eine Lösung von (1S,4S,5S)-6-Allyloxycarbonylmethyl-7-oxo-4-[(R)- und (S)-tetrahydropyran-2-yloxy]-2,6-diaza-bicyclo[3.2.0]heptan-2-carbonsäure-allylester (49.6 g, 0.125 mol) in THF (500 ml) wird bei -78°C vorgelegt und nacheinander mit Bis-trimethylsilyllithiumamid (1M in THF, 0.257 mol) und mit t-Butyl-dimethylsilyloxyacetylchlorid (28.7 g, 0.137 mol) versetzt. Nach 30 Minuten werden 1N wässrige Salzsäure (130 ml) und eine gesättigte wässrige Kochsalzlösung (130 ml) zugetropft. Das Reaktionsgemisch wird mit Aethylacetat verdünnt, getrocknet, eingeengt und über Kieselgel chromatographiert (Laufmittel Aethylacetat/n-Hexan 3.5/6.5). Man erhält 64 g (90%) eines gelblichen Oels.
IR(Film) 1779, 1743,1712 $cm^{-1}$

| Mikroanalyse: $C_{27}H_{42}N_2O_9$ | | | |
|---|---|---|---|
| Ber. | C 57.22 | H 7.47 | N 4.94 |
| Gef. | C 57.49 | H 7.67 | N 4.94 |

f) 1:1 Gemisch von (1S,4S,5S)-6-[(R)- und (S)-1-Allyloxycarbonyl-3-(t-butyldimethyl-silanyloxy)-2-oxo-propyl]-4-hydroxy-7-oxo-2,6-diazabicyclo[3.2.0]heptan-2-carbonsäure-allylester

Eine Lösung von (1S,4S,5S)-6-[(R)- und (S)-1-Allyloxycarbonyl-3-(t-butyldimethyl-silanyloxy)-2-oxo-propyl]-7-oxo-4-[(R)- und (S)-tetrahydropyran-2-yloxy]-2,6-diaza-bicyclo[3.2.0]heptan-2-carbonsäure-allylester (62.12 g; 109 mmol) in Aether (1l) wird mit fein gemahlenem Magnesiumbromidaetherat (83 g; 328 mmol) versetzt. Nach 45 Minuten wird die Suspension mit Wasser (1l) tropfenweise versetzt. Die organische Phase wird mit Wasser und gesättigter wässriger Kochsalzlösung gewaschen, getrocknet und eingeengt. Der Rückstand wird über Kieselgel chromatographiert (Laufmittel Aethylacetat/n-Hexan 1:1). Man erhält 38.8 g (73%) eines
gelblichen Oels.
IR(Film): 3434, 1776, 1746,1709 $cm^{-1}$

| Mikroanalyse: $C_{22}H_{34}N_2O_8Si$ | | | |
|---|---|---|---|
| Ber. | C 54.75 | H 7.10 | N 5.80 |
| Gef. | C 54.95 | H 7.22 | N 5.49 |

g) (1aS,3aR,6bS)-5-(t-Butyl-dimethyl-silanyloxymethyl)-1-oxo-1,1a,2,3,3a,6b-hexahydro-4-oxa-2,6a-diaza-cyclobut[cd]inden-2,6-dicarbonsäure-diallylester

Eine Lösung von (1S,4S,5S)-6-[(R)- und (S)-1-Allyloxycarbonyl-3-(t-butyl-dimethyl-silanyloxy)-2-oxo-propyl]-4-hydroxy-7-oxo-2,6-diazabicyclo[3.2.0]heptan-2-carbonsäure-allylester (38.7 g; 80 mmol) in THF (500 ml) wird nacheinander, bei -25 °C, mit Triphenylphosphin (31.47 g, 0.12 mol) und einer Lösung von Azodicarbonsäure-diäthylester (19.5 g, 0.112 mol) in THF (20 ml) unter Rühren versetzt. Das Reaktionsgemisch wird 5 Stunden bei Raumtemperatur weitergerührt. Das Lösungsmittel wird eingeengt und der Rückstand in Aethylacetat gelöst und mit gesättigter wässriger Ammoniumchloridlösung versetzt. Die organische Phase wird mit Wasser gewaschen und über Magnesiumsulfat getrocknet und eingeengt. Der Rückstand wird in Aether/n-Hexan (1:1; 500 ml) aufgenommen und die erhaltenen Kristalle abgenutscht. Die Mutterlauge wird eingeengt und über Kieselgel chromatographiert (Laufmittel Aethylacetat/n-Hexan 3:7). Man erhält 24.4 g (65.5%) eines gelblichen Oels.
IR(Film): 1788, 1716, 1619 $cm^{-1}$

| Mikroanalyse: $C_{22}H_{32}N_2O_7Si$ | | | |
|---|---|---|---|
| Ber. | C 56.88 | H 6.94 | N 6.03 |
| Gef. | C 56.92 | H 7.11 | N 6.05 |

h) (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester

Eine Lösung von (1aS,3aR,6bS)-5-(t-Butyl-dimethyl-silanyloxymethyl)-1-oxo-1,1a,2,3,3a,6b-hexahydro-4-oxa-2,6a-diaza-cyclobut[cd]inden-2,6-dicarbonsäure-diallylester (24.3 g; 52.3 mmol) in Aethanol (200 ml) und Wasser (1 ml) wird mit Pyridinium-(toluol-4-sulfonat) (6.6 g; 26 mmol) versetzt und während 4 Stunden auf 50 °C erwärmt. Das Lösungsmittel wird eingeengt, der Rückstand in Aethylacetat aufgenommen und mit Wasser und gesättigter wässriger Kochsalzlösung gewaschen, getrocknet und eingeengt. Der Rückstand wird auf Kieselgel chromatographiert (Laufmittel Aethylacetat/n-Hexan 7:3). Man erhält 13,43 g (73.3%) eines gelblichen Oels.
IR(Film): 1783, 1710, 1614, 1413 $cm^{-1}$

| Mikroanalyse: $C_{16}H_{318}N_2O_7$ | | | |
|---|---|---|---|
| Ber. | C 54.86 | H 5.18 | N 8.00 |
| Gef. | C 54.32 | H 5.35 | N 7.77 |

Beispiel 29

(a) (1aS,3aR,6bS)-5-(5-Methyl-1,3,4-thiadiazol-2-ylsulfanylmethyl)-1-oxo-1,1a,2,3,3a,6b-hexahydro-4-oxa-2,6a-diaza-cyclobut[cd]inden-6-carbonsäure-Natriumsalz

Eine Lösung von (1aS,3aR,6bS)-5-(5-Methyl-1,3,4-thiadiazol-2-ylsulfanylmethyl)-1-oxo-1,1a,2,3,3a,6b-hexahydro-4-oxa-2,6a-diazacyclobut[cd]inden-2,6-dicarbonsäure-diallylester (1.25 g; 2.69 mmol) in Aethylacetat (25 ml) wird mit Bis-(triphenylphosphin)-palladium(II)dichlorid (37 mg; 0.054 mmol) und Essigsäure (1.23 ml; 21 mmol) versetzt. Die erhaltene Lösung wird tropfenweise mit Tributylzinnhydrid (3.91 g; 13.45 mmol) versetzt. Nach 5-stündigem Rühren wird die Lösung mit n-Hexan verdünnt; die erhaltenen Kristalle werden abgenutscht und getrocknet (740 mg; 81%). Diese Kristalle werden in wenig Wasser gelöst, mit Natriumbicarbonat (180 mg) versetzt und über ein hydrophobes Polymer chromatographiert (Laufmittel: Wasser). Man

erhält 158 mg als farbloses Pulver.
IR(KBr): 1750, 1626, 1591 $cm^{-1}$
MS(ISP): 341.2 $(M+H)^+$

Die dabei eingesetzte Ausgangsverbindung wird wie folgt hergestellt:

Eine Lösung von (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (350 mg; 1 mmol) in THF (10 ml) wird mit 2-Mercapto-5-methyl-1,3,4-thiadiazol (158 mg; 1.2 mmol) und Triphenylphosphin versetzt. Die erhaltene Lösung wird tropfenweise bei -20 °C mit einer Lösung von Azodicarbonsäure-diaetylester (226 mg; 1.3 mmol) in THF (5 ml) unter Rühren versetzt. Die Lösung wird während 3 Stunden bei 0 °C weitergerührt. Das Lösungsmittel wird eingeengt und der Rückstand auf Kieselgel mit Methylenchlorid:Aether (7:3) chromatographiert. Man erhält ein gelblichen Oels (266 mg; 57%).
IR(KBr): 1784, 1712, 1613 $cm^{-1}$
MS(ISP): 465.4 $(M+H)^+$

In Analogie hierzu werden hergestellt:

(b) (1aS,3aR,6bS)-1-Oxo-5-(pyridin-4-ylsulfanylmethyl)-1,1a,2,3,3a,6b-hexahydro-4-oxa-2,6a-diaza-cyclobut-[cd]inden-6-carbonsäure

Ausgehend von (1aS,3aR,6bS)-1-Oxo-5-pyridin-4-ylsulfanylmethyl-1,1a,2,3,3a,6b-hexahydro-4-oxa-2,6a-diaza-cyclobut[cd]inden-2,6-dicarbonsäure-diallylester (1.14 g; 2.57 mmol) werden 40 mg (5%) als farbloser Feststoff erhalten.
IR(KBr): 1750, 1623, 1578 $cm^{-1}$
MS(ISP): 320.3 $(M\text{-}Na+2H)^+$

Der als Ausgangsverbindung eingesetzte (1aS,3aR,6bS)-1-Oxo-5-(pyridin-4-ylsulfanylmethyl)-1,1a,2,3,3a,6b-hexahydro-4-oxa-2,6a-diazacyclobut[cd]inden-2,6-dicarbonsäure-diallylester wird ausgehend von (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (1.98 g; 5.66 mmol) und 4-Mercaptopyridin (0.756 g; 6.8 mmol) wie im Beispiel 29(a) erhalten: 1.47 g (58%).
IR(KBr): 1784, 1709, 1612, 1573 $cm^{-1}$
MS(EI): 444 $(M+H)^+$, 333 (M-SPh)

(c) (1aS,3aR,6bS)-5-(5-Amino-1,3,4-thiadiazol-2-ylsulfanylmethyl)-1-oxo-4-oxa-1,1a,2,3,3a,6b-hexahydro-2,6a-diaza-cyclobut[cd]inden-6-carbonsäure-Natriumsalz

Ausgehend von (1aS,3aR,6bS)-5-(5-Amino-1,3,4-thiadiazol-2-ylsulfanylmethyl)-1-oxo-4-oxa-1,1a,2,3,3a,6b-hexahydro-2,6a-diaza-cyclobut[cd]inden-2,6-dicarbonsäure-diallylester (1.4 g; 3 mmol) werden 320 mg (29%) als farbloser Feststoff erhalten.
IR(KBr): 1748, 1622, 1583, 1496 $cm^{-1}$
MS(ISN): 340.2 $(M\text{-}Na)^-$

Als Nebenprodukt wird (1aS,3aR,6bS)-2-Allyloxycarbonyl-5-(5-amino-1,3,4-thiadiazol-2-ylsulfanylmethyl)-1-oxo-4-oxa-1,1a,2,3,3a,6b-hexahydro-2,6a-diaza-cyclobut[cd]inden-6-carbonsäure-Natriumsalz isoliert: 49 mg (4%).
IR(KBr): 1761, 1704, 1630, 1596, 1494 $cm^{-1}$
MS(ISP): 448 $(M+H)^+$, 426.3 (M-Na + H)

Der als Ausgangsverbindung eingesetzte (1aS,3aR,6bS)-5-(5-Amino-1,3,4-thiadiazol-2-ylsulfanylmethyl)-1-oxo-4-oxa-1,1a,2,3,3a,6b-hexahydro-2,6a-diaza-cyclobut[cd]inden-2,6-dicarbonsäure-diallylester kann in Analogie zu Beispiel 16 ausgehend von (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-di-allylester (100 mg; 0.28 mmol), 2-Amino-5-mercapto-1,3,4-thiadiazol (41 mg; 0.31 mmol) und Mesylchlorid (36 mg; 0.31 mmol) hergestellt werden: 117 mg (90%).
IR(KBr): 1782, 1709, 1611, 1493 $cm^{-1}$
MS(ISP): 488.3 $(M+Na)^+$; 466.3 $(M+H)^+$

(d) (1aS,3aR,6bS)-5-(2-Carbamoyl-5-methyl[1,2,4]triazolo[1,5-a]pyrimidin-7-ylsulfanylmethyl)-1-oxo-1,1a,2,3,3a,6b-hexahydro-4-oxa-2,6a-diaza-cyclobut[cd]inden-6-carbonsäure

Ausgehend von (1aS,3aR,6bS)-5-(2-Carbamoyl-5-methyl[1,2,4]triazolo[1,5-a]pyrimidin-7-ylsulfanylmethyl)-1-oxo-1,1a,2,3,3a,6b-hexahydro-4-oxa-2,6a-diaza-cyclobut[cd]inden-2,6-dicarbonsäure-diallylester (190

mg; 3 mmol) werden 62 mg (42%) als farbloser Feststoff erhalten.
IR(KBr): 1753, 1691, 1525, 1594, 1512 $cm^{-1}$
MS(ISP): 440.3 $(M+Na)^+$; 418.4 $(M+H)^+$; 390.3 (M-CO)

Der als Ausgangsverbindung eingesetzte (1aS,3aR,6bS)-5-(2-Carbamoyl-5-methyl[1,2,4]triazolo[1,5-a]-pyrimidin-7-ylsulfanylmethyl)-1-oxo-1,1a,2,3,3a,6b-hexahydro-4-oxa-2,6a-diaza-cyclobut[cd]inden-2,6-dicarbonsäure-diallylester wird ausgehend von (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (1.05 g; 3 mmol) und 7-Mercapto-5-methyl[1,2,4]triazolo[1,5-a]pyrimidin-2-carboxamid (648 mg; 3 mmol) wie im Beispiel 29(c) hergestellt: 1.15 g (70%).
IR(KBr):1785, 1707, 1596, 1511 $cm^{-1}$

| Mikroanalyse: $C_{23}H_{23}N_7O_7S$ | | | | |
|---|---|---|---|---|
| Ber. | C 51.01 | H 4.28 | N 18.11 | S 5.92 |
| Gef. | C 50.76 | H 4.36 | N 18.12 | S 5.95 |

(e) (1aS,3aR,6bS)-5-(1-Methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(1-Methyl-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester (122 mg; 0.25 mmol) werden 31 mg (36%) als farbloser Feststoff erhalten.
IR(KBr): 1752, 1626, 1588, 1387 $cm^{-1}$
MS(ISP): 347,3 $(M+Na)^+$; 342,4 $(M+NH_4)^+$; 325,3 $(M+H)^+$

Als Nebenprodukt wird (1aS,3aR,6bS)-5-(1-Methyl-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-2-allylester-Natriumsalz als farbloser Feststoff isoliert (34 mg; 32%).
IR(KBr): 1763, 1710, 1631, 1597 $cm^{-1}$
MS(ISP): 431.4 $(M+Na)^+$; 426.4 $(M+NH_4)^+$; 409.4 $(M+H)^+$

Der als Ausgangsverbindung eingesetzte (1aS,3aR,6bS)-5-(1-Methyl-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester wird ausgehend von (1aS,3aR,6bS)-5-Hydroxymethyl-1-oxo-1,1a,2,3,3a,6b-hexahydro-4-oxa-2,6a-diaza-cyclobut[cd]-inden-2,6-dicarbonsäure-diallylester (3 g; 8,6 mmol) und bis-(5-Mercapto-1-methyltetrazolyl)-dithiocarbonat (2.54 g; 9.84 mmol) in Analogie zu Beispiel 15 hergestellt: 2.47 g; 64%.
IR (KBr): 1784, 1710, 1615, 1412 $cm^{-1}$
MS(ISP): 471.4 $(M+Na)^+$; 466.4 $(M+NH_4)^+$; 449.4 $(M+H)^+$

(f) (1aS,3aR,6bS)-5-(Carbamoyloxymethyl)-1-oxo-1,1a,2,3,3a,6b-hexahydro-4-oxa-2,6a-diaza-cyclobut[cd]-inden-6-carbonsäure

Diese Verbindung wird ausgehend von (1aS,3aR,6bS)-5-(Carbamoyloxymethyl)-1-oxo-1,1a,2,3,3a,6b-hexahydro-4-oxa-2,6a-diaza-cyclobut[cd]inden-2,6-dicarbonsäure-diallylester erhalten.
IR (KBr): 1770, 1692, 1645, 1414 $cm^{-1}$

Der als Ausgangsverbindung eingesetzte (1aS,3aR,6bS)-5-(Carbamoyloxymethyl)-1-oxo-1,1a,2,3,3a,6b-hexahydro-4-oxa-2,6a-diaza-cyclobut[cd]inden-2,6-dicarbonsäure-diallylester wird wie folgt hergestellt:

(1aS,3aR,6bS)-5-(2-Chloracetylaminocarbamoyloxymethyl)-1-oxo-1,1a,2,3,3a,6b-hexahydro-4-oxa-2,6a-diaza-cyclobut[cd]inden-2,6-dicarbonsäure-diallylester.

Ausgehend von (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (2 g; 5,7 mmol) und Chloracetylisocyanat (1.0g; 8.56 mmol) erhält man in Analogie zu Beispiel 26 2.56 g (95%) als farbloses Pulver.
IR(Film): 1787, 1713, 1625, 1497 $cm^{-1}$

| Mikroanalyse: $C_{19}H_{20}N_3O_9Cl$ | | | |
|---|---|---|---|
| Ber. | C 48.57 | H 4.29 | N 8.94 |
| Gef. | C 48.73 | H 4.51 | N 8.76 |

(1aS,3aR,6bS)-5-(Carbamoyloxymethyl)-1-oxo-1,1a,2,3,3a,6b-hexahydro-4-oxa-2,6a-diaza-cyclobut[cd]inden-2,6-dicarbonsäure-diallylester

Ausgehend von (1aS,3aR,6bS)-5-(2-Chloracetylaminocarbamoyloxymethyl)-1-oxo-1,1a,2,3,3a,6b-hexahydro-4-oxa-2,6a-diaza-cyclobut[cd]inden-2,6-dicarbonsäure-diallylester (2.56 g; 5.45 mmol) und $NaHCO_3$ - (0.915 g; 10.9 mmol) erhält man in Analogie zu Beispiel 26 1.33 g (62%) als farbloses Pulver.
IR(Film): 1798, 1708, 1646, 1414 $cm^{-1}$

| Mikroanalyse: $C_{17}H_{19}N_3O_8$ | | | |
|---|---|---|---|
| Ber. | C 51.91 | H 4.87 | N 10.68 |
| Gef. | C 51.67 | H 4.88 | N 10.52 |

(g) (1aS,3aR,6bS)-5-(1-(Cyclopropyl-carbamoylmethyl)-1H-tetrazol-5-ylsulfanylmethyl)-1-oxo-1,1a,2,3,3a,6b-hexahydro-4-oxa-2,6a-diaza-cyclobut[cd]inden-6-carbonsäure

Ausgehend von (1aS,3aR,6bS)-5-(1-(Cyclopropyl-carbamoylmethyl)-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester (722 mg; 1,36 mmol) werden 526 mg (95%) als gelblicher Feststoff erhalten.
IR(KBr): 3317, 1778, 1694, 1546, 1213 $cm^{-1}$ MS(ISP): 430,3 $(M+Na)^+$; 425 $(M+NH_4)^+$; 408,3 $(M+H)^+$

Der dabei als Ausgangsverbindung eingesetzte (1aS,3aR,6bS)-5-(1-(Cyclopropyl-carbamoylmethyl)-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester kann wie folgt hergestellt werden:

Eine Lösung von (1aS,3aR,6bS)-5-Hydroxymethyl-1-oxo-1,1a,2,3,3a,6b-hexahydro-4-oxa-2,6-diaza-cyclobut[cd]inden-2,6-dicarbonsäure-diallylester (500 mg; 1,4 mmol; aus Beispiel 28) in abs. THF wird bei 0 °C mit Triäthylamin (500 µl; 3,6 mmol) und Mesylchlorid (127 µl; 1,6 mmol) versetzt. Während 0,5 Stunden wird bei 0 °C gerührt und anschliessend mit einer Lösung von N-Cyclopropyl-2-(5-mercapto-tetrazol-1-yl)-acetamid (326 mg; 1,6mmol) in abs. THF (7 ml) versetzt. Bei Raumtemperatur wird 3,5 Stunden gerührt. Die enstandene weisse Suspension wird versetzt mit 50ml eines Gemisches bestehend aus 25 ml gesättigter, wässriger Kochsalzlösung, 12,5 ml Wasser und 12,5 ml eines wässrigen 2M Dikaliumhydrogenphosphat/ Kaliumdihydrogenphosphat-Puffers, pH 6. Das Gemisch wird mit Aethylacetat (2x100 ml) extrahiert. Die organischen Phasen werden mit gesättigter, wässriger Kochsalzlösung (50 ml) gewaschen, vereinigt, über Magnesiumsulfat getrocknet und eingeengt. Der Rückstand wird an Kieselgel mit Aethylacetat/n-Hexan 4:1 chromatographiert. Man erhält 734 mg (97%) eines weissen Schaumes.
IR(KBr): 1782, 1707, 1613, 1546, 1097 $cm^{-1}$
MS(ISP): 532,4 $(M+H)^+$

(h) (1aS,3aR,6bS)-1-Oxo-5-(1-(phenyläthyl-carbamoylmethyl)-1H-tetrazol-5-ylsulfanylmethyl)-1,1a,2,3,3a,6b-hexahydro-4-oxa-2,6a-diaza-cyclobut[cd]inden-6-carbonsäure

Ausgehend von (1aS,3aR,6bS)-1-Oxo-5-(1-(phenyläthyl-carbamoylmethyl)-1H-tetrazol-5-ylsulfanylmethyl)-1,1a,2,3,3a,6b-hexahydro-4-oxa-2,6a-diaza-cyclobut[cd]inden-2,6-dicarbonsäure-diallylester (297 mg; 0,50 mmol) werden 218 mg (93%) als gelber Feststoff erhalten.
IR(KBr): 1774, 1680, 1554, 1214, 750, 702 $cm^{-1}$
MS(ISP): 494 $(M+Na)^+$; 489,4 $(M+NH_4)^+$; 472,3 $(M+H)^+$

Der dabei als Ausgangsverbindung eingesetzte (1aS,3aR,6bS)-1-Oxo-5-(1-(phenyläthyl-carbamoylmethyl)-1H-tetrazol-5-ylsulfanylmethyl)-1,1a,2,3,3a,6b-hexahydro-4-oxa-2,6a-diaza-cyclobut[cd]inden-2,6-dicarbonsäure-diallylester kann in Analogie zu Beispiel 29(g) aus (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (194 mg; 0,55 mmol; aus Beispiel 28) und 2-(5-Mercapto-tetrazol-1-yl)-N-phenyläthyl-acetamid (167 mg; 0,64mmol) hergestellt werden. Man erhält 303 mg (92%) eines weissen Schaumes.
IR(KBr): 3340, 1785, 1708, 1614, 1549, 1214, 759, 701 $cm^{-1}$
MS(ISP): 618 $(M+Na)^+$; 613,3 $(M+NH_4)^+$; 596,3 $(M+H)^+$

(i) (1aS,3aR,6bS)-5-(1-(Carbamoylmethyl-carbamoylmethyl)-1H-tetrazol-5-ylsulfanylmethyl)-1-oxo-1,1a,2,3,3a,6b-hexahydro-4-oxa-2,6a-diaza-cyclobut[cd]inden-6-carbonsäure

Ausgehend von (1aS,3aR,6bS)-5-(1-(Carbamoylmethyl-carbamoylmethyl)-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester (424 mg; 0,77 mmol) werden 119 mg (36%) als weisser Feststoff erhalten.
IR(KBr): 3407, 1757, 1671, 1630, 1398, 1218 $cm^{-1}$
MS(ISP): 447,2 $(M+Na)^+$; 442,3 $(M+NH_4)^+$; 425,3 $(M+H)^+$

Der dabei als Ausgangsverbindung eingesetzte (1aS,3aR,6bS)-5-(1-(Carbamoylmethyl-carbamoylmethyl)-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester kann in Analogie zu Beispiel 29(g) aus (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (245 mg; 0,70 mmol) und N-Carbamoylmethyl-2-(5-mercapto-tetrazol-1-yl)-acetamide (175 mg; 0,81mmol) hergestellt werden. Man erhält 365 mg (95%) eines weissen Schaumes.
IR(KBr): 3432, 1784, 1706, 1613 $cm^{-1}$
MS(ISP): 571 $(M+Na)^+$; 566,3 $(M+NH_4)^+$; 549,3 $(M+H)^+$

(j) (1aS,3aR,6bS)-5-(1-Carbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure

Ausgehend von (1aS,3aR,6bS)-5-(1-Carbamoylmethyl-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester (460 mg; 0,96 mmol) werden 336 mg (95%) als weisser Feststoff erhalten.
IR(KBr): 3410, 1775, 1695, 1612, 1214 $cm^{-1}$
MS(ISP): 390,2 $(M+Na)^+$; 385,2 $(M+NH_4)^+$; 368,2 $(M+H)^+$

Der dabei als Ausgangsverbindung eingesetzte (1aS,3aR,6bS)-5-(1-Carbamoylmethyl-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester kann in Analogie zu Beispiel 29(g) aus (1aS,3aR,6bS)-5-Hydroxymethyl-1-oxo-1,1a,2,3,3a,6b-hexahydro-4-oxa-2,6a-diaza-cyclobut[cd]indene-2,6-dicarbonsäure-diallylester (400 mg; 1,1 mmol; aus Beispiel 28) und 2-(5-Mercapto-tetrazol-1-yl)-acetamid (210 mg; 1,3 mmol) hergestellt werden. Man erhält 445 mg (82%) eines gelblichen Schaumes.
IR(KBr): 3430, 1783, 1707, 1613, 1214 $cm^{-1}$
MS(ISP): 514,2 $(M+Na)^+$; 509,2 $(M+NH_4)^+$; 492,2 $(M+H)^+$

(k) (1aS,3aR,6bS)-5-(1-Methylcarbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure

Ausgehend von (1aS,3aR,6bS)-5-(1-Methylcarbamoylmethyl-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester (536 mg; 1,1 mmol) werden 420 mg (100%) als gelblicher Feststoff erhalten.
IR(KBr): 3407, 1781, 1693, 1616, 1558, 1411 $cm^{-1}$
MS(ISN): 380,2 $(M-H)^-$

Der dabei als Ausgangsverbindung eingesetzte (1aS,3aR,6bS)-5-(1-Methylcarbamoylmethyl-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester kann in Analogie zu Beispiel 29(g) aus (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (400 mg; 1,1 mmol; aus Beispiel 28) und 2-(5-Mercapto-tetrazol-1-yl)-N-methyl-acetamid (240 mg; 1,4 mmol) hergestellt werden. Man erhält 511 mg (89%) eines weissen Schaumes.
IR(KBr): 3374, 1707, 1614, 1553 $cm^{-1}$
MS(ISP): 528,3 $(M+Na)^+$; 523,3 $(M+NH_4)^+$; 506,4 $(M+H)^+$

(l) (1aS,3aR,6bS)-5-(1-(2-Morpholin-4-yl-2-oxo-äthyl)-1H-tetrazol-5-ylsulfanylmethyl)-1-oxo-1,1a,2,3,3a,6b-hexahydro-4-oxa-2,6a-diazacyclobut[cd]inden-6-carbonsäure

Ausgehend von (1aS,3aR,6bS)-5-(1-(2-Morpholin-4-yl-2-oxo-äthyl)-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester (589 mg; 1,1 mmol) werden 395 mg (86%) als gelblicher Feststoff erhalten.
IR(KBr): 3432, 1777, 1706, 1663, 1614 $cm^{-1}$
MS(ISN): 436,2 $(M-H)^-$

Der dabei als Ausgangsverbindung eingesetzte (1aS,3aR,6bS)-5-(1-(2-Morpholin-4-yl-2-oxo-äthyl)-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester kann in Analogie zu Beispiel 29(g) aus (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (407 mg; 1,2 mmol; aus Beispiel 28) und 2-(5-Mercapto-tetrazol-1-yl)-1-(morpholino-4-yl)-äthanon (306 mg; 1,3 mmol) hergestellt werden. Man erhält 595 mg (91%) eines weissen Schaumes.
IR(KBr): 1783, 1710, 1666, 1614, 1241, 975 $cm^{-1}$
MS(ISP): 584,2 $(M+Na)^+$; 579,3, $(M+NH_4)^+$; 562,3 $(M+H)^+$

| Mikroanalyse: $C_{23}H_{27}N_7O_8S \cdot 0{,}236\ AcOEt \cdot 0{,}200\ H_2O$ | | | | |
|---|---|---|---|---|
| Ber.<br>Gef. | C 49,13<br>C 49,35 | H 5,03<br>H 5,04 | N 16,72<br>N 16,73 | S 5,47<br>S 5,72 |

(m) (1aS,3aR,6bS)-5-(1-(4-Hydroxyphenyl-carbamoylmethyl)-1H-tetrazol-5-ylsulfanylmethyl)-1-oxo-1,1a,2,3,3a,6b-hexahydro-4-oxa-2,6a-diaza-cyclobut[cd]inden-6-carbonsäure

Ausgehend von (1aS,3aR,6bS)-5-(1-(4-Hydroxyphenyl-carbamoylmethyl)-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester (390 mg; 0,67 mmol) werden 293 mg (95%) als gelblicher Feststoff erhalten.
IR(KBr): 3422, 1775, 1691, 1613, 1513, 836 $cm^{-1}$
MS(EI): 208 (M - [N-(4-Hydroxy-phenyl)-2-(5-mercapto-tetrazol-1-yl)acetamid])$^+$

Der dabei als Ausgangsverbindung eingesetzte (1aS,3aR,6bS)-5-(1-(4-Hydroxyphenyl-carbamoylmethyl)-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester kann in Analogie zu Beispiel 29(g) aus (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (500 mg; 1,4 mmol; aus Beispiel 28) und N-(4-Hydroxy-phenyl)-2-(5-mercapto-tetrazol-1-yl)acetamid (431 mg; 1,7 mmol) hergestellt werden. Man erhält 636 mg (76%) eines weissen Schaumes.
IR(KBr): 3401, 1784, 1707, 1613, 1555, 1513, 836 $cm^{-1}$
MS(ISP): 606,3 $(M+Na)^+$; 601,3, $(M+NH_4)^+$; 584,3 $(M+H)^+$

(n) (1aS,3aR,6bS)-1-Oxo-5-(1-(trityloxy-carbamoylmethyl)-1H-tetrazol-5-ylsulfanylmethyl)-1,1a,2,3,3a,6b-hexahydro-4-oxa-2,6a-diaza-cyclobut[cd]inden-6-carbonsäure

Ausgehend von (1aS,3aR,6bS)-1-Oxo-5-(1-(trityloxy-carbamoylmethyl)-1H-tetrazol-5-ylsulfanylmethyl)-1,1a,2,3,3a,6b-hexahydro-4-oxa-2,6a-diaza-cyclobut[cd]inden-2,6-dicarbonsäure-diallylester (523 mg; 0,68 mmol) werden 381 mg (89%) als gelblicher Feststoff erhalten.
IR(KBr): 3426, 1779, 1708, 1613, 1400, 761, 704 $cm^{-1}$
MS(ISP): 648,4 $(M+Na)^+$; 643, $(M+NH_4)^+$; 626,4 $(M+H)^+$

Die Verbindung wird in üblicher Weise durch saure Hydrolyse (z.B. wässrige Salzsäure) oder Hydrogenolyse mit Pd/C in die 1-(Hydroxycarbamoylmethyl)-Verbindung (bezogen auf die Tetrazolylgruppe) übergeführt.

Der dabei als Ausgangsverbindung eingesetzte (1aS,3aR,6bS)-1-Oxo-5-(1-(trityloxy-carbamoylmethyl)-1H-tetrazol-5-ylsulfanylmethyl)-1,1a,2,3,3a,6b-hexahydro-4-oxa-2,6a-diaza-cyclobut[cd]inden-2,6-dicarbonsäure-diallylester kann in Analogie zu Beispiel 29(g) aus (1aS,3aR,6bS)-5-Hydroxymethyl-1-oxo-1,1a,2,3,3a,6b-hexahydro-4-oxa-2,6a-diaza-cyclobut[cd]inden-2,6-dicarbonsäure-diallylester (460 mg; 1,3 mmol; aus Beispiel 28) und 2-(5-Mercapto-tetrazol-1-yl)-N-trityloxy-acetamid (660 mg; 1,6 mmol) hergestellt werden. Man erhält 953 mg (97%) eines gelben Schaumes.
IR(KBr): 1786, 1711, 1615, 1448, 763, 704 $cm^{-1}$

MS(ISP): 772,2 $(M+Na)^+$; 767,3 $(M+NH_4)^+$; 750,3 $(M+H)^+$

Beispiel 30

(1aS,3aR,6bS)-2-Allyl-5-(1-methyl-1H-tetrazol-5-ylsulfanylmethyl)-1-oxo-4-oxa-1,1a,2,3,3a,6b-hexahydro-2,6a-diaza-cyclobut[cd]inden-6-carbonsäure-Natriumsalz

Eine Lösung von (1aS,3aR,6bS)-5-(1-Methyl-1H-tetrazol-5-ylsulfanylmethyl)-1-oxo-1,1a,2,3,3a,6b-hexahydro-4-oxa-2,6a-diaza-cyclobut[cd]inden-2,6-dicarbonsäure-diallylester (257 mg; 0.57 mmol; aus Beispiel 29(e)) in THF (1 ml) wird mit Tetrakis-(triphenylphosphin)-palladium (37 mg; 0.027 mmol) und Dimedon (240 mg; 1.71 mmol) versetzt und über Nacht bei Raumtemperatur gerührt. Das Lösungsmittel wird eingeengt. Der Rückstand wird in Aethylacetat verdünnt und mit gesättigter, wässriger Natriumbicarbonatlösung (5 ml) gewaschen. Die organische Phase wird mit Wasser (5 ml) gewaschen. Die wässerige Phase wird lyophilisiert und über ein hydrophobes Polymer chromatographiert (Laufmittel: Wasser). Man erhält 41 mg (18%) eines weisses Pulver.
IR(KBr): 1745, 1631, 1595, 1391 $cm^{-1}$
MS(ISP): 365.2 $(M+H)^+$

Beispiel 31

(a) (1aS,3aR,6bS)-2-(4-Hydroxy-phenylcarbamoyl)-5-(5-methyl-1,3,4-thiadiazol-2-ylsulfanylmethyl)-1-oxo-1,1a,2,3,3a,6b-hexahydro-4-oxa-2,6a-diaza-cyclobut[cd]inden-6-carbonsäure-Natriumsalz

Diese Verbindung wird in Analogie zu Beispiel 3(a) ausgehend von (1aS,3aR,6bS)-5-(5-Methyl-1,3,4-thiadiazol-2-ylsulfanylmethyl)-1-oxo-1,1a,2,3,3a,6b-hexahydro-4-oxa-2,6a-diaza-cyclobut[cd]inden-6-carbonsäure (aus Beispiel 29(a)) und 4-Hydroxy-phenylcarbaminsäure-2,5-dioxopyrrolidin-1-yl-ester in 39% Ausbeute als farbloses Pulver erhalten.
IR(KBr): 1759, 1630, 1599, 1513 $cm^{-1}$
MS(ISP): 498.2 $(M+Na)^+$; 493.2 $(M+NH_4)^+$; 476.2 $(M+H)^+$

In Analogie hierzu werden hergestellt:

(b) (1aS,3aR,6bS)-2-(4-Carbamoyl-phenylcarbamoyl)-5-(5-methyl-1,3,4-thiadiazol-2-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(5-Methyl-1,3,4-thiadiazol-2-ylsulfanylmethyl)-1-oxo-1,1a,2,3,3a,6b-hexahydro-4-oxa-2,6a-diaza-cyclobut[cd]inden-6-carbonsäure und 4-Carbamoyl-phenylcarbaminsäure-2,5-dioxopyrrolidin-1-yl-ester in 50% Ausbeute als farbloses Pulver erhalten.
IR(KBr): 1761, 1663, 1596, 1526 $cm^{-1}$
MS(ISN): 501.3 $(M\text{-}Na)^-$; 369.3 $(M\text{-}C_3H_4N_2S_2)$

(c) (1aS,3aR,6bS)-5-(5-Amino-1,3,4-thiadiazol-2-ylsulfanylmethyl)-2-(4-hydroxy-phenylcarbamoyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(5-Amino-1,3,4-thiadiazol-2-ylsulfanylmethyl)-1-oxo-1,1a,2,3,3a,6b-hexahydro-4-oxa-2,6a-diaza-cyclobut[cd]inden-6-carbonsäure und 4-Hydroxy-phenylcarbaminsäure-2,5-dioxopyrrolidin-1-yl-ester in 38% Ausbeute als farbloses Pulver erhalten.
IR(KBr): 1755, 1629, 1600, 1513 $cm^{-1}$
MS(ISP): 499.3 $(M+H)^+$; 477.3 $(M\text{-}Na+2H)^+$

(d) (1aS,3aR,6bS)-2-(4-Hydroxy-phenylcarbamoyl)-1-oxo-5-(pyridin-4-ylsulfanylmethyl)-1,1a,2,3,3a,6b-hexahydro-4-oxa-2,6a-diaza-cyclobut[cd]inden-6-carbonsäure-Natriumsalz

Ausgehend von (1aS,3aR,6bS)-1-Oxo-5-(pyridin-4-ylsulfanylmethyl)-1,1a,2,3,3a,6b-hexahydro-4-oxa-2,6a-diaza-cyclobut[cd]inden-6-carbonsäure und 4-Hydroxy-phenylcarbaminsäure-2,5-dioxopyrrolidin-1-yl-ester in 33% Ausbeute als farbloses Pulver erhalten.
IR(KBr): 1759, 1626, 1581, 1513 $cm^{-1}$
MS(ISP): 477.4 $(M+H)^+$; 455.4 $(M\text{-}Na+2H)^+$

(e) (1aS,3aR,6bS)-2-(4-Carbamoyl-phenylcarbamoyl)-1-oxo-5-(pyridin-4-ylsulfanylmethyl)-1,1a,2,3,3a,6b-hexahydro-4-oxa-2,6a-diaza-cyclobut[cd]inden-6-carbonsäure-Natriumsalz

Ausgehend von (1aS,3aR,6bS)-1-Oxo-5-(pyridin-4-ylsulfanylmethyl)-1,1a,2,3,3a,6b-hexahydro-4-oxa-2,6a-diaza-cyclobut[cd]inden-6-carbonsäure und 4-Carbamoyl-phenylcarbaminsäure-2,5-dioxopyrrolidin-1-yl-ester in 40% Ausbeute als farbloses Pulver erhalten.
IR(KBr): 1761, 1664, 1583, 1526 $cm^{-1}$
MS(ISP): 504.4 $(M+H)^+$; 482.4 $(M\text{-}Na+2H)^+$

(f) (1aS,3aR,6bS)-2-(4-Carbamoyl-phenylcarbamoyl)-5-(carbamoyloxymethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(Carbamoyloxymethyl)-1-oxo-1,1a,2,3,3a,6b-hexahydro-4-oxa-2,6a-diaza-cyclobut[cd]inden-6-carbonsäure und 4-Carbamoyl-phenylcarbaminsäure-2,5-dioxopyrrolidin-1-yl-ester in 61% Ausbeute als farbloses Pulver erhalten.
IR(KBr): 1759, 1718, 1657, 1609, 1524 $cm^{-1}$
MS(ISP): 410.4 $(M+Na)^+$; 405.5 $(M+NH_4)^+$; 388.4 $(M+H)^+$

(g) (1aS,3aR,6bS)-5-(Carbamoyloxymethyl)-2-(4-hydroxy-phenylcarbamoyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(Carbamoyloxymethyl)-1-oxo-1,1a,2,3,3a,6b-hexahydro-4-oxa-2,6a-diaza-cyclobut[cd]inden-6-carbonsäure und 4-Hydroxy-phenylcarbaminsäure-2,5-dioxopyrrolidin-1-yl-ester in 50% Ausbeute als farbloses Pulver erhalten.
IR(KBr): 1757, 1716, 1655, 1513 $cm^{-1}$
MS(ISP): 383.4 $(M+Na)^+$; 378.4 $(M+NH_4)^+$; 361.4 $(M+H)^+$

(h) (1aS,3aR,6bS)-2-(4-Hydroxy-phenylcarbamoyl)-5-(1-methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(1-Methyl-1H-tetrazol-5-ylsulfanyl-methyl)-1-oxo-1,1a,2,3,3a,6b-hexahydro-4-oxa-2,6a-diaza-cyclobut[cd]inden-6-carbonsäure und 4-Hydroxy-phenylcarbaminsäure-2,5-dioxopyrrolidin-1-yl-ester in 12% Ausbeute als farbloses Pulver erhalten.
IR(KBr): 1758, 1631, 1602, 1513 $cm^{-1}$
MS(ISN): 458.4 $(M\text{-}Na)^-$; 414.4 $(M\text{-}Na\text{-}CO_2)^-$

(i) (1aS,3aR,6bS)-2-(4-Carbamoyl-phenylcarbamoyl)-5-(1-methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(1-Methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure und 4-Carbamoyl-phenylcarbaminsäure-2,5-dioxopyrrolidin-1-yl-ester in 12% Ausbeute als farbloses Pulver erhalten.
IR(KBr): 1761, 1662, 1599, 1526 $cm^{-1}$
MS(ISP): 509.4 $(M+H)^+$; 487.5 $(M\text{-}Na+2H)^+$

Beispiel 32

(a) (1aS,3aR,6bS)-2-Acetyl-5-(pyridin-4-ylsulfanylmethyl)-1-oxo-1,1a,2,3,3a,6b-hexahydro-4-oxa-2,6a-diaza-cyclobut[cd]inden-6-carbonsäure-Natriumsalz

Wird in Analogie zu Beispiel 10(b) ausgehend von (1aS,3aR,6bS)-1-Oxo-(5-pyridin-4-ylsulfanylmethyl)-1,1a,2,3,3a,6b-hexahydro-4-oxa-2,6a-diaza-cyclobut[cd]inden-6-carbonsäure (aus Beispiel 29(b)) und Essigsäureanhydrid in Essigsäure in 48% Ausbeute als farbloses Pulver erhalten.
IR(KBr): 1764, 1627, 1580, 1410 $cm^{-1}$
In Analogie hierzu werden hergestellt:

(b) (1aS,3aR,6bS)-2-Acetyl-5-(carbamoyloxymethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(Carbamoyloxymethyl)-1-oxo-1,1a,2,3,3a,6b-hexahydro-4-oxa-2,6a-diaza-cyclobut[cd]inden-6-carbonsäure (aus Beispiel 29(f)) in 41% Ausbeute als farbloses Pulver.
IR(KBr): 1765, 1723, 1659, 1455, 1348 $cm^{-1}$

(c) (1aS,3aR,6bS)-2-Acetyl-5-(2-Carbamoyl-5-methyl[1,2,4]triazolo[1,5-a]pyrimidin-7-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(2-Carbamoyl-5-methyl[1.2.4]triazolo[1,5-a]pyrimidin-7-ylsulfanylmethyl)-1-oxo-1,1a,2,3,3a,6b-hexahydro-4-oxa-2,6a-diaza-cyclobut[cd]inden-6-carbonsäure (aus Beispiel 29(d)) in 30% Ausbeute als farbloses Pulver.
IR(KBr): 1762, 1690, 1628, 1595, 1512 $cm^{-1}$
MS(ISP): 504.3 $(M+Na)^+$; 482.2 $(M+H)^+$; 460.5 $(M-Na+2H)^+$

(d) (1aS,3aR,6bS)-2-Acetyl-5-(1-cyclopropylcarbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(1-Cyclopropylcarbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure (150 mg; 0,37 mmol; Beispiel 29(g)) werden 10 mg (6%) als weisses Pulver isoliert.
IR(KBr): 1762, 1630, 1395 $cm^{-1}$
MS(ISP): 472,3 $(M+H)^+$; 450,3 $(M-Na+2H)^+$

(e) (1aS,3aR,6bS)-2-Acetyl-5-(1-carbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(1-Carbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure (100 mg; 0,27 mmol; Beispiel 29(j)) werden 23 mg (20%) als weisses Pulver isoliert.
IR(KBr): 1761, 1694, 1630, 1394, 1084 $cm^{-1}$
MS(ISP): 454,2 $(M+Na)^+$; 432,2 $(M+H)^+$; 410,2 $(M-Na+2H)^+$

(f) (1aS,3aR,6bS)-2-Acetyl-5-(1-methyl-carbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(1-Methylcarbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure (125 mg; 0,33 mmol; Beispiel 29(k)) werden 7 mg (5%) als weisses Pulver isoliert.
IR(KBr): 1763, 1630, 1600, 1408 $cm^{-1}$
MS(ISP): 446,3 $(M+H)^+$; 441,4 $(M-Na+H+NH_4)^+$; 424,3 $(M-Na+2H)^+$

(g) (1aS,3aR,6bS)-2-Acetyl-5-(1-(2-morpholin-4-yl-2-oxo-äthyl)-1H-tetrazol-5-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(1-(2-Morpholin-4-yl-2-oxo-äthyl)-1H-tetrazol-5-ylsulfanylmethyl)-1-oxo-1,1a,2,3,3a,6b-hexahydro-4-oxa-2,6a-diaza-cyclobut[cd]inden-6-carbonsäure (114 mg; 0,26 mmol; Beispiel 29(l)) werden 26 mg (20%) als weisses Pulver isoliert.
IR(KBr): 1764, 1659, 1598, 1395 $cm^{-1}$
MS(ISP): 502,2 $(M+H)^+$; 497,2 $(M-Na+H+NH_4)^+$; 480,2 $(M-Na+2H)^+$

(h) (1aS,3aR,6bS)-2-Acetyl-1-oxo-5-(1-(trityloxycarbamoyl-methyl)-1H-tetrazol-5-ylsulfanylmethyl)-1,1a,2,3,3a,6b-hexahydro-4-oxa-2,6a-diaza-cyclobut[cd]inden-6-carbonsäure-Natriumsalz

Ausgehend von (1aS,3aR,6bS)-1-Oxo-5-(1-(trityloxycarbamoyl-methyl)-1H-tetrazol-5-ylsulfanylmethyl)-1,1a,2,3,3a,6b-hexahydro-4-oxa-2,6a-diaza-cyclobut[cd]inden-6-carbonsäure (120 mg; 0,19 mmol; Beispiel 29(n)) werden 40 mg (31%) als weisses Pulver isoliert.

IR(KBr): 1757, 1690, 1628, 1386, 763, 703 $cm^{-1}$
MS(ISP): 690 $(M+H)^+$; 685,4 $(M\text{-}Na+H+NH_4)^+$; 668,3 $(M\text{-}Na+2H)^+$

Beispiel 33

(1aS,3aR,6bS)-2-Acetyl-5-(5-amino-1,3,4-thiadiazol-2-ylsulfanylmethyl)-1-oxo-1,1a,2,3,3a,6b-hexahydro-4-oxa-2,6a-diaza-cyclobut[cd]inden-6-carbonsäure-Natriumsalz

Wird in Analogie zu Beispiel 19(a) ausgehend von (1aS,3aR,6bS)-5-(5-Amino-1,3,4-thiadiazol-2-ylsulfanylmethyl)-1-oxo-1,1a,2,3,3a,6b-hexahydro-4-oxa-2,6a-diaza-cyclobut[cd]inden-6-carbonsäure (aus Beispiel 29(c)) und Acetylchlorid in 39% Ausbeute als rosa Pulver erhalten.
IR(KBr): 1758, 1626, 1595, 1495 $cm^{-1}$
MS(ISP): 428,2 $(M+Na)^+$; 406,2 $(M+H)^+$; 384,2 $(M\text{-}Na+2H)^+$

Beispiel 34

(1aS,3aR,6bS)-2-(1-Methyl-1H-tetrazol-5-ylsulfanylacetyl)-5-(5-methyl-1,3,4-thiadiazol-2-ylsulfanylmethyl)-1-oxo-1,1a,2,3,3a,6b-hexahydro-4-oxa-2,6a-diaza-cyclobut[cd]inden-6-carbonsäure

Eine Lösung von (1aS,3aR,6bS)-5-(5-methyl-1,3,4-thiadiazol-2-ylsulfanyl-methyl)-1-oxo-1,1a,2,3,3a,6b-hexahydro-4-oxa-2,6a-diaza-cyclobut[cd]inden-6-carbonsäure (120 mg; 0.246 mmol) in DMF (1 ml) wird mit (1-Methyl-1H-tetrazol-5-ylsulfanyl)-essigsäure-2,5-dioxo-pyrrolidin-1-yl-ester (80 mg; 0.296 mmol) versetzt. Die Lösung wird 2 Stunden gerührt, mit Kohle behandelt und filtriert. Das Filtrat wird mit Aethylacetat verdünnt. Die Kristalle werden abgenutscht, in Dimethylformamid (DMF) gelöst und mit 2N Natrium-2-Aethyl-capronat in Aethylacetat (1 ml) versetzt. Die erhaltenen Kristalle werden abgenutscht, in wenig Wasser gelöst und über ein hydrophobes Polymer chromatographiert (Laufmittel: Wasser). Man erhält 23 mg (18%) eines weissen Pulvers.
IR(KBr): 1760, 1631, 1599, 1388 $cm^{-1}$
MS(ISP): 541.3 $(M+Na)^+$; 519.3 $(M+H)^+$; 497.4 $(M\text{-}Na+2H)^+$

Beispiel 35

(1aS,3aR,6bS)-2-Acetyl-5-(1-methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-Natriumsalz

Wird in Analogie zu Beispiel 29(a) ausgehend von (1aS,3aR,6bS)-2-Acetyl-5-(1-methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-allylester in 26% Ausbeute als weisses Pulver hergestellt.
IR(KBr): 1762, 1650, 1631, 1600, 1390 $cm^{-1}$
MS(ISP): 389.3 (M+H); 384.3 $(M+NH_4+H\text{-}Na)^+$; 367.4 $(M\text{-}Na+2H)^+$

Der als Ausgangsverbindung eingesetzte (1aS,3aR,6bS)-2-Acetyl-5-(1-methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-allylester wird wie folgt hergestellt:

a) 1:1 Gemisch von (1S,4S,5S)-4-[(R)- und (S)-Tetrahydropyran-2-yloxy]-2,6-diazabicyclo[3.2.0]heptan-7-on

(1S,4S,5S)-2-Benzyloxycarbonyl-4-[(R)-und (S)-tetrahydropyran-2-yloxy]-2,6-diaza-bicyclo[3.2.0]heptan-7-on (1:1 Gemisch; 11 g; 0.32 mol; Europäische Offenlegungsschrift 508 234, Beispiel 14) in Aethanol (250 ml) wird über Pd/C (10%; 100 mg) hydriert. Der Katalysator wird abgenutscht, das Filtrat eingeengt und über Kieselgel mit Aethylacetat/Methanol 9:1 chromatographiert. Man erhält 3.30 g (49%) farbloses Material. Smp 153-54 °C (Aether)

| Mikroanalyse: $C_{10}H_{16}N_2O_3$ | | | |
|---|---|---|---|
| Ber. | C 56.59 | H 7.60 | N 13.20 |
| Gef. | C 56.55 | H 7.64 | N 12.96 |

b) 1:1 Gemisch von (1S,4S,5S)-2-Acetyl-4-[(R)- und (S)-tetrahydropyran-2-yloxy]-2,6-diazabicyclo[3.2.0]-heptan-7-on

Eine Lösung von (1S,4S,5S)-4-[(R)- und (S)-tetrahydropyran-2-yloxy]-2,6-diazabicyclo[3.2.0]heptan-7-on (4.84 g, 22.8 mmol) in Methylenchlorid (100 ml) wird bei 0°C vorgelegt und nacheinander mit Pyridin (2 ml; 25 mmol) und einer Lösung von Essigsäureanhydrid (2.26 ml; 24 mmol) in Methylenchlorid (10 ml) versetzt. Nach 10 Minuten wird das Reaktionsgemisch mit Methylenchlorid verdünnt und nacheinander mit gesättigter Natriumbicarbonat- und Kochsalzlösungen gewaschen. Die organische Lösung wird getrocknet und eingeengt und der Rückstand aus Aether/n-Hexan kristallisiert. Man erhält 5.44g (93.6%) farbloses Material. Smp 132-34°C (Aether)

| Mikroanalyse: $C_{12}H_{18}N_2O_4$ | | | |
|---|---|---|---|
| Ber. | C 56.68 | H 7.14 | N 11.02 |
| Gef. | C 56.58 | H 7.22 | N 10.88 |

c) 1:1 Gemisch von (1S,4S,5S)-[2-Acetyl-7-oxo-4-[(R)- und (S)-tetrahydropyran-2-yloxy]-2,6-diazabicyclo-[3.2.0]heptan-6-yl]-essigsäure-allylester

Eine Lösung von von (1S,4S,5S)-2-Acetyl-4-[(R)- und (S)-tetrahydropyran-2-yloxy]-2,6-diazabicyclo-[3.2.0]heptan-7-on (5.76 g, 22.7 mmol) in THF (100 ml) wird bei -78°C vorgelegt und nacheinander mit Bistrimethylsilyllithiumamid (1M in THF, 0.27 mol) und Bromessigsäure-allylester (3 ml; 25 mmol) versetzt. Das Reaktionsgemisch wird während 30 Minuten bei 0°C gerührt, mit einer gesättigten wässrigen Ammoniumchloridlösung versetzt und mit Aethylacetat und Wasser verdünnt. Die organische Phase wird mit gesättigter wässriger Kochsalzlösung gewaschen, getrocknet und eingeengt. Der Rückstand wird über Kieselgel mit Aethylacetat chromatographiert. Man erhält 5.0g (62%) eines gelblichen Oels.
IR(Film): 1770, 1740, 1655, 1417 $cm^{-1}$
MS(ISP): 370.5 $(M+NH_4)^+$; 353.4 $(M+H)^+$; 269.4 (M-dihydropyran)

d) 1:1:1:1 Gemisch von [(R)- und (S)-2-(1S,4S,5S)-2-Acetyl-7-oxo-4-[(R)- und (S)-(tetrahydropyran-2-yloxy]-2,6-diazabicyclo[3.2.0]heptan-6-yl]-4-(t-butyl-dimethylsilanyloxy)-3-oxo-buttersäure-allylester

Wird in Analogie zu Beispiel 28e) ausgehend von einem 1:1 Gemisch von (1S,4S,5S)-[2-Acetyl-7-oxo-4-[(R)- und (S)-tetrahydropyran-2-yloxy]-2,6-diazabicyclo[3.2.0]heptan-6-yl]-essigsäure-allylester und (t-Butyldimethylsilyl)oxyacetylchlorid in 32% Ausbeute als gelbliches Oel erhalten.
IR(Film): 1776, 1740, 1660, 1418 $cm^{-1}$
MS(ISP): 542.5 $(M+NH_4)^+$; 525.5 $(M+H)^+$

e) 1:1 Gemisch von (R)- und (S)-2-(1S,4S,5S)-(2-Acetyl-7-oxo-4-hydroxy-2,6-diazabicyclo[3.2.0]heptan-6-yl)-4-(t-butyl-dimethylsilanyloxy)-3-oxo-buttersäure-allylester

Wird in Analogie zu Beispiel 28f) ausgehend von [(R)- und (S)-2-(1S,4S,5S)-2-Acetyl-7-oxo-4-[(R) und (S)-tetrahydropyran-2-yloxy]-2,6-diazabicyclo[3.2.0]heptan-6-yl]-4-(t-butyl-dimethylsilanyloxy)-3-oxo-buttersäure-allylester in 44% Ausbeute als farblose Kristalle (Aether) erhalten.
IR(KBr): 1747, 1640, 1513, 1420 $cm^{-1}$
MS(ISP): 458.5 $(M+NH_4)^+$; 441.5 $(M+H)^+$

f) (1aS,3aR,6bS)-2-Acetyl-5-(t-butyl-dimethyl-silanyloxymethyl)-1-oxo-1,1a,2,3,3a,6b-hexahydro-4-oxa-2,6a-diaza-cyclobut[cd]inden-6-carbonsäure-allylester

Wird in Analogie zu Beispiel 28(g) ausgehend von einem 1:1 Gemisch von (R)- und (S)-2-(1S,4S,5S)-[2-Acetyl-7-oxo-4-hydroxy-2,6-diazabicyclo[3.2.0]heptan-6-yl]-4-(t-butyl-dimethylsilanyloxy)-3-oxo-buttersäure-allylester in 52% Ausbeute als gelbliches Oel erhalten.
IR(Film): 1783, 1716, 1666, 1618, 1415 $cm^{-1}$

| Mikroanalyse: $C_{20}H_{30}N_2O_6Si$ | | | |
|---|---|---|---|
| Ber. | C 56.85 | H 7.16 | N 6.63 |
| Gef. | C 56.75 | H 7.25 | N 6.60 |

g) (1aS,3aR,6bS)-2-Acetyl-5-(hydroxymethyl)-1-oxo-1,1a,2,3,3a,6b-hexahydro-4-oxa-2,6-diaza-cyclobut[cd]-inden-6-carbonsäure-allylester

Wird in Analogie zu Beispiel 28h) ausgehend von (1aS,3aR,6bS)-2-Acetyl-5-(t-butyl-dimethyl-silanyloxymethyl)-1-oxo-1,1a,2,3,3a,6b-hexahydro-4-oxa-2,6a-diaza-cyclobut[cd]inden-6-carbonsäure-allylester in 56% Ausbeute erhalten.
IR(Film): 1779, 1712, 1658, 1617, 1419 $cm^{-1}$
MS(ISP): 326.4 $(M+NH_4)^+$; 309.5 $(M+H)^+$; 281.4 $(M+H\text{-}CO)^+$; 263.4 $(M+H\text{-}CO\text{-}H_2O)^+$

h) (1aS,3aR,6bS)-2-Acetyl-5-(1-methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-allylester

Wird in Analogie zu Beispiel 29(d) ausgehend von (1aS,3aR,6bS)-2-Acetyl-5-hydroxymethyl-1-oxo-1,1a,2,3,3a,6b-hexahydro-4-oxa-2,6-diaza-cyclobut[cd]inden-6-carbonsäure-allylester und bis-(5-Mercapto-1-methyltetrazolyl)-dithiocarbonat in 59% Ausbeute erhalten. IR(KBr): 1781, 1711, 1660, 1615, 1415 $cm^{-1}$ MS-(ISP): 424.5 $(M+NH_4)^+$; 407.4 $(M+H)^+$

Beispiel 36

(1aS,3aR,6bR)-2-t-Butoxycarbonyl-5-methyl-1-oxo-1a,2,3,3a,4,6b-hexahydro-4-oxa-2,6a-diaza-cyclobut[cd]-inden-6-carbonsäure-Natriumsalz (1:1)

(1aS,3aR,6bR)-5-Methyl-1-oxo-1a,2,3,3a,4,6b-hexahydro-4-oxa-2,6a-diaza-cyclobut[cd]inden-2,6-dicarbonsäure-dibenzylester (334 mg; 0.76 mmol) wird in THF/Wasser (8:2; 15 ml) gelöst und über 10%ige Pd/C (100 mg) hydriert. Man filtriert und engt ein. Der Rückstand besteht aus roher 5-Methyl-1-oxo-1a,2,3,3a,4,6b-hexahydro-4-oxa-2,6a-diaza-cyclobut[cd]inden-6-carbonsäure; diese wird in Dioxan/Wasser (2:1; 5 ml) gelöst und mit Natriumbicarbonat (57 mg; 0.68 mmol) und Di-t-butyl-dicarbonat (149 mg; 0.68 mmol) versetzt. Das Gemisch wird über Nacht gerührt. Das Dioxan wird abgedampft. Der Rückstand wird über ein polymeres, hydrophobes Gel mit Wasser und Wasser:Methanol 9:1 fraktioniert. Die das Produkt enthaltenden Fraktionen werden vereinigt und lyophilisiert. Ausbeute: 44 mg (21%) farbloses Pulver.
IR(KBr): 1759, 1703, 1636 $cm^{-1}$
MS(ISP): $(M+Na^+)$ 333

Das Produkt kann mit Trifluoressigsäure gemäss Beispiel 2(a) in (1aS,3aR,6bR)-5-Methyl-1-oxo-1a,2,3,3a,4,6b-hexahydro-4-oxa-2,6a-diaza-cyclobut[cd]inden-6-carbonsäure-trifluoracetat übergeführt werden.

Der als Ausgangsverbindung eingesetzte (1aS,3aR,6bR)-5-Methyl-1-oxo-1a,2,3,3a,4,6b-hexahydro-4-oxa-2,6a-diaza-cyclobut[cd]inden-2,6-dicarbonsäure-dibenzylester kann wie folgt hergestellt werden:

a) (1S,4S,5S)-6-Benzyloxycarbonylmethyl-7-oxo-4-(tetrahydropyran-2-yloxy)-2,6-diaza-bicyclo[3.2.0]heptan-2-carbonsäure-benzylester (Diastereoisomeren-Gemisch)

(1S,4S,5S)-2-Benzyloxycarbonyl-4-[(R)-und (S)-tetrahydropyran-2-yloxy]-2,6-diaza-bicyclo[3.2.0]heptan-7-on (1:1 Gemisch; 3.46 g; 10 mmol) (Europäische Offenlegungsschrift 508 234, Beispiel 14) wird in abs. THF gelöst (100 ml) und bei -78°C mit 1M Bistrimethylsilyllithiumamidlösung in THF (11.5 ml) und Bromessigsäure-benzylester (2.51 g; 11 mmol) versetzt. Das Reaktionsgemisch wird auf -10°C aufgewärmt, mit gesättigter wässriger Natriumchloridlösung verdünnt und mit Aethylacetat extrahiert. Die organische Phase wird über Magnesiumsulfat getrocknet und eingeengt. Der Rückstand wird über Kieselgel (0,0062-0,2 mm Korngrösse) mit Aethylacetat/n-Hexan 1:1 chromatographiert. Ausbeute: 1.83 g (38%) farbloses Oel.
IR (KBr): 1770, 1740, 1705 $cm^{-1}$
MS (ISP): 517.5 $(M+Na^+)$ 512.5 $(M+NH_4{}^+)$, 512 $(M+H^+)$

b) (1S,4S,5S)-6-(1-Benzyloxycarbonyl-2-oxo-propyl)-4-hydroxy-7-oxo-2,6-diaza-bicyclo[3.2.0]heptan-2-carbonsäure-benzylester (Diastereoisomeren-Gemisch)

Eine Lösung von (1S,4S,5S)-6-Benzyloxycarbonylmethyl-7-oxo-4-(tetrahydropyran-2-ylozy)-2,6-diaza-bicyclo[3.2.0]heptan-2-carbonsäure-benzylester (1.88g, 3,82 mmol) wird in abs. THF(40 ml) gelöst und bei -70 °C mit einer 1M Lithium Bis-trimethylsilyllithiumamidlösung in THF (4.77ml, 4.77 mmol) versetzt. Das Reaktionsgemisch wird auf -40 °C erwärmt, nach 20 Minuten wieder auf -70 °C abgekühlt und mit einer Lösung von Acetylchlorid (0,59 ml, 8.40 mmol) in THF (10 ml) versetzt. Das Reaktionsgemisch wird über Nacht bei Raumtemperatur gerührt, mit gesättigter wässriger Ammoniumchloridlösung verdünnt und mit Aethylacetat extrahiert (3x 50 ml). Die organischen Phasen werden mit gesättigter wässriger Kochsalzlösung gewaschen, über Magnesiumsulfat getrocknet und eingeengt. Der Rückstand wird über Kieselgel (0,0062-0,2 mm Korngrösse) mit Aethylacetat/n-Hexan 3:7 chromatographiert. Ausbeute: 1.1 g (54%) von rohem (1S, 4S, 5S)-6-(1-Benzyloxycarbonyl-2-oxo-propyl)-7-oxo-4-(tetrahydropyran-2-yloxy)-2,6-diaza-bicyclo[3.2.0]heptan-2-carbonsäure-benzylester als farbloses Oel. Dieses Oel wird in Aethanol gelöst (15 ml) und mit Pyridinium-(toluol-4-sulfonat) (110 mg) bei 60 °C während 2 Stunden versetzt. Das Lösungsmittel wird eingeengt und der Rückstand in Aethylacetat aufgenommen und mit Wasser gewaschen. Die organische Phase wird über Magnesiumsulfat getrocknet, eingeengt und über Kieselgel (0,0062-0,2 mm Korngrösse) mit Aethylacetat/n-Hexan 1:1 chromatographiert. Ausbeute 483 mg (52%) farbloses Oel.
IR (KBr): 3447, 1773, 1710, 1773 $cm^{-1}$
MS 475,3 $(M+Na)^+$

c) (1aS,3aR,6bR)-5-Methyl-1-oxo-1a,2,3,3a,4,6b-hexahydro-4-oxa-2,6a-diaza-cyclobut[cd]inden-2,6-dicarbonsäure-dibenzylester

Eine Lösung von (1S,4S,5S)-6-(1-Benzyloxycarbonyl-2-oxo-propyl)-4-hydroxy-7-oxo-2,6-diaza-bicyclo-[3.2.0]heptan-2-carbonsäure-benzylester (452 mg, 1 mmol) in 10 ml abs. THF wird bei -10 °C mit Triphenylphosphin (340 mg, 1.3 mmol) und Azodicarbonsäure-diethylester (226 mg, 1.3 mmol) versetzt. Das Reaktionsgemisch wird über Nacht gerührt, das Lösungsmittel wird eingeengt und der Rückstand über Kieselgel (0,0062-0,2 mm Korngrösse) mit Aethylacetat/$CH_2Cl_2$ 5:95 chromatographiert. Ausbeute : 362 mg (83%)
IR (KBr): 1781, 1713, 1617, 1423, 1214, 1097 $cm^{-1}$
MS: 457.3 $(M+Na^+)$, 452.4 $(M+NH_4^+)$, 435.3 $(M+H^+)$

## Beispiel 37

(1aS,3aR,6bS)-5-(1-Cyclopropylcarbamoylmethyl-1H-tetrazol-5-ylsulfanylmethyl)-2-formyl-1-oxo-1,1a,2,3,3a,6b-hexahydro-4-oxa-2,6a-diaza-cyclobut[cd]inden-6-carbonsäure-Natriumsalz

Zu einer auf 0 °C gekühlten Lösung von (1aS,3aR,6bS)-5-(1-Cyclopropylcarbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure (120 mg; 0,29 mmol; Beispiel 29(g)) in DMF wird Ameisensäure-essigsäureanhydrid (133 mg; 1,5 mmol) getropft. Das Ganze wird 1 Stunde bei Raumtemperatur gerührt. Das Reaktionsgemisch wird auf 10 ml Wasser gegossen, mit Natriumbicarbonat auf pH 7 gestellt und eingeengt. Der Rückstand wird in wenig Wasser aufgenommen und über ein hydrophobes Polymer chromatographiert (Laufmittel: Wasser/Acetonitril). Man erhält 36 mg (27%) als weisses Pulver.
IR(KBr): 1765, 1667, 1594, 1390 $cm^{-1}$
MS(ISP): 480,2 $(M+Na)^+$; 458,2 $(M+H)^+$; 436,2 $(M-Na+2H)^+$

## Beispiel 38

(a) (1aS,3aR,6bS)-2-Formyl-5-(1-methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(1-Methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure (67 mg; 0,21 mmol; Beispiel 29(e)), Ameisensäure (0,12 ml; 3,2 mmol) und Dicyclohexylcarbodiimid (85 mg; 0,41 mmol) werden in Analogie zu Beispiel 39 17 mg (22%) als weisses Pulver erhalten.
IR(KBr): 1765, 1662, 1590, 1388 $cm^{-1}$

MS(ISP): 375,2 $(M+H)^+$; 371,4 $(M\text{-}Na+H+NH_4)^+$; 353,2 $(M\text{-}Na+2H)^+$

In Analogie hierzu werden hergestellt:

(b) (1aS,3aR,6bS)-5-(1-Carbamoylmethyl-1H-tetrazol-5-ylsulfanylmethyl)-2-formyl-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(1-Carbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure (100 mg; 0,27 mmol; Beispiel 29(j)) werden 26 mg (23%) als weisses Pulver erhalten.

IR(KBr): 1763, 1661, 1628, 1594, 1391 $cm^{-1}$

MS(ISP): 440,3 $(M+Na)^+$; 418,3 $(M+H)^+$; 413,3 $(M\text{-}Na+H+NH_4)^+$; 396,3 $(M\text{-}Na+2H)^+$

(c) (1aS,3aR,6bS)-2-Formyl-5-(1-(2-morpholin-4-yl-2-oxo-äthyl)-1H-tetrazo-5-ylsulfanylmethyl)-1-oxo-1,1a,2,3,3a,6b-hexahydro-4-oxa-2,6a-diaza-cyclobut[cd]inden-6-carbonsäure-Natriumsalz

Ausgehend von (1aS,3aR,6bS)-5-(1-(2-Morpholin-4-yl-2-oxo-äthyl)-1H-tetrazol-5-ylsulfanylmethyl)-1-oxo-1,1a,2,3,3a,6b-hexahydro-4-oxa-2,6a-diaza-cyclobut[cd]inden-6-carbonsäure (106 mg; 0,24 mmol; Beispiel 29(l)) werden 51 mg (43%) weisses Pulver isoliert.

IR(KBr): 1764, 1661, 1594, 1391 $cm^{-1}$

MS(ISN): 464,2 $(M\text{-}Na)^-$

(d) (1aS,3aR,6bS)-2-Formyl-1-oxo-5-(1-(trityloxycarbamoyl-methyl)-1H-tetrazol-5-ylsulfanylmethyl)-1,1a,2,3,3a,6b-hexahydro-4-oxa-2,6a-diaza-cyclobut[cd]inden-6-carbonsäure-Natriumsalz

Ausgehend von (1aS,3aR,6bS)-1-Oxo-5-(1-(trityloxycarbamoyl-methyl)-1H-tetrazol-5-ylsulfanylmethyl)-1,1a,2,3,3a,6b-hexahydro-4-oxa-2,6a-diaza-cyclobut[cd]inden-6-carbonsäure (84 mg; 0,13 mmol; Beispiel 29(n)) werden 34 mg (38%) weisses Pulver isoliert.

IR(KBr): 1761, 1665, 1629, 1599, 1388, 764, 704 $cm^{-1}$

MS(ISP): 698,4 $(M+Na)^+$; 676,3 $(M+H)^+$; 671 $(M\text{-}Na+H+NH_4)^+$; 654,3 $(M\text{-}Na+2H)^+$

Beispiel 39

(1aS,3aR,6bS)-5-(1-Carbamoylmethyl-1H-tetrazol-5-ylsulfanylmethyl)-1-oxo-2-trifluoracetyl-1,1a,2,3,3a,6b-hexahydro-4-oxa-2,6a-diaza-cyclobut[cd]inden-6-carbonsäure-Natriumsalz

Zu einer Lösung von (1aS,3aR,6bS)-5-(1-Carbamoylmethyl-1H-tetrazol-5-ylsulfanylmethyl)-1-oxo-1,1a,2,3,3a,6b-hexahydro-4-oxa-2,6a-diaza-cyclobut[cd]inden-6-carbonsäure (100 mg; 0,27 mmol; Beispiel 29(j)) in DMF wird Trifluoressigsäure (0,31 ml; 4,1 mmol) und Dicyclohexylcarbodiimid (111 mg; 0,54 mmol) gegeben. Das Reaktionsgemisch wird 1 Stunde gerührt. Der entstandene Niederschlag wird abfiltriert. Das Filtrat wird eingeengt, in wenig Wasser aufgenommen, mit Natriumbicarbonat auf pH 7 gestellt und über ein hydrophobes Polymer chromatographiert (Laufmittel: Wasser/Acetonitril). Man erhält 30 mg (23%) als weisses Pulver.

IR(KBr): 1772, 1696, 1628, 1596, 1394, 1212 $cm^{-1}$

MS(ISP): 486,3 $(M+H)^+$; 481,2 $(M\text{-}Na+H+NH_4)^+$; 464,3 $(M\text{-}Na+2H)^+$

Beispiel 40

a) (1aS,3aR,6bS)-5-(1-Methyl-1H-tetrazol-5-ylsulfanylmethyl)-1-oxo-4-thia-1,1a,2,3,3a,6b-hexahydro-2,6a-diaza-cyclobut[cd]inden-6-carbonsäure

Wird in Analogie zu Beispiel 29(a) ausgehend von (1aS,3aR,6bS)-5-(1-Methyl-1H-tetrazol-5-ylsulfanylmethyl)-1-oxo-4-thia-1,1a,2,3,3a,6b-hexahydro-2,6a-diaza-cyclobut[cd]inden-2,6-dicarbonsäure-diallylester in 26% Ausbeute als weisses Pulver hergestellt.

IR(KBr): 1766, 1703, 1617, 1580, 1377 $cm^{-1}$

MS(ISN): 339.2 $(M\text{-}Na)^-$; 295.2 $(M\text{-}CO_2\text{-}Na)^-$

Der als Ausgangsverbindung eingesetzte (1aS,3aR,6bS)-5-(1-Methyl-1H-tetrazol-5-ylsulfanylmethyl)-1-oxo-4-thia-1,1a,2,3,3a,6b-hexahydro-2,6a-diaza-cyclobut[cd]inden-2,6-dicarbonsäure-diallylester kann wie folgt hergestellt werden:

a) 1:1:1:1 Gemisch von (1S, 4S, 5S)-6-[(R) und (S)-1-Allyloxycarbonyl-3-(t-butyl-dimethyl-silanyloxy)-2-oxo-propyl]-4-methylsulfonyloxy-7-oxo-2,6-diaza-bicyclo[3.2.0]heptan-2-carbonsäure-allylester

Eine Lösung von 1:1:1:1 Gemisch von (1S,4S,5S)-6-[(R)-und (S)-1-Allyloxycarbonyl-3-(t-butyl-dimethyl-silanyloxy)-2-oxo-propyl]-4-hydroxy-7-oxo-2,6-diazabicyclo[3.2.0]heptan-2-carbonsäure-allylester (3.08 g; 6.16 mmol; aus Beispiel 28f)) in Methylenchlorid (50 ml) wird bei -10 °C tropfenweise mit Mesylchlorid (1.15 ml, 14.78 mmol) und einer Lösung von DABCO (1.8 g; 16.01 mmol) in Methylenchlorid (20 ml)versetzt. Die Lösung wird 20 Minuten bei Raumtemperatur gerührt und dann mit kaltem Wasser gewaschen. Die organische Phase wird getrocknet und eingeengt. Der Rückstand wird über Kieselgel mit Aethylacetat/n-Hexan 4:6 chromatographiert. Ausbeute : 2.8 g; (71%).
IR (Film): 2934, 1786, 1716, 1412, 1366, 1174 $cm^{-1}$
MS (ISP): 656.5 ($M+NH_4^+$)

b) (1aS,3aR, 6bS)-5-(t-Butyl-dimethyl-silanyloxymethyl)-1-oxo-1,1a,2,3,3a,6b-hexahydro-4-thia-2,6a-diaza-cyclobut[cd]inden-2,6-dicarbonsäure-diallylester

Auminiumoxid (5 g; basisch, Aktivitätstufe I) wird in DMSO (15 ml) suspendiert und unter Rühren mit Natriumhydrogensulfid (780 mg) versetzt. Die Suspension wird eine Stunde weitergerührt und dann eingeengt. Das erhaltene Pulver wird am Hochvakuum getrocknet.

Eine Lösung von 1:1:1:1 Gemisch von (1S, 4S, 5S)-6-[(R) und (S)-1-Allyloxycarbonyl-3-(t-butyl-dimethyl-silanyloxy)-2-oxo-propyl]-4-methylsulfonyloxy-7-oxo-2,6-diaza-bicyclo[3.2.0]heptan-2-carbonsäure-allylester (913 mg, 1.42 mmol) in Methylenchlorid (20 ml) wird mit dem oben beschriebenen Reagenz (2.84 g) versetzt. Die Suspension wird über Nacht gerührt, abgenutscht und mit Methylenchlorid nachgewaschen. Das Filtrat wird mit Wasser gewaschen, getrocknet und eingeengt. Der Rückstand wird über Kieselgel mit Aethylacetat/n-Hexan (3:7) chromatographiert. Ausbeute : 540 mg (79%.)
IR (Film): 2931, 1780, 1709, 1571, 1411 $cm^{-1}$
MS (ISP): 498.6 ($M+NH_4^+$); 481.6 ($M+H^+$)

c) (1aS,3aR,6bS)-5-Hydroxymethyl-1-oxo-1,1a,2,3,3a,6b-hexahydro-4-thia-2,6a-diaza-cyclobut[cd]inden-2,6-dicarbonsäure-diallylester

Eine Lösung von (1aS,3aR,6bS)-5-(t-Butyl-dimethyl-silanyloxymethyl)-1-oxo-1,1a,2,3,3a,6b-hexahydro-4-thia-2,6a-diaza-cyclobut[cd]inden-2,6-dicarbonsäure-diallylester (7.5 g; 162 mmol) in Acetonitril (30 ml) wird mit einer 2N Fluorwasserstoff-Lösung in Acetonitril (24.3 ml; 486 mmol) versetzt. Die Lösung wird bei Raumtemperatur 1.5 Stunden gerührt, dann mit Aethylacetat verdünnt und mit einem Gemisch von Wasser und verdünnter Natriumbicarbonatlösung gewaschen. Die organische Phase wird getrocknet und eingeengt. Der Rückstand wird über Kieselgel mit Aethylacetat/n-Hexan (2:3) chromatographiert. Ausbeute : 4.0 g (68%.)
IR(KBr): 1775, 1707, 1648, 1574, 1412 $cm^{-1}$
MS(ISP): 482.3 $(M+NH_4)^+$; 465.4 $(M+H)^+$

d) (1aS,3aR,6bS)-5-Methylsulfonyloxymethyl-1-oxo-1,1a,2,3,3a,6b-hexahydro-4-thia-2,6a-diaza-cyclobut[cd]-inden-2,6-dicarbonsäure-diallylester

Eine Lösung von (1aS,3aR,6bS)-5-Hydroxymethyl-1-oxo-1,1a,2,3,3a,6b-hexahydro-4-thia-2,6a-diaza-cyclobut[cd]inden-2,6-dicarbonsäure-diallylester (4.02 g; 10.97 mmol) in Methylenchlorid (75 ml) wird bei -15 °C mit Triaethylamin (1.44 g; 1.98 ml; 14.26 mmol) und Mesylchlorid (1.50 g; 1.02 ml; 13.16 mmol) versetzt. Das Reaktionsgemisch wird 30 Minuten bei dieser Temperatur gerührt, mit Methylenchlorid verdünnt und nacheinander mit gesättigter wässriger Natriumbicarbonatlösung und gesättigter wässriger Kochsalzlösung gewaschen Die organische Phase wird getrocknet und eingeengt. Der Rückstand wird über Kieselgel mit Aethylacetat/n-Hexan (1:1) chromatographiert. Ausbeute : 4.12g (84%).
IR (film): 1770, 1702, 1577, 1415, 1363 $cm^{-1}$
MS (ISP): 462.4 ($M+NH_4^+$); 445.3 ($M+H^+$)

e) (1aS,3aR,6bS)-5-(1-Methyl-1H-tetrazol-5-ylsulfanylmethyl)-1-oxo-1,1a,2,3,3a,6b-hexahydro-4-thia-2,6a-diaza-cyclobut[cd]inden-2,6-dicarbonsäure-diallylester

Wird in Analogie zu Beispiel 29(c) ausgehend von (1aS,3aR,6bS)-5-Methylsulfonyloxymethyl-1-oxo-1,1a,2,3,3a,6b-hexahydro-4-thia-2,6a-diaza-cyclobut[cd]inden-2,6-dicarbonsäure-diallylester und 5-Mercapto-1-methyl-1H-tetrazol hergestellt.
IR(KBr): 1776, 1709, 1647, 1581, 1411 $cm^{-1}$
MS(ISP): 389.4 $(M+Na)^+$;384 $(M+NH_4)^+$; 367.4 $(M+H)^+$; 339.3 $(M+H\text{-}CO)^+$

In Analogie zur unter (a) angeführten Verbindung werden hergestellt:

(b) (1aS,3aR,6bS)-1-(6-Carboxy-1-oxo-1,1a,2,3,,3a,6b-hexahydro-4-thia-2,6a-diaza-cyclobut[cd]inden-5-ylmethyl)-pyridinium-methylsulfonat

Wird ausgehend von (1aS,3aR,6bS)-1-(2,6-Bis-allyloxycarbonyl-1-oxo-1,1a,2,3,,3a,6b-hexahydro-4-thia-2,6a-diaza-cyclobut[cd]inden-5-ylmethyl)pyridinium-methylsulfonat hergestellt.
IR(KBr): 1777, 1700, 1632, 1483 $cm^{-1}$
MS(ISP): 304.2 $(M+H)^+$

Das als Ausgangsverbindung eingesetzte (1aS,3aR,6bS)-1-(2,6-Bis-allyloxycarbonyl-1-oxo-1,1a,2,3,,3a,6b-hexahydro-4-thia-2,6a-diaza-cyclobut[cd] inden-5-ylmethyl)-pyridinium-methylsulfonat kann in Analogie zu Beispiel 40(a) ausgehend von (1aS,3aR,6bS)-5-Methylsulfonyloxymethyl-1-oxo-1,1a,2,3,3a,6b-hexahydro-4-thia-2,6a-diaza-cyclobut[cd]inden-2,6-dicarbonsäure-diallylester und Pyridin hergestellt werden:
IR(KBr): 1777, 1706, 1632, 1584 $cm^{-1}$
MS(ISP): 428.4 $(M)^+$

(c) (1aS,3aR,6bS)-5-(1-Methyl-imidazol-2-ylsulfanylmethyl)-1-oxo-4-thia-1,1a,2,3,3a,6b-hexahydro-2,6a-diaza-cyclobut[cd]inden-6-carbonsäure

Wird ausgehend von (1aS,3aR,6bS)-5-(1-Methyl-imidazol-2-ylsulfanylmethyl)-1-oxo-4-thia-1,1a,2,3,3a,6b-hexahydro-2,6a-diaza-cyclobut[cd]inden-2,6-dicarbonsäure-diallylester hergestellt.
IR(KBr): 1741, 1608, 1374 $cm^{-1}$
MS(ISP): 383.1 $(M+2Na\text{-}H)^+$;361.1 $(M+Na)^+$; 339.2 $(M+H)^+$

Der als Ausgangsverbindung eingesetzte (1aS,3aR,6bS)-5-(1-Methylimidazol-2-ylsulfanylmethyl)-1-oxo-4-thia-1,1a,2,3,3a,6b-hexahydro-2,6a-diaza-cyclobut[cd]inden-2,6-dicarbonsäure-diallylester kann in Analogie zu Beispiel 40(a) ausgehend von (1aS,3aR,6bS)-5-Methylsulfonyloxymethyl-1-oxo-1,1a,2,3,3a,6b-hexahydro-4-thia-2,6a-diaza-cyclobut[cd]inden-2,6-dicarbonsäure-diallylester und 2-Mercapto-1-methyl-imidazol hergestellt werden:
IR(KBr): 1771, 1698, 1581 $cm^{-1}$
MS(ISP): 463.3 $(M+H)^+$

(d) (1aS,3aR,6bS)-5-(5-Hydroxy-4-methyl-4H-[1,2,4]-triazol-3-ylsulfanylmethyl)-1-oxo-4-thia-1,1a,2,3,3a,6b-hexahydro-2,6a-diaza-cyclobut[cd]inden-6-carbonsäure

Wird ausgehend von (1aS,3aR,6bS)-5-(5-Hydroxy-4-methyl-4H-[1,2,4]triazol-3-ylsulfanylmethyl)-1-oxo-4-thia-1,1a,2,3,3a,6b-hexahydro-2,6a-diaza-cyclobut[cd]inden-2,6-dicarbonsäure-diallylester hergestellt:
IR(KBr): 1741, 1707, 1606, 1577, 1376 $cm^{-1}$
MS(ISP): 356.2 $(M+2H\text{-}Na)^+$

Der als Ausgangsverbindung eingesetzte (1aS,3aR,6bS)-5-(5-Hydroxy-4-methyl-4H-[1,2,4]-triazol-3-ylsulfanylmethyl)-1-oxo-4-thia-1,1a,2,3,3a,6b-hexahydro-2,6a-diaza-cyclobut[cd]inden-2,6-dicarbonsäure-diallylester kann in Analogie zu Beispiel 40(a) ausgehend von (1aS,3aR,6bS)-5-Methylsulfonyloxymethyl-1-oxo-1,1a,2,3,3a,6b-hexahydro-4-thia-2,6a-diaza-cyclobut[cd]inden-2,6-dicarbonsäure-di-allylester und 5-Hydroxy-4-methyl-4H-[1,2,4]-triazol hergestellt werden:
IR(KBr): 1778, 1708, 1411 $cm^{-1}$
MS(ISP): 480.2 $(M+H)^+$

(e) (1aS,3aR,6bS)-5-(5-Amino-1,3,4-thiadiazol-2-ylsulfanylmethyl)-1-oxo-4-thia-1,1a,2,3,3a,6b-hexahydro-2,6a-diaza-cyclobut[cd]inden-6-carbonsäure

Wird ausgehend von (1aS,3aR,6bS)-5-(5-Amino-1,3,4-thiadiazol-2-ylsulfanylmethyl)-1-oxo-4-thia-1,1a,2,3,3a,6b-hexahydro-2,6a-diaza-cyclobut[cd]inden-2,6-dicarbonsäure-diallylester hergestellt:
IR(KBr): 1739, 1602, 1494 $cm^{-1}$
MS(ISP): 358.3 $(M+H)^+$

Der als Ausgangsverbindung eingesetzte (1aS,3aR,6bS)-5-(5-Amino-1,3,4-thiadiazol-2-ylsulfanylmethyl)-1-oxo-4-thia-1,1a,2,3,3a,6b-hexahydro-2,6a-diaza-cyclobut[cd]inden-2,6-dicarbonsäure-diallylester kann in Analogie zu Beispiel 40(a) ausgehend von (1aS,3aR,6bS)-5-Methylsulfonyloxymethyl-1-oxo-1,1a,2,3,3a,6b-hexahydro-4-thia-2,6a-diaza-cyclobut[cd]inden-2,6-dicarbonsäure-di-allylester und 5-Amino-2-mercapto-1,3,4-thiadiazol hergestellt werden:
IR(KBr): 1775, 1707, 1493 $cm^{-1}$
MS(ISP): 482.3 $(M+H)^+$

(f) (1aS,3aR,6bS)-1-Oxo-5-(pyridin-4-ylsulfanylmethyl)-4-thia-1,1a,2,3,3a,6b-hexahydro-2,6a-diaza-cyclobut-[cd]inden-6-carbonsäure

Wird ausgehend von (1aS,3aR,6bS)-1-Oxo-5-(pyridin-4-ylsulfanylmethyl)-4-thia-1,1a,2,3,3a,6b-hexahydro-2,6a-diaza-cyclobut[cd]inden-2,6-dicarbonsäure-diallylester hergestellt:
MS(ISP): 358.2 $(M+H)^+$; 336.2 $(M\text{-}Na+2H)^+$

Der als Ausgangsverbindung eingesetzte (1aS,3aR,6bS)-1-Oxo-5-(pyridin-4-ylsulfanylmethyl)-4-thia-1,1a,2,3,3a,6b-hexahydro-2,6a-diaza-cyclobut[cd]inden-2,6-dicarbonsäure-diallylester kann in Analogie zu Beispiel 40(a) ausgehend von (1aS,3aR,6bS)-5-Methylsulfonyloxymethyl-1-oxo-1,1a,2,3,3a,6b-hexahydro-4-thia-2,6a-diaza-cyclobut[cd]inden-2,6-dicarbonsäure-diallylester und 4-Mercapto-pyridin hergestellt werden:
IR(Film): 1775, 1709, 1647, 1574 $cm^{-1}$

| Mikroanalyse: $C_{21}H_{21}N_3O_5S_2$ | | | |
|---|---|---|---|
| Ber.<br>Gef. | C 54.89<br>C 55.06 | H 4.61<br>H 4.70 | N 9.14<br>N 8.90 |

(g) (1aS,3aR,6bS)-5-[1-(2-Morpholin-4-yl-2-oxo-äthyl)-1H-tetrazol-5-ylsulfanylmethyl]-1-oxo-4-thia-1,1a,2,3,3a,6b-hexahydro-2,6a-diaza-cyclobut[cd]inden-6-carbonsäure

Wird in Analogie zu Beispiel 29(a) ausgehend von (1aS,3aR,6bS)-5-[1-(2-Morpholin-4-yl-2-oxo-äthyl)-1H-tetrazol-5-ylsulfanylmethyl]-1-oxo-4-thia-1,1a,2,3,3a,6b-hexahydro-2,6a-diaza-cyclobut[cd]inden-2,6-dicarbonsäure-diallylester in 90% Ausbeute als weisses Pulver hergestellt.
IR(KBr): 1742, 1660, 1609, 1465, 1376 $cm^{-1}$
MS(ISP): 476.3 $(M+H)^+$; 471.4 $(M+H+Na+NH_4)^+$; 454.4 $(M+2H\text{-}Na)^+$

Der als Ausgangsverbindung eingesetzte (1aS,3aR,6bS)-5-[1-(2-Morpholin-4-yl-2-oxo-äthyl)-1H-tetrazol-5-ylsulfanylmethyl]-1-oxo-4-thia-1,1a,2,3,3a,6b-hexahydro-2,6a-diaza-cyclobut[cd]inden-2,6-dicarbonsäure-diallylester kann in Analogie zu Beispiel 40(a) ausgehend von (1aS,3aR,6bS)-5-Methylsulfonyloxymethyl-1-oxo-1,1a,2,3,3a,6b-hexahydro-4-thia-2,6a-diazacyclobut[cd]inden-2,6-dicarbonsäure-diallylester und 5-Mercapto-1-(2-morpholin-4-yl-2-oxo-ethyl)-1H-tetrazol hergestellt werden:
IR(KBr): 1777, 1708, 1631, 1666, 1580 $cm^{-1}$
MS(ISP): 600 $(M+Na)^+$; 595.3 $(M+NH_4)^+$; 578.3 $(M\text{-}H)^+$

(h) (1aS,3aR,6bS)-5-[5-Methyl-1,3,4-thiadiazol-2-ylsulfanylmethyl]-1-oxo-4-thia-1,1a,2,3,3a,6b-hexahydro-2,6a-diaza-cyclobut[cd]inden-6-carbonsäure

Wird in Analogie zu Beispiel 29(a) ausgehend von (1aS,3aR,6bS)-5-[5-Methyl-1,3,4-thiadiazol-2-ylsulfanylmethyl]-1-oxo-4-thia-1,1a,2,3,3a,6b-hexahydro-2,6a-diaza-cyclobut[cd]inden-2,6-dicarbonsäure-diallylester in 27% Ausbeute als weisses Pulver hergestellt.
IR(KBr): 1742, 1608, 1378 $cm^{-1}$
MS(ISN): 355.2 $(M\text{-}Na)^-$; 311.1 $(M\text{-}Na\text{-}CO_2)^-$;

Der als Ausgangsverbindung eingesetzte (1aS,3aR,6bS)-5-[5-Methyl-1,3,4-thiadiazol-2-ylsulfanylmethyl]-

1-oxo-4-thia-1,1a,2,3,3a,6b-hexahydro-2,6a-diaza-cyclobut[cd]inden-2,6-dicarbonsäure-di-allylester kann in Analogie zu Beispiel 40(a) ausgehend von (1aS,3aR,6bS)-5-Methylsulfonyloxymethyl-1-oxo-1,1a,2,3,3a,6b-hexahydro-4-thia-2,6a-diaza-cyclobut[cd] inden-2,6-dicarbonsäure-diallylester und 5-Mercapto-2-methyl-1,3,4-thiadiazol hergestellt werden:
IR(Film): 1775, 1707, 1579, 1411 $cm^{-1}$

Beispiel 41

(a) (1aS,3aR,6bS)-2-(4-Hydroxy-phenylcarbamoyl)-5-(6,7-dihydro-5H-1-pyrindin-4-ylsulfanylmethyl)-1-oxo-4-thia-1,1a,2,3,3a,6b-hexahydro-2,6a-diaza-cyclobut[cd]inden-6-carbonsäure-Natriumsalz

Wird in Analogie zu Beispiel 31(a) ausgehend von (1aS,3aR,6bS)-5-(6,7-Dihydro-5H-1-pyrindin-4-ylsulfanylmethyl)-1-oxo-4-thia-1,1a,2,3,3a,6b-hexahydro-2,6a-diaza-cyclobut[cd]inden-6-carbonsäure und 4-Hydroxyphenylcarbaminsäure-2,5-dioxopyrrolidin-1-yl-ester in 44% Ausbeute als farbloses Pulver erhalten.
IR(KBr): 1756, 1611, 1572, 1512 $cm^{-1}$
MS(ISP): 511.3 $(M\text{-}Na+2H)^+$

Der als Ausgangsverbindung eingesetzte (1aS,3aR,6bS)-5-(6,7-dihydro-5H-1-pyrindin-4-ylsulfanylmethyl)-1-oxo-4-thia-1,1a,2,3,3a,6b-hexahydro-2,6a-diaza-cyclobut[cd]inden-6-carbonsäure kann in Analogie zu Beispiel 29(a) und 40(a) ausgehend von (1aS,3aR,6bS)-5-methylsulfonyloxymethyl-1-oxo-1,1a,2,3,3a,6b-hexahydro-4-thia-2,6a-diaza-cyclobut[cd]inden-2,6-dicarbonsäure-diallylester und 2,3-Cyclopenteno-1H-pyridin-4-thion hergestellt werden.
IR(KBr): 1775, 1708, 1567, 1411 $cm^{-1}$
MS(ISP): 500.3 $(M+H)^+$

In Analogie hierzu werden hergestellt:

(b) (1aS,3aR,6bS)-2-(4-Hydroxy-phenylcarbamoyl)-1-oxo-5-(pyridin-4-ylsulfanylmethyl)-4-thia-1,1a,2,3,3a,6b-hexahydro-2,6a-diaza-cyclobut[cd]inden-6-carbonsäure-Natriumsalz

Wird in Analogie zu Beispiel 31(a) ausgehend von (1aS,3aR,6bS)-5-(Pyridin-4-ylsulfanylmethyl)-1-oxo-4-thia-1,1a,2,3,3a,6b-hexahydro-2,6a-diaza-cyclobut[cd]inden-6-carbonsäure und 4-Hydroxy-phenylcarbaminsäure-2,5-dioxopyrrolidin-1-yl-ester in 68% Ausbeute als farbloses Pulver erhalten.
IR(KBr): 1750, 1609, 1580, 1538, 1512 $cm^{-1}$
MS(ISP): 493.2 $(M+H)^+$; 471.2 $(M\text{-}Na+2H)^+$

(c) (1aS,3aR,6bS)-2-(4-Hydroxy-phenylcarbamoyl)-5-(5-methyl-1,3,4-thiadiazol-2-ylsulfanylmethyl)-1-oxo-4-thia-1,1a,2,3,3a,6b-hexahydro-2,6a-diaza-cyclobut[cd]inden-6-carbonsäure-Natriumsalz

Wird in Analogie zu Beispiel 31(a) ausgehend von (1aS,3aR,6bS)-5-(5-Methyl-1,3,4-thiadiazol-2-ylsulfanylmethyl)-1-oxo-4-thia-1,1a,2,3,3a,6b-hexahydro-2,6a-diaza-cyclobut[cd]inden-6-carbonsäure und 4-Hydroxyphenylcarbaminsäure-2,5-dioxopyrrolidin-1-yl-ester in 68% Ausbeute als farbloses Pulver erhalten.
IR(KBr): 1751, 1609, 1541, 1513 $cm^{-1}$
MS(ISN): 490.2 $(M\text{-}Na)^-$; 446.3 $(M\text{-}Na\text{-}CO_2)^-$;

(d) (1aS,3aR,6bS)-2-(4-Hydroxy-phenylcarbamoyl)-5-(1-methyl-1H-tetrazol-5-ylsulfanylmethyl)-1-oxo-4-thia-1,1a,2,3,3a,6b-hexahydro-2,6a-diaza-cyclobut[cd]inden-6-carbonsäure-Natriumsalz

Wird in Analogie zu Beispiel 31(a) ausgehend von (1aS,3aR,6bS)-5-(1-Methyl-1H-tetrazol-5-ylsulfanylmethyl)-1-oxo-4-thia-1,1a,2,3,3a,6b-hexahydro-2,6a-diaza-cyclobut[cd]inden-6-carbonsäure und 4-Hydroxyphenylcarbaminsäure-2,5-dioxopyrrolidin-1-yl-ester in 16% Ausbeute als farbloses Pulver erhalten.
IR(KBr): 1750, 1613, 1513 $cm^{-1}$

Als Nebenprodukt wird (1aS,3aR,6bS)-5-Hydroxymethyl-2-(4-hydroxyphenylcarbamoyl)-1-oxo-4-thia-1,1a,2,3,3a,6b-hexahydro-2,6a-diaza-cyclobut[cd]inden-6-carbonsäure erhalten.
IR(KBr): 1748, 1609, 1513, 1438$cm^{-1}$
MS(ISN): 398.3 $(M)^-$; 376.3 $(M\text{-}Na)^-$.

Beispiel 42

(a) (1aS,3aR,6bS)-2-Acetyl-5-(5-methyl-1,3,4-thiadiazol-2-ylsulfanylmethyl)-1-oxo-4-thia-1,1a,2,3,3a,6b-hexahydro-2,6a-diaza-cyclobut[cd]inden-6-carbonsäure-Natriumsalz

Wird in Analogie zu Beispiel 33 ausgehend von (1aS,3aR,6bS)-5-(5-Methyl-1,3,4-thiadiazol-2-ylsulfanylmethyl)-1-oxo-4-thia-1,1a,2,3,3a,6b-hexahydro-2,6a-diaza-cyclobut[cd]inden-6-carbonsäure und Acetylchlorid in 77% Ausbeute als farbloses Pulver erhalten.
IR(KBr): 1759, 1616 $cm^{-1}$
MS(ISP): 443.2 $(M+Na)^+$; 421.2 $(M+H)^+$; 399.2 $(M+H-Na)^+$

(b) (1aS,3aR,6bS)-2-Acetyl-1-oxo-5-(pyridin-4-ylsulfanylmethyl)-4-thia-1,1a,2,3,3a,6b-hexahydro-2,6a-diaza-cyclobut[cd]inden-6-carbonsäure-Natriumsalz

Wird in Analogie zu Beispiel 32 ausgehend von (1aS,3aR,6bS)-5-(Pyridin-4-ylsulfanylmethyl)-1-oxo-4-thia-1,1a,2,3,3a,6b-hexahydro-2,6a-diaza-cyclobut[cd]inden-6-carbonsäure und Essigsäureanhydrid in 42% Ausbeute als farbloses Pulver erhalten.
IR(KBr): 1752, 1614, 1576 $cm^{-1}$
MS(ISN): 393.3 $(M-Na+NH_3)^-$; 376.2 $(M-Na)^-$

(c) (1aS,3aR,6bS)-2-(2-Amino-1,3,4-thiadiazol-5-ylsulfanylacetyl)-5-(1-methyl-1H-tetrazol-5-ylsulfanylmethyl)-1-oxo-4-thia-1,1a,2,3,3a,6b-hexahydro-2,6a-diaza-cyclobut[cd]inden-6-carbonsäure-Natriumsalz

Wird in Analogie zu Beispiel 11 ausgehend von (1aS,3aR,6bS)-5-(1-Methyl-1H-tetrazol-5-ylsulfanylmethyl)-1-oxo-4-thia-1,1a,2,3,3a,6b-hexahydro-2,6a-diaza-cyclobut[cd]inden-6-carbonsäure, Bromacetyl-chlorid und 2-Amino-5-mercapto-1,3,4-thiadiazol in 27% Ausbeute als graues Pulver erhalten.
IR(KBr): 1756, 1616, 1497, 1411 $cm^{-1}$
MS(ISP): 536.2 $(M+H)^+$; 514.2 $(M-Na+2H)^+$

Beispiel 43

(1aS,3aR,6bS)-5-(1-Methyl-1H-tetrazol-5-ylmethylsulfanylmethyl)-1-oxo-4-thia-1,1a,2,3,3a,6b-hexahydro-2,6a-diaza-cyclobut[cd]inden-6-carbonsäure-Natriumsalz

Wird in Analogie zu Beispiel 29(a) ausgehend von (1aS,3aR,6bS)-5-(1-Methyl-1H-tetrazol-5-ylmethylsulfanylmethyl)-1-oxo-4-thia-1,1a,2,3,3a,6b-hexahydro-2,6a-diaza-cyclobut[cd]inden-2,6-dicarbonsäure-diallylester in 28% Ausbeute als weisses Pulver hergestellt.
IR(KBr): 1740, 1606, 1466, 1573$cm^{-1}$
MS(ISP): 377.2 $(M+H)^+$; 355.2 $(M+2H-Na)^+$

Der als Ausgangsverbindung eingesetzte (1aS,3aR,6bS)-5-(1-Methyl-1H-tetrazol-5-ylmethylsulfanylmethyl)-1-oxo-4-thia-1,1a,2,3,3a,6b-hexahydro-2,6a-diaza-cyclobut[cd]inden-2,6-dicarbonsäure-diallylester kann wie folgt hergestellt werden:

Eine Lösung von (1aS,3aR,6bS)-5-Methylsulfonyloxymethyl-1-oxo-1,1a,2,3,3a,6b-hexahydro-4-thia-2,6a-diaza-cyclobut[cd]inden-2,6-dicarbonsäure-diallylester (11 mg; 0.25 mmol) in 1N Natronlauge (0.5 ml) wird bei 0 °C mit Tetrabutylammoniumbromid (32 mg; 0.1 mmol) und einer Lösung von 5-Mercaptomethyl-1H-1-methyltetrazol (65 mg; 0.5 mmol) in Methylenchlorid versetzt. Das Reaktionsgemisch wird 4 Stunden weitergerührt. Die organische Phase wird abgetrennt und mit Wasser gewaschen, dann getrocknet und eingeengt. Der Rückstand wird mit Aethylacetat/n-Hexan (1:1) chromatographiert. Ausbeute 73 mg (63%)
IR(KBr): 1774, 1707, 1579, 1411 $cm^{-1}$
MS(ISP): 479.3 $(M+H)^+$

Beispiel 44

(1aS,3aR,6bS)-5-(5-Methylsulfanyl-1H-tetrazol-1-ylmethyl)-1-oxo-1,1a,2,3,3a,6b-hexahydro-4-thia-2,6a-diaza-cyclobut[cd]inden-6-carbonsäure-Natriumsalz

Wird in Analogie zu Beispiel 29(a) ausgehend von (1aS,3aR,6bS)-5-(5-Methylsulfanyl-1H-tetrazol-1-ylmethyl)-1-oxo-1,1a,2,3,3a,6b-hexahydro-4-thia-2,6a-diaza-cyclobut[cd]inden-2,6-dicarbonsäure-diallylester

in 33% Ausbeute erhalten.
IR(KBr): 1745, 1614, 1384, 1351 $cm^{-1}$
MS(ISP): 363.2 $(M+H)^+$; 341.3 $(M\text{-}Na+2H)^+$

Die dabei eingesetzte Ausgangsverbindung wird in Analogie zu Beispiel 29(c) aus (1aS,3aR,6bS)-5-Hydroxymethyl-1-oxo-1,1a,2,3,3a,6b-hexahydro-4-oxa-2,6-diaza-cyclobut[cd]inden-2,6-dicarbonsäure-diallylester und 5-Methylsulfanyl-1H-tetrazol in 41% Ausbeute hergestellt.
IR(KBr): 1774, 1701, 1700, 1418 $cm^{-1}$
MS(ISP): 487.3 $(M+Na)^+$; 482.3 $(M+NH_4)^+$ ; 365.3 $(M+H)^+$

Beispiel 45

(1aS,3aR,6bR)-1-Oxo-5-vinyl-1a,2,3,3a,4,6b-hexahydro-1H-2,6a-diaza-cyclobut[cd]inden-6-carbonsäure-trifluoracetat

Ausgehend von (1aS,3aR,6bR)-1-Oxo-5-vinyl-1a,2,3,3a,4,6b-hexahydro-1H-2,6a-diaza-cyclobut[cd]inden-2,6-dicarbonsäure-di-t-butylester (185mg; 0,49mmol) werden in Analogie zu Beispiel 2(a) 120 mg (73%) als gelblicher Festkörper erhalten.
IR(KBr): 1771, 1678, 1662, 1201 $cm^{-1}$
MS (ISP): $(M+H)^+$ 221,3

Der als Ausgangsverbindung eingesetzte (1aS,3aR,6bR)-1-Oxo-5-vinyl-1a,2,3,3a,4,6b-hexahydro-1H-2,6a-diaza-cyclobut[cd]inden-2,6-dicarbonsäure-di-t-butylester kann wie folgt hergestellt werden:

Zu einer Lösung von (1aS,3aR,6bR)-1-Oxo-5-trifluormethylsulfonyloxy-1a,2,3,3a,4,6b-hexahydro-1H-2,6a-diaza-cyclobut[cd]inden-2,6-dicarbonsäure-di-t-butylester (250 mg; 0.50 mmol; aus Beispiel 2(d)) in 1-Methyl-2-pyrrolidinon (2 ml) werden nacheinander Tris(dibenzylidenaceton)-dipalladium(0) (10 mg; 0,011 mmol), Zinkchlorid (136 mg; 1,0 mmol), Tri-(2-furyl)-phosphin(4,5 mg; 0,019 mmol) und schliesslich Trimethyl-vinylstannane (114 mg; 0,60 mmol) gegeben. Bei Raumtemperatur wird 4,5 Stunden gerührt. Das Reaktionsgemisch wird auf Wasser (20 ml) gegossen und mit Aethylacetat (25 ml) extrahiert. Die organische Phase wird mit Wasser (20 ml) gewaschen, über Magnesiumsulfat getrocknet und eingeengt. Der Rückstand wird an Kieselgel chromatographiert (Laufmittel: Aethylacetat/n-Hexan 1:4). Man erhält 110 mg (61%) als weissen Festkörper.
IR(KBr): 1765, 1708, 1242, 1161, 990, 926 $cm^{-1}$
MS (ISP): 399,4 $(M+Na)^+$; 394,4 $(M+NH_4)^+$; 377,4 $(M+H)^+$

Beispiel 46

(a) (1aS,3aR,6bR)-2-(4-Hydroxy-phenylcarbamoyl)-1-oxo-5-vinyl-1a,2,3,3a,4,6b-hexahydro-1H-2,6a-diaza-cyclobut[cd]inden-6-carbonsäure-Natriumsalz

Ausgehend von (1aS,3aR,6bR)-1-Oxo-5-vinyl-1a,2,3,3a,4,6b-hexahydro-1H-2,6a-diaza-cyclobut[cd]inden-6-carbonsäure-trifluoracetat (133 mg; 0,40 mmol; aus Beispiel 45) werden in Analogie zu Beispiel 3(a) 75 mg (50%) als weisses Pulver isoliert.
IR(KBr): 3417, 1745, 1606, 1513, 1393, 1244, 990, 905 $cm^{-1}$
MS (ISN): $(M\text{-}Na)^-$ 354,4

(b) (1aS,3aR,6bR)-2-(4-Carbamoyl-phenylcarbamoyl)-1-oxo-5-vinyl-1a,2,3,3a,4,6b-hexahydro-1H-2,6a-diaza-cyclobut[cd]inden-6-carbonsäure-Natriumsalz

Ausgehend von (1aS,3aR,6bR)-1-Oxo-5-vinyl-1a,2,3,3a,4,6b-hexahydro-1H-2,6a-diaza-cyclobut[cd]inden-6-carbonsäure-trifluoroacetat (133 mg; 0,40 mmol; aus Beispiel 45) werden in Analogie zu Beispiel 3(a) 66 mg (41%) als weisses Pulver isoliert.
IR(KBr): 3423, 1748, 1663, 1605, 1524, 1411, 990, 905, 853 $cm^{-1}$
MS (ISN): $(M\text{-}Na)^-$ 381,4

Beispiel 47

(1aS,3aR,6bR)-2-Acetyl-1-oxo-5-vinyl-1a,2,3,3a,4,6b-hexahydro-1H-2,6a-diaza-cyclobut[cd]inden-6-carbonsäure-Natriumsalz

Ausgehend von (1aS,3aR,6bR)-1-Oxo-5-vinyl-1a,2,3,3a,4,6b-hexahydro-1H-2,6a-diaza-cyclobut[cd]inden-6-carbonsäure-trifluoracetat (133 mg; 0,40 mmol; aus Beispiel 45) werden in Analogie zu Beispiel 3(ah) 54 mg (48%) als beiges Pulver isoliert.
IR(KBr): 1751, 1615, 1399, 990, 905 $cm^{-1}$
MS (ISN): $(M\text{-}Na)^-$ 278,4

Beispiel 48

(a) (Z)-(1aS,3aR,6bR)-5-(2-Cyanvinyl)-1-oxo-1a,2,3,3a,4,6b-hexahydro-1H-2,6a-diaza-cyclobut[cd]inden-6-carbonsäure-trifluoracetate

Diese Verbindung wird in der gleichen weise wie in Beispiel 2(a) angegeben ausgehend von (Z)-(1aS,3aR,6bR)-5-(2-Cyanvinyl)-1-oxo-1a,2,3,3a,4,6b-hexahydro-1H-2,6a-diaza-cyclobut[cd]inden-2,6-dicarbonsäure-di-t-butylester (500 mg; 1,25 mmol) hergestellt. Ausbeute: 375 mg (84 %) als gelber Feststoff.
IR (KBr): 2208, 1781, 1676, 1596, 1201 $cm^{-1}$
MS (ISP): $(M+H)^+$ 246,2

(b) (E)-(1aS,3aR,6bR)-5-(2-Cyanvinyl)-1-oxo-1a,2,3,3a,4,6b-hexahydro-1H-2,6a-diaza-cyclobut[cd]inden-6-carbonsäure-trifluoracetat

Diese Verbindung wird in der gleichen weise wie in Beispiel 2(a) angegeben ausgehend von (E)-(1aS,3aR,6bR)-5-(2-Cyanvinyl)-1-oxo-1a,2,3,3a,4,6b-hexahydro-1H-2,6a-diaza-cyclobut[cd]inden-2,6-dicarbonsäure-di-t-butylester (500 mg; 1,25 mmol) hergestellt. Ausbeute: 353 mg (79 %) als beiger Feststoff.
IR (KBr): 2215, 1782, 1676, 1602, 1201 $cm^{-1}$
MS (ISP): $(M+H)^+$ 246,3
Die hierfür verwendeten Ausgangsverbindungen werden wie folgt hergestellt:

a) (1aS,3aR,6bR)-5-Formyl-1-oxo-1a,2,3,3a,4,6b-hexahydro-1H-2,6a-diaza-cyclobut[cd]inden-2,6-dicarbonsäure-di-t-butylester

Diese Verbindung wird in der gleichen Weise wie in Beispiel 1(c) angegeben ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (760 mg, 2,00 mmol) hergestellt. Man erhält 757 mg (100%) als beigen Feststoff.
IR (KBr): 2760, 1789, 1711, 1672 $cm^{-1}$
MS (EI): (M- $^t$BuO) 305

b) (Z)- und (E)-(1aS,3aR,6bR)-5-(2-Cyanvinyl)-1-oxo-1a,2,3,3a,4,6b-hexahydro-1H-2,6a-diaza-cyclobut[cd]-inden-2,6-dicarbonsäure-di-t-butylester

(1aS,3aR,6bR)-5-Formyl-1-oxo-1a,2,3,3a,4,6b-hexahydro-1H-2,6a-diaza-cyclobut[cd]inden-2,6-dicarbonsäure-di-t-butylester(757 mg; 2,00 mmol) wird in Acetonitril gelöst, und bei -20 °C mit Lithiumperchlorat (212 mg; 2,00 mmol) und Cyanmethylentriphenylphosphoran (602 mg; 2,00 mmol) versetzt. Nach 2 Stunden wird die Lösung mit Essigester (100 ml) verdünnt und nacheinander mit 1N wässriger Salzsäure (50 ml) und gesättigter wässriger Kochsalzlösung (50 ml) gewaschen. Die organische Phase wird über Magnesiumsulfat getrocknet und eingeengt. Die Trennung der Z- und E-Isomeren erfolgt durch Chromatographie über Kieselgel (50 g: 0,040-0,063 mm Korngrösse) mit Essigester/n-Hexan 1:1.
Ausbeute Z-Isomer: 217 mg (27%) als gelber Feststoff.
IR (KBr): 2208, 1780, 1707, 1597 $cm^{-1}$
MS (ISP): $(M+NH_4^+)$ 419,4
Ausbeute E-Isomer: 289 mg (36%) als gelbes dickflüssiges Öl.

IR (KBr): 2216, 1781, 1707, 1602 $cm^{-1}$
MS (ISP): ($M + NH_4^+$) 419,4

Beispiel 49

(a) (Z)-(1aS,3aR,6bR)-5-(2-Cyanvinyl)-2-(4-hydroxy-phenylcarbamoyl)-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 3(a) angegeben ausgehend von (Z)-(1aS,3aR,6bR)-5-(2-Cyanvinyl)-1-oxo-1a,2,3,3a,4,6b-hexahydro-1H-2,6a-diaza-cyclobut[cd]inden-6-carbonsäure-trifluoracetat (195 mg; 0,6 mmol) hergestellt. Ausbeute: 85 mg (35%) als oranges Pulver.
IR (KBr): 2200, 1756, 1609, 1390, 1239 $cm^{-1}$
MS (ISN): $(M\text{-}Na)^-$ 379,2

(b) (E)-(1aS,3aR,6bR)-5-(2-Cyanvinyl)-2-(4-hydroxy-phenylcarbamoyl)-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 3(a) angegeben ausgehend von (E)-(1aS,3aR,6bR)-5-(2-Cyanvinyl)-1-oxo-1a,2,3,3a,4,6b-hexahydro-1H-2,6a-diaza-cyclobut[cd]inden-6-carbonsäure-trifluoracetat (195 mg; 0,6 mmol) hergestellt. Ausbeute: 98 mg (41%) als rosarotes Pulver.
IR (KBr): 2216, 1756, 1609, 1391, 1248 $cm^{-1}$
MS (ISN): $(M\text{-}Na)^-$ 379,0

Beispiel 50

(1aS,3aR,6bR)-[4-(6-Carboxy-1-oxo-1a,2,3,3a,4,6b-hexahydro-1H-2,6a-diazacyclobut[cd]inden-5-ylmethylsulfanyl)-pyridin-1-ylio]acetat-trifluoracetat

Diese Verbindung wird in der gleichen Weise wie in Beispiel 2(a) angegeben ausgehend von (1aS,3aR,6bR)-4-(2,6-Bis-t-butoxycarbonyl-1-oxo-1a,2,3,3a,4,6b-hexahydro-1H-2,6a-diazacyclobut[cd]inden-5-ylmethylsulfanyl)-1-t-butoxycarbonylmethyl-pyridinium-bromid (370 mg; 0,604 mmol) hergestellt. Ausbeute: 289 mg (98 %) als leicht gelber Feststoff.
IR (KBr): 1777, 1679, 1390, 1197 $cm^{-1}$
MS (ISP): $(M+H)^+$ 376,2
Die Ausgangsverbindung wird in Analogie zu Beispiel 20 ausgehend von (1aS,3aR,6bR)-1-oxo-5-(pyridin-4-ylsulfanylmethyl)-1a,2,3,3a,4,6b-hexahydro-1H-2,6a-diaza-cyclobut[cd]inden-2,6-dicarbonsäure-di-t-butylester (452 mg; 0,874 mmol; aus Beispiel 17) und Bromessigsäure-t-butylester (0,64 ml; 4,37 mmol) hergestellt. Ausbeute: 487 mg (85%) als farbloses Pulver.
IR (KBr): 1777, 1742, 1705, 1631, 1494 $cm^{-1}$
MS (ISP): $M^+$ 588,5

Beispiel 51

(1aS,3aR,6bR)-[4-(2-Benzylcarbamoyl-6-carboxy-1-oxo-1a,2,3,3a,4,6b-hexahydro-1H-2,6a-diaza-cyclobut[cd]inden-5-ylmethylsulfanyl)-pyridin-2-ylio]-acetat-Natriumsalz

Diese Verbindung wird in der gleichen Weise wie in Beispiel 27(c) angegeben ausgehend von (1aS,3aR,6bR)-[4-(6-Carboxy-1-oxo-1a,2,3,3a,4,6b-hexahydro-1H-2,6a-diazacyclobut [cd] inden-5-ylmethylsulfanyl)-pyridin-1-ylio]acetat-trifluoracetat (377 mg; 0,77 mmol) hergestellt. Ausbeute: 41 mg (10%) als leicht gelbes Pulver.
IR (KBr): 1752, 1632, 1541, 1492, 1374 $cm^{-1}$
MS (ISN): $(M\text{-}H)^-$ 507,2

Beispiel 52

(a) (1aS,3aR,6bR)-[4-(2-Acetyl-6-carboxy-1-oxo-1a,2,3,3a,4,6b-hexahydro-1H-2,6a-diaza-cyclobut[cd]inden-5-ylmethylsulfanyl)-pyridinio]-acetat-bromacetat (1:3,18) Natriumsalz (1:4,18)

(1aS,3aR,6bR)-1-Oxo-5-(pyridin-4-ylsulfanylmethyl)-1a,2,3,3a,4,6b-hexahydro-1H-2,6a-diaza-cyclobut-[cd]inden-6-carbonsäure-trifluoracetat (165 mg; 0,3 mmol) wird in Dimethylformamid (2 ml) bei 0°C vorgelegt und mit Natriumbicarbonat (108 mg; 1,29 mmol) und Acetylchlorid (0,026 ml; 0,36 mmol) versetzt. Nach 1 Stunde wird Natriumbicarbonat (60 mg; 0,72 mmol) und 2-Bromessigsäure (100 mg; 0,72 mmol) zugegeben. Das Reaktionsgemisch wird anschliessend 20 Stunden bei Raumtemperatur gerührt und eingeengt. Der Rückstand wird in Wasser (2 ml) gelöst und der pH-Wert mit gesättigter wässriger Natriumbicarbonatlösung auf 7 eingestellt. Die Lösung wird über ein polymeres hydrophobes Gel mit Wasser chromatographiert und lyophilisiert. Ausbeute: 167 mg (59%) als farbloses Pulver.
IR (KBr): 1752, 1689, 1632, 1414 $cm^{-1}$
MS (ISP): $(M+H)^+$ 418,4; $(M+Na)^+$ 440,4; $(M+2Na)^+$ 462,4

(b) (1aS,3aR,6bR)-[4-(2-Trifluoracetyl-6-carboxy-1-oxo-1a,2,3,3a,4,6b-hexahydro-1H-2,6a-diaza-cyclobut[cd]-inden-5-ylmethylsulfanyl)pyridin-1-ylio]-acetat-Natriumsalz

(1aS,3aR,6bR)-1-Oxo-5-(pyridin-4-ylsulfanylmethyl)-1a,2,3,3a,4,6b-hexahydro-1H-2,6a-diaza-cyclobut-[cd]inden-6-carbonsäure-trifluoracetat (165 mg; 0,3 mmol) wird in Dimethylformamid (2 ml) bei Raumtemperatur vorgelegt und mit Natriumbicarbonat (28 mg; 0,33 mmol) und Dicyclohexylcarbodiimid (74 mg; 0,36 mmol) versetzt. Nach 1 Stunde wird Natriumbicarbonat (38 mg; 0,45 mmol), 2-Bromessigsäure (63 mg; 0,45 mmol) und N-Methyl-N-trimethylsilyltrifluoracetamid (0,2 ml; 1,0 mmol) zugegeben. Das Reaktionsgemisch wird anschliessend 20 Stunden bei Raumtemperatur gerührt und eingeengt. Der Rückstand wird in Wasser (2 ml) gelöst und der pH-Wert mit gesättigter wässriger Natriumbicarbonatlösung auf 7 eingestellt. Die Lösung wird über ein polymeres hydrophobes Gel mit Wasser chromatographiert und lyophilisiert. Ausbeute: 32 mg (22%) als gelbes Pulver.
IR (KBr): 1765, 1695, 1631, 1369 $cm^{-1}$
MS (ISP): $(M+H)^+$ 494,4

(c) (1aS,3aR,6bR)-5-(1-Carbamoylmethyl-pyridin-4-yliosulfanylmethyl)-1-oxo-2-trifluoracetyl-1a,2,3,3a,4,6b-hexahydro-1H-2,6a-diaza-cyclobut[cd]inden-6-carboxylat

Diese Verbindung wird in Analogie zu Beispiel 52(b) ausgehend von (1aS,3aR,6bR)-1-Oxo-5-(pyridin-4-ylsulfanylmethyl)-1a,2,3,3a,4,6b-hexahydro-1H-2,6a-diaza-cyclobut[cd]inden-6-carbonsäure-trifluoracetat (165 mg; 0,3 mmol) und 2-Bromacetamid (125 mg; 0,90 mmol) hergestellt. Ausbeute: 12 mg (9%) als farbloses Pulver.
IR (KBr): 1768, 1692, 1631, 1600, 1393 $cm^{-1}$
MS (ISP): $(M+H)^+$ 471,4

(d) (1aS,3aR,6bR)-5-(1-Benzyl-pyridin-4-yliosulfanylmethyl)-1-oxo-2-trifluoracetyl-1a,2,3,3a,4,6b-hexahydro-1H-2,6a-diaza-cyclobut[cd]inden-6-carboxylat

Diese Verbindung wird in Analogie zu Beispiel 52(b) ausgehend von (1aS,3aR,6bR)-1-Oxo-5-(pyridin-4-ylsulfanylmethyl)-1a,2,3,3a,4,6b-hexahydro-1H-2,6a-diaza-cyclobut[cd]inden-6-carbonsäure-trifluoracetat (223 mg; 0,4 mmol) und Benzylbromid (0,12 ml; 1,0 mmol) hergestellt. Ausbeute: 17 mg (8%) als gelbes Pulver.
IR (KBr): 1767, 1693, 1626, 1387 $cm^{-1}$
MS (ISP): $(M+H)^+$ 504,3

Beispiel 53

(1aS,3aR,6bR)-5-(2,5-Dihydro-6-hydroxy-2-methyl-5-oxo-1,2,4-triazin-3-ylsulfanylmethyl)-1-oxo-1a,2,3,3a,4,6b-hexahydro-1H-2,6a-diaza-cyclobut[cd]inden-6-carbonsäure-trifluoracetat

(1aS,3aR,6bR)-5-acetoxymethyl-1-oxo-1a,2,3,3a,4,6b-hexahydro-1H-2,6a-diaza-cyclobut[cd]inden-6-carbonsäure-trifluoracetat (370 mg; 1,00 mmol) und Tetrahydro-2-methyl-3-thioxo-as-triazin-5,6-dion (167

mg; 1,05 mmol) werden in Acetonitril (2,5 ml) suspendiert und mit Bortrifluorid in Acetonitril (1,7 ml, 19%) versetzt. Nach 2 Stunden bei Raumtemperatur wird das Reaktionsgemisch eingeengt, mit abs. Aether verrührt und abgenutscht. Ausbeute: 606 mg (84%) als braun-beiges Pulver.
IR (KBr): 1765, 1730, 1629 $cm^{-1}$
MS (ISP): $(M+H)^+$ 366,4

Beispiel 54

(1aS,3aR,6bR)-5-(2,5-Dihydro-6-hydroxy-2-methyl-5-oxo-1,2,4-triazin-3-ylsulfanylmethyl)-2-(4-hydroxy-phenylcarbamoyl)-1-oxo-1a,2,3,3a,4,6b-hexahydro-1H-2,6a-diaza-cyclobut[cd]inden-6-carbonsäure-Natriumsalz (1:2)

Diese Verbindung wird in gleicher Weise wie in Beispiel 3(a) angegeben ausgehend von (1aS,3aR,6bR)-5-(2,5-Dihydro-6-hydroxy-2-methyl-5-oxo-1,2,4-triazin-3-ylsulfanylmethyl)-1-oxo-1a,2,3,3a,4,6b-hexahydro-1H-2,6a-diaza-cyclobut[cd]inden-6-carbonsäure-trifluoracetat (300 mg; 0,41 mmol) hergestellt. Ausbeute: 58 mg (17%) als beiges Pulver.
IR (KBr): 1748, 1630, 1604, 1546, 1401, 1241 $cm^{-1}$
MS (ISN): $(M\text{-}2Na+H)^-$ 499,2

Beispiel 55

(1aS,3aR,6bR)-2-Carboxymethylcarbamoyl-5-(piperidin-1-ylmethyl)-1-oxo-1a,2,3,3a,4,6b-hexahydro-1H-2,6a-diaza-cyclobut[cd]inden-6-carbonsäure

(1aS,3aR,6bR)-2-Benzyloxycarbonylmethylcarbamoyl-1-oxo-(5-pyridin-1-yliomethyl)-1a,2,3,3a,4,6b-hexahydro-1H-2,6a-diaza-cyclobut[cd]inden-6-carboxylat (400 mg; 0,84 mmol) wird in Wasser (100 ml) und Acetonitril (50 ml) über 10% Pd/C (100 mg) hydriert. Nach 2 Stunden wird das Reaktionsgemisch abgenutscht, eingeengt, und über ein polymeres hydrophobes Gel mit Wasser chromatographiert und lyophilisiert. Ausbeute: 200 mg (63%) als gelbes Pulver.
IR (KBr): 1757, 1700, 1608, 1537, 1395 $cm^{-1}$
MS (ISN): $(M\text{-}H)^-$ 391,4

Beispiel 56

(1aS,3aR,6bR)-5-(3-Benzyloxycarbonylmethyl-pyridin-1-yliomethyl)-1-oxo-1a,2,3,3a,4,6b-hexahydro-1H-2,6a-diaza-cyclobut[cd]inden-6-carboxylat-trifluoracetat

Diese Verbindung wird in gleicher Weise wie in Beispiel 2(a) angegeben ausgehend von (1aS,3aR,6bR)-3-Benzyloxycarbonylmethyl-1-(2,6-bis-t-butoxycarbonyl-1-oxo-1a,2,3,3a,4,6b-hexahydro-1H-2,6a-diaza-cyclobut[cd]inden-5-ylmethyl)-pyridinium-chlorid (314 mg; 0,5 mmol) hergestellt. Ausbeute: 179 mg (65%) als farbloses Pulver.
IR (KBr): 1782, 1737, 1677, 1636 $cm^{-1}$
MS (ISP): $M^+$ 434,5

Durch Abhydrierung der Benzylgruppe mit Pd/C erhält man die 3-Carboxymethyl-Verbindung in bezug auf die Pyridingruppe.

Die Ausgangsverbindung wird wie folgt hergestellt:

(1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (200 mg; 0,526 mmol) wird in abs. Methylenchlorid (3 ml) bei -40 °C vorgelegt und nacheinander mit 3-Pyridylessigsäure-benzylester (300 mg; 1,315 mmol) und Triflat-anhydrid (0,13 ml; 0,79 mmol) versetzt. Nach 1 Stunde bei dieser Temperatur wird das Reaktionsgemisch mit Methylenchlorid verdünnt (20 ml), mit gesättigter wässriger Kochsalzlösung (dreimal 10 ml) gewaschen, über Magnesiumsulfat getrocknet und eingeengt. Der erhaltene Rückstand wird mit abs. Aether (20 ml) verrührt und abgenutscht. Ausbeute: 327 mg (100%) als beiges Pulver.
IR (KBr): 1779, 1705, 1630, 1160 $cm^{-1}$
MS (ISP): $M^+$ 590,7

Beispiel 57

(a) (1aS,3aR,6bR)-5-(3-Benzyloxycarbonylmethyl-pyridin-1-yliomethyl)-2-(4-hydroxy-phenylcarbamoyl)-1-oxo-1a,2,3,3a,4,6b-hexahydro-1H-2,6a-diaza-cyclobut[cd]inden-6-carboxylat

Diese Verbindung wird in der gleichen Weise wie in Beispiel 3(a) angegeben ausgehend von (1aS,3aR,6bR)-5-(3-Benzyloxycarbonylmethylpyridin-1-yliomethyl)-1-oxo-1a,2,3,3a,4,6b-hexahydro-1H-2,6a-diaza-cyclobut[cd]inden-6-carboxylat-trifluoracetat (170 mg; 0,31 mmol; aus Beispiel 56) hergestellt. Ausbeute: 71 mg (40%) als beiges Pulver.
IR (KBr): 1765, 1616, 1512, 1381, 1243 $cm^{-1}$
MS (ISP): $(M+H)^+$ 569,5

(b) (1aS,3aR,6bR)-[1-[6-Carboxy-2-(4-hydroxy-phenylcarbamoyl)-1-oxo-1a,2,3,3a,4,6b-hexahydro-1H-2,6a-diaza-cyclobut[cd]inden-5-ylmethyl]pyridin-3-ylio]-acetat

(1aS,3aR,6bR)-5-(3-Benzyloxycarbonylmethyl-pyridin-1-yliomethyl)-2-(4-hydroxy-phenylcarbamoyl)-1-oxo-1a,2,3,3a,4,6b-hexahydro-1H-2,6a-diaza-cyclobut[cd]inden-6-carboxylat (66 mg; 0,116 mmol) wird in Wasser (10 ml) und Dimethylformamid (5 ml) gelöst und über 5% Pd/C hydriert. Nach 1 Stunde wird die Suspension abgenutscht und eingeengt. Der Rückstand wird in Wasser aufgenommen und Lyophilisiert. Ausbeute: 50 mg (90%) als gelbes Pulver.
IR (KBr): 1764, 1710, 1636, 1612, 1512, 1436, 1242 $cm^{-1}$
MS (ISP): $(M+H)^+$ 479,3

Beispiel 58

(a) (1aS,3aR,6bR)-2-Acetyl-5-carbamoyloxymethyl-1-oxo-1a,2,3,3a,4,6b-hexahydro-1H-2,6a-diaza-cyclobut-[cd]inden-6-carbonsäure-Natriumsalz

(1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (148 mg; 0,4 mmol) wird in Methylenchlorid (5 ml) suspendiert und mit N-Methyl-N-trimethylsilyltrifluoracetamid (0,171 ml; 0,88 mmol) versetzt. Nach 10 Minuten bei Raumtemperatur wird Natriumbicarbonat (41 mg; 0,48 mmol) und Acetylchlorid (0,035 ml; 0,48 mmol) zugegeben. Das Reaktionsgemisch wird 2 Stunden bei Raumtemperatur gerührt und anschliessend eingeengt. Der erhaltene Rückstand wird in Wasser (1 ml) aufgenommen und der pH-Wert mit gesättigter wässriger Natriumbicarbonatlösung auf 7 eingestellt. Die Lösung wird über ein polymeres hydrophobes Gel mit Wasser/Acetonitril chromatographiert und lyophilisiert. Ausbeute: 72 mg (54%) als gelbliches Pulver.
IR (KBr): 1755, 1710, 1640, 1609, 1402 $cm^{-1}$
MS (ISP): $(M+H)^+$ 332,4

(b) (1aS,3aR,6bR)-5-Carbamoyloxymethyl-1-oxo-2-(thiophen-2-yl-acetyl)-1a,2,3,3a,4,6b-hexahydro-1H-2,6a-diaza-cyclobut[cd]inden-6-carbonsäure-Natriumsalz

Diese Verbindung wird in Analogie zu Beispiel 19(d) ausgehend von (1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (148 mg; 0,4 mmol) und Thiophen-2-yl-essigester-2,5-dioxo-pyrrolidin-1-ylester (144 mg; 0,6 mmol) hergestellt. Ausbeute: 32 mg (19%) als beiges Pulver.
IR (KBr): 1751, 1645, 1610, 1402, 1239, 1191 $cm^{-1}$
MS (ISN): $(M-Na)^-$ 390,3

(c) (1aS,3aR,6bR)-5-Carbamoyloxymethyl-2-methylsulfonyl-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(e) ausgehend von (1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (148 mg; 0,4 mmol) hergestellt. Ausbeute: 29 mg (20%) als farbloses Pulver.
IR (KBr): 1751, 1607, 1402, 1333, 1154 $cm^{-1}$
MS (ISN): $(M-Na)^-$ 344,2

(d) (1aS,3aR,6bR)-5-Carbamoyloxymethyl-1-oxo-2-trifluoracetyl-1a,2,3,3a,4,6b-hexahydro-1H-2,6a-diaza-cyclobut[cd]inden-6-carbonsäure-Natriumsalz

Diese Verbindung wird in Analogie zu Beispiel 19(c) ausgehend von (1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (166 mg; 0,45 mmol) hergestellt. Ausbeute: 87 mg (50%) als farbloses Pulver.
IR (KBr): 1761, 1701, 1607, 1335, 1177 $cm^{-1}$
MS (ISN): $(M\text{-}Na)^-$ 362,4

(e) (1aS,3aR,6bR)-5-Carbamoyloxymethyl-2-(5-methyl-1,3,4-thiadiazol-2-ylsulfanylacetyl)-1-oxo-1a,2,3,3a,4,6b-hexahydro-1H-2,6a-diaza-cyclobut[cd]inden-6-carbonsäure-Natriumsalz

(1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (130 mg; 0,35 mmol) wird in Dimethylformamid (2 ml) bei -20°C vorgelegt und mit Natriumbicarbonat (118 mg; 1,4 mmol) und Bromessigsäurebromid (0,047 ml; 0,53 mmol) versetzt. Nach 2,5 Stunden bei dieser Temperatur wird 2-Methyl-5-mercapto-1,3,4-thiadiazol (56 mg; 0,42 mmol) und erneut Natriumbicarbonat (35 mg; 0,42 mmol) zugegeben. Nach 2 Stunden bei -20°C und 2 Stunden bei Raumtemperatur wird das Reaktionsgemisch eingeengt und der erhaltene Rückstand in Wasser (4 ml) aufgenommen. Der pH-Wert wird mit gesättigter wässriger Natriumbicarbonatlösung auf 7 eingestellt. Die Lösung wird über ein polymeres hydrophobes Gel mit Wasser/Acetonitril chromatographiert und lyophilisiert. Ausbeute: 71 mg (44%) als farbloses Pulver.
IR (KBr): 1754, 1710, 1650, 1606, 1399 $cm^{-1}$
MS (ISP): $(M+H)^+$ 462,4

(f) (1aS,3aR,6bR)-2-(5-Amino-1,3,4-thiadiazol-2-ylsulfanylacetyl)-5-carbamoyloxymethyl-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 58(e) ausgehend von (1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (130 mg; 0,35 mmol) und 2-Amino-5-mercapto-1,3,4-thiadiazol (58 mg; 0,42 mmol) hergestellt. Ausbeute: 67 mg (41%) als farbloses Pulver.
IR (KBr): 1750, 1606, 1402 $cm^{-1}$
MS (ISP): $(M+H)^+$ 463,4

(g) (1aS,3aR,6bR)-5-Carbamoyloxymethyl-1-oxo-2-pyridin-4-ylsulfanylacetyl-1a,2,3,3a,4,6b-hexahydro-1H-2,6a-diaza-cyclobut[cd]inden-6-carbonsäure-Natriumsalz

Diese Verbindung wird in Analogie zu Beispiel 58(e) ausgehend von (1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (130 mg; 0,35 mmol) und 4-Mercapto-pyridin (61 mg; 0,52 mmol) hergestellt. Ausbeute: 81 mg (53%) als farbloses Pulver.
IR (KBr): 1756, 1644, 1608, 1406, 1234 $cm^{-1}$
MS (ISP): $(M\text{-}Na+2H)^+$ 419,4; $(M+H)^+$ 441,4

(h) (1aS,3aR,6bR)-5-Carbamoyloxymethyl-1-oxo-2-phenylaminoacetyl-1a,2,3,3a,4,6b-hexahydro-1H-2,6a-diaza-cyclobut[cd]inden-6-carbonsäure-Natriumsalz

Diese Verbindung wird in Analogie zu Beispiel 58(e) ausgehend von (1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (130 mg; 0,35 mmol) und Anilin (0,048 ml; 0,52 mmol) hergestellt. Ausbeute: 57 mg (39%) als farbloses Pulver.
IR (KBr): 1751, 1650, 1604, 1405 $cm^{-1}$
MS (ISP): $(M\text{-}Na+2H)^+$ 401,4; $(M+H)^+$ 423,4

(i) (1aS,3aR,6bR)-2-Formyl-5-carbamoyloxymethyl-1-oxo-2-phenylaminoacetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(k) angegeben ausgehend von (1aS,3aR,6bR)-5-Carbamoyloxymethyl-1-oxo-1a,2,3,3a,4,6b-hexahydro-1H-2,6a-diaza-cyclobut[cd]inden-6-

carbonsäure-trifluoracetat (100 mg; 0,27 mmol) hergestellt. Ausbeute: 69 mg (81%) als gelbes Pulver.
IR (KBr): 1760, 1696, 1612, 1400 $cm^{-1}$
MS (ISN): $(M\text{-}Na)^-$ 294,1

Beispiel 59

(a) (1aS,3aR,6bR)-5-Carbamoyloxymethyl-2-(4-hydroxy-phenylcarbamoylmethyl)-1-oxo-1a,2,3,3a,4,6b-hexahydro-1H-2,6a-diaza-cyclobut[cd]inden-6-carbonsäure-Natriumsalz

(1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (148 mg; 0,4 mmol) wird in Dimethylformamid gelöst und mit N-Methyl-N-trimethylsilyltrifluoracetamid (0,17 ml; 0,88 mmol) bei Raumtemperatur versetzt. Nach 15 Minuten wird Natriumbicarbonat (41 mg; 0,48 mmol) und 2-Brom-4'-hydroxyacetanilid (111 mg; 0,48 mmol) zugegeben. Nach 5 Stunden wird das Reaktionsgemisch eingeengt und der erhaltene Rückstand in Wasser (2 ml) aufgenommen. Der pH-Wert wird mit gesättigter wässriger Natriumbicarbonatlösung auf 7 eingestellt und die Lösung über ein polymeres hydrophobes Gel mit Wasser/Acetonitril chromatographiert und lyophilisiert. Ausbeute: 99 mg (56%) als farbloses Pulver.
IR (KBr): 1750, 1728, 1668, 1602, 1402 $cm^{-1}$
MS (ISP): $(M+H)^+$ 439,5

(b) (1aS,3aR,6bR)-5-Carbamoyloxymethyl-2-methoxycarbonylmethyl-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 59(a) ausgehend von (1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (148 mg; 0,4 mmol) und Bromessigsäuremethylester (0,046 ml; 0,48 mmol) hergestellt. Ausbeute: 73 mg (50%) als gelbliches Pulver.
IR (KBr): 1750, 1734, 1602, 1401 $cm^{-1}$
MS (ISN): $(M\text{-}Na)^-$ 338,2

(c) (1aS,3aR,6bR)-5-Carbamoyloxymethyl-2-äthyl-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 59(a) ausgehend von (1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (148 mg; 0,4 mmol) und Aethyliodid (0,049 ml; 0,60 mmol) hergestellt. Ausbeute: 40 mg (31%) als gelbes Pulver.
IR (KBr): 1750, 1731, 1605, 1401 $cm^{-1}$
MS (ISN): $(M\text{-}Na)^-$ 294,3

(d) (1aS,3aR,6bR)-2-Carbamoylmethyl-5-carbamoyloxymethyl-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 59(a) ausgehend von (1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (148 mg; 0,4 mmol) und 2-Bromacetamid (68 mg; 0,48 mmol) hergestellt. Ausbeute: 19 mg (14%) als farbloses Pulver.
IR (KBr): 1750, 1700, 1676, 1602, 1400 $cm^{-1}$
MS (ISN): $(M\text{-}Na)^-$ 323,3

Beispiel 60

(Z)-(1aS,3aR,6bR)-2-[(2-Amino-thiazol-4-yl)-methoxyimino-acetyl]-5-carbamoyloxymethyl-1-oxo-1a,2,3,3a,4,6b-hexahydro-1H-2,6a-diaza-cyclobut[cd]inden-6-carbonsäure

(1aS,3aR,6bR)-5-Carbamoyloxymethyl-1-oxo-1a,2,3,3a,4,6b-hexahydro-1H-2,6a-diaza-cyclobut[cd]inden-6-carbonsäure-trifluoracetat (150 mg; 0,39 mmol) wird in Dimethylformamid (5 ml) gelöst und mit 2-(2-Aminothiazol-4-yl)-2-(Z)-methoxyimino-essigsäure-2-benzthiazolyl-thioester (151 mg; 0,43 mmol) bei Raumtemperatur versetzt. Nach 1 Stunde wird das Reaktionsgemisch eingeengt und der ölige Rückstand mit

Essigester (20 ml) verrührt. Das ausgefallene Produkt wird abgenutscht, mit Aceton und Aether gewaschen und getrocknet. Ausbeute: 82 mg (46%) als beiges Pulver.
IR (KBr): 1768, 1716, 1645, 1610, 1534, 1400, 1048 $cm^{-1}$
MS (ISP): $(M+H)^+$ 451,3

Beispiel 61

(a) (1aS,3aR,6bR)-2-Methoxycarbonylmethyl-5-(1-methyl-1H-tetrazol-5-ylsulfanylmethyl)-1-oxo-1a,2,3,3a,4,6b-hexahydro-1H-2,6a-diaza-cyclobut[cd]inden-6-carbonsäure

Diese Verbindung wird in Analogie zu Beispiel 59(a) ausgehend von (1aS,3aR,6bR)-5-(1-Methyl-tetrazol-5-yl-sulfanylmethyl)-1-oxo-1a,2,3,3a,4,6b-hexahydro-1H-2,6a-diaza-cyclobut[cd]inden-6-carbonsäure-trifluoracetat (200 mg; 0,415 mmol) und 2-Bromessigsäuremethylester (0,046 ml; 0,50 mmol) hergestellt. Ausbeute: 35 mg (21%) als gelbes Pulver.
IR (KBr): 1739, 1602, 1391 $cm^{-1}$
MS (ISP): $(M+H)^+$ 395,5

(b) (1aS,3aR,6bR)-2-(4-Hydroxy-phenylcarbamoylmethyl)-5-(1-methyl-1H-tetrazol-5-ylsulfanylmethyl)-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 59(a) angegeben ausgehend von (1aS,3aR,6bR)-5-(1-Methyl-tetrazol-5-yl-sulfanylmethyl)-1-oxo-1a,2,3,3a,4,6b-hexahydro-1H-2,6a-diaza-cyclobut[cd]inden-6-carbonsäure-trifluoracetat (200 mg; 0,415 mmol) hergestellt. Ausbeute: 25 mg (12%) als farbloses Pulver.
IR (KBr): 1741, 1670, 1603, 1513 $cm^{-1}$
MS (ISN): $(M\text{-}Na)^-$ 470,4

Beispiel 62

(1aS,3aR,6bR)-5-(1-Methyl-1H-tetrazol-5-ylsulfanylmethyl)-1-oxo-1a,2,3,3a,4,6b-hexahydro-1H-2,6a-diaza-cyclobut[cd]inden-2,6-dicarbonsäure-2-äthylester

Diese Verbindung wird in Analogie zu Beispiel 3(a) ausgehend von (1aS,3aR,6bR)-5-(1-Methyl-tetrazol-5-yl-sulfanylmethyl)-1-oxo-1a,2,3,3a,4,6b-hexahydro-1H-2,6a-diaza-cyclobut[cd]inden-6-carbonsäure-trifluoracetat (200 mg; 0,415 mmol) und Aethyl-N-hydroxysuccinimidyl-carbonat (217 mg; 1,16 mmol) hergestellt. Ausbeute: 62 mg (31%) als farbloses Pulver.
IR (KBr): 1774, 1707, 1628 $cm^{-1}$
MS (ISP): $(M+H)^+$ 395,4

Beispiel 63

(1aS,3aR,6bR)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-2,6-dicarbonsäure-di-t-butylester (2,3 g; 4,73 mmol) hergestellt. Ausbeute: 1,95 g (93%) als rötlisches Produkt.
IR (KBr): 1781, 1700, 1677, 1199 $cm^{-1}$
MS (ISN): $(M\text{-}H)^-$ 337,3

Die hierfür verwendete Ausgangsverbindung wird in Analogie zu Beispiel 17 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (1,8 g; 4,73 mmol) und 2-Methyl-5-mercapto-1,3,4-thiadiazol (937 mg; 7,09 mmol) hergestellt. Ausbeute: 2,3 g (100%) als farbloser fester Schaum.
IR (KBr): 1775, 1703, 1625 $cm^{-1}$
MS (ISP): $(M+H)^+$ 495,5

Beispiel 64

(1aS,3aR,6bR)-2-(4-Hydroxy-phenylcarbamoyl)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 3(a) angegeben ausgehend von (1aS,3aR,6bR)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat (133 mg; 0,3 mmol) hergestellt. Ausbeute: 60 mg (47%) als farbloses Pulver.
IR (KBr): 1747, 1650, 1603 $cm^{-1}$
MS (ISN): $(M-Na)^-$ 472,3

Beispiel 65

(a) (1aS,3aR,6bR)-2-Acetyl-5-(5-methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat (177 mg; 0,4 mmol) in DMF (5 ml) hergestellt. Ausbeute: 52 mg (38%) als oranges Pulver.
IR (KBr): 1751, 1660, 1601, 1412 $cm^{-1}$
MS (ISP): $(M+H)^+$ 381,3

(b) (1aS,3aR,6bR)-5-(5-methyl-1,3,4-thiadiazol-2-ylsulfanylmethyl)-1-oxo-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat (177 mg; 0,4 mmol) hergestellt. Ausbeute: 30 mg (19%) als oranges Pulver.
IR (KBr): 1759, 1693, 1609, 1390 $cm^{-1}$
MS (ISN): $(M-Na)^-$ 433,3

(c) (1aS,3aR,6bR)-5-(5-Methyl-1,3,4-thiadiazol-2-ylsulfanylmethyl)-1-oxo-2-(pyridin-4-ylsulfanylacetyl)-1a,2,3,3a,4,6b-hexahydro-1H-2,6a-diaza-cyclobut[cd]inden-6-carbonsäure-Natriumsalz

Diese Verbindung wird in Analogie zu Beispiel 58(e) ausgehend von (1aS,3aR,6bR)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat (177 mg; 0,4 mmol) und 4-Mercapto-pyridin (53 mg; 0,48 mmol) hergestellt. Ausbeute: 41 mg (20%) als gelbliches Pulver.
IR (KBr): 1754, 1647, 1604, 1409 $cm^{-1}$
MS (ISP): $(M-Na+2H)^+$ 490,4

(d) (1aS,3aR,6bR)-2-(3-Carbamoyl-pyridin-1-ylioacetyl)-5-(5-methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 58(e) ausgehend von (1aS,3aR,6bR)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat (295 mg; 0,666 mmol) und Isonicotinsäureamid (122 mg; 1,00 mmol) hergestellt. Ausbeute: 55 mg (16%) als gelbliches Pulver.
IR (KBr): 1757, 1669, 1604, 1386 $cm^{-1}$
MS (ISP): $(M+H)^+$ 501,4

(e) (1aS,3aR,6bR)-2-Formyl-5-(5-methyl-1,3,4-thiadiazol-2-ylsulfanyl-methyl)-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(k) angegeben ausgehend von (1aS,3aR,6bR)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat (177 mg; 0,4 mmol) hergestellt. Ausbeute: 59 mg (38%) als oranges Pulver.
IR (KBr): 1753, 1661, 1593, 1391 $cm^{-1}$
MS (ISP): $(M+H)^+$ 367,2

(f) (1aS,3aR,6bR)-2-(2-Amino-1,3,4-thiadiazol-5-ylsulfanylacetyl)-5-(5-methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 58(e) ausgehend von (1aS,3aR,6bR)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat (177 mg; 0,4 mmol) und 2-Amino-5-mercapto-1,3,4-thiadiazol (66 mg; 0,48 mmol) hergestellt. Ausbeute: 54 mg (27%).
IR (KBr): 1751, 1650, 1600, 1389 $cm^{-1}$
MS (ISP): $(M\text{-}Na+2H)^+$ 512,2

(g) (1aS,3aR,6bR)-2-Carbamoylmethylsulfanylacetyl-5-(5-methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 58(e) ausgehend von (1aS,3aR,6bR)-5-(5-Methyl-1,3,4-thiadiazol-2-ylsulfanylmethyl)-1-oxo-1a,2,3,3a,4,6b-hexahydro-1H-2,6a-diaza-cyclobut[cd]inden-6-carbonsäure-trifluoracetat (177 mg; 0,4 mmol) und 2-Mercapto-acetamid (44 mg; 0,48 mmol) hergestellt. Ausbeute: 43 mg (22%).
IR (KBr): 1752, 1673, 1596, 1382 $cm^{-1}$
MS (ISP): $(M\text{-}Na+2H)^+$ 470,3; $(M+H)^+$ 492,2

Beispiel 66

(1aS,3aR,6bR)-1-Oxo-5-(4-pyridin-3-yl-thiazol-2-ylsulfanylmethyl)-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-1-Oxo-5-(4-pyridin-3-yl-thiazol-2-ylsulfanylmethyl)-1a,2,3,3a,4,6b-hexahydro-1H-2,6a-diaza-cyclobut[cd]inden-2,6-dicarbonsaure-di-t-butylester (1,11 g; 2,00 mmol) hergestellt. Ausbeute: 966 mg (86%) als oranges Pulver.
IR (KBr): 1778, 1678, 1630 $cm^{-1}$
MS (ISP): $(M+H)^+$ 401,3

Die hierfür verwendete Ausgangsverbindung wird in Analogie zu Beispiel 17 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (760 mg; 2,0 mmol) und 2-Mercapto-4-pyridin-3-yl-1,3-thiazol (583 mg; 3,0 mmol) hergestellt. Ausbeute: 1,11 g (100%) als farbloser fester Schaum.
IR (KBr): 1775, 1703, 1625, 1250, 1164 $cm^{-1}$
MS (ISP): $(M+H)^+$ 557,4

Beispiel 67

(a) (1aS,3aR,6bR)-2-Acetyl-1-oxo-5-(4-pyridin-3-yl-thiazol-2-ylsulfanyl-methyl)-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-1-Oxo-5-(4-pyridin-3-yl-thiazol-2-ylsulfanylmethyl)-1a,2,3,3a,4,6b-hexahydro-1H-2,6a-diaza-cyclobut[cd]inden-6-carbonsäure-trifluoracetat (225 mg; 0,4 mmol) in DMF (5 ml) hergestellt. Ausbeute: 87 mg (47%) als farbloses Pulver.
IR (KBr): 1748, 1650, 1596, 1404 $cm^{-1}$
MS (ISP): $(M\text{-}Na+2H)^+$ 443,4

(b) (1aS,3aR,6bR)-1-Oxo-5-(4-pyridin-3-yl-thiazol-2-ylsulfanylmethyl)-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-1-Oxo-5-(4-pyridin-3-yl-thiazol-2-ylsulfanylmethyl)-1a,2,3,3a,4,6b-hexahydro-1H-2,6a-diaza-cyclobut[cd]inden-6-carbonsäure-trifluoracetat (225 mg; 0,4 mmol) hergestellt. Ausbeute: 127 mg (61%) als oranges Pulver.
IR (KBr): 1764, 1689, 1624, 1406 $cm^{-1}$
MS (ISP): $(M\text{-}Na+2H)^+$ 497,2

Beispiel 68

(1aS,3aR,6bR)-5-[(R)-2-Amino-2-(3-methyl-1,2,4-oxadiazol-5-yl)-äthylsulfanyl-methyl]-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-[(R)-2-t-butoxycarbonylamino-2-(3-methyl-1,2,4-oxadiazol-5-yl)-äthylsulfanylmethyl]-1-oxo-1a,2,3,3a,4,6b-hexahydro-1H-2,6a-diaza-cyclobut[cd]inden-2,6-dicarbonsäure-di-t-butylester (310 mg; 0,5 mmol) hergestellt. Ausbeute: 251 mg (100%) als beiges Pulver.
IR (KBr): 1776, 1677, 1203 $cm^{-1}$
MS (ISP): $(M+H)^+$ 366,4
Die hierfür verwendete Ausgangsverbindung wird in Analogie zu Beispiel 17 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (190 mg; 0,5 mmol) hergestellt. Ausbeute: 310 mg (100%) als gelber fester Schaum.
IR (KBr): 1778, 1710, 1585, 1513, 1251, 1165 $cm^{-1}$
MS (ISP): $(M+H)^+$ 622,4; $(M+NH_4)^+$ 639,4

Beispiel 69

(1aS,3aR,6bR)-5-(1-Carbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-(1-Carbamoylmethyl-1H-tetrazol-5-ylsulfanylmethyl)-1-oxo-1a,2,3,3a,4,6b-hexahydro-1H-2,6a-diaza-cyclobut[cd]inden-2,6-dicarbonsäure-di-t-butylester (1,00 g; 1,92 mmol) hergestellt. Ausbeute: 830 mg (90%) als blassrosa Feststoff.
IR (KBr): 1780, 1693, 1624 $cm^{-1}$
MS (ISP): $(M+H)^+$ 366,3
Die hierfür verwendete Ausgangsverbindung wird in Analogie zu Beispiel 17 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (700 mg; 1,84 mmol) und 5-Mercapto-1H-tetrazol-1-acetamid (439 mg; 2,76 mmol) hergestellt. Ausbeute: 960 mg (100%) als gelber Feststoff.
IR (KBr): 1777, 1703, 1625, 1251 $cm^{-1}$
MS (ISP): $(M+H)^+$ 522,5; $(M+NH_4)^+$ 539,5

Beispiel 70

(a) (1aS,3aR,6bR)-2-Acetyl-5-(1-carbamoylmethyl-1H-tetrazol-5-ylsulfanyl-methyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-(1-Carbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure-trifluoracetat (240 mg; 0,5 mmol) in Dimethylformamid (5 ml) hergestellt. Ausbeute: 123 mg (57%) als oranges Pulver.
IR (KBr): 1749, 1694, 1622, 1397 $cm^{-1}$
MS (ISP): $(M+H)^+$ 408,4; $(M+NH_4)^+$ 425,4; $(M+Na)^+$ 430,4

(b) (1aS,3aR,6bR)-5-(1-Carbamoylmethyl-1H-tetrazol-5-ylsulfanylmethyly)-2-trifluoracetyl-1-oxo-1a,2,3,3a,4,6b-hexahydro-1H-2,6a-diaza-cyclobut-[cd]inden-6-carbonsäure-Natriumsalz

Diese Verbindung wird in der gleichen Weise wie in Beispiel 19(c) angegeben ausgehend von (1aS,3aR,6bR)-5-(1-Carbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure-trifluoracetat (300 mg; 0,625 mmol) in Dimethylformamid (5 ml) hergestellt. Ausbeute: 160 mg (53%) als oranges Pulver.
IR (KBr): 1763, 1692, 1606 $cm^{-1}$
MS (ISP): $(M+H)^+$ 462,3; $(M+NH_4)^+$ 479,3; $(M+Na)^+$ 484,3

(c) (1aS,3aR,6bR)-5-(1-Carbamoylmethyl-1H-tetrazol-5-ylsulfanylmethyl)-2-formyl-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(k) angegeben ausgehend von (1aS,3aR,6bR)-5-(1-Carbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure-trifluoracetat (300 mg; 0,625 mmol) hergestellt. Ausbeute: 54 mg (42%) als oranges Pulver.
IR (KBr): 1753, 1693, 1659, 1601, 1395 $cm^{-1}$
MS (ISP): $(M+H)^+$ 394,1; $(M+NH_4)^+$ 411,3; $(M+Na)^+$ 416,2

Beispiel 71

(1aS,3aR,6bR)-1-Oxo-5-(pyrimidin-2-ylsulfanylmethyl)-1a,2,3,3a,4,6b-hexahydro-1H-2,6a-diaza-cyclobut[cd]-inden-6-carbonsäure-trifluoracetat

Diese Verbindung wird in der gleichen Weise wie in Beispiel 2(a) angegeben ausgehend von (1aS,3aR,6bR)-1-Oxo-5-(pyrimidin-2-ylsulfanyl-methyl)-1a,2,3,3a,4,6b-hexahydro-1H-2,6a-diaza-cyclobut[cd]-inden-2,6-dicarbonsäure-di-t-butylester (560 mg; 1,18 mmol) hergestellt. Ausbeute: 500 mg (93%) als gelbes Pulver.
IR (KBr): 1780, 1676, 1630, 1200 $cm^{-1}$
MS (ISN): $(M-H)^-$ 317,2
Die hierfür verwendete Ausgangsverbindung wird in Analogie zu Beispiel 17 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (450 mg; 1,18 mmol) und 2-Mercapto-pyrimidin (185 mg; 1,61 mmol) hergestellt. Ausbeute: 560 mg (100%) als gelbes Pulver.
IR (KBr): 1775, 1704, 1381, 1164 $cm^{-1}$
MS (ISP): $(M+H)^+$ 475,4; $(M+Na)^+$ 497,4

Beispiel 72

(a) (1aS,3aR,6bR)- 2-Acetyl-1-oxo-5-(pyrimidin-2-ylsulfanylmethyl)-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-1-Oxo-5-(pyrimidin-2-ylsulfanylmethyl)-1a,2,3,3a,4,6b-hexahydro-1H-2,6a-diaza-cyclobut[cd]inden-6-carbonsäure-trifluoracetat (182 mg; 0,4 mmol) in Dimethylformamid (4 ml) hergestellt. Ausbeute: 58 mg (35%) als oranges Pulver.
IR (KBr): 1749, 1650, 1599, 1380 $cm^{-1}$
MS (ISP): $(M+2H-Na)^+$ 361,2; $(M+H)^+$ 383,2; $(M+Na)^+$ 405,2

(b) (1aS,3aR,6bR)-1-Oxo-5-(pyrimidin-2-ylsulfanylmethyl)-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-1-Oxo-5-(pyrimidin-2-ylsulfanylmethyl)-1a,2,3,3a,4,6b-hexahydro-1H-2,6a-diaza-cyclobut[cd]-inden-6-carbonsäure-trifluoracetat (182 mg; 0,4 mmol) hergestellt. Ausbeute: 75 mg (43%) als gelbes Pulver.
IR (KBr): 1749, 1650, 1599, 1380 $cm^{-1}$
MS (ISP): $(M+2H-Na)^+$ 415,3; $(M+H)^+$ 437,3

Beispiel 73

(1aS,3aR,6bR)-5-(1-Methylcarbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-(1-Methylcarbamoylmethyl-1H-tetrazol-5-ylsulfanylmethyl)-1-oxo-1a,2,3,3a,4,6b-hexahydro-1H-2,6a-diaza-cyclobut[cd]inden-2,6-dicarbonsäure-di-t-butylester (632 mg; 1,18 mmol) hergestellt. Ausbeute: 600 mg (100%) als hellgelbes Pulver.
IR (KBr): 1781, 1680, 1630, 1570, 1200 $cm^{-1}$

Die hierfür verwendete Ausgangsverbindung wird in Analogie zu Beispiel 17 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (190 mg; 0,5 mmol) und N-Methyl-2-(5-mercapto-1H-tetrazol-1-yl)-acetamid (280 mg; 1,62 mmol) hergestellt. Ausbeute: 630 mg (100%) als gelber fester Schaum.
IR (KBr): 1777, 1701, 1640, 1557, 1251 $cm^{-1}$
MS (ISP): $(M+H)^+$ 536,4; $(M+NH_4)^+$ 553,4

Beispiel 74

(a) (1aS,3aR,6bR)-2-Acetyl-5-(1-methylcarbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-(1-Methylcarbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure-trifluoracetat (247 mg; 0,487 mmol) in Dimethylformamid (4 ml) hergestellt. Ausbeute: 71 mg (33%) als hellgelbes Pulver.
IR (KBr): 1751, 1686, 1640, 1603, 1550, 1409 $cm^{-1}$
MS (ISP): $(M+2H\text{-}Na)^+$ 422,4; $(M+H\text{-}Na+NH_4)^+$ 439,4; $(M+H)^+$ 444,3

(b) (1aS,3aR,6bR)-5-(1-Methylcarbamoylmethyl-1H-tetrazol-5-ylsulfanylmethyl)-1-oxo-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-(1-Methylcarbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure-trifluoracetat (247 mg; 0,487 mmol) hergestellt. Ausbeute: 63 mg (26%) als hellgelbes Pulver.
IR (KBr): 1764, 1692, 1605, 1560, 1399, 1155 $cm^{-1}$
MS (ISP): $(M+2H\text{-}Na)^+$ 476,3; $(M+H\text{-}Na+NH_4)^+$ 493,3; $(M+H)^+$ 498,2

Beispiel 75

(1aS,3aR,6bR)-1-Oxo-5-(1H-1,2,4-triazol-3-ylsulfanylmethyl)-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-1-Oxo-5-(1H-1,2,4-triazol-3-ylsulfanylmethyl)-1a,2,3,3a,4,6b-hexahydro-1H-2,6a-diaza-cyclobut[cd]inden-2,6-dicarbonsäure-di-t-butylester (533 mg; 1,18 mmol) hergestellt. Ausbeute: 430 mg (89%) als oranges Pulver.
IR (KBr): 1778, 1700, 1676 $cm^{-1}$
MS (ISP): $(M+H)^+$ 308,2

Die hierfür verwendete Ausgangsverbindung wird in Analogie zu Beispiel 17 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (450 mg; 1,18 mmol) und 1H-1,2,4-triazole-3-thiol (280 mg; 1,62 mmol) hergestellt. Ausbeute: 530 mg (100%) als hellgelber fester Schaum.
IR (KBr): 1775, 1704, 1633, 1368 $cm^{-1}$
MS (ISP): $(M+H)^+$ 464,4; $(M+Na)^+$ 486,4

Beispiel 76

(a) (1aS,3aR,6bR)-2-Acetyl-1-oxo-5-(1H-1,2,4-triazol-3-ylsulfanylmethyl)-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-1-Oxo-5-(1H-1,2,4-triazol-3-ylsulfanylmethyl)-1a,2,3,3a,4,6b-hexahydro-1H-2,6a-diaza-cyclobut[cd]inden-6-carbonsäure-trifluoracetat (205 mg; 0,5 mmol) in Dimethylformamid (5 ml) hergestellt. Ausbeute: 84 mg (45%) als gelbes Pulver.
IR (KBr): 1749, 1660, 1598, 1401 $cm^{-1}$
MS (ISN): $(M\text{-}Na)^-$ 348,2

(b) (1aS,3aR,6bR)-1-Oxo-5-(1H-1,2,4-triazol-3-ylsulfanylmethyl)-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-1-Oxo-5-(1H-1,2,4-triazol-3-ylsulfanylmethyl)-1a,2,3,3a,4,6b-hexahydro-1H-2,6a-diaza-cyclobut[cd]inden-6-carbonsäure-trifluoracetat (205 mg; 0,5 mmol) hergestellt. Ausbeute: 73 mg (34%) als beiges Pulver.
IR (KBr): 1762, 1695, 1598, 1399 $cm^{-1}$
MS (ISP): $(M+H)^+$ 404,3; $(M+Na)^+$ 426,3

Beispiel 77

(1aS,3aR,6bR)-5-[1-(4-Hydroxy-phenylcarbamoylmethyl)-1H-tetrazol-5-ylsulfanylmethyl]-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-[1-(4-Hydroxy-phenylcarbamoylmethyl)-1H-tetrazol-5-ylsulfanylmethyl]-1-oxo-1a,2,3,3a,4,6b-hexahydro-1H-2,6a-diaza-cyclobut[cd]inden-2,6-dicarbonsäure-di-t-butylester (724 mg; 1,18 mmol) hergestellt. Ausbeute: 650 mg (99%) als beiges Pulver.
IR (KBr): 1779, 1678, 1621, 1513, 1250, 1202 $cm^{-1}$
MS (ISN): $(M\text{-}H)^-$ 456,3
Die hierfür verwendete Ausgangsverbindung wird in Analogie zu Beispiel 17 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (450 mg; 1,18 mmol) und N-(4-Hydroxy-phenyl)-2-(5-mercapto-1H-tetrazol-5-yl)-acetamid (477 mg; 1,62 mmol) hergestellt. Ausbeute: 720 mg (100%) als beiges Pulver..
IR (KBr): 1776, 1701, 1680, 1615, 1557, 1367, 1250 $cm^{-1}$
MS (ISP): $(M+H)^+$ 614,3; $(M+NH_4)^+$ 631,3

Beispiel 78

(a) (1aS,3aR,6bR)-2-Acetyl-5-[1-(4-hydroxy-phenylcarbamoylmethyl)-1H-tetrazol-5-ylsulfanylmethyl]-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-[1-(4-Hydroxy-phenylcarbamoylmethyl)-1H-tetrazol-5-ylsulfanylmethyl]-1-oxo-1a,2,3,3a,4,6b-hexahydro-1H-2,6a-diazacyclobut[cd]inden-6-carbonsäure-trifluoracetat (279 mg; 0,5 mmol) in Dimethylformamid (5 ml) hergestellt. Ausbeute: 97 mg (37%) als beiges Pulver.
IR (KBr): 1750, 1686, 1614, 1399, 1251 $cm^{-1}$
MS (ISP): $(M+H)^+$ 500,4; $(M+Na)^+$ 522,3

(b) (1aS,3aR,6bR)-5-[1-(4-Hydroxy-phenylcarbamoylmethyl)-1H-tetrazol-5-ylsulfanylmethyl]-1-oxo-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-[1-(4-Hydroxy-phenylcarbamoylmethyl)-1H-tetrazol-5-ylsulfanylmethyl]-1-oxo-1a,2,3,3a,4,6b-hexahydro-1H-2,6a-diaza-cyclobut[cd]inden-6-carbonsäure-trifluoracetat (279 mg; 0,5 mmol)

hergestellt. Ausbeute: 79 mg (27%) als farbloses Pulver.
IR (KBr): 1763, 1680, 1605, 1398, 1250, 1208, 1157 $cm^{-1}$
MS (ISP): $(M+H)^+$ 554,2; $(M+Na)^+$ 576,2

Beispiel 79

(1aS,3aR,6bR)-1-Oxo-5-[1-(phenäthylcarbamoylmethyl)-1H-tetrazol-5-ylsulfanylmethyl]-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-1-Oxo-5-[1-(phenäthylcarbamoylmethyl)-1H-tetrazol-5-ylsulfanylmethyl]-1a,2,3,3a,4,6b-hexahydro-1H-2,6a-diaza-cyclobut[cd]inden-2,6-dicarbonsäure-di-t-butylester (1,18 g; 1,89 mmol) hergestellt. Ausbeute: 850 mg (81%) als rosarotes Pulver.
IR (KBr): 1778, 1678, 1650, 1558, 1242, 1200 $cm^{-1}$
MS (ISN): $(M-H)^-$ 468,4

Die hierfür verwendete Ausgangsverbindung wird in Analogie zu Beispiel 17 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (720 mg; 1,89 mmol) und N-(2-Phenyläthyl)-2-(5-mercapto-1H-tetrazol-5-yl)-acetamid (680 mg; 2,58 mmol) hergestellt. Ausbeute: 1,18 g (100%) als gelbes Pulver.
IR (KBr): 1773, 1699, 1670, 1554, 1252 $cm^{-1}$
MS (ISP): $(M+H)^+$ 626; $(M+Na)^+$ 648; $(M+K)^+$ 664

Beispiel 80

(a) (1aS,3aR,6bR)-2-Acetyl-1-oxo-5-[1-(phenäthylcarbamoylmethyl)-1H-tetrazol-5-ylsulfanylmethyl]-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-1-Oxo-5-[1-(phenäthylcarbamoylmethyl)-1H-tetrazol-5-ylsulfanylmethyl]-1a,2,3,3a,4,6b-hexahydro-1H-2,6a-diaza-cyclobut[cd]-inden-6-carbonsäure-trifluoracetat (278 mg; 0,5 mmol) in Dimethylformamid (5 ml) hergestellt. Ausbeute: 99 mg (37%) als beiges Pulver.
IR (KBr): 1750, 1685, 1606, 1560, 1403 $cm^{-1}$
MS (ISP): $(M+H)^+$ 512,2; $(M+NH_4)^+$ 529,2; $(M+Na)^+$ 534,2

(b) (1aS,3aR,6bR)-1-Oxo-5-[1-(phenäthylcarbamoylmethyl)-1H-tetrazol-5-ylsulfanylmethyl]-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-1-Oxo-5-[1-(phenäthylcarbamoylmethyl)-1H-tetrazol-5-ylsulfanylmethyl]-1a,2,3,3a,4,6b-hexahydro-1H-2,6a-diaza-cyclobut[cd]inden-6-carbonsäure-trifluoracetat (292 mg; 0,52 mmol) hergestellt. Ausbeute: 91 mg (29%) als beiges Pulver.
IR (KBr): 1763, 1692, 1606, 1551, 1398 $cm^{-1}$
MS (ISP): $(M+H)^+$ 565,9; $(M+NH_4)^+$ 582,9; $(M+Na)^+$ 587,9

Beispiel 81

(1aS,3aR,6bR)-5-Azidomethyl-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-Azidomethyl-1-oxo-1a,2,3,3a,4,6b-hexahydro-1H-2,6a-diaza-cyclobut[cd]inden-2,6-dicarbonsäure-di-t-butylester (450 mg; 1,2 mmol) hergestellt. Ausbeute: 390 mg (95%) als beiges Pulver.
IR (KBr): 2109, 1782, 1676, 1201 $cm^{-1}$
MS (ISP): $(M+H)^+$ 250,4; $(M+NH_4)^+$ 267,5

Die hierfür verwendete Ausgangsverbindung wird wie folgt hergestellt:

(1aS,3aR,6bR)-5-Azidomethyl-1-oxo-1a,2,3,3a,4,6b-hexahydro-1H-2,6a-diaza-cyclobut[cd]inden-2,6-dicarbonsäure-di-t-butylester

(1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (190 mg; 0,5 mmol) in Dimethylformamid (2,0 ml) wird bei -20°C mit Triäthylamin (0,097 ml; 0,7 mmol) und Methansulfochlorid (0,054 ml; 0,7 mmol) versetzt. Nach 15 Minuten bei dieser Temperatur wird mit Dimethylformamid (9 ml) verdünnt und Natriumazid (49 mg; 0,75 mmol) zugegeben. Anschliessend wird das Reaktionsgemisch 1 Stunde bei 0°C gerührt und auf ein Gemisch von Essigester (90 ml) und Wasser (45 ml) gegossen. Die organische Phase wird nacheinander mit Wasser (2x20 ml) und gesättigter wässriger Kochsalzlösung (30 ml) gewaschen, über Magnesiumsulfat getrocknet und eingeengt. Der Rückstand wird mit n-Hexan versetzt und abgenutscht. Ausbeute: 200 mg (97%) als leicht gelbes Pulver.
IR (KBr): 2110, 1768, 1708, 1644 $cm^{-1}$
MS (EI): (M-$^t$BUO•) 332

Beispiel 82

(1aS,3aR,6bR)-5-Azidomethyl-2-(4-hydroxy-phenylcarbamoyl)-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 3(a) angegeben ausgehend von (1aS,3aR,6bR)-5-Azidomethyl-1-oxo-1a,2,3,3a,4,6b-hexahydro-1H-2,6a-diaza-cyclobut[cd]inden-6-carbonsäure-trifluoracetat (130 mg; 0,37 mmol) hergestellt. Ausbeute: 50 mg (33%) als gelbliches Pulver.
IR (KBr): 2107, 1747, 1650, 1606 $cm^{-1}$
MS (ISP): $(M+H)^+$ 407,4; $(M\text{-}Na+2H)^+$ 385,5

Beispiel 83

(a) (1aS,3aR,6bR)-2-Acetyl-5-azidomethyl-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(a) angegeben ausgehend von (1aS,3aR,6bR)-5-Azidomethyl-1-oxo-1a,2,3,3a,4,6b-hexahydro-1H-2,6a-diaza-cyclobut[cd]inden-6-carbonsäure-trifluoracetat (130 mg; 0,37 mmol) hergestellt. Ausbeute: 35 mg (30%) als braunes Pulver.
IR (KBr): 2103, 1752, 1650, 1613 $cm^{-1}$
MS (ISN): $(M\text{-}Na)^-$ 290,3

(b) (1aS,3aR,6bR)-5-Azidomethyl-2-trifluoracetyl-1-oxo-1a,2,3,3a,4,6b-hexahydro-1H-2,6a-diaza-cyclobut[cd]-inden-6-carbonsäure-Natriumsalz

Diese Verbindung wird in der gleichen Weise wie in Beispiel 19(c) angegeben ausgehend von (1aS,3aR,6bR)-5-Azidomethyl-1-oxo-1a,2,3,3a,4,6b-hexahydro-1H-2,6a-diaza-cyclobut[cd]inden-6-carbonsäure-trifluoracetat (126 mg; 0,36 mmol) hergestellt. Ausbeute: 34 mg (26%) als braunes Pulver.
IR (KBr): 2107, 1762, 1696, 1612 $cm^{-1}$

Beispiel 84

(1aS,3aR,6bR)-5-Acetylaminomethyl-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-Acetylaminomethyl-1-oxo-1a,2,3,3a,4,6b-hexahydro-1H-2,6a-diaza-cyclobut[cd]inden-2,6-dicarbonsäure-di-t-butylester (350 mg; 0,83 mmol) hergestellt. Ausbeute: 255 mg (82%) als beiges Pulver.
IR (KBr): 1781, 1675, 1640, 1551, 1200 $cm^{-1}$
MS (ISN): $(M\text{-}H)^-$ 264,3

Die hierfür verwendete Ausgangsverbindung wird wie folgt hergestellt:

a) (1aS,3aR,6bR)-5-Aminomethyl-1-oxo-1a,2,3,3a,4,6b-hexahydro-1H-2,6a-diaza-cyclobut[cd]inden-2,6-dicarbonsäure-di-t-butylester-hydrochlorid

(1aS,3aR,6bR)-5-Azidomethyl-1-oxo-1a,2,3,3a,4,6b-hexahydro-1H-2,6a-diaza-cyclobut[cd]inden-2,6-dicarbonsäure-di-t-butylester-hydrochlorid (520 mg; 1,28 mmol; aus Beispiel 81) wird in Methanol (50 ml) und 1N wässrige Salzsäure (1,3 ml) gelöst und über 10% Pd/C (125 mg) hydriert. Nach 1 Stunde wird das Reaktionsgemisch abgenutscht und eingeengt. Ausbeute: 530 mg (100%) als farbloses Pulver.
IR (KBr):1777, 1705, 1368, 1163 $cm^{-1}$
MS (ISP): $(M+H)^+$ 380,5

b) (1aS,3aR,6bR)-5-Acetylaminomethyl-1-oxo-1a,2,3,3a,4,6b-hexahydro-1H-2,6a-diaza-cyclobut[cd]inden-2,6-dicarbonsäure-di-t-butylester

(1aS,3aR,6bR)-5-Aminomethyl-1-oxo-1a,2,3,3a,4,6b-hexahydro-1H-2,6a-diaza-cyclobut[cd]inden-2,6-dicarbonsäure-di-t-butylester-hydrochlorid (490 mg; 1,16 mmol) wird in Methylenchlorid (5 ml) gelöst und bei -15°C mit Triäthylamin (0,33 ml; 2,4 mmol) und Acetylchlorid (0,093 ml; 1,3 mmol) versetzt. Nach 10 Minuten wird das Reaktionsgemisch mit Methylenchlorid (25 ml) verdünnt und nacheinander mit Wasser (10 ml) und gesättigter wässriger Kochsalzlösung (10 ml) gewaschen. Die organische Phase wird über Magnesiumsulfat getrocknet und eingeengt. Ausbeute: 350 mg (60%) als farbloses Pulver.
IR (KBr): 1775, 1705, 1660, 1535 $cm^{-1}$
MS (ISP): $(M+NH_4)^+$ 439,6

## Beispiel 85

(1aS,3aR,6bR)-5-Acetylaminomethyl-2-(4-hydroxy-phenylcarbamoyl)-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 3(a) angegeben ausgehend von (1aS,3aR,6bR)-5-Acetylaminomethyl-1-oxo-1a,2,3,3a,4,6b-hexahydro-1H-2,6a-diaza-cyclobut[cd]inden-6-carbonsäure-trifluoracetat (100 mg; 0,267 mmol) hergestellt. Ausbeute: 34 mg (30%) als farbloses Pulver.
IR (KBr): 1747, 1646, 1604, 1513, 1374 $cm^{-1}$
MS (ISN): $(M-Na)^-$ 399,4

## Beispiel 86

(1aS,3aR,6bR)-5-Acetylaminomethyl-1-oxo-2-trifluoracetyl-1a,2,3,3a,4,6b-hexahydro-1H-2,6a-diaza-cyclobut-[cd]inden-6-carbonsäure-Natriumsalz

Diese Verbindung wird in der gleichen Weise wie in Beispiel 19(c) angegeben ausgehend von (1aS,3aR,6bR)-5-Acetylaminomethyl-1-oxo-1a,2,3,3a,4,6b-hexahydro-1H-2,6a-diaza-cyclobut[cd]inden-6-carbonsäure-trifluoracetat (130 mg; 0,348 mmol) hergestellt. Ausbeute: 24 mg (18%) als farbloses Pulver.
IR (KBr): 1759, 1698, 1607, 1542, 1401 $cm^{-1}$
MS (ISP): $(M-Na+2H)^+$ 362,4; $(M-Na+H+NH_4)^+$ 379,4; $(M+H)^+$ 384,4

## Beispiel 87

(1aS,3aR,6bR)-5-Methylsulfonylaminomethyl-1-oxo-1a,2,3,3a,4,6b-hexahydro-1H-2,6a-diaza-cyclobut[cd]-inden-6-carbonsäure-trifluoracetat

Diese Verbindung wird in der gleichen Weise wie in Beispiel 2(a) angegeben ausgehend von (1aS,3aR,6bR)-5-Methylsulfonylaminomethyl-1-oxo-1a,2,3,3a,4,6b-hexahydro-1H-2,6a-diaza-cyclobut[cd]-inden-2,6-dicarbonsäure-di-t-butylester (600 mg; 1,3 mmol) hergestellt. Ausbeute: 530 mg (97%) als beiges Pulver.
IR (KBr): 1779, 1677, 1630, 1315, 1148 $cm^{-1}$
MS (ISP): $(M+H)^+$ 302,3

Die hierfür verwendete Ausgangsverbindung wird wie folgt hergestellt:

(1aS,3aR,6bR)-5-Aminomethyl-1-oxo-1a,2,3,3a,4,6b-hexahydro-1H-2,6a-diaza-cyclobut[cd]inden-2,6-dicarbonsäure-di-t-butylester-hydrochlorid (800 mg; 1,9 mmol; aus Beispiel 84) wird in Methylenchlorid (8

ml) gelöst und bei -40 °C mit Triäthylamin (0,59 ml; 4,2 mmol) und Mesylchlorid (0,18 ml; 2,3 mmol) versetzt. Nach 20 Minuten wird das Reaktionsgemisch mit Essigester (40 ml) verdünnt und nacheinander mit Wasser (20 ml) und gesättigter wässriger Kochsalzlösung (20 ml) gewaschen. Die organische Phase wird über Magnesiumsulfat getrocknet und eingeengt. Der erhaltene Rückstand wird aus Essigester/n-Hexan kristallisiert und abgenutscht. Ausbeute: 660 mg (76%) als farbloses Pulver.
IR (KBr): 1757, 1693, 1639 $cm^{-1}$
MS (ISP): $(M+NH_4)^+$ 475,5

Beispiel 88

(a) (1aS,3aR,6bR)-2-Acetyl-5-methylsulfonylaminomethyl-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-Methylsulfonylaminomethyl-1-oxo-1a,2,3,3a,4,6b-hexahydro-1H-2,6a-diaza-cyclobut[cd]inden-6-carbonsäure-trifluoracetat (220 mg; 0,523 mmol) in Dimethylformamid (6 ml) hergestellt. Ausbeute: 105 mg (55%) als farbloses Pulver.
IR (KBr): 1751, 1614, 1403, 1311, 1148 $cm^{-1}$
MS (ISN): $(M\text{-}Na)^-$ 342,3

(b) (1aS,3aR,6bR)-5-Methylsulfonylaminomethyl-1-oxo-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-Methylsulfonylaminomethyl-1-oxo-1a,2,3,3a,4,6b-hexahydro-1H-2,6a-diaza-cyclobut[cd]-inden-6-carbonsäure-trifluoracetat (220 mg; 0,523 mmol) hergestellt. Ausbeute: 83 mg (38%) als farbloses Pulver.
IR (KBr): 1758, 1694, 1604, 1401, 1149 $cm^{-1}$
MS (ISN): $(M\text{-}Na)^-$ 396,3; $(M\text{-}Na+NH_3)^-$ 413,3

Beispiel 89

(1aS,3aR,6bR)-5-(4-Hydroxy-phenylcarbamoyloxymethyl)-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-(4-Hydroxy-phenylcarbamoyloxymethyl)-1-oxo-1a,2,3,3a,4,6b-hexahydro-1H-2,6a-diaza-cyclobut[cd]inden-2,6-dicarbonsäure-di-t-butylester (640 mg; 1,24 mmol) hergestellt. Ausbeute: 670 mg (100%) als farbloses Pulver.
IR (KBr): 1775, 1677, 1516 $cm^{-1}$
MS (ISN): $(M\text{-}H)^-$ 358,3

Die hierfür verwendete Ausgangsverbindung wird wie folgt hergestellt:

(1aS,3aR,6bR)-5-Hydroxymethyl-1-oxo-1a,2,3,3a,4,6b-hexahydro-1H-2,6a-diaza-cyclobut[cd]inden-2,6-dicarbonsäur-di-t-butylester (770 mg; 2,03 mmol) wird in Methylenchlorid (24 ml) gelöst und mit 0,4 nm Molekularsieb (1 g), Di-(N-succinimidyl)-carbonat (624 mg; 2,44 mmol) und Triäthylamin (0,68 ml; 4,86 mmol) bei Raumtemperatur versetzt. Nach 1 Stunde wird 4-Aminophenol (270 mg; 2,44 mmol) und Triäthylamin (0,56 ml; 4,05 mmol) zugegeben. Nach 1 Stunde wird das Reaktionsgemisch mit Methylenchlorid (100 ml) verdünnt und nacheinander mit gesättigter wässriger Natriumbicarbonatlösung (20 ml) und gesättigter wässriger Kochsalzlösung (20 ml) gewaschen, über Magnesiumsulfat getrocknet und eingeengt. Der erhaltene Rückstand wird über Kieselgel (50 g; 0,040-0,063 mm Korngrösse) mit Essigester/n-Hexan 6:4 chromatographiert. Ausbeute: 640 mg (61 %) als farbloser Feststoff.
IR (KBr): 1776, 1708, 1516 $cm^{-1}$
MS (ISP): $(M+H)^+$ 516,4; $(M+NH_4)^+$ 533,4; $(M+Na)^+$ 538,3

Beispiel 90

(a) (1aS,3aR,6bR)-2-Acetyl-5-(4-hydroxy-phenylcarbamoyloxymethyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-(4-Hydroxy-phenylcarbamoyloxymethyl)-1-oxo-1a,2,3,3a,4,6b-hexahydro-1H-2,6a-diaza-cyclobut[cd]inden-6-carbonsäure-trifluoracetat (220 mg; 0,47 mmol) in Dimethylformamid (6 ml) hergestellt. Ausbeute: 154 mg (77%) als gelbliches Pulver.
IR (KBr): 1751, 1720, 1606, 1404, 1221 $cm^{-1}$
MS (ISN): $(M-Na)^-$ 400,3

(b) (1aS,3aR,6bR)-5-(4-Hydroxy-phenylcarbamoyloxymethyl)-2-trifluoracetyl-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-(4-Hydroxy-phenylcarbamoyloxymethyl)-1-oxo-1a,2,3,3a,4,6b-hexahydro-1H-2,6a-diaza-cyclobut[cd]inden-6-carbonsäure-trifluoracetat (220 mg; 0,47 mmol) hergestellt. Ausbeute: 120 mg (53%) als gelbliches Pulver.
IR (KBr): 1759, 1694, 1605, 1402, 1222 $cm^{-1}$
MS (ISN): $(M-Na)^-$ 454,2

Beispiel 91

(1aS,3aR,6bR)-1-Oxo-5-(2,2,2-trifluoräthylcarbamoyloxymethyl)-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-1-Oxo-5-(2,2,2-trifluoräthylcarbamoyloxymethyl)-1a,2,3,3a,4,6b-hexahydro-1H-2,6a-diaza-cyclobut[cd]inden-2,6-dicarbonsäure-di-t-butylester (408 mg; 0,81 mmol) hergestellt. Ausbeute: 217 mg (69%) als gelbliches Pulver.
IR (KBr): 1773, 1725, 1679, 1625, 1549, 1403, 1241, 1156 $cm^{-1}$
MS (ISP): $(M+H)^+$ 350,3; $(M+NH_4)^+$ 367,3

Die hierfür verwendete Ausgangsverbindung wird in Analogie zu Beispiel 89 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester 400 mg; 1,05 mmol) und 2,2,2-Trifluoräthylamin (0,1 ml; 1,26 mmol) hergestellt. Ausbeute: 435 mg (82%) als farbloser Feststoff.
IR (KBr): 1776, 1709, 1539, 1240, 1158 $cm^{-1}$
MS (ISP): $(M+H)^+$ 506,4; $(M+NH_4)^+$ 523,4

Beispiel 92

(a) (1aS,3aR,6bR)-2-Acetyl-1-oxo-5-(2,2,2-trifluoräthylcarbamoyloxymethyl)-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-1-Oxo-5-(2,2,2-trifluoräthylcarbamoyloxymethyl)-1a,2,3,3a,4,6b-hexahydro-1H-2,6a-diaza-cyclobut[cd]inden-6-carbonsäure-trifluoracetat (95 mg; 0,24 mmol) in Dimethylformamid (3 ml) hergestellt. Ausbeute: 35 mg (35%) als gelbliches Pulver.
IR (KBr): 1758, 1730, 1618, 1408, 1151 $cm^{-1}$
MS (ISP): $(M+NH_4)^+$ 409,3; $(M+Na)^+$ 414,2

(b) (1aS,3aR,6bR)-1-Oxo-2-trifluoracetyl-5-(2,2,2-trifluoräthylcarbamoyloxymethyl)-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-1-Oxo-5-(2,2,2-trifluoräthylcarbamoyloxymethyl)-1a,2,3,3a,4,6b-hexahydro-1H-2,6a-diaza-cyclobut[cd]inden-6-carbonsäure-trifluoracetat (120 mg; 0,307 mmol) hergestellt. Ausbeute: 56

mg (39%) als beiges Pulver.
IR (KBr): 1762, 1695, 1608, 1546, 1403, 1152 $cm^{-1}$
MS (ISP): $(M+NH_4)^+$ 463,2

Beispiel 93

(1aS,3aR,6bR)-5-Cyclopropylcarbamoyloxymethyl-1-oxo-1a,2,3,3a,4,6b-hexahydro-1H-2,6a-diaza-cyclobut-[cd]inden-6-carbonsäure-trifluoracetat

Diese Verbindung wird in der gleichen Weise wie in Beispiel 2(a) angegeben ausgehend von (1aS,3aR,6bR)-5-Cyclopropylcarbamoyloxymethyl-1-oxo-1a,2,3,3a,4,6b-hexahydro-1H-2,6a-diaza-cyclobut-[cd]inden-2,6-dicarbonsäure-di-t-butylester (490 mg; 1,05 mmol) hergestellt. Ausbeute: 330 mg (78%) als beiges Pulver.
IR (KBr): 1780, 1700, 1681, 1625, 1410, 1203 $cm^{-1}$
MS (ISP): $(M+H)^+$ 308,3

Die hierfür verwendete Ausgangsverbindung wird in Analogie zu Beispiel 89 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (500 mg; 1,32 mmol) und Cyclopropylamin (0,11 ml; 1,58 mmol) hergestellt. Ausbeute: 533 mg (88%) als farbloses Pulver.
IR (KBr): 1781, 1709, 1686 $cm^{-1}$
MS (ISP): $(M+H)^+$ 464,4; $(M+NH_4)^+$ 481,4; $(M+Na)^+$ 486,4

Beispiel 94

(a) (1aS,3aR,6bR)-2-Acetyl-5-cyclopropylcarbamoyloxymethyl-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-Cyclopropylcarbamoyloxymethyl-1-oxo-1a,2,3,3a,4,6b-hexahydro-1H-2,6a-diaza-cyclobut[cd]inden-6-carbonsäure-trifluoracetat (150 mg; 0,37 mmol) in Dimethylformamid (2,5 ml) hergestellt. Ausbeute: 71 mg (51%) als farbloses Pulver.
IR (KBr): 1754, 1708, 1640, 1606, 1529, 1406, 1263 $cm^{-1}$
MS (ISP): $(M+H)^+$ 350,3; $(M+NH_4)^+$ 367,4; $(M+Na)^+$ 372,3

(b) (1aS,3aR,6bR)-5-Cyclopropylcarbamoyloxymethyl-1-oxo-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-Cyclopropylcarbamoyloxymethyl-1-oxo-1a,2,3,3a,4,6b-hexahydro-1H-2,6a-diaza-cyclobut-[cd]inden-6-carbonsäure-trifluoracetat (150 mg; 0,37 mmol) hergestellt. Ausbeute: 67 mg (42%) als hellbraunes Pulver.
IR (KBr): 1764, 1697, 1607, 1520, 1404 $cm^{-1}$
MS (ISN): $(M-Na)^-$ 402,2

Beispiel 95

(1aS,3aR,6bR)-5-Carbamoylmethylcarbamoyloxymethyl-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-Carbamoylmethylcarbamoyloxymethyl-1-oxo-1a,2,3,3a,4,6b-hexahydro-1H-2,6a-diaza-cyclobut[cd]inden-2,6-dicarbonsäure-di-t-butylester (317 mg; 0,66 mmol) hergestellt. Ausbeute: 236 mg (79%) als beiges Pulver.
IR (KBr): 1774, 1700, 1679, 1610, 1426, 1203 $cm^{-1}$
MS (ISP): $(M+H)^+$ 325,3

Die hierfür verwendete Ausgangsverbindung wird in Analogie zu Beispiel 89 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (500 mg; 1,31 mmol) und Glycinamid-hydrochlorid (175 mg; 1,58 mmol) hergestellt. Ausbeute: 260 mg (41%) als farbloser Feststoff.

IR (KBr): 1776, 1710, 1690, 1525, 1244 $cm^{-1}$
MS (ISP): $(M+H)^+$ 481,4; $(M+NH_4)^+$ 498,5; $(M+Na)^+$ 503,5

Beispiel 96

(a) (1aS,3aR,6bR)-2-Acetyl-5-carbamoylmethylcarbamoyloxymethyl-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-Carbamoylmethylcarbamoyloxymethyl-1-oxo-1a,2,3,3a,4,6b-hexahydro-1H-2,6a-diaza-cyclobut[cd]inden-6-carbonsäure-trifluoracetat (100 mg; 0,22 mmol) in Dimethylformamid (2 ml) hergestellt. Ausbeute: 26 mg (30%) als oranges Pulver.
IR (KBr): 1755, 1680, 1621, 1540, 1402 $cm^{-1}$
MS (ISN): $(M-Na)^-$ 365,2

(b) (1aS,3aR,6bR)-5-Carbamoylmethylcarbamoyloxymethyl-1-oxo-2-trifluoroacetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-Carbamoylmethylcarbamoyloxymethyl-1-oxo-1a,2,3,3a,4,6b-hexahydro-1H-2,6a-diaza-cyclobut[cd]inden-6-carbonsäure-trifluoracetat (100 mg; 0,22 mmol) hergestellt. Ausbeute: 28 mg (29%) als bräunliches Pulver.
IR (KBr): 1763, 1690, 1606, 1529, 1403 $cm^{-1}$
MS (ISP): $(M+H)^+$ 443,2

Beispiel 97

(1aS,3aR,6bR)-5-Methylcarbamoyloxymethyl-1-oxo-1a,2,3,3a,4,6b-hexahydro-1H-2,6a-diaza-cyclobut[cd]-inden-6-carbonsäure-trifluoracetat

Diese Verbindung wird in der gleichen Weise wie in Beispiel 2(a) angegeben ausgehend von (1aS,3aR,6bR)-5-Methylcarbamoyloxymethyl-1-oxo-1a,2,3,3a,4,6b-hexahydro-1H-2,6a-diaza-cyclobut[cd]-inden-2,6-dicarbonsäure-di-t-butylester (600 mg; 1,37 mmol) hergestellt. Ausbeute: 490 mg (92%) als leicht beiges Pulver.
IR (KBr): 1776, 1710, 1680, 1539, 1201 $cm^{-1}$
MS (ISN): $(M-H)^-$ 280,2
Die hierfür verwendete Ausgangsverbindung wird in Analogie zu Beispiel 89 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (600 mg; 1,58 mmol) und Methylamin-hydrochlorid (126 mg; 1,89 mmol) hergestellt. Ausbeute: 600 mg (87%) als farbloser Schaum.
IR (KBr): 1776, 1708, 1634, 1532, 1246 $cm^{-1}$
MS (ISP): $(M+H)^+$ 438,5; $(M+NH_4)^+$ 455,5; $(M+Na)^+$ 460,4

Beispiel 98

(a) (1aS,3aR,6bR)-2-Acetyl-5-methylcarbamoyloxymethyl-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-Methylcarbamoyloxymethyl-1-oxo-1a,2,3,3a,4,6b-hexahydro-1H-2,6a-diaza-cyclobut[cd]inden-6-carbonsäure-trifluoracetat (100 mg; 0,26 mmol) in Dimethylformamid (2 ml) hergestellt. Ausbeute: 41 mg (46%) als oranges Pulver.
IR (KBr): 1754, 1704, 1624, 1540, 1405 $cm^{-1}$
MS (ISN): $(M-Na)^-$ 322,2

(b) (1aS,3aR,6bR)-5-Methylcarbamoyloxymethyl-1-oxo-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-Methylcarbamoyloxymethyl-1-oxo-1a,2,3,3a,4,6b-hexahydro-1H-2,6a-diaza-cyclobut[cd]-inden-6-carbonsäure-trifluoracetat (200 mg; 0,506 mmol) hergestellt. Ausbeute: 70 mg (34%) als gelb-braunes Pulver.
IR (KBr): 1768, 1696, 1610, 1537, 1405 $cm^{-1}$
MS (ISP): $(M+H)^+$ 378,2; $(M+NH_4)^+$ 395,3; $(M+Na)^+$ 400,2

Beispiel 99

(1aS,3aR,6bR)-1-Oxo-5-(pyridin-4-ylmethylcarbamoyloxymethyl)-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-1-Oxo-5-(pyridin-4-ylmethylcarbamoyloxymethyl)-1-oxo-1a,2,3,3a,4,6b-hexahydro-1H-2,6a-diazacyclobut[cd]inden-2,6-dicarbonsäure-di-t-butylester (1,00g; 1,94 mmol) hergestellt. Ausbeute: 1,17 g (100%) als leicht beiges Pulver.
IR (KBr): 1782, 1710, 1678, 1511, 1198 $cm^{-1}$
MS (ISP): $(M+H)^+$ 359,3

Die hierfür verwendete Ausgangsverbindung wird in Analogie zu Beispiel 89 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (900 mg; 2,37 mmol) und 4-Picolylamin (0,29 ml; 2,8 mmol) hergestellt. Ausbeute: 657 mg (54%) als farbloser Feststoff.
IR (KBr): 1774, 1706, 1690, 1243 $cm^{-1}$
MS (ISP): $(M+H)^+$ 515,4

Beispiel 100

(a) (1aS,3aR,6bR)-2-Acetyl-1-oxo-5-(pyridin-4-ylmethylcarbamoyloxymethyl)-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-1-Oxo-5-(pyridin-4-ylmethylcarbamoyloxymethyl)-1a,2,3,3a,4,6b-hexahydro-1H-2,6a-diaza-cyclobut[cd]inden-6-carbonsäure-trifluoracetat (200 mg; 0,33 mmol) in Dimethylformamid (3 ml) hergestellt. Ausbeute: 80 mg (57%) als beiges Pulver.
IR (KBr): 1755, 1712, 1640, 1604, 1418 $cm^{-1}$
MS (ISP): $(M+2H\text{-}Na)^+$ 401,3

(b) (1aS,3aR,6bR)-1-Oxo-5-(pyridin-4-ylmethylcarbamoyloxymethyl)-2-trifluoracetyl-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-1-Oxo-5-(pyridin-4-ylmethylcarbamoyloxymethyl)-1a,2,3,3a,4,6b-hexahydro-1H-2,6a-diaza-cyclobut[cd]inden-6-carbonsäure-trifluoracetat (200 mg; 0,33 mmol) hergestellt. Ausbeute: 90 mg (57%) als farbloses Pulver.
IR (KBr): 1762, 1712, 1698, 1605, 1401 $cm^{-1}$
MS (ISN): $(M\text{-}Na)^-$ 453,3

(c) (1aS,3aR,6bR)-2-[(R)-(N-Benzyloxycarbonyl)-2-phenylglycyl]-1-oxo-5-(pyridin-4-ylmethylcarbamoyloxymethyl)-1a,2,3,3a,4,6b-hexahydro-1H-2,6a-diaza-cyclobut[cd]inden-6-carbonsäure-Kaliumsalz

Diese Verbindung wird in Analogie zu Beispiel 19(c) ausgehend von (1aS,3aR,6bR)-1-Oxo-5-(pyridin-4-ylmethylcarbamoyloxymethyl)-1a,2,3,3a,4,6b-hexahydro-1H-2,6a-diaza-cyclobut[cd]inden-6-carbonsäure-trifluoracetat (500 mg; 0,83 mmol), N-Benzyloxycarbonyl-D-(-)[R]-2-phenylglycin (515 mg; 2,5 mmol) und Kaliumcarbonat (250 mg; 1,8 mmol) in Dimethylformamid (5 ml) hergestellt. Ausbeute: 97 mg (19%) bräunliches Pulver.

IR (KBr): 1756, 1713, 1605, 1523 $cm^{-1}$
MS (ISP): $(M+H)^+$ 626,3

(d) (1aS,3aR,6bR)-2-[(R)-2-phenylglycyl]-1-oxo-5-(pyridin-4-ylmethylcarbamoyloxymethyl)-1a,2,3,3a,4,6b-hexahydro-1H-2,6a-diaza-cyclobut[cd]inden-6-carbonsäure-hydrochlorid

(1aS,3aR,6bR)-2-[(R)-(N-Benzyloxycarbonyl)-2-phenylglycyl]-1-oxo-5-(pyridin-4-ylmethylcarbamoyloxymethyl)-1a,2,3,3a,4,6b-hexahydro-1H-2,6a-diaza-cyclobut[cd]inden-6-carbonsäure-Kaliumsalz (85 mg; 0,136 mmol) wird in Methanol (20 ml) und 1N wässriger Salzsäure (0,41 ml) über 10% Pd/C (20 mg) hydriert. Nach 2,5 Stunden wird die Suspension abgenutscht, eingeengt, mit Aether verrührt und abgenutscht. Ausbeute: 43 mg (56%) als gelb-oranges Pulver.
IR (KBr): 1760, 1720, 1643 $cm^{-1}$
MS (ISP): $(M+H)^+$ 492,4

Beispiel 101

(1aS,3aR,6bR)-5-[(4-Hydroxy-benzyl)-carbamoyloxymethyl]-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-[(4-Hydroxy-benzyl)carbamoyloxymethyl]-1-oxo-1a,2,3,3a,4,6b-hexahydro-1H-2,6a-diaza-cyclobut[cd]inden-2,6-dicarbonsäure-di-t-butylester (380 mg; 0,72 mmol) hergestellt. Ausbeute: 280 mg (84%) als beiges Pulver.
IR (KBr): 1774, 1710, 1696, 1615, 1515, 1203 $cm^{-1}$
MS (ISP): $(M+H)^+$ 374,2; $(M+NH_4)^+$ 391,3; $(M+Na)^+$ 396,2

Die hierfür verwendete Ausgangsverbindung wird in Analogie zu Beispiel 89 ausgehend vor (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (600 mg; 1,58 mmol) und 4-Hydroxybenzylamin (270 mg; 2,2 mmol) hergestellt. Ausbeute: 320 mg (38%) als farbloser Feststoff.
IR (KBr): 1775, 1705, 1516, 1367, 1242, 1161 $cm^{-1}$
MS (ISP): $(M+H)^+$ 530,4; $(M+NH_4)^+$ 547,4; $(M+Na)^+$ 552,4

Beispiel 102

(a) (1aS,3aR,6bR)-2-Acetyl-5-[(4-hydroxy-benzyl)-carbamoyloxymethyl]-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-[(4-Hydroxy-benzyl)-carbamoyloxymethyl]-1-oxo-1a,2,3,3a,4,6b-hexahydro-1H-2,6a-diaza-cyclobut[cd]inden-6-carbonsäure-trifluoracetat (110 mg; 0,24 mmol) in Dimethylformamid (2 ml) hergestellt. Ausbeute: 36 mg (35%) als beiges Pulver.
IR (KBr): 1758, 1710, 1613, 1515, 1406 $cm^{-1}$
MS (ISP): $(M+H)^+$ 415,9; $(M+NH_4)^+$ 433,0; $(M+Na)^+$ 438,0

(b) (1aS,3aR,6bR)-5-[(4-Hydroxy-benzyl)-carbamoyloxymethyl]-2-trifluoracetyl-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-[(4-Hydroxy-benzyl)-carbamoyloxymethyl]-1-oxo-1a,2,3,3a,4,6b-hexahydro-1H-2,6a-diaza-cyclobut[cd]inden-6-carbonsäure-trifluoracetat (120 mg; 0,26 mmol) hergestellt. Ausbeute: 29 mg (23%) als braunes Pulver.
IR (KBr): 1765, 1694, 1612, 1516, 1403 $cm^{-1}$
MS (ISP): $(M+H)^+$ 470,0; $(M+NH_4)^+$ 487,1; $(M+Na)^+$ 492,1

Beispiel 103

(1aS,3aR,6bR)-5-(4-Methyl-piperazin-1-ylcarbonyloxymethyl)-1-oxo-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-5-(4-Methyl-piperazin-1-ylcarbonyloxymethyl)-1-oxo-1a,2,3,3a,4,6b-hexahydro-1H-2,6a-diaza-cyclobut[cd]inden-2,6-dicarbonsäure-di-t-butylester (900 mg; 1,78 mmol) hergestellt. Ausbeute: 807 mg (76%) als beiges Pulver.
IR (KBr): 1779, 1700, 1679, 1201 $cm^{-1}$
MS (ISP): $(M+H)^+$ 351

Die hierfür verwendete Ausgangsverbindung wird in Analogie zu Beispiel 89 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (600 mg; 1,58 mmol) und 4-Methyl-piperazin (0,19 ml; 1,89 mmol) hergestellt. Ausbeute: 805 mg (100%) als farbloser harzartiger Feststoff.
IR (KBr): 1774, 1702, 1366, 1160 $cm^{-1}$
MS (ISP): $(M+H)^+$ 507

Beispiel 104

(a) (1aS,3aR,6bR)-2-Acetyl-5-(4-methyl-piperazin-1-ylcarbonyloxymethyl)-1-oxo-1a,2,3,3a,4,6b-hexahydro-1H-2,6a-diaza-cyclobut[cd]inden-6-carbonsäure-Natriumsalz

Diese Verbindung wird in Analogie zu Beispiel 19(a) ausgehend von (1aS,3aR,6bR)-5-(4-Methyl-piperazin-1-ylcarbonyloxymethyl)-1-oxo-1a,2,3,3a,4,6b-hexahydro-1H-2,6a-diaza-cyclobut[cd]inden-6-carbonsäure-trifluoracetat (200 mg; 0,33 mmol) in Dimethylformamid (4 ml) hergestellt. Ausbeute: 40 mg (29%) als braunes Pulver.
IR (KBr): 1757, 1694, 1640, 1608, 1427, 1236 $cm^{-1}$
MS (ISP): $(M+H)^+$ 393,1

(b) (1aS,3aR,6bR)-5-(4-Methyl-piperazin-1-ylcarbonyloxymethyl)-2-trifluoracetyl-1-oxo-1a,2,3,3a,4,6b-hexahydro-1H-2,6a-diaza-cyclobut[cd]inden-6-carbonsäure-Natriumsalz

Diese Verbindung wird in der gleichen Weise wie in Beispiel 19(c) angegeben ausgehend von (1aS,3aR,6bR)-5-(4-methyl-piperazin-1-ylcarbonyloxymethyl)-1-oxo-1a,2,3,3a,4,6b-hexahydro-1H-2,6a-diaza-cyclobut[cd]inden-6-carbonsäure-trifluoracetat (200 mg; 0,33 mmol) hergestellt. Ausbeute: 82 mg (52%) als gelbliches Pulver.
IR (KBr): 1756, 1710, 1661, 1614, 1402, 1207 $cm^{-1}$
MS (ISN): $(M\text{-}Na)^-$ 445,2

Beispiel 105

(1aS,3aR,6bR)-1-Oxo-5-(1H-tetrazol-5-yl-amino-carbonyloxymethyl)-1a,2,3,3a,4,6b-hexahydro-1H-2,6a-diaza-cyclobut[cd]inden-6-carbonsäure-trifluoracetat

Diese Verbindung wird in der gleichen Weise wie in Beispiel 2(a) angegeben ausgehend von (1aS,3aR,6bR)-1-Oxo-5-(1H-tetrazol-5-yl-aminocarbonyloxymethyl)-1-oxo-1a,2,3,3a,4,6b-hexahydro-1H-2,6a-diaza-cyclobut[cd]inden-2,6-dicarbonsäure-di-t-butylester (750 mg; 1,52 mmol) hergestellt. Ausbeute: 410 mg (60%) als gelb-brauner Feststoff.
IR (KBr): 1773, 1700, 1677, 1611, 1401, 1203, 1135 $cm^{-1}$
MS (ISP): $(M+H)^+$ 336,3

Die hierfür verwendete Ausgangsverbindung wird in Analogie zu Beispiel 89 ausgehend von (1aS,3aR,6bR)-5-Hydroxymethyl-1-oxo-1a,2,3,3a,4,6b-hexahydro-1H-2,6a-diaza-cyclobut[cd]inden-2,6-dicarbonsäure-di-t-butylester (1,00 g; 2,63 mmol) und 5-Amino-1H-tetrazol (270 mg; 3,15 mmol) hergestellt. Ausbeute: 750 mg (58%) als farbloses Pulver.
IR (KBr): 1774, 1735, 1703, 1607, 1367, 1242 $cm^{-1}$
MS (ISP): $(M+Na)^+$ 514; $(M+K)^+$ 530

Beispiel 106

(1aS,3aR,6bR)-5-Methoxy-1-oxo-1a,2,3,3a,4,6b-hexahydro-1H-2,6a-diaza-cyclobut[cd]inden-6-carbonsäure-trifluoracetat

Ausgehend von (1aS,3aR,6bR)-5-Methoxy-1-oxo-1a,2,3,3a,4,6b-hexahydro-1H-2,6a-diaza-cyclobut[cd]-inden-2,6-dicarbonsäure-di-t-butylester (1,43 g; 3,8 mmol) werden in Analogie zu Beispiel 2(a) 1,12 g (70%) als gelblicher Festkörper erhalten.
IR(KBr): 1772, 1679, 1613, 1369, 1201, 1134 $cm^{-1}$
MS (ISP): $(M+H)^+$ 225,3

| Mikroanalyse: $C_{12}H_{13}N_2O_6F_3 \bullet 0{,}33\ H_2O \bullet 0{,}65\ (CH_3CH_2)_2O \bullet 0{,}27\ CF_3COOH$ | | | | |
|---|---|---|---|---|
| Ber. | C 42,97 | H 4,87 | N 6,62 | F 17,11 |
| Gef. | C 43,14 | H 4,85 | N 6,39 | F 17,14 |

Der als Ausgangsverbindung eingesetzte (1aS,3aR,6bR)-5-Methoxy-1-oxo-1a,2,3,3a,4,6b-hexahydro-1H-2,6a-diaza-cyclobut[cd]inden-2,6-dicarbonsäure-di-t-butylester kann wie folgt hergestellt werden:

Bei 0°C wird eine Lösung von (1aS,3aR,6bR)-5-Hydroxy-1-oxo-1a,2,3,3a,4,6b-hexahydro-2,6a-diazacyclobut[cd]inden-2,6-dicarbonsäure-di-tbutylester (2,00 g; 5,5 mmol; aus Beispiel 1) in THF (50 ml) mit einer Lösung von Diazomethan in Diäthylether (insgesamt 12 ml, aufgeteilt in Portionen zu 2 ml und 2x5 ml) versetzt. Bei Raumtemperatur wird 4 Stunden gerührt. Man verdünnt mit Aethanol (10ml) und engt ein. Der Rückstand wird an Kieselgel chromatographiert (Laufmittel Aethylacetat/n-Hexan 2:1). Man erhält 1,66 g (80%) als weissen Festkörper.
IR(KBr): 1761, 1705, 1627, 1236, 1162, 1112 $cm^{-1}$
MS (ISP): $(M+Na)^+$ 403; $(M+H)^+$ 381,5

| Mikroanalyse: $C_{19}H_{28}N_2O_6 \bullet 0{,}043\ H_2O$ | | | |
|---|---|---|---|
| Ber. | C 59,86 | H 7,43 | N 7,05 |
| Gef. | C 60,02 | H 7,52 | N 7,35 |

Beispiel 107

(1aS,3aR,6bR)-2-(4-Hydroxy-phenylcarbamoyl)-5-methoxy-1-oxo-1a,2,3,3a,4,6b-hexahydro-1H-2,6a-diaza-cyclobut[cd]inden-6-carbonsäure-Natriumsalz

Ausgehend von (1aS,3aR,6bR)-5-Methoxy-1-oxo-1a,2,3,3a,4,6b-hexahydro-1H-2,6a-diaza-cyclobut[cd]-inden-6-carbonsäure-trifluoracetat (150 mg; 0,35; mmol; aus Beispiel 106) werden in Analogie zu Beispiel 3-(a) 81 mg (61%) als weisses Pulver isoliert.
IR(KBr): 3400, 1745, 1633, 1513, 1410, 1244, 1112, 1006, 835 $cm^{-1}$
MS (ISN): $(M\text{-}Na)^-$ 358,3

Beispiel 108

(a) (1aS,3aR,6bR)-2-Acetyl-5-methoxy-1-oxo-1a,2,3,3a,4,6b-hexahydro-1H-2,6a-diaza-cyclobut[cd]inden-6-carbonsäure-Natriumsalz

Ausgehend von (1aS,3aR,6bR)-5-Methoxy-1-oxo-1a,2,3,3a,4,6b-hexahydro-1H-2,6a-diaza-cyclobut[cd]-inden-6-carbonsäure-trifluoracetat (200 mg; 0,47 mmol; aus Beispiel 106) werden in Analogie zu Beispiel 3-(aa) 90 mg (66%) eines weissen Pulvers erhalten.
IR (KBr): 1750, 1633, 1414, 1114 $cm^{-1}$
MS (ISP): $(M+Na)^+$ 311,3; $(M+H)^+$ 289,3; $(M\text{-}Na+2H)^+$ 267,3

(b) (1aS,3aR,6bR)-5-Methoxy-1-oxo-2-trifluoracetyl-1a,2,3,3a,4,6b-hexahydro-1H-2,6a-diaza-cyclobut[cd]-inden-6-carbonsäure-Natriumsalz

Ausgehend von (1aS,3aR,6bR)-5-Methoxy-1-oxo-1a,2,3,3a,4,6b-hexahydro-1H-2,6a-diaza-cyclobut[cd]-inden-6-carbonsäure-trifluoracetat (150 mg; 0,35 mmol; aus Beispiel 106) werden in Analogie zu Beispiel 3-(ab) 66 mg (49%) als weisses Pulver isoliert.
IR (KBr): 1757, 1699, 1633, 1603, 1409, 1156 $cm^{-1}$
MS (ISN): $(M-Na)^-$ 319,3

| Mikroanalyse: $C_{12}H_{10}N_2O_5F_3Na \cdot 1.96\ H_2O \cdot 0{,}05\ NaHCO_3$ | | | | | |
|---|---|---|---|---|---|
| Ber.<br>Gef. | C 37,92<br>C 37,79 | H 3,69<br>H 3,89 | N 7,34<br>N 7,41 | F 14,93<br>F 15,08 | Na 6,32<br>Na 6,44 |

Beispiel A

Herstellung von Trockenampullen für die intramuskuläre Verabreichung:

Es wird in üblicher Weise ein Lyophilisat von 0,5 g des Natriumsalzes der (1aS,3aR,6bR)-5-Carbamoyloxymethyl-2-(4-hydroxy-phenylcarbamoyl)-1-oxo-1a,2,3,3a,4,6b-hexahydro-1H-2,6a-diazacyclobut[cd]inden-6-carbonsäure sowie 1 g des Dinatriumsalzes der (6R,7R)-7-[(2-Amino-4-thiazolyl)-2-(Z-methoxyimino)-acetamido]-3-{[(2,5-dihydro-6-hydroxy-2-methyl-5-oxo-1,2,4-triazin-3-yl)sulfanyl]methyl}-8-oxo-5-thia-1-azabicyclo[4.2.0]oct-2-en-2-carbonsäure hergestellt und in eine Ampulle abgefüllt. Vor der Verabreichung wird das Lyophilisat mit 4 ml einer 2%igen wässrigen Lidocainhydrochlorid-Lösung versetzt.

Die beiden Wirkstoffe können wahlweise separat in zwei verschiedene Ampullen abgefüllt werden.

Als Wirkstoff kann auch eine andere Verbindung der Formel I eingesetzt werden, z.B.

(1aS,3aR,6bR)-5-Carbamoyloxymethyl-2-(4-carbamoyl-phenylcarbamoyl)-1-oxo-1a,2,3,3a,4,6b-hexahydro-1H-2,6a-diazacyclobut[cd]inden-6-carbonsäure-Natriumsalz,

(1aS,3aR,6bR)-2-(4-Carbamoyl-phenylcarbamoyl)-1-oxo-5-(pyridin-4-ylsulfanylmethyl)-1a,2,3,3a,4,6b-hexahydro-1H-2,6a-diazacyclobut[cd]inden-6-carbonsäure,

(1aS,3aR,6bR)-5-(5-Amino-1,3,4-thiadiazol-2-ylsulfanylmethyl)-2-(4-hydroxy-phenylcarbamoyl)-1-oxo-1a,2,3,3a,4,6b-hexahydro-1H-2,6a-diaza-cyclobut[cd]inden-6-carbonsäure-Natriumsalz,

(1aS,3aR,6bR)-5-(5-Amino-1,3,4-thiadiazol-2-ylsulfanyl)-2-(4-hydroxyphenylcarbamoyl)-1-oxo-1a,2,3,3a,4,6b-hexahydro-1H-2,6a-diazacyclobut[cd]inden-6-carbonsäure-Natriumsalz,

(1aS,3aR,6bR)-5-carbamoyloxymethyl-1-oxo-2-trifluoracetyl-1a,2,3,3a,4,6b-hexahydro-1H-2,6a-diaza-cyclobut[cd]inden-6-carbonsäure-Natriumsalz,

(1aS,3aR,6bR)-1-Oxo-5-(pyridin-4-ylsulfanylmethyl)-2-trifluoracetyl-1a,2,3,3a,4,6b-hexahydro-1H-2,6a-diaza-cyclobut[cd]inden-6-carbonsäure-Natriumsalz,

(1aS,3aR,6bR)-5-(1-Carbamoylmethyl-1H-tetrazol-5-ylsulfanylmethyl)-2-formyl-1-oxo-1a,2,3,3a,4,6b-hexahydro-1H-2,6a-diaza-cyclobut[cd]inden-6-carbonsäure-Natriumsalz,

(1aS,3aR,6bR)-2-Methylsulfonyl-5-(1-methyl-1H-tetrazol-5-ylsulfanylmethyl)-1-oxo-1a,2,3,3a,4,6b-hexahydro-1H-2,6a-diaza-cyclobut[cd]inden-6-carbonsäure-Natriumsalz,

(1aS,3aR,6bR)-2-Acetyl-5-(1-carbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure-Natriumsalz,

(1aS,3aR,6bS)-2-Acetyl-5-(1-methyl-1H-tetrazol-5-ylsulfanylmethyl)-1-oxo-1,1a,2,3,3a,6b-hexahydro-4-oxa-2,6a-diaza-cyclobut[cd]inden-6-carbonsäure-Natriumsalz.

## Patentansprüche

**1.** β-Lactame der allgemeinen Formel

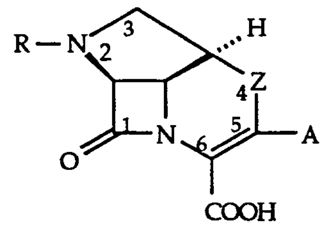

I

in der Z Methylen, Sauerstoff oder Schwefel und R Wasserstoff, ggfs. durch Carboxy, niederes Alkoxycarbonyl, Carbamoyl, niederes Alkylcarbamoyl, Phenylcarbamoyl oder Hydroxyphenylcarbamoyl substituiertes niederes (Cyclo)alkyl, niederes Alkenylmethyl, niederes Alkenylmethoxycarbonyl, Formyl, ggfs. durch Halogen, Cyan, Carbamoyl-niederes Alkoxy, Carbamoyl-niederes Alkylthio oder Carbamoyl-niederes Alkylamino substituiertes niederes (Cyclo)alkanoyl bzw. (Cyclo)alkylsulfonyl, ggfs. durch niederes (Cyclo)alkyl, niederes Alkoxycarbonyl-niederes Alkyl, Benzyloxycarbonyl-niederes Alkyl oder Carboxy-niederes Alkyl substituiertes Carbamoyl oder eine Ringstruktur der allgemeinen Formeln

Q-X-CO- (a1)

Q-X-$SO_2$- (a2)

bedeutet, worin Q einen 5- oder 6-gliedrigen, ggfs. stickstoff-, schwefel- und/oder sauerstoffhaltigen Ring und X eine direkte Bindung oder eine der Gruppen -$CH_2$-, -$CH_2CH_2$-, -CH=CH-, -NH-, -$NHCH_2$-, -$CH_2NH$-, -CH($NH_2$)-, -$CH_2CH_2NH$-, -C(=$NOCH_3$)-, -$OCH_2$- und -$SCH_2$- darstellt; und worin ferner A niederes Alkyl, Hydroxy-niederes Alkyl, Vinyl, Cyanvinyl, niederes Alkoxy, ggfs. phenylsubstituiertes niederes (Cyclo)alkanoyloxy bzw. (Cyclo)alkylsulfonyloxy, ggfs. nieder-(cyclo)alkyl-substituiertes Benzoyloxy bzw. Phenylsulfonyloxy, einen Rest -S-Het oder -S-$CH_2$-Het, worin Het einen 5- oder 6-gliedrigen, stickstoff-, schwefel- und/oder sauerstoffhaltigen Heterocyclus darstellt, oder einen Rest -$CH_2$-L bedeutet, worin L ggfs. phenylsubstituiertes niederes (Cyclo)alkanoyloxy bzw. (Cyclo)-alkylsulfonyloxy, ggfs. nieder(cyclo)alkyl-substituiertes Benzoyloxy bzw. Phenylsulfonyloxy, Carbamoyloxy, niederes (Cyclo)alkoxycarbonyl, Carboxy, Azido, Amino, niederes (Cyclo)alkanoylamino, niederes (Cyclo)alkylsulfonylamino, niederes (Cyclo)alkylamino, di-niederes (Cyclo)alkylamino, einen 5- oder 6-gliedrigen, an einem Stickstoffatom gebundenen Ring oder einen Rest -S-Het oder -S-$CH_2$-Het, worin Het die obige Bedeutung hat, darstellt,
und pharmazeutisch verträgliche, leicht hydrolysierbare Ester und Salze dieser Verbindungen.

**2.** Verbindungen gemäss Anspruch 1 der allgemeinen Formel

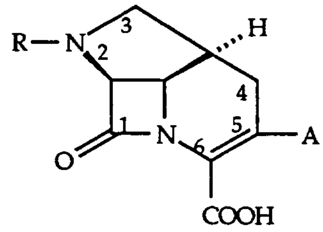

Ia

in der R Wasserstoff, Formyl, niederes (Cyclo)alkanoyl, Halogen-niederes Alkanoyl, Cyan-niederes Alkanoyl, niederes (Cyclo)alkylsulfonyl, Phenylsulfonyl, Phenyl-niederes Alkylsulfonyl oder eine (hetero)-aromatische Gruppe der allgemeinen Formel

Q-X-CO-     (a)

bedeutet, worin Q einen 5- oder 6-gliedrigen, ggfs. stickstoff-, schwefel- und/oder sauerstoffhaltigen Ring und X eine der Gruppen -NH-, $-CH_2-$, $-CH_2CH_2-$, $-CH_2NH-$, $-S-CH_2-$ und $-C(=NOCH_3)$ darstellt; und worin ferner A niederes (Cyclo)alkylsulfonyloxy, Phenylsulfonyloxy, niederes (Cyclo)-alkylphenylsulfonyloxy, einen Rest -S-Het, worin Het einen 5- oder 6-gliedrigen, stickstoff-, schwefel- und/oder sauerstoffhaltigen Heterocyclus darstellt, oder einen Rest $-CH_2-L$ bedeutet, worin L niederes Alkanoyloxy, Carbamoyloxy, niederes Alkoxycarbonyl, Carboxy, Azido, Amino, niederes Alkylamino, di-niederes Alkylamino, einen 5- oder 6-gliedrigen an einem Stickstoffatom quaternären Ring oder eine Gruppe -S-Het, worin Het die obige Bedeutung hat, darstellt,
und pharmazeutisch verträgliche, leicht hydrolysierbare Ester und Salze dieser Verbindungen.

**3.** Verbindungen gemäss Anspruch 1, dadurch gekennzeichnet, dass R eine Gruppe der allgemeinen Formel

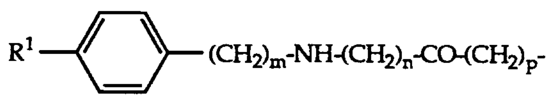

(b)

worin $R^1$ Wasserstoff, Hydroxy, Carbamoyl oder Sulfamoyl und m, n und p jeweils 0 oder 1 darstellen, bedeutet.

**4.** Verbindungen gemäss einem der Ansprüche 1-3, dadurch gekennzeichnet, dass R die Gruppe

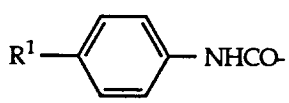

(b1)

worin $R^1$ die in Anspruch 3 gegebene Bedeutung hat, darstellt.

**5.** Verbindungen gemäss Anspruch 1 oder 3, dadurch gekennzeichnet, dass R die Gruppe

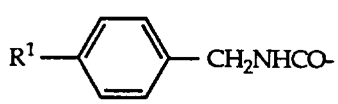

(b2)

worin $R^1$ die in Anspruch 3 gegebene Bedeutung hat, darstellt.

**6.** Verbindungen gemäss Anspruch 1 oder 3, dadurch gekennzeichnet, dass R die Gruppe

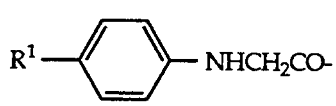

(b3)

worin $R^1$ die in Anspruch 3 gegebene Bedeutung hat, darstellt.

**7.** Verbindungen gemäss Anspruch 1 oder 3, dadurch gekennzeichnet, dass R die Gruppe

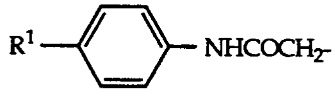

(b4)

worin $R^1$ die in Anspruch 3 gegebene Bedeutung hat, darstellt.

**8.** Verbindungen gemäss Anspruch 4, dadurch gekennzeichnet, dass R die 4-Hydroxyphenylcarbamoylgruppe darstellt.

**9.** Verbindungen gemäss Anspruch 4, dadurch gekennzeichnet, dass R die 4-Carbamoylphenylcarbamoylgruppe darstellt.

**10.** Verbindungen gemäss Anspruch 1 oder 2, dadurch gekennzeichnet, dass R eine ggfs. Fluor- oder Cyan-substituierte niedere Alkanoyl- bzw. niedere Alkylsulfonylgruppe darstellt.

**11.** Verbindungen gemäss Anspruch 10, dadurch gekennzeichnet, dass R die Formylgruppe darstellt.

**12.** Verbindungen gemäss Anspruch 10, dadurch gekennzeichnet, dass R die Acetylgruppe darstellt.

**13.** Verbindungen gemäss Anspruch 10, dadurch gekennzeichnet, dass R die Trifluoracetylgruppe darstellt.

**14.** Verbindungen gemäss Anspruch 10, dadurch gekennzeichnet, dass R die Cyanacetylgruppe darstellt.

**15.** Verbindungen gemäss Anspruch 10, dadurch gekennzeichnet, dass R die Methylsulfonylgruppe darstellt.

**16.** Verbindungen gemäss Anspruch 1 oder 2, dadurch gekennzeichnet, dass R Wasserstoff oder eine der Gruppen
2-Oxo-pyrrolidin-3-ylcarbamoyl
Thien-2-yl-methylcarbamoyl
3,4-Dihydroxy-benzylcarbamoyl
2-Oxo-tetrahydrothien-3-ylcarbamoyl
4-Sulfamoyl-benzylcarbamoyl
3-Methoxy-isoxazol-5-ylmethylcarbamoyl
3-Hydroxy-isoxazol-5-ylmethylcarbamoyl
1,1-Dioxo-tetrahydrothien-3-ylmethylcarbamoyl
(2-Amino-thiazol-4-yl)-methoxyiminoacetyl
1-Methyl-1H-tetrazol-5-ylsulfanylacetyl
3-Carbamoyl-pyridin-1-ylioacetyl
darstellt.

**17.** Verbindungen gemäss Anspruch 1, dadurch gekennzeichnet, dass R eine der Gruppen
2-t-Butoxycarbonyl-äthylcarbamoyl
4-Hydroxy-benzylcarbamoyl
Trifluormethylsulfonyl
Benzyloxycarbonylcarbamoyl
Benzylcarbamoyl
Cyclopropylcarbamoyl
4-Sulfamoyl-benzylcarbamoyl
2-Thiophen-2-yl-äthylcarbamoyl
5-Methyl-1,3,4-thiadiazol-2-yl-sulfonylacetyl
5-Amino-1,3,4-thiadiazol-2-yl-sulfonylacetyl
Pyridin-4-ylsulfanylacetyl
Phenylaminoacetyl
4-Hydroxy-phenylcarbamoylmethyl
Methoxycarbonylmethyl
Aethyl
Carbamoylmethyl
Pyridin-4-ylsulfanylacetyl
3-Carbamoyl-pyridin-1-ylioacetyl
Carbamoylmethylsulfanylacetyl
(R)-Benzyloxycarbonylamino-phenyl-acetyl

(R)-Amino-phenyl-acetyl
Carboxymethylcarbamoyl
darstellt.

**18.** Verbindungen gemäss einem der Ansprüche 1-17, dadurch gekennzeichnet, dass A einen Rest $-CH_2-L$ darstellt, worin L eine Gruppe der allgemeinen Formel

$-OCONR^2R^3$ (c)

bedeutet, worin $R^2$ Wasserstoff und $R^3$ Wasserstoff, niederes (Cyclo)alkyl, Halogen-niederes Alkyl, Carbamoylmethyl oder einen Rest $-(CH_2)_qQ$ bedeutet, worin q 0, 1 oder 2 ist und Q obige Bedeutung hat, oder auch $R^2$ und $R^3$ zusammen mit dem Stickstoffatom einen gesättigten, ggfs. Schwefel, Sauerstoff oder zusätzlichen Stickstoff enthaltenden N-Heterocyclus darstellt.

**19.** Verbindungen gemäss Anspruch 18, dadurch gekennzeichnet, dass $R^2$ Wasserstoff und $R^3$ Wasserstoff oder eine der Gruppen
Methyl
Cyclopropyl
2,2,2-Trifluoräthyl
Phenyl
p-Hydroxyphenyl
Benzyl
p-Hydroxybenzyl
4-Pyridylmethyl
Carbamoylmethyl
1H-Tetrazol-5-yl
darstellt.

**20.** Verbindungen gemäss Anspruch 18, dadurch gekennzeichnet, dass $-NR^2R^3$ eine die Gruppen
Piperazinyl
4-Methyl-piperazinyl
4-Morpholinyl
4-Thiomorpholinyl
darstellt.

**21.** Verbindungen gemäss Anspruch 19, dadurch gekennzeichnet, dass A die Carbamoyloxymethylgruppe darstellt.

**22.** Verbindungen gemäss einem der Ansprüche 1-17, dadurch gekennzeichnet, dass A eine Gruppe der allgemeinen Formel

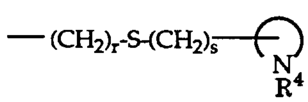 (d)

in der r und s jeweils 0 oder 1 und

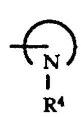

einen 5- oder 6-gliedrigen, ggfs. ein Schwefel- oder Sauerstoffatom enthaltenden N-Heterocyclus darstellt; und worin $R^4$ niederes Alkyl, Sulfonylmethyl oder eine Gruppe der allgemeinen Formel

$-CH_2CONR^5R^6$ (d1)

bedeutet und $R^5$ Wasserstoff und $R^6$ Wasserstoff, niederes (Cyclo)alkyl, Hydroxy, Carbamoylmethyl, Halogen-niederes Alkyl oder einen Rest $-(CH_2)_qQ$ bedeutet, worin q 0, 1 oder 2 ist und Q obige Bedeutung hat, oder auch $R^5$ und $R^6$ zusammen mit dem Stickstoffatom einen gesättigten, ggfs. Schwefel, Sauerstoff oder zusätzlichen Stickstoff enthaltenden N-Heterocyclus darstellt.

**23.** Verbindungen gemäss Anspruch 22, dadurch gekennzeichnet, dass der Rest

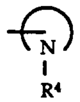

einen 1-$R^4$-substituierten 1H-Tetrazol-5-ylrest darstellt.

**24.** Verbindungen gemäss Anspruch 22 oder 23, dadurch gekennzeichnet, dass $R^4$ Methyl darstellt.

**25.** Verbindungen gemäss Anspruch 22 oder 23, dadurch gekennzeichnet, dass $R^4$ die Gruppe (d1) darstellt, in der $R^5$ Wasserstoff und $R^6$ Wasserstoff, Methyl, Cyclopropyl, Phenyl, p-Hydroxyphenyl, Benzyl, Phenäthyl, Carbamoylmethyl oder Hydroxy oder $R^5$ und $R^6$ zusammen mit dem Stickstoffatom Piperazinyl, 4-Methyl-piperazinyl, 4-Morpholinyl oder 4-Thiomorpholinyl bedeuten.

**26.** Verbindungen gemäss einem der Ansprüche 22-24, dadurch gekennzeichnet, dass A die 1-Methyl-1H-tetrazol-5-ylsulfanylmethylgruppe darstellt.

**27.** Verbindungen gemäss einem der Ansprüche 22, 23 und 25, dadurch gekennzeichnet, dass A die 1-Carbamoylmethyl-1H-tetrazol-5-ylsulfanylmethylgruppe darstellt.

**28.** Verbindungen gemäss einem der Ansprüche 1-17, dadurch gekennzeichnet, dass A eine Gruppe der allgemeinen Formel

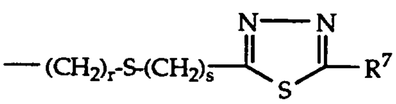 (e)

bedeutet, in der r und s jeweils 0 oder 1 und $R^7$ Methyl, Amino, Acetylamino oder Pyridinioacetylamino darstellt.

**29.** Verbindungen gemäss Anspruch 28, dadurch gekennzeichnet, dass A die 5-Amino-1,3,4-thiadiazol-2-yl-sulfanylmethylgruppe oder die 5-Amino-1,3,4-thiadiazol-2-yl-sulfanylgruppe darstellt.

**30.** Verbindungen gemäss einem der Ansprüche 1-17, dadurch gekennzeichnet, dass A eine Gruppe der allgemeinen Formel

$-(CH_2)_r-S-(CH_2)_s-R^8$ (f)

bedeutet, in der r und s jeweils 0 oder 1 ist und $R^8$ die Pyridin-4-yl-Gruppe oder die Gruppe

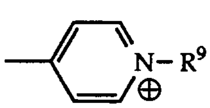 (g)

darstellt und $R^9$ Methyl, Benzyl, Carboxymethyl oder Carbamoylmethyl bedeutet.

**31.** Verbindungen gemäss Anspruch 30, dadurch gekennzeichnet, dass A die Pyridin-4-ylsulfanylmethylgruppe darstellt.

**32.** Verbindungen gemäss einem der Ansprüche 1-17, dadurch gekennzeichnet, dass A eine der Gruppen
1-Methyl-1H-tetrazol-5-ylsulfanyl
5-Methyl-1,3,4-thiadiazol-2-ylsulfanyl
5-(Pyridin-1-ylioacetylamino)-1,3,4-thiadiazol-2-ylsulfanyl
1-Methyl-pyridin-4-yliosulfanylmethyl
Pyridin-1-yliomethyl
Methylsulfonyloxy
4-Methyl-phenylsulfonyloxy
Carboxymethyl
Methoxycarbonylmethyl
Methyl
Vinyl
Acetoxymethyl
darstellt.

**33.** Verbindungen gemäss einem der Annsprüche 1-17, dadurch gekennzeichnet, dass A eine der Gruppen
2-Carbamoyl-5-methyl[1,2,4]triazolo[1,5-a]pyrimidin-7-ylsulfanylmethyl
1-(Cyclopropyl-carbamoylmethyl)-1H-tetrazol-5-ylsulfanylmethyl
1-(Phenyläthyl-carbamoylmethyl)-1H-tetrazol-5-ylsulfanylmethyl
1-(Carbamoylmethyl-carbamoylmethyl)-1H-tetrazol-5-ylsulfanylmethyl
1-Methylcarbamoylmethyl-1H-tetrazol-5-ylsulfanylmethyl
1-(2-Morpholin-4-yl-2-oxo-äthyl)-1H-tetrazol-5-ylsulfanylmethyl
1-(4-Hydroxyphenyl-carbamoylmethyl)-1H-tetrazol-5-ylsulfanylmethyl
1-(Hydroxy-carbamoylmethyl)-1H-tetrazol-5-ylsulfanylmethyl
1-Sulfonylmethyl-1H-tetrazol-5-ylsulfanylmethyl
1-Methyl-imidazol-2-ylsulfanylmethyl
5-Hydroxy-4-methyl-4H-[1,2,4]-triazol-3-ylsulfanylmethyl
6,7-Dihydro-5H-1-pyrindin-4-ylsulfanylmethyl
5-Methyl-1,3,4-thiadiazol-2-ylsulfanylmethyl
1-Methyl-1H-tetrazol-5-yl-methylsulfanylmethyl
5-Methylsulfanyl-1H-tetrazol-1-ylmethyl
4-Methyl-4-pyridiniosulfanyl
Carbamoylmethylsulfanyl
5-(1,4-Dimethyl-1H-1,2,4-triazol-4-ium)-methylsulfanyl
Pyridin-4-ylsulfanyl
5-Acetylamino-1,3,4-thiadiazol-2-ylsulfanylmethyl
2-Cyanvinyl (Z- und E-Isomere)
1-Carboxymethyl-pyridin-4-yliosulfanylmethyl
1-Carbamoylmethyl-pyridin-4-yliosulfanylmethyl
1-Benzyl-pyridin-4-yliosulfanylmethyl
2,5-Dihydro-6-hydroxy-2-methyl-5-oxo-1,2,4-triazin-3-ylsulfanylmethyl
Piperidin-1-ylmethyl
3-Benzyloxycarbonylmethyl-pyridin-1-yliomethyl
3-Carboxymethyl-pyridin-1-yliomethyl
4-Pyridin-3-yl-thiazol-2-ylsulfanylmethyl
Pyrimidin-2-ylsulfanyl
1H-1,2,4-Triazol-3-ylsulfanylmethyl
Azidomethyl
Acetylaminomethyl
Methylsulfonylaminomethyl
4-Hydroxy-phenylcarbamoyloxymethyl
2,2,2-Trifluoräthylcarbamoyloxymethyl
Cyclopropylcarbamoyloxymethyl
Carbamoylmethylcarbamoyloxymethyl
Methylcarbamoyloxymethyl

Pyridincarbamoyloxymethyl
4-Hydroxy-benzylcarbamoyloxymethyl
4-Methyl-piperazin-1-ylcarbonyloxymethyl
1H-Tetrazol-5-yl-amino-carbonyloxymethyl
Methoxy
darstellt.

**34.** (1aS,3aR,6bR)-5-Carbamoyloxymethyl-2-(4-hydroxy-phenylcarbamoyl)-1-oxo-1a,2,3,3a,4,6b-hexahydro-1H-2,6a-diazacyclobut[cd]inden-6-carbonsäure sowie entsprechende, pharmazeutisch verträgliche Salze davon, insbesondere das Natriumsalz.

**35.** (1aS,3aR,6bR)-5-Carbamoyloxymethyl-2-(4-carbamoyl-phenylcarbamoyl)-1-oxo-1a,2,3,3a,4,6b-hexahydro-1H-2,6a-diazacyclobut[cd]inden-6-carbonsäure sowie entsprechende, pharmazeutisch verträgliche Salze davon, insbesondere das Natriumsalz.

**36.** (1aS,3aR,6bR)-2-(4-Carbamoyl-phenylcarbamoyl)-1-oxo-5-(pyridin-4-ylsulfanylmethyl)-1a,2,3,3a,4,6b-hexahydro-1H-2,6a-diazacyclobut[cd]inden-6-carbonsäure sowie entsprechende, pharmazeutisch verträgliche Salze davon, insbesondere das Natriumsalz.

**37.** (1aS,3aR,6bR)-5-(5-Amino-1,3,4-thiadiazol-2-ylsulfanylmethyl)-2-(4-hydroxy-phenylcarbamoyl)-1-oxo-1a,2,3,3a,4,6b-hexahydro-1H-2,6a-diaza-cyclobut[cd]inden-6-carbonsäure sowie entsprechende, pharmazeutisch verträgliche Salze davon, insbesondere das Natriumsalz.

**38.** (1aS,3aR,6bR)-5-(5-Amino-1,3,4-thiadiazol-2-ylsulfanyl)-2-(4-hydroxyphenylcarbamoyl)-1-oxo-1a,2,3,3a,4,6b-hexahydro-1H-2,6a-diazacyclobut[cd]inden-6-carbonsäure sowie entsprechende, pharmazeutisch verträgliche Salze davon, insbesondere das Natriumsalz.

**39.** (1aS,3aR,6bR)-5-Carbamoyloxymethyl-1-oxo-2-trifluoracetyl-1a,2,3,3a,4,6b-hexahydro-1H-2,6a-diaza-cyclobut[cd]inden-6-carbonsäure sowie entsprechende, pharmazeutisch verträgliche Salze davon, insbesondere das Natriumsalz.

**40.** (1aS,3aR,6bR)-1-Oxo-5-(pyridin-4-ylsulfanylmethyl)-2-trifluoracetyl-1a,2,3,3a,4,6b-hexahydro-1H-2,6a-diaza-cyclobut[cd]inden-6-carbonsäure sowie entsprechende, pharmazeutisch verträgliche Salze davon, insbesondere das Natriumsalz.

**41.** (1aS,3aR,6bR)-5-(1-Carbamoylmethyl-1H-tetrazol-5-ylsulfanylmethyl)-2-formyl-1-oxo-1a,2,3,3a,4,6b-hexahydro-1H-2,6a-diaza-cyclobut[cd]inden-6-carbonsäure sowie entsprechende, pharmazeutisch verträgliche Salze davon, insbesondere das Natriumsalz.

**42.** (1aS,3aR,6bR)-2-Methylsulfonyl-5-(1-methyl-1H-tetrazol-5-ylsulfanylmethyl)-1-oxo-1a,2,3,3a,4,6b-hexahydro-1H-2,6a-diaza-cyclobut[cd]inden-6-carbonsäure sowie entsprechende, pharmazeutisch verträgliche Salze davon, insbesondere das Natriumsalz.

**43.** (1aS,3aR,6bR)-2-Acetyl-5-(1-carbamoylmethyl-1H-tetrazol-5-ylsulfanylmethyl)-1-oxo-1a,2,3,3a,4,6b-hexahydro-1H-2,6a-diaza-cyclobut[cd]inden-6-carbonsäure sowie entsprechende, pharmazeutisch verträgliche Salze davon, insbesondere das Natriumsalz.

**44.** (1aS,3aR,6bS)-2-Acetyl-5-(1-methyl-1H-tetrazo-5-ylsulfanylmethyl)-1-oxo-1,1a,2,3,3a,6b-hexahydro-4-oxa-2,6a-diaza-cyclobut[cd]inden-6-carbonsäure sowie entsprechende, pharmazeutisch verträgliche Salze davon, insbesondere das Natriumsalz.

**45.** Verbindungen gemäss einem der Ansprüche 1-3 ausgewählt unter
(1aS,3aR,6bR)-2-(4-Hydroxy-phenylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure
(1aS,3aR,6bR)-2-(4-Carbamoyl-phenylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure
(1aS,3aR,6bR)-2-[(S)-2-Oxo-pyrrolidin-3-ylcarbamoyl]-5-(1-methyl-1H-tetrazol-5-ylsulfanyl)-1-oxo-1a,2,3,3a,4,6b-hexahydro-1H-2,6a-diazacyclobut[cd]inden-6-carbonsäure

(1aS,3aR,6bR)-5-(1-Methyl-1H-tetrazol-5-ylsulfanyl)-1-oxo-2-(thien-2-ylmethylcarbamoyl)-1a,2,3,3a,4,6b-hexahydro-1H-2,6a-diazacyclobut[cd]inden-6-carbonsäure

(1aS,3aR,6bR)-2-(3,4-Dihydroxy-benzylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure

(1aS,3aR,6bR)-5-(1-Methyl-1H-tetrazol-5-ylsulfanyl)-1-oxo-2-[(R)- und [(S)-2-oxo-tetrahydro-thien-3-ylcarbamoyl]-1a,2,3,3a,4,6b-hexahydro-1H-2,6a-diazacyclobut[cd]inden-6-carbonsäure

(1aS,3aR,6bR)-5-(1-Methyl-1H-tetrazol-5-ylsulfanyl)-1-oxo-2-(4-sulfamoyl-benzylcarbamoyl)-1a,2,3,3a,4,6b-hexahydro-1H-2,6a-diazacyclobut[cd]inden-6-carbonsäure

(1aS,3aR,6bR)-2-(3-Methoxy-isoxazol-5-ylmethylcarbamoyl)-5-(1-methyl-1H-tetrazol-5-ylsulfanyl)-1-oxo-1a,2,3,3a,4,6b-hexahydro-1H-2,6a-diazacyclo-but[cd]inden-6-carbonsäure

(1aS,3aR,6bR)-2-[(R)- und [(S)-1,1-Dioxo-tetrahydrothien-3-ylcarbamoyl)-5-(1-methyl-1H-tetrazol-5-ylsulfanyl)-1-oxo-1a,2,3,3a,4,6b-hexahydro-1H-2,6a-diazacyclobut[cd]inden-6-carbonsäure

(1aS,3aR,6bR)-2-(4-Hydroxyphenylcarbamoyl)-5-(5-methyl-1,3,4-thiadiazol-2-ylsulfanyl)-1-oxo-1a,2,3,3a,4,6b-hexahydro-1H-2,6a-diazacyclobut[cd]inden-6-carbonsäure

(1aS,3aR,6bR)-2-(4-Carbamoyl-phenylcarbamoyl)-5-(5-methyl-1,3,4-thiadiazol-2-ylsulfanyl)-1-oxo-1a,2,3,3a,4,6b-hexahydro-1H-2,6a-diazacyclo-but[cd]inden-6-carbonsäure

(1aS,3aR,6bR)-5-(5-Amino-1,3,4-thiadiazol-2-ylsulfanyl)-2-(4-carbamoylamino-phenylcarbamoyl)-1-oxo-1a,2,3,3a,4,6b-hexahydro-1H-2,6a-diazacyclo-but[cd]inden-6-carbonsäure

(1aS,3aR,6bR)-2-(4-Hydroxy-phenylcarbamoyl)-1-oxo-5-(5-pyridin-1-ylacetylamino-1,3,4-thiadiazol-2-ylsulfanyl)-1a,2,3,3a,4,6b-hexahydro-1H-2,6a-diazacyclobut[cd]inden-6-carbonsäure

(Z)-(1aS,3aR,6bR)-2-[(2-Amino-thiazol-4-yl)-methoxyiminoacetyl]-1-oxo-5-(1-methyl-1H-tetrazol-5-ylsulfanyl)-1a,2,3,3a,4,6b-hexahydro-1H-2,6a-diaza-cyclobut[cd]inden-6-carbonsäure

(1aS,3aR,6bR)-2-(4-Hydroxy-phenylcarbamoyl)-5-methylsulfonyloxy-1-oxo-1a,2,3,3a,4,6b-hexahydro-1H-2,6a-diazacyclobut[cd]inden-6-carbonsäure-

(1aS,3aR,6bR)-2-(4-Carbamoyl-phenylcarbamoyl)-5-methylsulfonyloxy-1-oxo-1a,2,3,3a,4,6b-hexahydro-1H-2,6a-diazacyclobut[cd]inden-6-carbonsäure-

(1aS,3aR,6bR)-5-Methylsulfonyloxy-2-(thien-2-ylmethylcarbamoyl)-1-oxo-1a,2,3,3a,4,6b-hexahydro-1H-2,6a-diazacyclobut[cd]inden-6-carbonsäure

(1aS,3aR,6bR)-2-(3,4-Dihydroxy-benzylcarbamoyl)-5-methylsulfonyloxy-1-oxo-1a,2,3,3a,4,6b-hexahydro-1H-2,6a-diazacyclobut[cd]inden-6-carbonsäure

(1aS,3aR,6bR)-5-Methylsulfonyloxy-1-oxo-2-[(S)-2-oxo-pyrrolidin-3-ylcarbamoyl]-1a,2,3,3a,4,6b-hexahydro-1H-2,6a-diazacyclobut[cd]inden-6-carbonsäure

(1aS,3aR,6bR)-5-Methylsulfonyloxy-1-oxo-2-[(R) und -[(S)-2-oxo-tetrahydro-thien-3-ylcarbamoyl]-1a,2,3,3a,4,6b-hexahydro-1H-2,6a-diazacyclobut[cd]inden-6-carbonsäure

(1aS,3aR,6bR)-2-[(R)- und [(S)-1,1-Dioxo-tetrahydrothien-3-ylcarbamoyl]-5-methylsulfonyloxy-1-oxo-1a,2,3,3a,4,6b-hexahydro-1H-2,6a-diazacyclobut[cd]inden-6-carbonsäure

(1aS,3aR,6bR)-5-Methylsulfonyloxy-1-oxo-2-(4-sulfamoylbenzylcarbamoyl)-1a,2,3,3a,4,6b-hexahydro-1H-2,6a-diazacyclobut[cd]inden-6-carbon säure

(1aS,3aR,6bR)-2-(4-Hydroxy-phenylcarbamoyl)-5-(4-methyl-phenylsulfonyloxy)-1-oxo-1a,2,3,3a,4,6b-hexahydro-1H-2,6a-diazacyclobut[cd]inden-6-carbonsäure

(1aS,3aR,6bR)-2-(3-Carbamoyl-pyridin-1-ylioacetyl)-5-methylsulfonyloxy-1-oxo-1a,2,3,3a,4,6b-hexahydro-1H-2,6a-diazacyclobut[cd]inden-6-carboxylat

(1aS,3aR,6bR)-5-Methylsulfonyloxy-2-(1-methyl-1H-tetrazol-5-ylsulfanylacetyl)-1-oxo-1a,2,3,3a,4,6b-hexahydro-1H-2,6a-diazacyclobut[cd]inden-6-carbonsäure

(1aS,3aR,6bR)-5-Carboxymethyl-2-(4-hydroxy-phenylcarbamoyl)-1-oxo-1a,2,3,3a,4,6b-hexahydro-1H-2,6a-diazacyclobut[cd]inden-6-carbonsäure

(1aS,3aR,6bR)-2-(4-Hydroxy-phenylcarbamoyl)-5-methoxycarbonylmethyl-1-oxo-1a,2,3,3a,4,6b-hexahydro-1H-2,6a-diazacyclobut[cd]inden-6-carbonsäure

(1aS,3aR,6bR)-2-(4-Hydroxy-phenylcarbamoyl)-5-(1-methyl-1H-tetrazol-5-ylsulfanylmethyl)-1-oxo-1a,2,3,3a,4,6b-hexahydro-1H-2,6a-diazacyclo-but[cd]inden-6-carbonsäure

(1aS,3aR,6bR)-2-(4-Carbamoyl-phenylcarbamoyl)-5-(1-methyl-1H-tetrazol-5-ylsulfanylmethyl)-1-oxo-1a,2,3,3a,4,6b-hexahydro-1H-2,6a-diaza-cyclobut[cd]inden-6-carbonsäure

(1aS,3aR,6bR)-2-[(S)-2-Oxo-pyrrolidin-3-ylcarbamoyl]-5-(1-methyl-1H-tetrazol-5-ylsulfanylmethyl]-1-oxo-1a,2,3,3a,4,6b-hexahydro-1H-2,6a-diaza-cyclobut[cd]inden-6-carbonsäure

(1aS,3aR,6bR)-2-Acetyl-5-(1-methyl-1H-tetrazol-5-ylsulfanylmethyl)-1-oxo-1a,2,3,3a,4,6b-hexahydro-1H-2,6a-diazacyclobut[cd]inden-6-carbonsäure

(1aS,3aR,6bR)-2-Acetyl-1-oxo-5-(pyridin-4-ylsulfanylmethyl)-1a,2,3,3a,4,6b-hexahydro-1H-2,6a-diazacyclobut[cd]inden-6-carbonsäure

(1aS,3aR,6bR)-2-(4-Carbamoyl-phenylcarbamoyl)-5-(1-methyl-pyridin-1-yliosulfanylmethyl)-1-oxo-1a,2,3,3a,4,6b-hexahydro-1H-2,6a-diazacyclobut[cd]inden-6-carboxylat
(1aS,3aR,6bR)-2-(4-Hydroxy-phenylcarbamoyl)-1-oxo-5-(pyridin-1-yliomethyl)-1a,2,3,3a,4,6b-hexahydro-1H-2,6a-diazacyclobut[cd]inden-6-carboxylat
(1aS,3aR,6bR)-2-(3-Hydroxy-isoxazol-5-ylmethylcarbamoyl)-1-oxo-5-(pyridin-1-yliomethyl)-1a,2,3,3a,4,6b-hexahydro-1H-2,6a-diazacyclobut[cd]inden-6-carboxylat
(1aS,3aR,6bR)-2-(4-Carbamoyl-phenylcarbamoyl)-1-oxo-5-(pyridin-1-yliomethyl)-1a,2,3,3a,4,6b-hexahydro-1H-2,6a-diazacyclobut[cd]inden-6-carboxylat
(1aS,3aR,6bR)-2-Acetyl-1-oxo-5-(pyridin-1-yliomethyl)-1a,2,3,3a,4,6b-hexahydro-1H-2,6a-diazacyclobut[cd]inden-6-carboxylat
(1aS,3aR,6bR)-1-Oxo-5-(pyridin-1-yliomethyl)-2-trifluoracetyl-1a,2,3,3a,4,6b-hexahydro-1H-2,6a-diazacyclobut[cd]inden-6-carboxylat
(1aS,3aR,6bR)-5-Acetoxymethyl-2-(4-hydroxy-phenylcarbamoyl)-1-oxo-1a,2,3,3a,4,6b-hexahydro-1H-2,6a-diazacyclobut[cd]inden-6-carbonsäure
sowie pharmazeutisch verträgliche Salze, insbesondere die Natriumsalze dieser Verbindungen.

**46.** Verbindungen der allgemeinen Formel

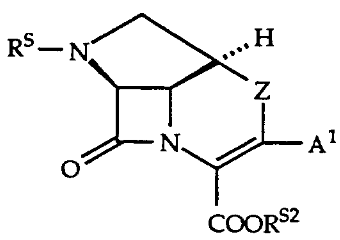

II

in der Z die in Anspruch 1 gegebene Bedeutung hat, $A^1$ wie A in Anspruch 1 ist, jedoch nicht niederes Alkoxy bedeutet, $R^S$ die für R in Anspruch 1 gegebene Bedeutung hat oder eine Aminoschutzgruppe bedeutet und $R^{S2}$ eine Carboxyschutzgruppe darstellt.

**47.** Verbindungen gemäss Anspruch 46, dadurch gekennzeichnet, dass Z Methylen, $R^{S2}$ t-Butyl und $R^S$ t-Butoxycarbonyl darstellt.

**48.** Verbindungen unter Anspruch 46, dadurch gekennzeichnet, dass Z Sauerstoff oder Schwefel, $R^{S2}$ Allyl und $R^S$ Allyloxycarbonyl darstellt.

**49.** Verbindungen der allgemeinen Formel

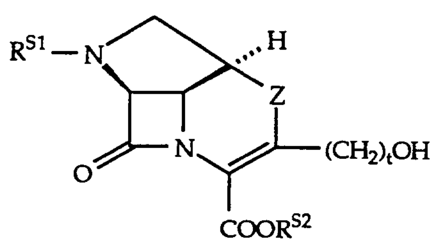

in der Z die in Anspruch 1 gegebene Bedeutung hat, $R^{S1}$ eine Aminoschutzgruppe, $R^{S2}$ eine Carboxyschutzgruppe und t die Zahl 0 oder 1 darstellt.

**50.** Verbindungen nach Anspruch 49, dadurch gekennzeichnet, dass Z Methylen, $R^{S2}$ t-Butyl und $R^{S1}$ t-Butoxycarbonyl oder Acetyl darstellt.

**51.** Verbindungen nach Anspruch 49, dadurch gekennzeichnet, dass Z Sauerstoff oder Schwefel, $R^{S2}$ Allyl und $R^{S1}$ Allyloxycarbonyl oder Acetyl darstellt.

**52.** Verbindungen gemäss einem der Ansprüche 1-45 als pharmazeutische Wirkstoffe.

**53.** Verbindungen gemäss einem der Ansprüche 1-45 als $\beta$-Lactamase hemmende Wirkstoffe.

**54.** Verfahren zur Herstellung von Verbindungen gemäss einem der Ansprüche 1-45, dadurch gekennzeichnet, dass man

a) in einer Verbindung der allgemeinen Formel

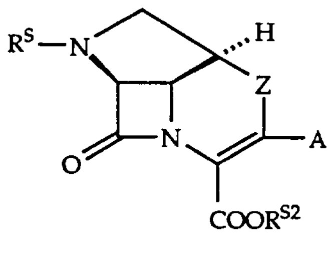

II

in der A und Z die oben gegebene Bedeutung haben, $R^S$ die für R angegebene Bedeutung hat oder eine Aminoschutzgruppe bedeutet und $R^{S2}$ eine Carboxyschutzgruppe darstellt, die Carboxyschutzgruppe und eine allfällige Aminoschutzgruppe abspaltet und ein allenfalls erhaltenes Säureadditionssalz einer Verbindung der allgemeinen Formel

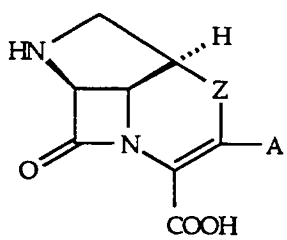

III

in der A und Z die oben gegebene Bedeutung haben, gewünschtenfalls mit den Rest R abgebenden Mitteln behandelt und ggfs. allfällig noch vorhandene Schutzgruppen abspaltet, oder dass man

b) zur Herstellung eines leicht hydrolysierbaren Esters einer Verbindung der Formel I eine Carbonsäure der Formel I einer entsprechenden Veresterung unterwirft, oder dass man

c) zur Herstellung von pharmazeutisch verträglichen Salzen einer Verbindung der Formel I eine Verbindung der Formel I in ein solches Salz überführt.

**55.** Arzneimittel enthaltend eine Verbindung gemäss einem der Ansprüche 1-45.

**56.** $\beta$-Lactamase hemmende Arzneimittel enthaltend eine Verbindung gemäss einem der Ansprüche 1-45.

**57.** Arzneimittel gemäss Anspruch 55 oder 56 enthaltend zusätzlich ein $\beta$-Lactam-Antibiotikum.

**58.** Arzneimittel gemäss Anspruch 57 enthaltend ein Penicillin, Cephalosporin, Penem oder Carbapenem als $\beta$-Lactam-Antibiotikum.

**59.** Arzneimittel gemäss Anspruch 58 enthaltend Benzylpenicillin, Piperacillin, Phenoxymethylpenicillin, Carbenicillin, Apalcillin, Methicillin, Propicillin, Tricarcillin, Ampicillin, Amoxicillin oder Mecillinam, oder Ceftazidim, Cefetamet, Cefatamet Pivoxil, Cefotaxim, Cefmenoxim, Ceftizoxim, Cefuroxim, Cephaloridin, Cephalotin, Cefazolin, Cephalexin, Cefoxitin, Cephacetril, Cefamandol, Cephapirin, Cephradin, Cephaloglycin, (6R,7R)-7-[(Z)-2-(2-Amino-4-thiazolyl)-2-(methoxyimino)acetamido]-3-(azidomethyl)-8-oxo-5-thia-1-azabicyclo[4.2.0]oct-2-en-2-carbonsäure oder (E)-2-(Isobutoxycarbonyl)-2-pentenyl (6R,7R)-7-[(Z)-2-(2-Amino-4-thiazolyl)-2-(methoxyimino)acetamido]-3-(azidomethyl)-8-oxo-5-thia-1-azabicyclo[4.2.0]oct-2-en-2-carboxylat; oder Imipenem oder Meropenem; oder ein pharmazeutisch verträgliches Salz einer

dieser Verbindungen.

**60.** Arzneimittel gemäss Anspruch 58 enthaltend Ceftriaxon oder eines seiner pharmazeutisch verträglichen Salze.

**61.** Arzneimittel gemäss Anspruch 60 enthaltend Ceftriaxon-di-Natriumsalz-hemiheptahydrat.

**62.** Arzneimittel gemäss einem der Ansprüche 57-61 als Kombinationspräparat zur gleichzeitigen, getrennten oder zeitlich abgestuften Anwendung in der antibakteriellen Therapie.

**63.** Verwendung von Verbindungen gemäss einem der Ansprüche 1-45 bei der Bekämpfung oder Verhütung von Krankheiten.

**64.** Verwendung von Verbindungen gemäss einem der Ansprüche 1-45 bei der Bekämpfung oder Verhütung von Infektionskrankheiten.

**65.** Verwendung von Verbindungen gemäss einem der Ansprüche 1-45 bei der Herstellung von antibakteriell wirksamen Mitteln.

Europäisches Patentamt

# EUROPÄISCHER RECHERCHENBERICHT

Nummer der Anmeldung: EP 95102819.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 6) |
|---|---|---|---|
| X | EP - A - 0 508 234 (HOFFMANN) * Ansprüche 43,47-57; Seite 18, Zeile 30,40, Formeln IVa, 1b2 * -- | 1,46, 49, 55-65 | C 07 D 513/14<br>C 07 D 507/08<br>C 07 D 498/14<br>C 07 D 463/10<br>C 07 D 487/04<br>A 61 K 31/40<br>A 61 K 31/535<br>A 61 K 31/54 |
| A | WO - A - 93/15 085 (SMITHKLINE) * Ansprüche 1,22,23 * ---- | 1,46, 49,55, 63-65 | |

RECHERCHIERTE SACHGEBIETE (Int. Cl 6)

C 07 D 513/00
C 07 D 507/00
C 07 D 498/00
C 07 D 463/00
C 07 D 487/00

Der vorliegende Recherchenbericht wurde für alle Patentansprüche erstellt.

| Recherchenort | Abschlußdatum der Recherche | Prüfer |
|---|---|---|
| WIEN | 02-05-1995 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument